# EUROPEAN PATENT SPECIFICATION

(11) **EP 1 078 002 B1**
(45) Date of publication and mention of the grant of the patent: **21.05.2008**
(21) Application number: 99927091.1
(22) Date of filing: 21.05.1999
(51) Int. Cl.: C07K 14/78, A61K 38/39, G01N 33/68

(54) **PEPTIDE ANTIANGIOGENIC DRUGS**
PEPTIDE ALS ANTIANGIOGENE ARZNEIMITTEL
MEDICAMENTS PEPTIDIQUES ANTI-ANGIOGENIQUES

(30) Priority: 22.05.1998 US 83745; 16.02.1999 US 250574; 26.03.1999 US 277466
(43) Date of publication of application: 28.02.2001
(73) Proprietor: ABBOTT LABORATORIES, Abbott Park, IL 60064-6050 (US)
(72) Inventor: HENKIN, Jack, Highland Park, IL 60035 (US); HAVIV, Fortuna, Deerfield, IL 60015 (US); BRADLEY, Michael, F., Wadsworth, IL 60083 (US); KALVIN, Douglas, M., Buffalo Grove, IL 60089 (US); SCHNEIDER, Andrew, J., Gurnee, IL 60031 (US)
(74) Representative: Modiano, Micaela Nadia
(86) International application number: PCT/US1999/011448
(87) International publication number: WO 1999/061476

(56) References cited:
- EP-A- 0 443 404
- WO-A-93/16716
- WO-A-97/41824
- WO-A-98/41542

### SEQUENCE LISTING

<110> Abbott Laboratories
   Henkin, J.
   Haviv, F.
   Bradley, M. F.
   Kalvin, D. M.
   Schneider, A. J.
<120> Peptide Antiangiogenic Drugs
<130> 6356.PC.01
<140> PCT/US99/11448
   <141> 1999-05-21
<i50> US 09/277,466
   <151> 1999-03-26
<150> US 09/250,574
   <151> 1999-02-16
<150> US 09/083,745
   <1.51> 1998-05-22
<160> 6
<170> FastSEQ for Windows Version 3.0
<210> 1
   <211> 10
   <212> PRT
   <213> Artificial Sequence
<220>
   <221> PEPTIDE
   <222> (1) ... (1)
   <223> Xaa is Ala, Asx, citrullyl (Cit), Glx, EtGly, Met, N-methylAla(MeAla), Pro, pyro-Glx, MeGly, Ser, or Thr
<221> PEPTIDE
   <222> (2) ... (2)
   <223> Xaa is Ala, Asx, Glx, Leu, Met, Phe, Pro, or Ser
<221> PEPTIDE
   <222> (3) ... (3)
   <223> Xaa is Ala, Asx, Cit, Cha, cyclohexylGly, Glx, Gly, Ile, Leu, Met, Nva, Phe, Ser, t-buGly, Thr, Val, or Cys
<221> PEPTIDE
   <222> (4) ... (4)
   <223> Xaa is aIle, Gly, Ile, Pro, dehydroLeu, D-Ala, D-3-(naphth-1-yl)Ala, D-3-(naphth-2-yl)Ala, D-(3-pyridyl)-Ala, D-Abu, D-alle, D-alloThr, D-allylGly, D-Asx, D-benzothienylAla,
<221> PEPTIDE
   <222> (4)...(4)
   <223> Xaa at position 4 can also be D-3-(4,4'-biphenyl)Ala, D-chloroPhe, D-3-(3-trifluoromethylphenyl)Ala, D-3-(3-cyanophenyl)Ala,
<221> PEPTIDE
   <222> (4)... (4)
   <223> Xaa at position 4 can also be D-3-(3,4-difluorophenyl)Ala, D-Cit, D-Cha, D-cyclohexylGly, D-Cys, D-Cys(S-t-bu), D-Glx, D-His, D-homoIle, D-homoPhe, D-homoSer, D-Ile,
<221> PEPTIDE
   <222> (4) ... (4)
   <223> Xaa at position 4 can also be D-Leu, D-Lys(N-epsilon-nicotinyl), D-Lys, D-Met, D-neopentylGly, D-Nle, D-Nva, D-Orn, D-Phe, D-3-(4-aminophenyl)Ala, D-3-(4-methylphenyl)Ala,
<221> PEPTIDE
   <222> (4) ... (4)
   <223> Xaa at position 4 can also be D-3-(4-nitrophenyl)Ala, D-3-(3,4-dimethoxyphenyl)Ala, D-3-(3,4,5-trifluorophenyl)Ala, D-Pro, D-Ser,
<221> PEPTIDE
   <222> (4) ... (4)
   <223> Xaa at position 4 can also be D-Ser(O-benzyl), D-t-buGly, D-thienylAla, D-Thr, D-Thr(O-benzyl), D-Trp, D-Tyr(O-benzyl), D-Tyr(O-ethyl), D-Tyr, or D-Val
<221> PEPTIDE
   <222> (5) ... (5)
   <223> Xaa is Ala, (3-pyridyl)Ala, 3-(naphth-1-yl)Ala, 3-(naphth-2-yl)Ala, alloThr, allylGly, Glx, Gly, His, homoSer, Ile, Lys(N-epsilon-acetyl), Met, Nva, octylGly, Om, 3-(4-hydroxymethylphenyl)Ala,
<221> PEPTIDE
   <222> (5) ... (5)
   <223> Xaa at position 5 can also be Pro, Ser, Thr, Trp, Tyr, D-alloThr, D-homoser, D-Ser, D-Thr, or Cys
<221> PEPTIDE
   <222> (6)...(6)
   <223> Xaa is Ala, 3-(naphth-1-yl)Ala, 3-(naphth-2-yl)Ala, (3-pyridyl)Ala, Abu, allylGly, Arg, Asx, Cit, Cha, Glx, Gly, His, homoAla, homoLeu, homoSer, Ile, Leu,
<221> PEPTIDE
   <22.2> (6) ... (6)
   <223> Xaa at position 6 can also be Lys(N-epsilon-acetyl), Lys(N-epsilon-isopropyl), Met(sulfone), Met(sulfoxide), Met, Nle, Nva, octylGly, Phe,
<221> PEPTIDE
   <222> (6) ... (6)
   <223> Xaa at position 6 can also be 3-(4-carboxyamidephenyl)Ala, propargylGly, Ser, Thr, Trp, Tyr, Val, D-3-(naphth-1-yl)Ala, D-3-(naphth-2-yl)Ala, D-Glx, D-homoSer, D-Leu,
<221> PEPTIDE
   <222> (6) ... (6)
   <223> Xaa at position 6 can also be D-Nva, D-Ser, or Cys
<221> PEPTIDE
   <222> (7) ... (7)
   <223> Xaa is Ala, allylGly, Asx, Cit, cyclohexylGly, Glx, Gly, homoSer, Ile, aIle Leu, Lys(N-epsilon-acetyl), Met, 3-(naphth-1-yl)Ala, 3-(naphth-2-yl)Ala, Nva, Phe, Pro, Ser, t-buGly,
<221> PEPTIDE
   <222> (7) ... (7)
   <223> Xaa at position 7 can also be Trp, Tyr, Val, D-aIle D-Ile, or Cys
<221> PEPTIDE
   <222> (8) ... (8)
   <223> Xaa is Ala(3-guanidino), Ala[3-pyrrolidinyl(2-N-amidino)], Ala[4-piperidinyl(N-amidino)], Arg,
<221> PEPTIDE
   <222> (8) ... (8)
   <223> Xaa at position 8 can also be Arg (NGNG'diethyl), Cit, 3-(cyclohexyl)Ala(4-N'-isopropyl), Gly[4-piperidinyl(N-amidino)], His, homoArg,
<221> PEPTIDE
   <222> (8) ... (8)
   <223> Xaa at position 8 can also be Lys, Lys(N-epsilon-isopropyl), Lys(N-epsilon-nicotinyl), norArg, Orn(N-delta-isopropyl), Orn(N-delta-nicotinyl),
<221> PEPTIDE
   <222> (8) ... (8)
   <223> Xaa at position 8 can also be Orn[N-delta-(2-imidazolinyl)], [(4-amino(N-isopropyl)methyl)phenyl]Ala, 3-(4-guanidinophenyl)Ala, or
<221> PEPTIDE
   <222> (8) ... (8)
   <223> Xaa at position 8 can also be 3-(4-amino-N-isopropylphenyl)Ala
<221> PEPTIDE
   <222> (9)...(9)
   <223> Xaa is Abu, Aib, homoPro, hydroxyPro, Ile, Leu, Phe, Pro, Ser, t-buGly, Thr, Val, D-Ala, or D-Pro
<221> PEPTIDE
   <222> (10)...(10)
   <223> Xaa is azaGlyamide, D-Alaamide, D-Alaethylamide, Glyamide, Glyethylamide, MeGlyamide, Seramide, or D-Seramide
<400> 1
   Xaa Xaa Xaa Xaa Xaa Xaa Xaa Xaa Xaa Xaa
   1 5 10
<210> 2
   <211> 9
   <212> PRT
   <213> Artificial Sequence
<220>
   <221> PEPTIDE
   <222> (1) ... (1)
   <223> Xaa is MeGly
<221> PEPTIDE
   <222> (4) ... (4)
   <223> Xaa at position 4 here is the same as position 4 in SEQ ID NO:1
<221> PEPTIDE
   <222> (5)...(5)
   <223> Xaa at position 5 here is the same as position 5 in SEQ ID NO:1
<221> PEPTIDE
   <222> (6)...(6)
   <223> Xaa at position 6 here is the same as position 6 in SEQ ID N0:1
<400> 2
   Xaa Gly Val Xaa Xaa Xaa Ile Arg Pro
   1 5
<210> 3
   <211> 9
   <212> PRT
   <213> Artificial Sequence
<220>
   <221> PEPTIDE
   <222> (1) ... (1)
   <223> Xaa is MeGly
<222> (8) ... (8)
   <223> Xaa at position 8 can also be 3-(4-amino-N-isopropylphenyl)Ala
<221> PEPTIDE
   <222> (9) ... (9)
   <223> Xaa is Abu, Aib, homoPro, hydroxyPro, Ile, Leu, Phe, Pro, Ser, t-buGly, Thr, Val, D-Ala, or D-Pro
<221> PEPTIDE
   <222> (10)...(10)
   <223> Xaa is azaGlyamide, D-Alaamide, D-Alaethylamide, Glyamide, Glyethylamide, MeGlyamide, Seramide, or D-Seramide
<400> 1
<210> 2
   <211> 9
   <212> PRT
   <213> Artificial Sequence
<220>
   <221> PEPTIDE
   <222> (1) ... (1)
   <223> Xaa is MeGly
<221> PEPTIDE
   <222> (4) ... (4)
   <223> Xaa at position 4 here is the same as position 4 in SEQ ID NO:1
<221> PEPTIDE
   <222> (5) ... (5)
   <223> Xaa at position 5 here is the same as position 5 in SEQ ID NO:1.
<221> PEPTIDE
   <222> (6)...(6)
   <:223> Xaa at position 6 here is the same as position 6 in SEQ ID NO:1
<400> 2
   Xaa Gly Val Xaa Xaa Xaa Ile Arg Pro
   1 5
<210> 3
   <211> 9
   <212> PRT
   <213> Artificial Sequence
<220>
   <221> PEPTIDE
   <222> (1) ... (1)
   <223> Xaa is MeGly
<221> PEPTIDE
   <222> (6)...(6)
   <223> Xaa is Nva
<400> 3
   Xaa Gly Val Ile Thr Xaa Ile Arg Pro
   1 5
<210> 4
   <211> 9
   <212> PRT
   <213> Artificial Sequence
<220>
   <221> PEPTIDE.
   <222> (1) ... (1)
   <223> Xaa is MeGly
<221> PEPTIDE
   <222> (6) ... (6)
   <223> Xaa is Nva
<400> 4
   Xaa Gly Val Gly Thr Xaa Ile Arg Pro
   1 5
<210> 5
   <211> 9
   <212> PRT
   <213> Artificial Sequence
<220>
   <221> PEPTIDE
   <222> (1) ... (1)
   <223> Xaa is MeGly
<221> PEPTIDE
   <222> (4) ... (4)
   <223> Xaa is aile
<221> PEPTIDE
   <222> (6) ... (6)
   <223> Xaa is Nva
<400> 5
   Xaa Gly Val Xaa Thr Xaa Ile Arg Pro
   1 5
<210> 6
   <211> 9
   <212> PRT
   <213> Artificial Sequence
<220>
   <221> PEPTIDE
   <222> (1) ... (1)
   <223> Xaa is MeGly
<221> PEPTIDE
   <222> (4)...(4)
   <223> Xaa is dehydroLeu
<221> PEPTIDE
   <222> (6) ... (6)
   <223> Xaa is Nva
<400> 6
   Xaa Gly Val Xaa Thr Xaa Ile Arg Pro
    1               5

## Description

### Technical Field

The invention relates to novel compounds having activity useful for treating conditions which arise or are exacerbated by angiogenesis, pharmaceutical compositions comprising these compounds, a method of treating using said compounds, and a method of inhibiting angiogensis.

### Background of the Invention

Angiogenesis is the fundamental process by which new blood vessels are formed and is essential to a variety of normal body activities (such as reproduction, development and wound repair). Although the process is not completely understood, it is believed to involve a complex interplay of molecules which both stimulate and inhibit the growth of endothelial cells, the primary cells of the capillary blood vessels. Under normal conditions, these molecules appear to maintain the microvasculature in a quiescent state (i.e. one of no capillary growth) for prolonged periods which may last for as long as weeks or in some cases, decades. When necessary however (such as during wound repair), these same cells can undergo rapid proliferation and turnover within a five day period. (Folkman, J. and Shing, Y., The Journal of Biological Chemistry, 267(16): 10931-10934, and Folkman, J. and Klagsbrun, M., Science, 235: 442-447 (1987).

Although angiogenesis is a highly regulated process under normal conditions, many diseases (characterized as "angiogenic diseases") are driven by persistent unregulated angiogenesis. Otherwise stated, unregulated angiogenesis may either cause a particular disease directly or exascerbate an existing pathological condition. For example, ocular neovacularization has been implicated as the most common cause of blindness. In certain existing conditions such as arthritis, newly formed capillary blood vessels invade the joints and destroy cartilage. In diabetes, new capillaries formed in the retina invade the vitreous, bleed, and cause blindness. Growth and metastasis of solid tumors are also angiogenesis-dependent (Folkman, J., Cancer Research, 46: 467-473 (1986), Folkman, J., Journal of the National Cancer Institute, 82: 4-6 (1989)). It has been shown for example that tumors which enlarge to greater than 2 mm, must obtain their own blood supply and do so by inducing the growth of new capillary blood vessels. Once these new blood vessels become embedded in the tumor, they provide a means for tumor cells to enter the circulation and metastasize to distant sites, such as liver, lung or bone (Weidner, N., et al., The New England Journal of Medicine, 324(1); 1-8 (1991)).

Although several angiogenesis inhibitors are currently under development for use in treating angiogenic diseases (Gasparini, G. and Harris, A.L., J Clin Oncol 13(3): 765-782, (1995)), there are disadvantages associated with several of these compounds. For example, suramin is a potent angiogenesis inhibitor, but causes (at doses required to reach antitumor activity) severe systemic toxicity in humans. Other compounds, such as retinoids, interferons and antiestrogens are safe for human use but have only a weak anti-angiogenic effect.

### Summary of the Invention

In one aspect, the present invention provides a compound the formula of:

A₀-A₁-A₂-A₃-A₄-A₅-A₆-A₇-A₈-A₉-A₁₀ (I)

or a pharmaceutically acceptable salt, ester, solvate or prodrug thereof, wherein:
A₁ is sarcosyl, A₂ is glycyl, A₃ is valyl, A₇ is isoleucyl, A₈ is arginyl, A₉ is prolyl, and A₀, A₄, A₅, A₆, and A₁₀ are as follows:
   A₀ is hydrogen or an acyl group selected from:
      (1) R-(CH₂)ₙ-C(O)-; wherein n is an integer selected from 0, 1, 2, 3 and 5 and R is selected from methyl; N-acetylamino; methoxyl; carboxyl; cyclohexyl; cyclohexyl containing one double bond and substituted with three hydroxyl groups; and a 5- or 6-membered aromatic or nonaromatic ring ooptionally containing one heteroatom selected from nitrogen and oxygen, wherein the ring is optionally substituted with alkyl; and
      (2) R¹-CH₂CH₂-(OCH₂CH₂O)p-CH₂-C(O)-; wherein R¹ is N-acetylamino, and p is 1;
   A₄ is an amino acyl residue of L or D configuration selected from:
      (1) allo-isoleucyl,
      (2) glycyl,
      (3) isoleucyl,
      (5) dehydroleucyl.
      (6) D-alanyl,
      (7) D-3 -(naphth-1 -yl)alanyl,
      (8) D-3-(naphth-2-yl)alanyl,
      (9) D-(3-pyridyl)-alanyl,
      (10) D-2-aminobutyryl,
      (11) D-allo-isoleucyl,
      (12) D-allo-threonyl,
      (14) D-asparaginyl,
      (15) D-aspartyl,
      (16) D-3-(3-benzothienyl)alanyl
      (17) D-3-(4,4-biphenyl)alanyl,
      (18) D-3-(3-chlorophenyl)alanyl
      (19) D-3-(3-trifluoromethylphenyl)alanyl,
      (20) D-3-(3-cyanophenyl)alanyl;
      (21) D-3-(3,4-difluorophenyl)alanyl;
      (22) D-citrullyl,
      (23) D-cyclohexylalanyl,
      (24) D-cyclohexyglycyl,
      (25) D-cystyl,
      (26) D-cystyl(*S*-t-butyl),
      (27) D-glutaminyl,
      (28) D-glutamyl,
      (29) D-histidyl,
      (31) D-homophenylalanyl,
      (32) D-homoseryl;
      (33) D-isoleucyl,
      (34) D-leucyl,
      (36) D-lysyl,
      (37) D-methionyl,
      (38) D-neopentylglycyl,
      (39) D-norleucyl,
      (41) D-ornithyl,
      (42) D-penicillaminyl,
      (43) -D-penicillaminyl(acetamidomethyl),
      (44) D-penicillaminyl(S-benzyl),
      (45) D-phenylalanyl,
      (46) D-3-(4-aminophenyl)alanyl,
      (47) D-3-(4-methylphenyl)alanyl,
      (48) D-3-(4-nitrophenyl)alanyl,
      (49) D-3-(3,4-dimethoxyphenyl)alanyl,
      (50) D-3-(3,4,5-trifluorophenyl)alanyl,
      (52) D-seryl,
      (53) D-seryl(O-benzyl),
      (54) D-*t*-butylglycyl,
      (55) D-thienylalanyl,
      (56) D-threonyl,
      (57) D-threonyl (*O*-benzyl),
      (58) D-tryptyl,
      (59) D-tyrosyl(O-benzyl),
      (60) D-tyrosyl(O-ethyl),
      (61) D-tyrosyl,
      (62) D-valyl; and
      (63) D-3-(4-chlorophenyl)alanyl;
   A₅ is an amino acyl residue selected from:
      (1) alanyl,
      (3) 3-(naphth-1-yl)alanyl,
      (4) 3-(naphth-2-yl)alanyl,
      (6) allylglycyl,
      (7) glutaminyl,
      (8) glycyl,
      (10) homoseryl,
      (11) isoleucyl,
      (12) lysyl(N-epsilon-acetyl),
      (13)methionyl,
      (14) norvalyl,
      (15) octylglycyl,
      (17) 3-(4-hydroxymethylphenyl)alanyl,
      (18) prolyl,
      (19) seryl,
      (20) threonyl,
      (21) tryptyl,
      (22) tyrosyl,
      (26) D-threonyl, and
      (27) penicillaminyl;
   A₆ is an amino acyl residue selected from:
      (1) alanyl,
      (5) 2-aminobutyryl,
      (6) allylglycyl,
      (7) arginyl
      (8) asparaginyl,
      (9) aspartyl,
      (10) citrullyl,
      (11) 3-(cyclohexyl)alanyl,
      (12) glutaminyl,
      (13)glutamyl,
      (14) glycyl,
      (19) isoleucyl,
      (20) leucyl,
      (21) lysyl(N-epsilon-acetyl),
      (23) methionyl(sulfone),
      (24) methionyl(sulfoxide),
      (25) methionyl,
      (26) norleucyl,
      (27) norvalyl,
      (28) octylglycyl;
      (30) 3-(4-carboxyamidephenyl)alanyl,
      (31) propargylglycyl,
      (32) seryl,
      (35) tyrosyl,
      (36) valyl,
      (42) D-norvalyl, and
      (44) penicillaminyl;
   A₁₀ is selected from:
      (1) hydroxyl,
      (2) azaglycylamide,
      (3) D-alanylamide,
      (7) sarcosylamide,
      (8) serylamide,
      (9) D-serylamide,
      (10) a group represented by the formula 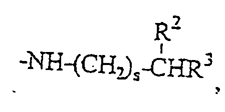 and
      (11) a group represented by the formula-NH-R⁴; wherein:
         s is an integer selected from 0 and 1,
         R² is selected from hydrogen, alkyl, and a 6-membered cycloalkyl ring;
         R³ is selected from hydrogen, hydroxy, alkyl, alkoxy, and a 6-membered ring containing one nitrogen, provided that s is not zero when R³ is hydroxy or alkoxy; and
         R⁴ is selected from hydrogen and hydroxy.

In another aspect, the present invention provides a composition for treating a patient in need of anti-angiogenesis therapy comprising a peptide defined above in combination with a pharmaceutically acceptable carrier.

Yet another aspect of the present invention provides a method for treating a patient in need of anti-angiogenesis therapy comprising administering to the patient a therapeutically effective amount of a peptide as defined above.

Still yet another aspect of the present invention provides a composition for the treatment of a disease selected from cancer, arthritis, psoriasis, angiogenesis of the eye associated with infection or surgical intervention, macular degeneration and diabetic retinopathy comprising a peptide as defined above in combination with a pharmaceutically acceptable carrier.

In yet another aspect, the present invention provides a method of isolating a receptor from an endothelial cell comprising binding a peptide as defined above to the receptor to form a peptide receptor complex, isolating the peptide receptor complex, and purifying the receptor.

### Detailed Description of the Invention

### Definition of Terms

The term "alkyl" as used herein refers to a monovalent group derived from a straight or branched chain saturated hydrocarbon by the removal of a hydrogen atom. Examples of alkyl include, but are not limited to, methyl, ethyl, propyl, isopropyl, butyl, sec-butyl, iso-butyl, tert-butyl, pentyl, hexyl, and the like. Preferred alkyl groups for the invention are C₁-C₆ alkyl groups having from one to six carbon atoms. Alkyl groups of one to three carbon atoms (C₁-C₃ alkyl) are more preferred for the invention.

The term "nicotinyl" as used herein refers to the acyl group derived from nicotinic acid, i.e. pyridine-3-carboxylic acid. The term "2-Me-nicotinyl" or "2-methylnicotinyl" refers to a nicotinyl moiety substituted with a methyl group at the carbon adjacent to the nitrogen atom.

The term "shikimyl" as used herein refers to the acyl residue derived from shikimic acid or [3R-(3α,4α,5β-3.4.5-trihydroxy]-1-cyclohexene-1-carboxylic acid. A "dihydroshikimyl" group denotes the fully saturated analog of shikimic acid.

The term "succinyl" as used herein refers to the acyl residue derived from succinic acid or (1,4-dioxobutyl)-1-carboxylic acid

The term "N-acetylamino" as used herein refers to an amino moiety (-NH₂) substituted on the nitrogen atom with an acetyl (CH₃C(O)-) group.

The term "carbonyl" as used herein refers to the group -C(O)-.

The term "carboxy" or "carboxyl" as used herein refers to the group -C(O)OH.

The term "alkoxy" as used herein refers to an alkyl group as defined above attached to a parent molecular moiety via an ether linkage. Exemplary alkoxy groups include, but are not limited to, methoxy, ethoxy, isopropoxy, and the like.

The term "aromatic ring" as used herein refers to an unsaturated cyclic hydrocarbon associated with a system of π-electron bonds. One to two carbon atoms of the hydrocarbon ring can be substituted with a heteroatom selected from nitrogen, oxygen, or sulfur. Exemplary 5- or 6-membered aromatic rings include, but are not limited to, benzyl, pyridyl, furyl, tetrahydrofuryl, thienyl, and pyrrolyl. An aromatic ring, including rings substituted with a heteroatom, can be optionally substituted on one or more carbon atoms with substituents selected from alkyl, alkoxy, carboxy, and halogen, for example, tolyl, bromobenzyl *t*-butylbenzyl, nicotinyl, 2-methylnicotinyl, 2-furoic acid, and the like.

The term "nonaromatic ring" as used herein refers to a saturated or unsaturated cyclic hydrocarbon ring, which can be optionally substituted with one or two heteroatoms selected from nitrogen, oxygen, or sulfur. Exemplary nonaromatic rings are cyclohexyl, tetrahydropyranyl, pyrrolidinyl, and piperidinyl.

The term "N-protecting group" as used herein refers to an easily removable group which is known in the art to protect an amino group against undesirable reaction during synthetic procedures and to be selectively removable. The use of N-protecting groups is well known in the art for protecting groups against undesirable reactions during a synthetic procedure and many such protecting groups are known, cf, for example, T.H. Greene and P.G.M. Wuts, Protective Groups in Organic Synthesis, 2nd edition, John Wiley & Sons, New York (1991). Examples ofN-protecting groups include, but are not limited to, acyl groups including acetyl, trifluoroacetyl, acylisothiocyanate, aminocaproyl, benzoyl and the like, and acyloxy groups, including t-butyloxycarbonyl (Boc) and carbobenzyloxy (Cbz), 9-fluorenylmethoxycarbonyl (Fmoc), and the like.

As used herein the terms "Leu," "Sar," "Gln," "Gly," "Val," "Ile," "Thr," "Nva," "Arg," "Asn," "pyroGlu," "Ser," "Ala," "Homoala," "Cha," "Pro", "Phe," "Trp," "1-Nal," "2-Nal," "Azagly" and "Nle" refer to leucine, sarcosine (N-methylglycine), glutamine, glycine, valine, isoleucine, threonine, norvaline, arginine, aspargine, pyroglutamic acid, serine, alanine, homoalanine, cyclohexylalanine, proline, phenylalanine, tryptophan, 1-naphthylalanine, 2-naphthylalanine, azaglycine, and norleucine, respectively, in their L-, D- or DL forms. Unless indicated otherwise by a "D" prefix, e.g. D-Ala or D-Ile (also D-Ile), the stereochemistry of the α-carbon of the amino acids and aminoacyl residues in peptides described in this specification and the appended claims is the natural or "L" configuration. The Cahn-Ingold-Prelog "R" and "S" designations are used to specify the stereochemistry of chiral centers in certain of the acyl substituents at the N-terminus of the peptides of this invention. The designation "R,S" is meant to indicate a racemic mixture of the two enantiomeric forms. This nomenclature follows that described in R.S. Cahn, et al., Angew. Chem. Int. Ed. Engl., 5, 385-415 (1966).

For the most part, the names on naturally occurring and non-naturally occurring aminoacyl residues used herein follow the naming conventions suggested by the IUPAC Commission on the Nomenclature of Organic Chemistry and the IUPAC-IUB Commission on Biochemical Nomenclature as set out in "Nomenclature of α-Amino Acids (Recommendations, 1974) " Biochemistry, 14(2), (1975). To the extent that the names and abbreviations of amino acids and aminoacyl residues employed in this specification and appended claims differ from those suggestions, they will be made clear to the reader. Some abbreviations useful in describing the invention are defined below in the following Table 1.

**Table 1**

| Abbreviation | Definition |
|---|---|
| Abu | 2-aminobutyric acid |
| 6-Ac-Aca | 6-NAc-caproyl, 6-N-Ac-(CH2)5C(O)-, or 6-N-acetyl-aminocaproic acid |
| Aib | 2-aminoisobutyric acid |
| Ala(3-guanidino) | alanine(3-guanidino) |
| Ala(3-pyrrolidinylamidino) | alanine[3 -pyrrolidinyl(2-N -arnidino)] |
| Ala(4-Pip(N-amidino)) | alanine(4-piperidinyl(N-amidino)) |
| Allylgly | 2-(allyl)glycine |
| AM | aminomethyl |
| Aminopyrimidinobutanoyl | 2-amino-4-[(2-amino)pyrimidinyl]butanoic acid |
| Azagly | azaglycine |
| 3-Ac-Bala | 3-N-acetyl-beta-alanine |
| Bala | beta-alanine |
| Cha | 3-(cyclohexyl)alanine |
| Cha(4-NIsp) | 3-(cycloliexyl)alanine(4-N'-isopropyl) |
| Cit | citrulline |
| 2ClTrt | 2-chloro-trityl |
| Cys(tBu) | cysteine(S-t-butyl) |
| D-2-Thienylala | D-3-(2-thienyl)alanine |
| D-3.3-Diphenylala | D-3,3-(diphenyl)alanine |
| D-3.4-diClPhe | D-3-(3,4-dichlorophenyl)alanine |
| D-3.4-diFPhe | D-3-(3,4-difluorophenyl)alanine |
| D-3-Benzothienylala | D-3-(3-benzothienyl)alanine |
| D-3-CF₃Phe | D-3-(3-trifluoromethylphenyl)alanine |
| D-3-ClPhe | D-3-(3-chlorophenyl)alanine |
| D-3-CNPhe | D-3-(3-cyanophenyl)alanine |
| D-3-Pal | D-(3-pyridyl)alanine |
| D-4,4-Biphenylala | D-3-(4,4 -biphenyl)alanine |
| D-4-ClPhe | D-3-(4-chtoro-phenyl)alanine |
| D-Cha | D-3-(cyclohexyl)alanine |
| D-Chg | D-cyclohexylglycine |
| Dehydroleu | dehydroleucine |
| D-Hphe | D-homophenylalanine |
| D-Ile | D-isoleucine |
| D-alloIle | D-allo-isoleucine |
| D-Lys(Nic) | D-lysine(N-epsilon-nicotinyl) |
| D-Leu | D-leucine |
| D-pentaFPhe | D-3-(pentafluorophenyl)alanine |
| D-Val | D-valine |
| 4-Ac-Gaba | 4-N-acetyl-gamma-aminobutyric acid or 4-N-acetyl-4-aminobutyric acid |
| Gaba | gamma-aminobutyric acid or 4-aminobutyric acid |
| Gly[4-Pip(N-amidino)] | glycine[4-piperidinyl(N-amidino)] |
| Harg | homoarginine |
| Hle | homoleucine |
| Hser | homoserine |
| Hyp | 4-hydroxyproline |
| Isp | isopropyl |
| Lys(Ac) | lysine(N-epsilon-acetyl) |
| Lys(Isp) | lysine(N-epsilon-isopropyl) |
| Lys(Nic) | lysine(N-epsilon-nicotinyl) |
| Met(O) | methionine sulfoxide |
| Met(O₂) | methionine sulfone |
| MeOAc or (MeO)acetyl | methoxyacetyl |
| 1 Nal | 3-(naphth-1-yl)alanine |
| 2Nal | 3-(naphth-2-yl)alanine |
| N-Ac-Sar | N-acetylsarcosine |
| Neopentylgly | neopentylglycine |
| NEtGly | N-ethylglycine |
| Norarg | norarginine |
| Octylgly | 2-(octyl)glycine |
| Orn(Ac) | ornithine(N-delta-acetyl) |
| Orn(2-imidazo) | ornithine [N-delta-(2-imidazolinyl)] |
| Orn(Isp) | ornithine(N-delta-isopropyl) |
| Orn(Nic) | ornithine(N-delta-nicotinyl) |
| O-TBDMS | O-t-butyldimethylsilyl |
| Pen | penicillamine or β,β-dimethylcysteine |
| Pen(Acm) | penicillamine(acetamidomethyl) |
| D-Phe(3,4,5-triF) | D-3-(3,4,5-trifluorophenyl)alanine |
| D-Phe(3,4-diMeO) | D-3-(3,4-dimethoxyphenyl)alanine |
| Phe(4-CH₂OH) | 3-(4-hydroxymethylphenyl)alanine |
| Phe(4-CONH₂) | 3-(4-carboxyamidephenyl)alanine |
| Phe(4-guanidino) | 3-(4-guanidinophenyl)alanine |
| D-Phe(4-Me) | D-3-(4-methylphenyl)alanine |
| D-Phe(4-NH₂) | D-3-(4-aminophenyl)alanine |
| Phe(4-NIsp) | 3-(4-amino-N-isopropylphenyl)alanine |
| Phe(4-CH₂NHIsp) | [(4-amino(N-isopropyl)methyl)phenyl]alanine |
| D-Phe(4-NO₂) | D-3-(4-nitrophenyl)alanine |
| Propargylgly | propargylglycine |
| Pip | pipecolic acid or homoproline |
| pyBrop | bromo-tris-pyrrolidinophosphoniumhexafluorophosphate |
| Ser(Bzl) | serine(*O*-benzyl) |
| tButylgly | *t*-butylglyine |
| Thr(Bzl) | threonine(*O*-benzyl) |
| Tic | 1,2,3,4-tetrahydroisoquinoline-3-carboxylic acid |
| Trt | trityl |
| Tyr(Bzl) | tyrosine(*O*-benzyl) |
| Tyr(Et) | tyrosine(*O*-ethyl) |
| THF | tetrahydrofuryl or tetrahydrofuran |
| 2-THFcarbonyl | (tetrahydro-2-furyl)carbonyl |

When not found in the table above, nomenclature and abbreviations may be further clarified by reference to the Calbiochem-Novabiochem Corp. 1999 Catalog and Peptide Synthesis Handbook or the Chem-Impex International, Inc. Tools for Peptide & Solid Phase Synthesis 1998-1999 Catalogue*.*

The term "pharmaceutically acceptable salt" as used herein refers to salts which are, within the scope of sound medical judgement, suitable for use in contact with the tissues of humans and lower animals without undue toxicity, irritation, allergic response and the like, and are commensurate with a reasonable benefit/risk ratio. Pharmaceutically acceptable salts are well known in the art . For example, S. M. Berge, et al. describe pharmaceutically acceptable salts in detail in J. Pharmaceutical Sciences, 1977, 66: 1-19. The salts can be prepared *in situ* during the final isolation and purification of the compounds of the invention, or separately by reacting the free base function with a suitable organic acid. Representative acid addition salts include acetate, adipate, alginate, ascorbate, aspartate, benzenesulfonate, benzoate, bisulfate, borate, butyrate, camphorate, camphersulfonate, citrate. cyclopentanepropionate, digluconate, dodecylsulfate, ethanesulfonate, fumarate, glucoheptonate, glycerophosphate, hemisulfate, heptonate, hexanoate, hydrobromide, hydrochloride, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, palmoate, pectinate, persulfate, 3-phenylpropionate, phosphate, picrate, pivalate, propionate, stearate, succinate, sulfate, tartrate, thiocyanate, toluenesulfonate, undecanoate, valerate salts, and the like. Representative alkali or alkaline earth metal salts include sodium, lithium, potassium, calcium, magnesium, and the like, as well as nontoxic ammonium, quaternary ammonium, and amine cations, including, but not limited to ammonium, tetramethylammonium, tetraethylammonium, methylamine, dimethylamine, trimethylamine, triethylamine, ethylamine, and the like.

As used herein, the term "pharmaceutically acceptable ester" refers to esters which hydrolyze *in vivo* and include those that break down readily in the human body to leave the parent compound or a salt thereof. Suitable ester groups include, for example, those derived from pharmaceutically acceptable aliphatic carboxylic acids, particularly alkanoic, alkenoic, cycloalkanoic and alkanedioic acids, in which each alkyl or alkenyl moiety advantageously has not more than 6 carbon atoms. Examples of particular esters include formates, acetates, propionates, butyrates; acrylates and ethylsuccinates.

The term "pharmaceutically acceptable solvate" represents an aggregate that comprises one or more molecules of the solute, such as a formula (I) compound, with one or more molecules of solvent_

The term "pharmaceutically acceptable prodrugs" as used herein refers to those prodrugs of the compounds of the present invention which are, within the scope of sound medical judgement, suitable for use in contact with with the tissues of humans and lower animals with undue toxicity, irritation, allergic response, and the like, commensurate with a reasonable benefit/risk ratio, and effective for their intended use, as well as the zwitterionic forms, where possible, of the compounds of the invention. The term "prodrug" refers to compounds that are rapidly transformed *in* vivo to yield the parent compound of the above formula, for example by hydrolysis in blood. A thorough discussion is provided in T. Higuchi and V. Stella, Pro-drugs as Novel Delivery Systems, Vol. 14 of the A.C.S. Symposium Series, and in Edward B. Roche, ed., Bioreversible Carriers in Drug Design, American Pharmaceutical Association and Pergamon Press, 1987.

The term "receptor" as used herein refers to a chemical group or molecule on the cell surface or in the cell interior that has an affinity for a specific chemical group, molecule, or virus. Isolation of receptors relevant to the antiangiogenic activity of the peptide of the invention can provide useful diagnostic tools.

In one embodiment, the present invention relates to compounds of the structure

A₀-A₁-A₂-A₃-A₄-A₅-A₆-A₇-A₈-A₉-A₁₀ (I)

wherein A₀, A₁, A₂, A₃, A₇, A₈, A₉, and A₁₀ are as defined above. The N-terminus of a nonapeptide represented by A₁-A₉ can be modified by an amino acyl group represented by A₀. A₁₀ represents a group suitable for modifying the C-terminus of the compound.

In the present embodiment, A₄ is an amino acyl residue having a D configuration selected from D-allo-isoleucyl, , D-3-(3-cyanophenyl)alanyl, D-cystyl, D-isoleucyl, D-leucyl, D-penicillaminyl, D-phenylalanyl, D-3-(3,4,5-trifluorophenyl)alanyl, and D-3-(4-aminophenyl)alanyl; A₅ is an amino acyl residue selected from octylglycyl, glycyl, penicillaminyl, seryl, threonyl, and tyrosyl; and A₆ is an amino acyl residue selected from glutaminyl, leucyl, norvalyl, and seryl.

In another embodiment of the invention, the compounds have the structure (I) as defined above wherein A₁ is sarcosyl, A₂ is glycyl, A₃ is valyl, A₇ is isoleucyl, A₈ is arginyl, and A₉ is prolyl. Compounds of the present embodiment can be represented by the structure

A₀-Sar-Gly-Val-A₄-A₅-A₆-Ile-Arg-Pro-A₁₀ (II)

wherein A₀ is hydrogen or an acyl group modifying the N-terminus. Suitable groups for A₀ can represented by the formula R-(CH₂)ₙ-C(O)-; wherein n is an integer selected from 0,1, 2, 3 and 5 and R is selected from methyl; N-acetylamino; methoxyl; carboxyl; cyclohexyl; cyclohexyl containing one double bond and substituted with three hydroxyl groups; and a 5- or 6-membered aromatic or nonaromatic ring optionally containing one heteroatom selected from nitrogen, and oxygen wherein the ring is optionally substituted with alkyl; or R¹-CH₂CH₂-(OCH₂CH₂₀)ₚ-CH₂-C(O)-; wherein R¹ is N-acetylamino, and p is 1.

A₄ is an amino acyl residue of L or D configuration selected from allo-isoleucyl, dehydroleucyl, glycyl, isoleucyl, D-alanyl, D-3-(naphth-1-yl)alanyl, D-3-(naphth-2-yl)alanyl, D-(3-pyridyl)-alanyl, D-2-aminobutyryl, D-allo-isoleucyl, D-allo-threonyl, D-asparaginyl, D-aspartyl, D-3-(3-benzothienyl)alanyl, D-3-(4,4'-biphenyl)alanyl, D-3-(3-chlorophenyl)alanyl; D-3-(3-trifluoromethylphenyl)alanyl, D-3-(3-cyanophenyl)alanyl, D-3-(3,4-difluorophenyl)alanyl, D-citrullyl, D-cyclohexylalanyl, D-cyclohexylglycyl, D-cystyl, D-cystyl(S-t-butyl). D-glutaminyl, D-glutamyl, D-histidyl, D-homophenylalanyl, D-homoseryl, D-isoleucyl, D-leucyl, D-lysyl, D-methionyl, D-neopentylglycyl, D-norleucyl, D-ornithyl, D-penicillaminyl, D-penicillaminyl(acetamidomethyl), D-penicillaminyl(S-benzyl), D-phenylalanyl, D-3-(4-aminophenyl)alanyl, D-3-(4-methylphenyl)alanyl, D-3-(4-nitrophenyl)alanyl, D-3-(3,4-dimethoxyphenyl)alanyl, D-3-(3,4,5-trifluorophenyl)alanyl, D-seryl, D-seryl(O-benzyl), D-*t*-butylglycyl, D-thienylalanyl, D-threonyl, D-threonyl(*O*-benzyl), D-tryptyl, D-tyrosyl(O-benzyl), D-tyrosyl(O-ethyl), D-tyrqsyl, D-valyl, and D-3-(4-chlorophenyl)alanyl.

A₃ is an amino acyl residue of L or D configuration selected from alanyl, 3 -(naphth-1-yl)alanyl, 3-(naphth-2-yl)alanyl, allylglycyl, glutaminyl, glycyl, homoseryl, isoleucyl, lysyl(N-epsilon-acetyl), methionyl, norvalyl, octylglycyl, 3-(4-hydroxymethylphenyl)alanyl, prolyl, seryl, threonyl, tryptyl, tyrosy, D-threonyl, and penicillaminyl.

A₆ is an amino acyl residue of L or D configuration selected from alanyl, 2-aminobutyryl, allylglycyl, arginyl, asparaginyl, aspartyl, citrullyl, 3-(cyclohexyl)alanyl, glutaminyl, glutamyl, glycyl, isoleucyl, leucyl, lysyl(N-epsilon-acetyl), methionyl(sulfone), methionyl(sulfoxide), methionyl, norleucyl, norvalyl, octylglycyl, 3-(4-carboxyamidephenyl)alanyl, propargylglycyl, seryl, tyrosyl, valyl, D-norvalyl, and penicillaminyl.

A₁₀ is a hydroxyl group or an amino acid amide selected from azaglycylamide. D-alanylamide, sarcosylamide, serylamide, D-serylamide, or A₁₀ is a group represented by the formula 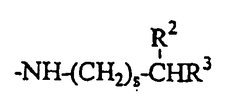 or a group represented by the formula -NH-R⁴, wherein s is an integer selected from 0 and 1; R² is selected from hydrogen, alkyl, and a 6-membered cycloalkyl ring; R³ is selected from hydrogen, hydroxy, alkyl, alkoxy, and a 6-membered ring containing one nitrogen, provided that s is not zero when R³ is hydroxy or alkoxy; and R⁴ is selected from hydrogen and hydroxy.

Preferred compounds of the invention have the structure (II) as defined above, wherein A₄ is an amino acyl residue having a D configuration selected from D-alanyl, D-3-(naphth-1-yl)alanyl, D-3-(naphth-2-yl)alanyl, D-(3-pyridyl)-alanyl, D-2-aminobutyryl, D-allo-isoleucyl, D-allo-threonyl, D-asparaginyl, D-aspartyl, D-3-(3-chlorophenyl)alanyl, D-3-(3-trifluoromethylphenyl)alanyl, D-3-(3-cyanophenyl)alanyl, D-3-(3,4-difluorophenyl)alanyl, D-cyclohexylalanyl, D-cyclohexylglycyl, D-cystyl, D-glutaminyl, D-glutamyl, D-histidyl, D-homophenylalanyl, D-homoseryl, D-isoleucyl, D-leucyl. D-methionyl, D-neopentylglycyl, D-norleucyl, D-penicillaminyl, D-penicillaminyl(acetamidomethyl), D-penicillaminyl(*S*-benzyl). D-phenylalanyl, D-3-(4-aminophenyl)alanyl, D-3-(4-methylphenyl)alanyl, D-3-(4-nitrophenyl)alanyl, D-3-(3,4-dimethoxyphenyl)alanyl, D-3-(3,4,5-trifluorophenyl)alanyl, D-seryl, D-seryl(*O*-benzyl), D-*t*-butylglycyl, D-thienylalanyl, D-threonyl, D-threonyl(O-benzyl), D-tyrosyl(*O*-ethyl), D-tyrosyl, D-valyl, and D-3-(4-chlorophenyl)alanyl.

Other preferred compounds of the present invention have the structure of formula (II), wherein A₅ is selected from glycyl, octylglycyl, penicillaminyl, seryl, threonyl, and tyrosyl.

Additional preferred compounds of the present invention have the structure represented by formula (II), wherein A₆ is selected from glutaminyl, leucyl, norvalyl, and seryl.

The more preferred amino acid residues for substituting the position represented by A₄ are D configuration amino acids selected from D-allo-isoleucyl, .., D-3-(3-cyanophenyl)alanyl, D-cystyl, D-isoleucyl, D-leucyl, D-penicillaminyl, D-phenylalanyl, D-3-(3,4,5-trifluorophenyl)alanyl, and D-3-(4-aminophenyl)alanyl.

Preferred A₀ groups for modifying the N-terminus of the compounds in the scope of the invention are selected from acetyl, butyryl, N-acetyl-beta-alanyl, (6-N-acetylamino)caproyl, cyclohexylacetyl, furoyl, gamma-aminobutyryl, 2-methoxyacetyl, methylnicotinyl, nicotinyl, phenylacetyl, propionyl, shikimyl, succinyl, and tetrahydrofuroyl.

The preferred A₁₀ groups for modifying the C-terminus of the invention are selected from D-alanylamide, azaglycylamide, serylamide, ethylamide, hydroxylamide, isopropylamide, propylamide, 2-(cyclohexyl)ethylamide, 2-(1-pyrrolidine)ethylamide, 1-(cyclohexyl)ethylanude, 2-(methoxy)ethylamide, and 2-(hydroxy)ethylamide.

Compounds contemplated as failing within the scope of the present invention include, but are not limited to:
(1) N-Ac-Sar-Gly-Val-D-Ile-Thr-Nva-Ile-Arg-ProNHCH₂CH₃,
(2) pyroGlu-Gly-Val-D-Ile-Thr-Nva-Ile-Arg-ProNHCH₂CH₃,
(3) N-Ac-Sar-Gly-Val-D-Ile-Thr-Nva-Ile-Arg-ProNHCH₃,
(4) N-Ac-Sar-Gly-Val-D-Ile-Thr-Nva-Ile-Arg-ProNHCH(CH₃)₂,
(5) N-Ac-Sar-Gly-Val-D-Ile-Thr-Nva-Ile-Arg-ProNHCH₂CH₂-(1-pyrrolidine),
(6) N-Ac-Sar-Gly-Val-D-Ile-Thr-Nva-Ile-Arg-ProNHethyl(1-piperidine),
(7) N-Ac-Sar-Gly-Val-D-Ile-Thr-Nva-Ile-Arg-ProNHmethylcyclopropyl,
(8) N-Ac-Sar-Gly-Val-D-Ile-Thr-Nva-Ile-Arg-ProNH (ethyl-1-(R)-cyclohexyl),
(9) N-Ac-Sar-Gly-Val-D-Ile-Thr-Nva-Ile-Arg-ProNH₂,
(10) N-Ac-Sar-Gly-Val-D-Ile-Thr-Nva-Ile-Arg-ProNHCH₂CH₂OCH₃,
(11) N-Ac-Sar-Gly-Val-D-Ile-Thr-Nva-Ile-Arg-ProNHCH₂CH₂cyclohexyl,
(12) N-Ac-Sar-Gly-Val-D-Ile-Thr-Nva-Ile-Arg-ProNHCH₂CH₂CH₃,
(13) N-Ac-Sar-Gly-Val-D-alloIle-Thr-Nva-Ile-Arg-ProNHCH₂CH₃,
(14) N-Ac-Sar-Gly-Val-D-Leu-Thr-Nva-Ile-Arg-ProNHCH₂CH₃,
(15) N-Ac-Sar-Gly-Val-De-Thr-Nva-Ile-Arg-ProNHCH₂CH₃,
(16) N-Ac-Sar-Gly-Val-Gly-Thr-Nva-Ile-Arg-ProNHCH₂CH₃,
(17) N-Ac-Sar-Gly-Val-D-Val-Thr-Nva-Ile-Azg-ProNHCH₂CH₃,
(18) N-Ac-Sar-Gly-Val-D-Ala-Thr-Nva-lie-Arg-ProNHCH₂CH₃,
(19) N-Ac-Sar-Gly-Val-D-Met-Thr-Nva-Ile-Arg-ProNHCH₂CH₃,
(20) N-Ac-Sar-Gly-Val-D-Nle-Thr-Nva-Ile-Arg-ProNHCH₂CH₃,
(21) N-Ac-Sar-Gly-Val-D-Phe-Thr-Nva-Ile-Arg-ProNHCH₂CH₃,
(22) N-Ac-Sar-Gly-Val-D-Tyr-Thr-Nva-Ile-Arg-ProNHCH₂CH₃,
(23) N-Ac-Sar-Gly-Val-D-4,4-Biphenylala-Thr-Nva-Ile-Arg-ProNHCH₂CH₃,
(24) N-Ac-Sar-Gly-Val-D-Cha-Thr-Nva-Ile-Arg-PrONHCH₂CH₃,
(25) N-Ac-Sar-Gly-Val-D-Chg-Thr-Nva-Ile-Arg-ProNHCH₂CH₃,
(26) N-Ac-Sar-Gly-Val-D-4-ClPhe-Thr-Nva-Ile-Arg-ProNHCH₂CH₃,
(27) N-Ac-Sar-Gly-Val-D-Hphe-Thr-Nva-Ile-Arg-ProNHCH₂CH₃,
(28) N-Ac-Sar-Gly-Val-Dehydroleu-Thr-Nva-Ile-Arg-ProNHCH₂CH₃,
(29) N-Ac-Sar-Gly-Val-D-3-CF3Phe-Thr-Nva-Ile-Arg-ProNHCH₂CH₃,
(32) N-Ac-Sar-Gly-Val-D-3-CIPhe-Thr-Nva-Ile-Arg-ProNHCH₂CH₃,
(33) N-Ac-Sar-Gly-Val-D-2-Thienylala-Tbr-Nva-Ile-Arg-ProNHCH₂CH₃,
(34) N-Ac-Sar-Gly-Val-D-3-CNPhe-Thr-Nva-Ile-Arg-ProNHCH₂CH₃,
(35) N-Ac-Sar-Gly-Val-D-Ile-Thr-DNva-Ile-Arg-ProNHCH₂CH₃,
(36) N-Ac-Sar-Gly-Val-D-Ile-Thr-Gln-Ile-Arg-ProNHCH,CH₃,
(37) N-Ac-Sar-Gly-Val-D-Ile-fhr-C'na-Ile-Arc, ProNHCH₂CH₃,
(38) N-Ac-Sar-Gly-Val-D-Ile-Tk-Gly-Ile-Arg-ProNHCH₂CH₃,
(39) N-Ac-Sar-Gly-Val-D-Ile-Thr-Ala-Ile-Arg-ProNHCH₂CH₃,
(40) N-Ac-Sar-Gly-Val-D-Ile-Thr-Val-Ile-Arg-ProNHCH₂CH₃,
(41) N-Ac-Sar-Gly-Val-D-Ile-Thr-Abu-Ile-Arg-ProNHCH₂CH₃,
(42) N-Ac-Sar-Gly-Val-D-Ile-Thr-Allylgly-Ile-Arg-ProNHCH₂CH₃,
(43) N-Ac-Sar-Gly-Val-D-Ile-Thr-Octylgly-Ile-Arg-ProNHCH₂CH₃,
(44) N-Ac-Sar-Gly-Val-D-Ile-Thr-Met-Ile-Arg ProNHCH₂CH₃,
(45) N-Cyclohexylacetyl-Sar-Gly-Val-D-Ile-Thr-Nva-Ile-Arg-ProNHCH₂CH₃,
(46) N-(2-Me-Nicotinyl)-Sar-Gly-Val-D-Ile-Thr-Nva-Ile-Arg-ProNHCH₂CH,
(47) N-Succinyl-Sar-Gly-Val-D-Ile-Thr-Nva-De-Arg-ProNHCH₂CH₃,
(48) N-Nicotinyl-Sar-Gly-Val-D-Ile-Thr-Nva-Ile-Arg-ProNHCH₂CH₃,
(49) N-Propionyl-Sar-Gly-Val-D-Ile-Thr-Nva-Ile-Arg-ProNHCH₂CH₃,
(50) N-(MeO)acetyl-Sar-Gly-Val-D-Ile-Thr-Nva-Ile-Arg-ProNHCH₂CH₃,
(51) N-(Shikimyl)-Sar-Gly-Val-D-lle-Thr-Nva-Ile-Arg-ProNHCH₂CH₃,
(52) N-(2-Furoyl)-Sar-Gly-Val-D-Ile-Thr-Nva-Ile-Arg-ProNHCH₂CH₃,
(53) N-Butyryl-Sar-Gly-Val-D-Ile-.Thr-Nva-Ile-Aro,-ProNHCH₂CH₃,
(54) N-[2-THFcarbonyl]-Sar-Gly-Val-D-Ile-Thr-Nva-Ile-Arg-ProNHCH₂CH;
(55) N-[CH₃C(O)NH-(CH₂)₂-O-(CH₂)₂-O-CH₂-C(O)]-Sar-Gly-Val-D-Ile-Thr-Nva-Ile-Arg-PrONHCH₂CH₃,
(56) N-[6-N-acetyl-(CH₂)₅C(O)]-Sar-Gly-VaI-D-Ile-Thr-Nva-Ile-Arg-ProNHCH₂CH₃,
(58) N-[4-N-Acetylaminobutyryl]-Sar-Gly-Val-D-Ile-Thr-Nva-Ile-Arg-ProNHCH₂CH₃,
(59) H-Sar-Gly-Val-D-Ile-Thr-Nva-Ile-Arg-ProNHCH₂CH₃,
(66) N-Ac-Sar-Gly-VaI-D-Ile-Thr-Leu-Ile-Arg-ProNHCH₂CH₃,
(67) N-Ac-Sar-Gly-Val-D-Ile-Thr-Ser-Ile-Arg-ProNHCH₂CH₃,
(68) N-Ac-Sar-Gly-Val-D-Ile-Thr-Nva-Ile-Arg-Pro-D-AlaNH₂,
(77) N-Ac-Sar-Gly-Val-D-Tyr (Et)-Thr-Nva-Ile-Arg-ProNHCH₂CH₃,
(78) N-Ac-Sar-Gly-Val-D-Cys (tBu)-Thr-Nva-ne-Arg-ProNHCH₂CH₃,
(79) N-Ac-Sar-Gly-Val-D-Cys-Thr-Nva-Ile-Arg-ProNHCH₂CH₃,
(80) N-Ac-Sar-Gly-Val-D-Tyr(Bzl)-Thr-Nva-Ile-Arg-ProNHCH₂CH₃,
(81) N-Ac-Sar-Gly-Val-D-Ser(Bzl)-Thr-Nva-Ile-Arg-ProNHCH₂CH₃,
(82) N-Ac-Sar-Gly-Val-D-lNal-Thr-Nva-Ile-Arg-ProNHCH₂CH₃,
(83) N-Ac-Sar-Gly-Val-D-tButylgly-Tbr-Nva-Ile-Arg-ProNHCH₂CH₃,
(84) N-Ac-Sar-Gly-Val-D-Orn-Thr-Nva-Ile-Arg-ProNHCH₂CH₃,
(85) N-Ac-Sar-Gly-Val-D-Thr (Bz1)-Thr-Nva-Ile-Arg-ProNHCH₂CH₃,
(86) N-Ac-Sar-Gly-Val-D-2Nal-Tbr-Nva-Ile-Arg-ProNHCH₂CH₃,
(87) N-Ac-Sar-Gly-Val-D-Phe (4-Me)-Thr-Nva-Ile-Arg-ProNHCH₂CH₃,
(88) N-Ac-Sar-Gly-Val-D-Phe (3,4-diMeO)-Thr-Nva-Ile-Arg-ProNHCH₂CH₃,
(89) N-Ac-Sar-Gly-Val-D-Phe (3,4,5-triF)-Thr-Nva-Ile-Arg-ProNHCH₂CH₃,
(90) N-Ac-Sar-Gly-Val-D-Phe (4-NO₂)-Thr-Nva-Ile-Arg-ProNHCH₂CH₃,
(91) N-Ac-Sar-Gly-Val-D-Pen-Thr-Nva-Ile-Arg-ProNHCH₂CH₃,
(92) N-Ac-Sar-Gly-Val-D-Pen (Acm)-Thr-Nva-Ile-Arg-ProNHCH₂CH₃,
(93) N-Ac-Sar-Gly-Val-D-Abu-Thr-Nva-Ile-Arg-ProNHCH₂CH₃,
(94) N-Ac-Sar-Gly-Val-D-Phe (4-NH₂)-Thr-Nva-Ile-Arg-ProNHCH₂CH₃,
(108) N-Ac-Sar-Gly-Val-D-Leu-Ala-Nva-Ile-Arg-ProNHCH₂CH₃,
(109) N-Ac-Sar-Gly-Val-D-Leu-Pro-Nva-Ile-Arg-ProNHCH₂CH₃,
(110) N-Ac-Sar-Gly-Val-D-Leu-Trp-Nva-Ile-Arg-ProNHCH₂CH₃,
(111) N-Ac-Sar-Gly-Val-D-Leu-Tyr-Nva-ne-Arg-ProNHCH₂CH₃,
(112) N-Ac-Sar-Gly-Val-D-Leu-Nva-Nva-Ile-Arg-ProNHCH₂CH₃,
(113) N-Ac-Sar-Gly-Val-D-Leu-Gly-Nva-Ile-Arg-ProNHCH₂CH₃,
(114) N-Ac-Sar-Gly-Val-D-Leu-Lys(Ac)-Nva-IIe-Arg-ProNHCH₂CH₃,
(115) N-Ac-Sar-Gly-Val-D-Leu-2Nal-Nva-Ile-Arg-ProNHCH₂CH₃
(116) N-Ac-Sar-Gly-Val-D-Leu-INal-Nva-Ile-Arg-ProNHCH₂CH₃,
(117) N-Ac-Sar-Gly-Val-D-Leu-Octylgly-Nva-Ile-Arg-ProNHCH₂CH₃,
(118) N-Ac-Sar-Gly-Val-D-Leu-Gln-Nva-Ile-Arg-ProNHCH₂CH₃,
(119) N-Ac-Sar-Gly-Val-D-Leu-Met-Nva-Ile-Arg-ProNHCH₂CH₃,
(120) N-Ac-Sar-Gly-Val-D-Leu-Ser-Nva-Ile-Arg-ProNHCH₂CH₃,
(121) N-Ac-Sar-Gly-Val-D-Leu-Allylgly-Nva-Ile-Arg-ProNHCH₂CH₃,
(122) N-Ac-Sar-Gly-Val-D-Leu-Ile-Nva-Ile-Arg-ProNHCH₂CH₃,
(123) N-Ac-Sar-Gly-Val-D-Leu-D-Thr-Nva-De-Arg-ProNHCH₂CH₃,
(124) N-Ac-Sar-Gly-Val-D-Ile-Thr-Ile-Ile-Arg-ProNHCH₂CH₃,
(125) N-Ac-Sar-Gly-Val-D-Ile-Thr-Nle-Ile-Arg-ProNHCH₂CH₃,
(126) N-Ac-Sar-Gly-Val-D-Ile-Thr-Cit-Ile-Arg-ProNHCH₂CH₃,
(127) N-Ac-Sar-Gly-Val-D-Ile-Thr-Met(O₂)-Ile-Arg-ProNHCH₂CH₃,
(128) N-Ac-Sar-Gly-Val-D-Ile-Thr-Arg-Ile-Arg-ProNHCH₂CH₃,
(129) N-Ac-Sar-Gly-Val-D-Ile-Thr-Tyr-Ile-Arg-ProNHCH₂CH₃,
(130) N-Ac-Sar-Gly-Val-D-Ile-Thr-Glu-Ile-Arg-ProNHCH₂CH₃,
(131) N-Ac-Sar-Gly-Val-D-Ile-Thr-Lys (Ac)-Ile-Arg-ProNHCH₂CH₃,
(132) N-Ac-Sar-Gly-Val-D-Ile-Thr-Propargylgly-Ile-Arg-ProNHCH₂CH₃,
(133) N-Ac-Sar-Gly-Val-D-alloIle-Thr-Gln-Ile-Arg-ProNHCH₂CH₃,
(134) N-Ac-Sar-Gly-Val-D-Leu-Thr-Gln-Ile-Arg-ProNHCH₂CH₃,
(135) N-Ac-Bala-Sar-Gly-Val-D-Ile-Thr-Nva-Ile-Arg-ProNHCH₂CH₃,
(136) N-Phenylacetyl-Sar-Gly-Val-D-Ile-Thr-Nva-Ile-Arg-ProNHCH₂CH₃,
(137) N-Ac-Sar-Gly-Val-D-Ile-Thr-Nva-Ile-Arg-Pro-AzaglyNH₂,
(139) N-Ac-Sar-Gly-Val-D-Ile-Thr-Nva-Ile-Arg-Pro-SerNH₂,
(140) N-Succinyl-Sar-Gly-Val-D-Leu-Thr-Nva-Ile-Arg-ProNHCH₂CH₃,
(158) N-Ac-Sar-Gly-Val-D-Ile-Thr-Gln-De-Arg-ProNHCH(CH₃)₂,
(159) N-Succinyl-Sar-Gly-VaI-D-Ile-Thr-Gln-Ile-Arg-ProNHCH₂CH₃,
(160) N-Succinyl-Sar-Gly-Val-D-Leu-Thr-Gln-Ile-Arg-PrONHCH2CH3,
(161) N-Succinyl-Sar-Gly-Val-D-Leu-Thr-G1n-Ile-Arg-PrONHCH(CH₃)₂,
(162) N-Ac-Sar-Gly-Val-D-Leu-Thr-Asp-fle-Arg-ProNHCH₂CH₃,
(163) N-Ac-Sar-Gly-Val-D-IIe-Thr-Asp-Ile-Arg-ProNHCH₂CH₃,
(164) N-A:c-Sar-Gly-Val-D-Ile-Thr-Asn-Ile-Ara ProNHCH₂CH₃,
(165) N-Ac-Sar-Gly-Val-D-Ile-Thr-Met(O)-Ile-Arg-ProNHCH₂CH₃,
(166) N-Ac-Sar-Gly-Val-D-Leu-Thr-Asn-Ile-Arg-ProNHCH₂CH₃,
(167) N-Ac-Sar-Gly-Val-D-Thr-Thr-Nva-Ile-Arg-ProNHCH₂CH₃,
(168) N-Ac-Sar-Gly-Val-D-Ser-Thr-Nva-Ile-Arg-ProNHCH₂CH₃,
(169) N-Ac-Sar-Gly-Val-D-Hser-Thr-Nva-Ile-Arg-ProNHCH₂CH₃,
(170) N-Ac-Sar-Gly-Val-D-Gln-Thr-Nva-Ile-Arg-ProNHCH₂CH₃,
(171) N-Ac-Sar-Gly-Val-D-Asn-Thr-Nva-ne-Arg-ProNHCH₂CH₃,
(172) N-Ac-Sar-Gly-Val-D-Cit-Thr-Nva-Ile-Arg-ProNHCH₂CH₃,
(173) N-Ac-Sar-Gly-Val-D-Hcit-Thr-Nva-De-Arg-ProNHCH₂CH₃,
(174) N-Ac-Sar-Gly-Val-D-Hle-Thr-Nva-Ile-Arg-ProNHCH₂CH₃,
(175) N-Ac-Sar-Gly-val-D-Neopentylgly-Thr-Nva-Ile-Arg-ProNHCH₂CH₃,
(176) N-Ac-Sar-Gly-Val-D-Ile-Thr-Phe(4-CONH₂)-Ile-Arg-ProNHCH₂CH₃,
(197) N-Succinyl-Sar-Gly-Val-D-alloIle-Thr-Nva-Ile-Arg-ProNHCH₂CH₃,
(1989) N-Succinyl-Sar-Gly-Val-D-Ile-Thr-Gln-Ile-Arg-ProNHCH₂CH₃,
(199) N-Succinyl-Sar-Gly-Val-D-Ile-Thr-Gln-Ile-Arg-Pro-D-AlaNH₂,
(200) N-Succinyl-Sar-Gly-Val-D-alloIle-Thr-Gln-Ile-Arg-ProNHCH₂CH₃,
(201) N-Succinyl-Sar-Gly-Val-D-allolle-T'nr-GIn-Ile-Arg-Pro-D-AlaNH₂,
(202) N-Succinyl-Sar-Gly-Val-D-allolle-Thr-Gln-Ile-Arg-ProNHCH₂CH₃,
(203) N-Succinyl-Sar-Gly-Val-D-Ile-Thr-Gln-Ile-Arg-ProNHCH₂CHs,
(204) N-Ac-Sar-Gly-Val-D-alloIle-Thr-Nva-Ile-Arg Pro-D-AlaNH₂,
(205) N-Ac-Sar-Gly-Val-D-alloIle-Thr-Nva-Ile-Arg-ProNHCH(CH3)2.
(206) N-Ac-Sar-Gly-Val-D-Ile-Thr-Gln-Ile-Arg-Pro-D-AlaNH₂,
(207) N-Ac-Sar-Gly-Val-D-Ile-Thr-GIn-Ile-Arg-PrONHCH(CH₃)₂,
(208) N-Ac-Sar-Gly-Val-D-alloIle-Thr-Gln-Ile-Arg-Pro-D-AlaNH₂,
(209) N-Ac-Sar-Gly-Val-D-alloIle-Thr-Gln-Ile-Arg-ProNHCH(CH₃)₂,
(210) N-Ac-Sar-Gly-Val-D-Ile-Thr-Nva-Ile-Arg-Pro-SarNH₂,
(211) N-Ac-Sar-Gly-Val-D-allolle-Thr-Nva-lie-Arg-Pro-SarNH₂,
(212) N-Ac-Sar-Gly-Val-D-Ile-Thr-Gln-Ile-Arg-Pro-SarNH₂,
(213) N-Ac-Sar-Gly-Val-D-allolle-Thr-Gln-Ile-Arg-Pro-SarNH₂,
(214) N-Ac-Sar-Gly-Val-D-alloIle-Thr-Ser-Ile-Arg-Pro-D-AlaNH₂,
(215) N-Ac-Sar-Gly-Val-D-allolle-Thr-Ser-Ile-A-rg-ProNHCH(CH₃)₂,
(216)N-Ac-Sar-Gly-Val-D-allolle-Thr-Ser-Ile-Arg-ProNHCH₂CH₃,
(217) N-Ac-Sar-Gly-Val-D-Ile-Thr-Orn(Ac)-Ile-Arg-ProNHCH₂CH₃,
(218) N-Ac-Sar-GIy-Val-D-Ile-Thr-Gln-Tle-Arg-Pro-AzaglyNH₂,
(219) N-Ac-Sar-Gly-Val-D-alloIle-Thr-Nva-Ile-Arg-Pro-AzaglyNH₂,
(220) N-Ac-Sar-Gly-Val-D-alloIle-Thr-Gln-Ile-Arg-Pro-AzaglyNH₂,
(221) N-(2-THFcarbonyl)-Sar-Gly-Val-D-alloIle-Thr-Nva-Ile-Arg-ProNHCH₂CH₃,
(222) N-(2-THFcarbonyl)-Sar-Gly-Val-D-Ile-Thr-Gln-Ile-Arg-ProNHCH₂CH₃,
(223) N-(2-7TWcarbonyl)-Sar-Gly-Val-D-alloIle-Thr-Gln-Ile-Arg-Pro NHCH₂CH₃.
(224) N-(2-THFcarbonyl)-Sar-Gly-Val-D-Ile-Thr-Gln-Ile-Arg-Pro-D-AlaNH₂,
(225) N-(2-THFcarbonyl)-Sar-Gly-Val-D-alloIle-Thr-Gln-Ile-Arg-Pro-D-AlaNH₂,
(226) N-(2-THFcarbonyl)-Sar-Gly-Val-D-alloIle-Thr-Gln-Ile-Arg-Pro NHCH(CH₃)₂,
(227) N-(6-Ac-Aca)-Sar-Gly-Val-D-alloIle-Thr-Nva-Ile-Arg-ProNHCH₂CH₃,
(228) N-(6-Ac-Aca)-Sar-Gly-Val-D-Ile-Thr-Gin-Ile-Arg-ProNHCH₂CH₃,
(229) N-(6-Ac-Aca)-Sar-Gly-Val-D-alloIle-Thr-Gln-Ile-Arg-ProNHCH₂CH₃,
(230) N-(6-Ac-Aca)-Sar-Gly-Val-D-Ile-Thr-Gln-Ile-Arg-Pro-D-AlaNH₂,
(231) N-(6-Ac-Aca)-Sar-Gly-Val-D-alloIle-Thr-Gln-Ile-Arg-Pro-D-AlaNH₂,
(232) N-(6-Ac-Aca)-Sar-Gly-Val-D-alloIle-Thr-Gln-Ile-Arg-ProNHCH(CH₃)₂,
(233) N-(4-Ac-Gaba)-Sar-Gly-Val-D-allolle-Thr-Nva-Ile-Arg-PrONHCH₂CH₃,
(234) N-(4-Ac-Gaba)-Sar-Gly-Val-D-Ile-Thr-Gln-Ile-Arg-ProNHCH₂CH₃,
(235) N-(4-Ac-Gaba)-Sar-Gly-Val-D-allolle-Thr-GIn-Ile-Arg-PrONHCH₂CH₃
(236) N-(4-Ac-Gaba)-Sar-Gly-Val-D-Ile-Thr-Gln-Ile-Arg-Pro-D-AlaNH₂,
(237) N-(4-Ac-Gaba)-Sar-Gly-Val-D-alloIle-Thr-Gln-Ile-Arg-Pro-D-AlaNH₂,
(238) N-(4-Ac-Gaba)-Sar-Gly-Val-D-alloIle-Thr-Gln-Ile-Arg-Pro NHCH(CH₃)₂,
(239) N-(2-Furoyl)-Sar-Gly-Val-D-alloIle-Thr-Nva-Ile-Arg-ProNHCH₂CH₃,
(240) N-(2-Furoyl)-Sar-Gly-Val-D-Ile-Thr-GIn-ile-Arg-PrONHCH₂CH₃;
(241) N-(2-Furoyl)-Sar-Gly-Val-D-alloIle-Thr-Gln-Ile-Arg-ProNHCH₂CH₃,
(242) N-(2-Furoyl)-Sar-Gly-Val-D-Ile-Thr-Gln-Ile-Arg-Pro-D-AlaNH₂,
(243) N-(2-Furoyl)-Sar-Gly-Val-D-alloIle-Thr-Gln-Ile-Arg-Pro-D-AlaNH₂,
(244) N-(2-Furoyl)-Sar-Gly-Val-D-allolle-Thr-Gln-Ile-Arg-ProNHCH(CH₃)₂,
(245) N-(Shikimyl)-Sar-Gly-Val-D-allolle-Thr-Nva-Ile-Arg-ProNHCH₂CH₃,
(246) N-(Shikimyl)-Sar-Gly-Val-D-Ile-Thr-Gln-Ile-Arg-ProNHCH₂CH₃,
(247) N-(Shikimyl)-Sar-Gly-Val-D-alloIle-Thr-Gln-Ile-Arg-ProNHCH₂CH₃,
(248) N-(Shikimyl)-Sar-Gly-Val-D-Ile-Thr-Gln-Ile-Arg-Pro-D-AlaNH₂,
(249) N-(Shikimyl)-Sar-Gly-Val-D-alloIle-Thr-Gln-Ile-Arg-Pro-D-AlaNH₂,
(250) N-(Shikimyl)-Sar-Gly-Val-D-alloIle-Thr-Gln-Ile-Arg-ProNHCH(CH₃)₂,
(251) N-(2-Me-Nicotinyl)-Sar-Gly-Val-D-allolle-Thr-Nva-Ile-Arg-ProNHCH₂CH₃.
(252) N-(2-Me-Nicotinyl)-Sar-Gly-Val-D-Ile-Thr-Gln-Ile-Arg-ProNHCH₂CH₃,
(253) N-(2-Me-Nicotinyl)-Sar-Gly-Val-D-alloIle-Thr-Gln-Ile-Arg-Pro NHCH₂CH₃.
(254) N-(2-Me-Nicotinyl)-Sar-Gly-Val-D-Ile-Thr-Gln-Ile-Arg-Pro-D-AlaNH₂,
(255) N-(2-Me-Nicotinyl)-Sar-Gly-Val-D-alloIle-Thr-Gln-Ile-Arg-Pro-D-AlaNH₂,
(256) N-(2-Me-Nicotinyl)-Sar-Gly-Val-D-alloIle-Thr-Gln-Ile-Arg-ProNHCH(CH₃)₂,
(257) N-Ac-Sar-Gly-Val-D-alloIle-Thr-Leu-Ile-Arg-Pro-D-AlaNH₂,
(258) N-Ac-Sar-Gly-Val-D-Ile-Thr-Leu-Ile-Arg-ProNHCH(CH₃)₂,
(259) N-Ac-Sar-Gly-Val-D-alloIle-Thr-Leu-Ile-Arg-ProN-HCH₂CH₃,
(260) N-Ac-Sar-Gly-Val-D-Ile-Thr-Leu-Ile-Arg-Pro-D-AlaNH₂,
(261) N-Succinyl-Sar-Gly-Val-D-Ile-Thr-Leu-Ile-Arg-Pro-D-AlaNH₂,
(262) N-Succinyl-Sar-Gly-Val-D-Ile-Thr-Leu-Ile-Arg-ProNHCH₂CH₃,
(263) N-Succinyl-Sar-Gly-Val-D-Ile-Thr-Leu-Ile-Arg-ProNHCH₂CH₃,
(264) N-Succinyl-Sar-Gly-Val-D-alloIle-Thr-Leu-Ile-Arg-ProNHCH₂CH₃,
(265) N-Succinyl-Sar-Gly-Val-D-alloIle-Thr-Leu-Ile-Arg-Pro-D-AlaNH₂,
(266) N-Succinyl-Sar-Gly-Val-D-Ile-Thr-Leu-Ile-Axg-Pro-AzaglyNH₂,
(267) N-Ac-Sar-Gly-Val-D-alloIle-Thr-Nva-Ile-Arg-ProNHethyl-(1-pyrrolidine),
(268) N-Ac-Sar-Gly-Val-D-alloIle-Thr-Nva-Ile-Arg-ProNH (ethyl-1-cyclohexyl),
(269) N-Ac-Sar-Gly-Val-D-Ile-Thr-Gln-Ile-Arg-ProNHethyl-(1-pyrrolidine),
(270) N-Ac-Sar-Gly-Val-D-Ile-Thr-Gln-Ile-Arg-ProNH (ethyl-1-cyclohexyl),
(271) N-Succinyl-Sar-Gly-Val-D-Ile-Thr-Gln-Ile-Arg-ProNH (ethyl-1-cyclohexyl).
(272) N-Ac-Sar-Gly-Val-D-alloIle-Thr-Nva-Ile-Arg-ProNHCH₂CH₂OCH₃,
(273) N-Ac-Sar-Gly-Val-D-Ile-Thr-Gln-Ile-Arg-ProNHCH₂CH₂OCH₃,
(274) N-Ac-Sar-Gly-Val-D-Ile-Thr-Ser-Ile-Ara ProNHCH₂CH₂OCH₃,
(275) N-Ac-Sar-Gly-Val-D-Ile-Thr-Leu-Ile-Arg-ProNHCH2CH₂OCH₃,
(276) N-Succinyl-Sar-Gly-Val-D-Ile-Thr-Nva-Ile-Arg-ProNHCH₂CH₂OCH₃,
(277) N-Succinyl-Sar-Gly-Val-D-Ile-Thr-Gln-Ile-Arg-ProNHCH₂CH₂OCH₃,
(278) N-Succinyl-Sar-Gly-Val-D-alloIle-Thr-Gln-Ile-Arg-ProNHCH₂CH₂OCH₃,
(279) N-Ac-Sar-Gly-Val-D-Ile-Ser-Nva-Ile-Arg-ProNHCH₂CH₂OCH₃,
(280) N-Ac-Sar-Gly-Val-D-Leu-Ser-Nva-Ile-Arg-ProNHCH₂CH₂OCH₃,
(281) N-Ac-Sar-Gly-Val-D-alloIle-Thr-Allygly-Ile-Arg-ProNHCH₂CH₃,
(282) N-Ac-Sar-Gly-Val-D-Ile-Tbr-Allygly-Ile-Arg-ProNHCH(CH₃)₂,
(283) N-Ac-Sar-Gly-Val-D-Ile-Thr-Allygly-Ile-Arg-Pro-D-AlaNH₂,
(284) N-Ac-Sar-Gly-Val-D-alloIle-Thr-Allygly-Ile-Arg-Pro-D-AlaNH₂,
(285) N-Succinyl-Sar-Gly-Val-D-Ile-Thr-Allygly-Ile-Arg-Pro-D-AlaNH₂,
(286) N-Ac-Sar-Gly-Val-D-Ile-Ser-Allygly-Ile-Arg-Pro-ProNHCH₂CH₃,
(287) N-Ac-Sar-Gly-Val-D-Leu-Ser-Allygly-Ile-Arg-Pro-ProNECH₂CH₃,
(288) N-Ac-Sar-Gly-Val-D-Ile-Thr-Nva-Ile-Arg-Pro-SarNH₂,
(289) N-Ac-Sar-Gly-Val-D-Ile-Thr-Nva-Ile-Arg-ProNHOH,
(290) N-Ac-Sar-Gly-Val-D-Ile-Ser-Nva-Ile-Arg ProNHCH₂CH₃,
(291) N-Ac-Sar-Gly-Val-D-alloIle-Ser-Nva-Ile-Arg-PrONHCH₂CH₃,
(292) N-Ac-Sar-Gly-Val-D-Leu-Hser-Nva-Ile-Arg-ProNHCH₂CH₃,
(300) N-Ac-Sar-Gly-Val-D-alloIle-Thr-Ala-Ile-Arg-ProNHCH₂CH₃,
(301) N-Ac-Sar-Gly-Val-D-Ile-Thr-Ala-Ile-Arg-ProNHCH(CH₃)₂,
(302) N-Ac-Sar-Gly-Val-D-Ile-Thr-Ala-Ile-Arg-Pro-D-AlaNH₂,
(303) N-Ac-Sar-Gly-Val-D-alloIle-Thr-Ala-Ile-Arg-Pro-D-AlaNH₂,
(304) N-Succinyl-Sar-Gly-Val-D-Ile-Thr-Ala-Ile-Arg-Pro-D-AlaNH₂,
(305) N-Ac-Sar-Gly-Val-D-Ile-Ser-Ala-Ile-Arg-ProNHCH₂CH₃,
(306) N-Ac-Sar-Gly-Val-D-Leu-Ser-Ala-Ile-Arg-ProNHCH₂CH₃,
(307) N-Ac-Sar-Gly-Val-D-alloIle-T'hr-Val-Ile-Arg-ProNHCH₂CH₃,
(308) N-Ac-Sar-Gly-Val-D-Ile-Thr-Val-Ile-Arg-ProNHCH(CH₃)₂,
(309) N-Ac-Sar-Gly-Val-D-Ile-Thr-Val-Ile-Arg-Pro-D-AlaNH₂,
(310) N-Ac-Sar-Gly-Val-D-alloIle-Thr-Val-Ile-Arg-Pro-D-AlaNH2,
(311) N-Succillyl-Sar-Gly-Val-D-Ile-Thr-Val-Ile-Arg-Pro-D-AlaNH₂,
(312) N-Ac-Sar-Gly-Val-D-Ile-Ser-Val-Ile-Arg-ProNHCH₂CH₃,
(313) N-Ac-Sar-Gly-Val-D-Leu-Ser-Val-Ile-Arg-ProNHCH₂CH₃,
(314) N-Ac-Sar-Gly-Val-D-alloIle-Thr-D-Nva-Ile-Arg-ProNHCH₂CH₃,
(315)N-Ac-Sar-Gly-Val-D-Ile-Thr-D-Nva-Ile-Arg-ProNHCH(CH₃)₂,
(316) N-Ac-Sar-Gly-Val-D-Ile-Thr-D-Nva-Ile-Arg-Pro-D-AlaNH₂,
(317) N-Ac-Sar-Gly-Val-D-alloIle-Thr-D-Nva-Ile-Arg-Pro-D-AlaNH₂,
(318) N-Succinyl-Sar-Gly-Val-D-IIe-Thr-D-Nva-Ile-Arg-Pro-D-AlaNH₂,
(319) N-Ac-Sar-Gly-Val-D-Ile-Ser-D-Nva-Ile-Arg-ProNHCH₂CH₃,
(320) N-Ac-Sar-Gly-Val-D-Leu-Ser-D-Nva-Ile-Arg-ProNHCH₂CH₃,
(321) N-Ac-Sar-Gly-Val-D-Ile-Ser-Gln-Ile-Arg-ProNHCH₂CH₃,
(322) N-Ac-Sar-Gly-Val-D-Leu-Ser-Gln-IIe-Arg-ProNHCH₂CH₃,
(323) N-Ac-Sar-Gly-Val-D-Leu-Ser-Nva-Ile-Arg-Pro-D-AlaNH₂,
(324) N-Ac-Sar-Gly-Val-D-Ile-Ser-Nva-Ile-Arg-Pro-D-AlaNH₂,
(325) N-Succinyl-Sar-Gly-Val-D-Leu-Ser-Nva-Ile-Arg-ProNHCH₂CH₃
(326) N-Succinyl-Sar-Gly-Val-D-Ile-Ser-Nva-Ile-Arg-ProNHCH₂CH₃,
(327) N-Succinyl-Sar-Gly-Val-D-Leu-Ser-Gln-Ile-Arg-ProNHCH₂CH₃,
(328) N-Succinyl-Sar-Gly-Val-D-Ile-Ser-Gln-Ile-Arg-ProNHCH₂CH₃,
(329) N-Ac-Sar-Gly-Val-D-Ile-Ser-Ser-Ile-Arg-ProNHCH₂CH₃,
(330) N-Ac-Sar-Gly-Val-D-Leu-Ser-Ser-Ile-Arg-ProNHCH₂CH₃,
(331) N-Ac-Sar-Gly-Val-D-Leu-Ser-Nva-Ile-Arg ProNHCH₂CH₂CH₃,
(332) N-Ac-Sar-Gly-Val-D-Ile-Ser-Nva-Ile-Arg-ProNHCH₂CH₂CH₃,
(333) N-Ac-Sar-Gly-Val-D-Leu-Ser-Leu-Ile-Arg-ProNHCH₂CH₃,
(334) N-Ac-Sar-Gly-Val-D-Ile-Ser-Leu-Ile-A-rg-ProNHCH₂CH₃,
(335) N-Ac-Sar-Gly-Val-D-alloIle-Ser-Nva-Ile-Arg-ProNHCH₂CH₃,
(336) N-Ac-Sar-Gly-Val-D-alloIle-Ser-Gln-Ile-Arg-ProNHCH₂CH₃,
(337) N-Succinyl-Sar-Gly-Val-D-alloIle-Ser-Nva-Ile-Arg-ProNHCH₂CH₂,
(338) N-Ac-Sar-Gly-Val-D-alloIle-Ser-Nva-Ile-Arg-ProNHCH₂CH₂CH₃,
(339) N-Ac-Sar-Gly-Val-D-alloIle-Ser-Nva-Ile-Arg-Pro-D-AlaNH₂,
(340) N-Ac-Sar-Gly-Val-D-alloIle-Ser-Leu-Ile-Arg-ProNHCH₂CH₃,
(341) N-Ac-Sar-Gly-Val-D-alloIle-Ser-Ser-Ile-Arg-ProNHCH₂CH₃,
(342) N-Ac-Sar-Gly-Val-D-IIe-Gly-Nva-Ile-Arg-ProNHCH₂CH₃,
(343) N-Ac-Sar-Gly-Val-D-alloIle-Gly-Nva-Ile-Arg-ProNHCH₂CH₃,
(344) N-Ac-Sar-Gly-Val-D-Leu-Gly-Gln-Ile-Arg-ProNHCH₂CH₃,
(345) N-Ac-Sar-Gly-Val-D-Ile-Gly-Gln-Ile-Arg-ProNHCH₂CH₃,
(346) N-Ac-Sar-Gly-Val-D-alloIle-Gly-Gln-De-Arg-ProNHCH₂CH₃,
(347) N-Ac-Sar-Gly-Val-D-Ile-Tyr-Nva-Ile-Arg-ProNHCH₂CH₃,
(348) N-Ac-Sar-Gly-Val-D-alloIle-Tyr-Nva-Ile-Arg-ProNHCH₂CH),
(349) N-Ac-Sar-Gly-Val-D-Leu-Tyr-Gln-Ile-Arg-ProNHCH₂CH₃,
(350) N-Ac- Sar-Gly-Val-D-Ile-Tyr-Gln-Ile-Arg-PrONHCH₂CH₃,
(351) N-Ac-Sar-Gly-Val-D-alloIle-Tyr-Gln-Ile-Arg-ProNHCH₂CH₃,
(352) N-Ac-Sar-Gly-Val-D-Ser-Thr-Nva-Ile-Arg-ProNHCH₂CH₃,
(353) N-Ac-Sar-Gly-Val-D-Thr-Thr-Nva-Ile-Arg-ProNHCH₂CH₃,
(354) N-Ac-Sar-Gly-Val-D-Gln-Thr-Nva-Ile-Arg-ProNHCH₂CH₃,
(355) N-Ac-Sar-Gly-Val-D-Asn-Thr-Nva-Ile-Arg-ProNHCH₂CH₃,
(356) N-Ac-Sar-Gly-Val-D-Arg-Thr-Nva-Ile-Arg-ProNHCH₂CH₃,
(357) N-Ac-Sar-Gly-Val-D-3-Pal-Thr-Nva-Ile-Arg-ProNHCH₂CH₃,
(358) N-Ac-Sar-Gly-Val-D-Glu-Thr-Nva-Ile-Arg-ProNHCH₂CH₃,
(359) N-Ac-Sar-Gly-Val-D-Asp-Thr-Nva-lie-Arg-ProNHCH₂CH₃,
(360) N-Ac-Sar-Gly-Val-D-His-Thr-Nva-Ile-Arg-PrONHCH₂CH₃,
(361) N-Ac-Sar-Gly-Val-D-Hser-Thr-Nva-Ile-Arg-ProNHCH₂CH₃,
(362) N-Ac-Sar-Gly-Val-D-alloThr-Thr-Nva-Ile-Arg-ProNHCH₂CH₃,
(363) N-Ac-Sar-Gly-Val-D-Ile-Thr-Nva-D-Ile-Arg-ProNHCH₂CH₃,
(364) N-Ac-Sar-Gly-Val-D-Ser-Thr-Gln-Ile-Arg-ProNHCH₂CH₃,
(365) N-Ac-Sar-Gly-Val-D-Thr-Thr-Gln-Ile-Arg-ProNHCH₂CH₃,
(366) N-Ac-Sar-Gly-Val-D-alloThr-Thr-GIn-Ile-Arg-PrONHCH₂CH₃,
(367) N-Ac-Sar-Gly-Val-D-Ser-Ser-Nva-Ile-Arg-ProNHCH₂CH₃,
(368) N-Ac-Sar-Gly-Val-D-Thr-Ser-Nva-Ile-Mg-ProNHCH₂CH₃,
(369) N-Ac-Sar-Gly-Val-D-alloThr-Ser-Nva-Ile-Arg-ProNHCH₂CH₃,
(370) N-Ac-Sar-Gly-Val-D-alloThr-Ser-Gln-Ile-Arg-ProNHCH₂CH₃,
(371) N-Ac-Sar-Gly-Val-D-Thr-Ser-Gln-Ile-Arg-ProNHCH₂CH₃,
(372) N-(6-Ac-Aca)-Sar-Gly-Val-D-Leu-Ser-Gln-Ile-Arg-ProNHCH₂CH₃,
(373) N-(6-Ac-Aca)-Sar-Gly-Val-D-Leu-Ser-Nva-Ile-Arg-ProNHCH₂CH₃,
(374) N-(4-Ac-Gaba)-Sar-Gly-Val-D-Leu-Ser-Gln-Ile-Arg-ProNHCH₂CH₃,
(375) N-(4-Ac-Gaba)-Sar-Gly-Val-D-Leu-Ser-Nva-Ile-Arg-ProNHCH₂CH₃,
(376) N-(2-Furoyl)-Sar-Gly-Val-D-Leu-Ser-Gln-Ile-Arg-ProNHCH₂CH₃,
(377) N-(2-Furoyl)-Sar-Gly-Val-D-Leu-Ser-Nva-Ile-Arg-ProNHCH₂CH₃,
(378) N-(Shikimyl)-Sar-Gly-Val-D-Leu-Ser-Gln-Ile-Arg-ProN-HCH₂CH₃,
(379) N-(Shikimyl)-Sar-Gly-Val-D-Leu-Ser-Nva-Ile-Arg-ProNHCH₂CH₃,
(382) N-(2-Me-nicotinyl)-Sar-Gly-Val-D-Leu-Ser-Gln-Ile-Arg-ProNHCH₂CH₃,
(383) N-(2-Me-nicotinyl)-Sar-Gly-Val-D-Leu-Ser-Nva-Ile-Arg-ProNHCH₂CH₃,
(384) N-Ac-Sar-Gly-Val-D-Leu-Ser-Nva-Ile-Arg-ProNHethyl-1-(R)-cyclohexyl,
(385) N-Ac-Sar-Gly-Val-D-Leu-Ser-Gln-Ile-Arg-ProNHethyl-1-(R)-cyclohexyl,
(386) N-Ac-Sar-Gly-Val-DIle-Thr-Ser-Ile-Arg-ProNHethyl-1-(R)-cyclohexyl,
(387) N-Ac-S ar-Gly-Val-D-Leu-Thr-Nva-Ile-Arg-ProNHethyl-1-(R)-cyclohehyl,
(388) N-Ac-Sar-Gly-Val-D-Leu-Ser-Ser-Ile-Arg-ProNHethyl-1-(R)-cyclohexyl,
(389) N-Ac-Sar-Gly-Val-DIle-Thr-Nva-Ile-Arg-ProNHethyl-1-(S)-cyclohexyl,
(390) N-Ac-Sar-Gly-Val-D-Pen-Ser-Nva-Ile-Arg ProNHCH₂CH₃,
(391) N-Ac-Sar-Gly-Val-D-Pen-Gly-Nva-Ile-Arg-ProNHCH₂CH₃,
(392) N-Ac-Sar-Gly-Val-D-Pen-Thr-Gln-Ile-Arg-ProNHCH₂CH₃,
(393) N-Ac-Sar-Gly-Val-D-Pen-Ser-Nva-Ile-Arg-ProNHCH(CH₃)₂,
(394) N-Succinyl-Sar-Gly-Val-D-Pen-Ser-Nva-Ile-Arg-ProNHCH₂CH₃,
(395) N-Ac-Sar-Gly-Val-D-Pen-Ser-Nva-Ile-Arg-Pro-D-AlaNH₂,
(396) N-Ac-Sar-Gly-Val-D-Pen-Ser-Gln-Ile-Arg-ProNHCH₂CH₃,
(397) N-Ac-Sar-Gly-Val-D-Pen-Gly-Gln-Ile-Arg-ProNHCH₂CH₃,
(398) N-Ac-Sar-Gly-Val-D-Pen-Ser-Ser-Ile-Arg-ProNHCH₂CH₃,
(399) N-Ac-Sar-Gly-Val-D-Pen-Thr-Ser-Ile-Arg-ProNHCH₂CH₃,
(400) N-Ac-Sar-Gly-Val-D-Pen-Thr-Leu-Ile-Arg-ProNHCH₂CH₃,
(401) N-Ac-Sar-Gly-Val-D-Pen-Ser-Leu-Ile-Arg-ProNMCH₂CH₃
(402) N-Succinyl-Sar-Gly-Val-D-Pen-Ser-Ser-Ile-Arg-ProNHCH₂CH3,
(403) N-Succinyl-Sar-Gly-Val-D-Pen-Ser-Leu-Ile-Arg-ProNHCH₂CH₃,
(404) N-Succinyl-Sar-Gly-Val-D-Pen-Thr-Gln-Ile-Arg-ProNHCH (CH₃)₂,
(405) N-Ac-Sar-Gly-Val-D-Cys-Thr-Nva-Ile-Arg-ProNHCH₂CH₃,
(406) N-Ac-Sar-Gly-Val-D-Cys-Ser-Nva-Ile-Arg-ProNHCH₂CH₃,
(407) N-Ac-Sar-Gly-Val-D-Cys-Gly-Nva-Ile-Arg-ProNHCH₂CH₃,
(408) N-Ac-Sar-Gly-Val-D-Cys-Thr-Gln-Ile-Arg-ProNHCH₂CH₃,
(409) N-Ac-Sar-Gly-Val-D-Cys-Ser-Nva-Ile-Arg-ProNHCH (CH₃)₂,
(410) N-Succinyl-Sar-Gly-Val-D-Cys-Ser-Nva-Ile-Arg-ProNHCH₂CH₃,
(411) N-Ac-Sar-Gly-Val-D-Cys-Ser-Nva-Ile-Arg-Pro-D-AlaNH₂,
(412) N-Ac-Sar-Gly-Val-D-Cys-Ser-Gln-Ile-Arg-ProNHCH₂CH₃,
(413) N-Ac-Sar-Gly-Val-D-Cys-Gly-Gln-Ile-Arg-ProNHCH₂CH₃,
(414) N-Ac-Sar-Gly-Val-D-Cys-Ser-Ser-Ile-Arg-ProNHCH₂CH₃,
(415) N-Ac-Sar-Gly-Val-D-Cys-Thr-Ser-Ile-Arg-ProNHCH₂CH₃,
(416) N-Ac-Sar-Gly-Val-D-Cys-Thr-Leu-Ile-Arg-ProNHCH₂CH₃,
(417) N-Ac-Sar-Gly-Val-D-Cys-Ser-Leu-Ile-Arg-ProNHCH₂CH₃,
(418) N-Succinyl-Sar-Gly-Val-D-Cys-Ser-Ser-Ile-Arg-ProNHCH₂CH₃,
(419) N-Succinyl-Sar-Gly-Val-D-Cys-Ser-Leu-Ile-Arg-ProNHCH₂CH₃,
(431) N-Ac-Sar-Gly-Val-D-Leu-Pen-Nva-Ile-Ara ProNHCH₂CH₃,
(432) N-Ac-Sar-Gly-Val-D-Ile-Pen-Nva-Ile-Arg-ProNHCH₂CH₃,
(433) N-Ac-Sar-Gly-Val-D-alloIle-Pen-Nva-Ile-Arg-ProNHCH₂CH₃,
(434) N-Ac-Sar-Gly-Val-D-Ile-Pen-Gln-Ile-Arg-ProNHCH₂CH₃,
(435) N-Ac-Sar-Gly-Val-D-Ile-Pen-Ser-Ile-Arg-ProNHCH₂CH₃,
(436) N-Ac-Sar-Gly-Val-D-Ile-Pen-Leu-Ile-Arg-ProNHCH₂CH₃,
(437) N-Ac-Sar-Gly-Val-D-IIe-Pen-Nva-Ile-Arg-ProNHCH(CH₃)₂,
(438) N-Ac-Sar-Gly-Val-D-Ile-Pen-Nva-Ile-Arg-Pro-D-AlaNH₂,
(439) N-Succinyl-Sar-Gly-Val-D-Ile-Pen-Nva-Ile-Arg-ProNHCH₂CH₃,
(440) N-Succinyl-Sar-Gly-Val-D-Ile-Pen-Gln-Ile-Arg-ProNHCH₂CH₃,
(441) N-Suceinil-Sar-Gly-Val-D-Ile-Pen-Gln-Ile-Arg-ProNHCH(CH₃)₂,
(442) N-Ac-Sar-Gly-Val-D-Ile-Thr-Pen-Ile-Arg-ProNHCH₂CH₂OCH₃,
(443) N-Ac-Sar-Gly-Val-D-allolle-Thr-Pen-Ile-Arg-ProNHCH₂CH₃,
(444) N-Ac-Sar-Gly-Val-D-Leu-Thr-Pen-Ile-Arg-ProNHCH₂CH₃,
(445) N-Ac-Sar-Gly-Val-D-Ile-Thr-Pen-Ile-Arg-Pro-D-AlaNH₂,
(446) N-Succinyl-Sar-Gly-Val-D-Ile-Thr-Pen-Ile-Arg-ProNHCH₂CH₃,
(447) N-Ac-Sar-Gly-Val-D-Ile-Thr-Pen-Ile-Arg-ProNHCH(CH₃)₂,
(448) N-Ac-Sar-Gly-Val-D-Leu-Ser-Pen-Ile-Arg-ProNHCH₂CH₃,
(449) N-Ac-Sar-Gly-Val-D-Leu-Gly-Pen-Ile-Arg-ProNHCH₂CH₃,
(450) N-Suceinyl-Sar-Gly-Val-D-Leu-Ser-Pen-Ile-Arg-ProNHCH₂CH₃,
(451) N-Ac-Sar-Gly-Val-D-Phe (3,4,5-triF)-Thr-Gln-Ile-Arg-ProNHCH₂CH₃,
(452) N-Ac-Sar-Gly-Val-D-Phe (3,4,5-triF)-Ser-Nva-Ile-Arg-ProNHCH₂CH₃,
(453) N-Ac-Sar-Gly-Val-D-Phe (3,4,5-triF)-Gly-Nva-Ile-Arg-ProNHCH₂CH₃,
(454) N-Ac-Sar-Gly-Val-D-Phe (3,4,5-triF)-Ser-Leu-Ile-Arg-ProNHCH₂CH₃,
(455) N-Ac-Sar-Gly-Val-D-Phe (3,4,5-triF)-Ser-Nva-Ile-Arg-Pro-D-AlaNH₂,
(456) N-Succinyl-Sar-Gly-Val-D-Phe (3,4,5-triF)-Thr-Gln-Ile-Arg-ProNHCH₂CH₃,
(457) N-Succinyl-Sar-Gly-Val-D-Phe (3,4,5-triF)-Ser-Gln-Ile-Arg ProNHCH₂CH₃,
(458) N-Succinyl-Sar-Gly-Val-D-Phe (3,4,5-triF)-Thr-Gln-Ile-Arg-ProNHCH (CH₃)₂,
(459) N-Ac-Sar-Gly-Val-D-Phe (3,4,5-triF)-Ser-Gln-Ile-Arg-ProNHCH₂CH₃,
(460) N-Ac-Sar-Gly-Val-D-Phe (3,4,5-triF)-Ser-Ser-Ile-Arg-ProNHCH₂CH₃,
(470) N-(3-Ac-Bala)-Sar-Gly-Val-D-alloIle-Thr-Nva-Ile-Arg-ProNHCH₂CH₃,
(471) N-(3-Ac-Bala)-Sar-Gly-Val-D-Ile-Thr-Gln-Ile-Arg-ProNHCH₂CH₃,
(472) N-(3-Ac-Bala)-Sar-Gly-Val-D-alloIle-Thr-Gln-Ile-Arg-ProNHCH₂CH₃,
(473) N-(3-Ac-Bala)-Sar-Gly-Val-D-Ile-Thr-Gln-Ile-Arg-Pro-DAlaNH₂,
(474) N-(3-Ac-Bala)-Sar-Gly-Val-D-alloIle-Thr-Gln-Ile-Arg-Pro-DAlaNH₂,
(475) N-(3-Ac-Bala)-Sar-Gly-Val-D-alloIle-Thr-Gln-Ile-Arg-ProNHCH(CH₃)₂,
(476) N-(3-Ac-Bala)-Sar-Gly-Val-D-Leu-Ser-Nva-Ile-Arg-ProNHCH₂CH₃,
(477) N-(3-Ac-Bala)-Sar-Gly-Val-D-Leu-Thr-Nva-Ile-Arg-ProNHCH₂CH₃,
(478) N-(3-Ac-Bala)-Sar-Gly-Val-D-Pen-hr-Nva-Ile-Arg-ProNHCH₂CH₃,
(479) N-(3-Ac-Bala)-Sar-Gly-Val-D-Ile-Ser-Nva-Ile-Arg-ProNHCH₂CH₃,
(484) N-Ac-Sar-Gly-Val-D-Ile-Thr-Nva-Ile-Arg-Pro-OH,
(485) N-Ac-Sar-Gly-Val-D-alloIle-Thr-NNra-Ile-Ara,-Pro-OH,
(486) N-Ac-Sar-Gly-Val-D-Leu-Thr-Nva-Ile-Arg-Pro-OH,
(487) N-Ac-Sar-Gly-Val-D-Pen-Thr-Nva-Ile-Arg-Pro-OH,
(488) N-Ac-Sar-Gly-Val-D-Phe (3,4,5-triF)-Thr-Nva-Ile-Arg-Pro-OH,
(489) N-Ac-Sar-Gly-Val-D-Ile-Thr-Gln-Ile-Arg-Pro-OH,
(490) N-Ac-Sar-Gly-Val-D-Leu-Ser-Nva-Ile-Arg-Pro-OH,
(492) N-Ac-Sar-Gly-Val-D-Ile-Ser-Gln-Ile-Arg-Pro-OH,
(493) N-Succinyl-Sar-Gly-Val-D-Ile-l'hr-Nva-Ile-Arg-Pro-OH,
(494) N-Succinyl-Sar-Gly-Val-D-Leu-Tbr-Gln-Ile-Arg-Pro-OH,
(495) N-Ac-Sar-Gly-Val-allo-Ile-Thr-Nva-Ile-Arg-ProNHCH₂CH₃,
(496) N-Ac-Sar-Crly-Val-D-Gly-VAl-Thr-Nva-Ile-Arg-ProNHCH₂CH₃,
(497) N-Ac-Sar-Gly-Val-D-Trp-Thr-Nva-Ile-Arg-ProNHCH₂CH₃,
(498) N-Ac-Sar-Gly-Val-D-33-Dipheylala-Thr-Nva-Ile-Arg-ProNHCH₂CH₃,
(499) N-Ac-Sar-Gly-Val-D-3-Benzothienylala-Thr-Nva-Ile-Arg-ProNHCH₂CH₃,
(500) N-Ac-Sar-Gly-Val-D-3,4-diF-Phe-Thr-Nva-Ile-Arg-PrQNHCH₂CH₃,
(501) N-Ac-Sar-Gly-Val-D-Pen(Bzl)-Thr-Nva-Ile-Arg-ProNHCH₂CH₃,
(502) N-Ac-Sar-Gly-Val-D-Leu-Thr-Gln-Ile-Arg-ProNH(CH₃)CH₂,
(503) H-Sar-Gly-Val-D-Leu-Thr-Gln-Ilz-Arg-ProNHCH₂CH₃
(508) N-Ac-Sar-Glv-Val-D-Ile Thr-Nya-Ile-Arg-ProNHCH₂CH₂OH and
(510) N-Ac-Sar-Gly-Val-D-Ile -Thr-Leu-Ile-Arg-ProNH((R)-1-cyclohexylethyl)

It is well known in the art that modifications and changes can be made in the structure of a polypeptide without substantially altering the biological function of that peptide. For example, certain amino acids can be substituted for other amino acids in a given polypeptide without any appreciable loss of function. In making such changes, substitutions of like amino acid residues can be made on the basis of relative similarity of side-chain substituents, for example, their size, charge, hydrophobicity, hydrophilicity, and the like.

In describing the invention, certain abbreviations are used for the sake of convenience throughout the specification, including the examples, to refer to reagents and compounds useful for preparing the compounds of the invention. When so used, the following abbreviations are meant to refer to the following: DMF for dimethylformamide; DMA for dimethylacetamide; DIEA for diisopropylethylamine; HATU for 0-(7-azabenzotriazol-l-yl)-N,N,N',N'-tetramethyluronium haxafluorophosphate; NMP for N-methylpyrrolidone; and TFA for trifluoroacetic acid.

### Determination of Biological Activity

### Pellet Preparation

Ten microliters of a mixture containing a final concentration of 1, 5, or 10 mM of the peptides of invention, 100 ng of bFGF (Collaborative Biomedical Products, Bedford, MA), and 6% Hydron (Sigma, St. Louis, MO) were pipetted into the tip of a sterile Teflon rod. After drying for 1-2 hours, the pellets were stored at 4 °C.

### Pellet Implantation

A small (about 2 mm) radial incision at 1 mm from the center of the cornea was performed in anesthetized Sprague Dawley rats. With a curved iris spatula, an intrastromal pocket was made to a distance of 1 mm from the limbus-the circular blood vessels that surround the cornea. A single pellet was implanted. Antibiotic ointment (neosporin) was applied post surgery to the operated eye to prevent infection and to decrease inflammation.

### Data Analysis

At day seven post-implantation, neovascularization was measured through a slitlamp biomicroscopy (Nikon NS-1), connected to an image analysis system (Leica Qwin). The response was calculated by colorimetrically detecting the area of new blood vessels, and calculating the new vessel surface area in µm². The compounds of the invention inhibit rat cornea neovascularization as shown in Table 2.

**Table 2**

| Effect of the Compounds of the Invention on Rat Cornea Neovascularization | | |
|---|---|---|
| Peptide | Number of Comeas/Dose | % Inhibition |
| Example 1 | 6/10 µM | 92.6 |
| Example 1 | 5/5 µM | 74.8 |
| Example 1 | 4/6 µM | 71.5 |
| untreated | 5/- | - |

The compounds of the invention, including but not limited to those specified in the examples, possess anti-angiogenic activity. As angiogenesis inhibitors, such compounds are useful in the treatment of both primary and metastatic solid tumors, including carcinomas of breast, colon. rectum, lung, oropharynx, hypopharynx, esophagus, stomach, pancreas, liver, gallbladder and bile ducts, small intestine, urinary tract (including kidney, bladder and urothelium), female genital tract, (including cervix, uterus, and ovaries as well as choriocarcinoma and gestational trophoblastic disease), male genital tract (including prostate, seminal vesicles, testes and and germ cell tumors), endocrine glands (including the thyroid, adrenal, and pituitary glands), and skin, as well as hemangiomas, melanomas, sarcomas (including those arising from bone and soft tissues as well as Kaposi's sarcoma) and tumors of the brain, nerves, eyes, and meninges (including astrocytomas, gliomas, glioblastomas, retinoblastomas, neuromas, neuroblastbmas, Schwannomas, and meningiomas). Such compounds may also be useful in treating solid tumors arising from hematopoietic malignancies such as leukemias (i.e. chloromas, plasmacytomas and the plaques and tumors of mycosis fungoides and cutaneous T-cell lymphoma/leukemia) as well as in the treatment of lymphomas (both Hodgkin's and non-Hodgkin's lymphomas). In addition, these compounds may be useful in the prevention of metastases from the tumors described above either when used alone or in combination with radiotherapy and/or other chemotherapeutic agents.

Further uses include the treatment and prophylaxis of autoimmune diseases such as rheumatoid, immune and degenerative arthritis; various ocular diseases such as diabetic retinopathy, retinopathy of prematurity, corneal graft rejection, retrolental fibroplasia, neovascular glaucoma, rubeosis, retinal neovascularization due to macular degeneration, hypoxia, angiogenesis in the eye associated with infection or surgical intervention, and other abnormal neovascularization conditions of the eye; skin diseases such as psoriasis; blood vessel diseases such as hemagiomas, and capillary proliferation within atherosclerotic plaques; Osler-Webber Syndrome; myocardial angiogenesis; plaque neovascularization; telangiectasia; hemophiliac joints; angiofibroma; and wound granulation. Other uses include the treatment of diseases characterized by excessive or abnormal stimulation of endothelial cells, including but not limited to intestinal adhesions, Crohn's disease, atherosclerosis, scleroderma, and hypertrophic scars, i.e. keloids. Another use is as a birth control agent, by inhibiting ovulation and establishment of the placenta. The compounds of the invention are also useful in the treatment of diseases that have angiogenesis as a pathologic consequence such as cat scratch disease (*Rochele minalia quintosa*) and ulcers (*Helicobacter pylori*)*.* The compounds of the invention are also useful to reduce bleeding by administration prior to sugery, especially for the treatment of resectable tumors.

The compounds of the invention may be used in combination with other compositions and procedures for the treatment of diseases. For example, a tumor may be treated conventionally with surgery, radiation or chemotherapy combined with a peptide of the present invention and then a peptide of the present invention may be subsequently administered to the patient to extend the dormancy of micrometastases and to stabilize and inhibit the growth of any residual primary tumor. Additionally, the compounds of the invention may be combined with pharmaceutically acceptable excipients, and optionally sustained-release matrices, such as biodegradable polymers, to form therapeutic compositions.

A sustained-release matrix, as used herein, is a matrix made of materials, usually polymers, which are degradable by enzymatic or acid-base hydrolysis or by dissolution. Once inserted into the body, the matrix is acted upon by enzymes and body fluids. A sustained-release matrix desirably is chosen from biocompatible materials such as liposomes, polylactides (polylactic acid), polyglycolide (polymer of glycolic acid), polylactide co-glycolide (copolymers of lactic acid and glycolic acid) polyanhydrides, poly(ortho)esters, polypeptides, hyaluronic acid, collagen, chondroitin sulfate, carboxylic acids, fatty acids, phospholipids, polysaccharides, nucleic acids, polyamino acids, amino acids such as phenylalanine, tyrosine, isoleucine, polynucleotides, polyvinyl propylene, polyvinylpyrrolidone and silicone. A preferred biodegradable matrix is a matrix of one of either polylactide, polyglycolide, or polylactide co-glycolide (co-polymers of lactic acid and glycolic acid).

When used in the above or other treatments, a therapeutically effective amount of one of the compounds of the present invention may be employed in pure form or, where such forms exist, in pharmaceutically acceptable salt form. By a "therapeutically effective amount" of the compound of the invention is meant a sufficient amount of the compound to treat an angiogenic disease, (for example, to limit tumor growth or to slow or block tumor metastasis) at a reasonable benefit/risk ratio applicable to any medical treatment. It will be understood, however, that the total daily usage of the compounds and compositions of the present invention will be decided by the attending physician within the scope of sound medical judgment. The specific therapeutically effective dose level for any particular patient will depend upon a variety of factors including the disorder being treated and the severity of the disorder; activity of the specific compound employed; the specific composition employed, the age, body weight, general health, sex and diet of the patient; the time of administration, route of administration, and rate of excretion of the specific compound employed; the duration of the treatment; drugs used in combination or coincidential with the specific compound employed; and like factors well known in the medical arts. For example, it is well within the skill of the art to start doses of the compound at levels lower than those required to achieve the desired therapeutic effect and to gradually increase the dosage until the desired effect is achieved.

The compounds of the present invention can be used in the form of salts derived from inorganic or organic acids. These salts include but are not limited to the following: acetate, adipate, alginate, citrate, aspartate, benzoate, benzenesulfonate, bisulfate, butyrate, camphorate, camphorsufonate, digluconate, glycerophosphate, hemisulfate, heptanoate, hexanoate, fumarate; hydrochloride, hydrobromide, hydroiodide, 2-hydroxy-ethansulfonate (isothionate), lactate, maleate, methanesulfonate, nicotinate, 2-naphthalenesulfonate, oxalate, palmoate, pectinate, persulfate, 3-phenylpropionate, picrate, pivalate, propionate, succinate, tartrate, thiocyanate, phosphate, glutamate, bicarbonate, *p*-toluenesulfonate and undecanoate. Water or oil-soluble or dispersible products are thereby obtained.

Examples of acids which may be employed to form pharmaceutically acceptable acid addition salts include such inorganic acids as hydrochloric acid, sulphuric acid and phosphoric acid and such organic acids as acetic acid, maleic acid, succinic acid and citric acid. Other salts include salts with alkali metals or alkaline earth metals, such as sodium, potassium, calcium or magnesium or with organic basis. Preferred salts of the compounds of the invention include phosphate, tris and acetate.

Alternatively, a compound of the present invention may be administered as pharmaceutical compositions containing the compound of interest in combination with one or more pharmaceutically acceptable excipients. A pharmaceutically acceptable carrier or excipient refers to a non-toxic solid, semi-solid or liquid filler, diluent, encapsulating material or formulation auxiliary of any type. The compositions may be administered parenterally, intracistemally, intravaginally, intraperitoneally, topically (as by powders, ointments, drops or transdermal patch), rectally, or bucally. The term "parenteral" as used herein refers to modes of administration which include intravenous, intramuscular, intraperitoneal, intrastemal, subcutaneous and intraarticular injection and infusion.

Pharmaceutical compositions for parenteral injection comprise pharmaceutically-acceptable sterile aqueous or nonaqueous solutions, dispersions, suspensions or emulsions, as well as sterile powders for reconstitution into sterile injectable solutions or dispersions just prior to use. Examples of suitable aqueous and nonaqueous carriers, diluents, solvents or vehicles include water, ethanol, polyols (such as glycerol, propylene glycol, polyethylene glycol, and the like), carboxymethylcellulose and suitable mixtures thereof, vegetable oils (such as olive oil), and injectable organic esters such as ethyl oleate. Proper fluidity may be maintained, for example, by the use of coating materials such as lecithin, by the maintenance of the required particle size in the case of dispersions, and by the use of surfactants.

These compositions may also contain adjuvants such as preservative, wetting agents, emulsifying agents, and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents such as sugars, sodium chloride, and the like. Prolonged absorption of the injectable pharmaceutical form may be brought about by the inclusion of agents which delay absorption, such as aluminum monostearate and gelatin.

Injectable depot forms are made by forming microencapsule matrices of the drug in biodegradable polymers such as polylactide-polyglycolide, poly(orthoesters), poly(anhydrides), and (poly)glycols, such as PEG. Depending upon the ratio of drug to polymer and the nature of the particular polymer employed, the rate of drug release can be controlled. Depot injectable formulations are also prepared by entrapping the drug in liposomes or microemulsions which are compatible with body tissues.

The injectable formulations may be sterilized, for example, by filtration through a bacterial-retaining filter, or by incorporating sterilizing agents in the form of sterile solid - compositions which can be dissolved or dispersed in sterile water or other sterile injectable medium just prior to use.

Topical administration includes administration to the skin or mucosa, including surfaces of the lung and eye. Compositions for topical administration, including those for inhalation, may be prepared as a dry powder which may be pressurized or non-pressurized. In non-pressurized powder compositions, the active ingredient in finely divided form may be used in admixture with a larger-sized pharmaceutically-acceptable inert carrier comprising particles having a size, for example, of up to 100 micrometers in diameter. Suitable inert carriers include sugars such as lactose. Desirably, at least 95% by weight of the particles of the active ingredient have an effective particle size in the range of 0.01 to 10 micrometers.

Alternatively, the composition may be pressurized and contain a compressed gas, such as nitrogen or a liquified gas propellant. The liquified propellant medium and indeed the total composition is preferably such that the active ingredient does not dissolve therein to any substantial extent. The pressurized composition may also contain a surface active agent, such as a liquid or solid non-ionic surface active agent or may be a solid anionic surface active agent. It is preferred to use the solid anionic surface active agent in the form of a sodium salt.

A further form of topical administration is to the eye. A compound of the invention is delivered in a pharmaceutically acceptable ophthalmic vehicle, such that the compound is maintained in contact with the ocular surface for a sufficient time period to allow the compound to penetrate the corneal and internal regions of the eye, as for example the anterior chamber, posterior chamber, vitreous body, aqueous humor, vitreous humor, cornea, iris/ciliary, lens, choroid/retina and sclera. The pharmaceutically-acceptable ophthalmic vehicle may, for example, be an ointment, vegetable oil or an encapsulating material. Alternatively, the compounds of the invention may be injected directly into the vitreous and aqueous humour.

Compositions for rectal or vaginal administration are preferably suppositories which may be prepared by mixing the compounds of this invention with suitable nonirritating excipients or carriers such as cocoa butter, polyethylene glycol or a suppository wax which are solid at room temperature but liquid at body temperature and therefore melt in the rectum or vaginal cavity and release the active compound.

Compounds of the present invention may also be administered in the form of liposomes. As is known in the art, liposomes are generally derived from phospholipids or other lipid substances. Liposomes are formed by mono- or multi-lamellar hydrated liquid crystals that are dispersed in an aqueous medium. Any non-toxic, physiologically-acceptable and metabolizable lipid capable of forming liposomes can be used. The present compositions in liposome form can contain, in addition to a compound of the present invention, stabilizers, preservatives, excipients, and the like. The preferred lipids are the phospholipids and the phosphatidyl cholines (lecithins), both natural and synthetic. Methods to form liposomes are known in the art. See, for example, Prescott, Ed., Methods in Cell Biology, Volume XIV, Academic Press, New York, N.Y. (1976), p. 33 et seq*.*

While the compounds of the invention can be administered as the sole active pharmaceutical agent, they may also be used in combination with one or more agents which are conventionally administered to patients for treating angiogenic diseases. For example, the compounds of the invention are effective over the short term to make tumors more sensitive to traditional cytotoxic therapies such as chemicals and radiation. The compounds of the invention also enhance the effectiveness of existing cytotoxic adjuvant anti-cancer therapies. The compounds of the invention may also be combined with other antiangiogenic agents to enhance their effectiveness, or combined with other antiangiogenic agents and administered together with other cytotoxic agents. In particular, when used in the treatment of solid tumors, compounds of the invention may be administered with IL-12, retinoids, interferons, angiostatin, endostatin, thalidomide, thrombospondin-1. thrombospondin-2, captopryl, angioinhibins, TNP-470, pentosan polysulfate, platelet factor 4, LM-609, SU-5416, CM-101, Tecogalan, plasminogen-K-5, vasostatin, vitaxin, vasculostatin, squalamine, marimastat or other MMP inhibitors, antineoplastic agents such as alpha inteferon, COMP (cyclophosphamide, vincristine, methotrexate and prednisone), etoposide, mBACOD (methortrexate, bleomycin, doxorubicin, cyclophosphamide, vincristine and dexamethasone), PRO-MACE/MOPP (prednisone, methotrexate (w/leucovin rescue), doxorubicin, cyclophosphamide, cisplatin, taxol, etoposide/mechlorethamine, vincristine, prednisone and procarbazine), vincristine, vinblastine, and the like as well as with radiation.

Total daily dose of the compositions of the invention to be administered to a human or other mammal host in single or divided doses may be in amounts, for example, from 0.0001 to 300 mg/kg body weight daily and more usually 1 to 300 mg/kg body weight.

It will be understood that agents which can be combined with the compound of the present invention for the inhibition, treatment or prophylaxis of angiogenic diseases are not limited to those listed above, but include in principle any agents useful for the treatment or prophylaxis of angiogenic diseases.

The peptides of the invention may be used for the development of affinity columns for isolation of receptors relevant to the antiangiogenic activity of the peptide of the invention, e.g. TSP-1 receptor, in, for example, cultured endothelial cells. As is known in the art, isolation and purification of the receptor may be followed by amino acid sequencing to identify and isolate polynucleotides which encode the receptor. Recombinant expression of this receptor would allow greater amounts of receptor to be produced, e.g. to produce a sufficient quantity for use in high throughput screening assays to identify other angiogenesis inhibitors.

The peptides of the present invention may be chemically coupled to isotopes, enzymes, carrier proteins, cytotoxic agents, fluorescent molecules, chemiluminescent, bioluminescent and other compounds for a variety of applications. For example, a peptide may be labeled to facilitate testing of its ability to bind antisera or to detect cell types which possess a relevant receptor. The coupling technique is generally chosen on the basis of the functional groups available on the amino acids of the peptide including, but not limited to amino, sulfhydral, carboxyl, amide, phenol, and imidazole. Various reagents used to effect such couplings include among others, glutaraldehyde, diazodized benzidine, carbodiimide, and p-benzoquinone.

The efficiency of the coupling reaction is determined using different techniques appropriate for the specific reaction. For example, radiolabeling of the peptide with I¹²⁵ may be accomplished using chloramine T and NaI¹²⁵ of high specific activity. The reaction is terminated with sodium metabisulfite and the mixture is desalted on disposable columns. The labeled peptide is eluted from the column and fractions are collected. Aliquots are removed from each fraction and radioactivity measured in a gamma counter. In this manner, a labeled peptide may be obtained which is free from unreacted NaI¹²⁵

The peptides of the present invention can also be used as antigens to generate polyclonal or monoclonal antibodies. Such antibodies can be used in diagnostic methods and kits to detect or quantify the peptide of the invention, or peptides related thereto, in a body fluid or tissue. Results from these tests could be used to diagnose or determine the prognostic relevance of such peptides.

The use of the peptides of the present invention to generate monoclonal antibodies in animals such as the mouse, rabbit or sheep, follows techniques well known in the art. If desired, the antibodies can then be used to make anti-idiotype antibodies which in turn can be humanized as is known in the art to prevent immunological responses. The humanized antibodies can be used to inhibit angiogenesis or to make kits to detect the receptor as described herein.

For the production of polyclonal antisera in rabbits, sheep, goats or other animals the peptides of the invention are coupled, for example through lysine residues, to purified bovine serum albumin using glutaraldehyde. The efficiency of this reaction may be determined by measuring the incorporation of radiolabeled peptide. Unreacted glutaraldehyde and peptide may be separated by dialysis and the conjugate stored for subsequent use.

Serum samples from generation of polyclonal antisera or media samples from production of monoclonal antisera may be analyzed for determination of antibody titer and in particular, for the determination of high titer antisera. Subsequently, the highest titer antisera may be tested to establish the following: a) optimal antiserum dilution for highest specific binding of the antigen and lowest non-specific binding, b) ability to bind increasing amounts of peptide in a standard displacement curve, c) potential cross-reactivity with immunologically-related peptides and proteins (including plasminogen, TSP-1, and TSP-1 of related species), and d) ability to detect the peptide of the invention in extracts of plasma, urine, tissues, and in cell culture media.

Titer may be established through several means known in the art, such as by dot blot and density analysis, and also by precipitation of radiolabeled peptide-antibody complexes using protein A, secondary antisera, cold ethanol or charcoal-dextran followed by activity measurement with a gamma counter. If desired, the highest titer antisera may be purified on affinity columns. For example, the peptides of the invention may be coupled to a commercially available resin and used to form an affinity column. Antiserum samples may then be passed through the column so that antibodies to the peptides of the invention bind (via the peptide) to the column. These bound antibodies are subsequently eluted, collected and evaluated for determination of titer and specificity.

Kits for measurement of the compounds of the invention are also contemplated as part of the present invention. Antisera that possess the highest titer and specificity and can detect the peptides of the invention in extracts of plasma, urine, tissues, and in cell culture media may be used to establish assay kits for rapid, reliable, sensitive, and specific measurement and localization of peptides of the invention. These assay kits may employ (but are not limited to) the following techniques: competitive and non-competitive assays, radioimmunoassay (RIA), bioluminescence and chemiluminescence assays, fluorometric assays, sandwich assays, immunoradiometric assays, dot blots, enzyme linked assays including ELISA, microtiter plates, antibody coated strips or dipsticks for rapid monitoring of urine or blood, and immunocytochemistry. For each kit the range, sensitivity, precision, reliability, specificity and reproducibility of the assay are established by means well known to those skilled in the art.

The above described assay kit would provide instructions, antiserum, one or more peptides of the invention, and possibly radiolabeled peptides of the invention and/or reagents for precipitation of bound peptide/antibody complexes. Such a kit would be useful for the measurement of the peptide of the invention in biological fluids and tissue extracts of animals and humans with and without tumors, as is well known in the art.

Another kit may be used to visualize or localize the peptide of the invention in tissues and cells. Immunohistochemistry techniques and kits, for example, which employ such techniques are well known to those of ordinary skill in the art. Such a kit provides antisera to the peptide of the invention, and possibly blocking serum and secondary antiserum linked to a fluorescent molecule such as fluorescein isothiocyanate, or to some other reagent used to visualize the primary antiserum. Using this methodology, biopsied tumors may be examined for sites of peptide production or for sites of the peptide receptor. Alternatively, a kit may supply radiolabeled nucleic acids for use in *in situ* hybridization to probe for messenger RNA which encodes the compound of the invention.

### Synthesis of the Peptides

The polypeptides of the present invention may be synthesized by any techniques that are known to those skilled in the art. For solid phase peptide synthesis, a summary of the many techniques may be found in J.M. Stewart and J.D. Young, Solid Phase Peptide Synthesis, W.H. Freeman Co. (San Francisco), 1963 and J. Meienhofer, Hormonal Proteins and Peptides, vol. 2, p. 46, Academic Press (New York), 1973. For classical solution synthesis see G. Schroder and K. Lupke, The Peptides, vol. 1, Acacemic Press (New York), 1965.

Reagents, resins, amino acids, and amino acid derivatives are commercially available and can be purchased from Chem-Impex International, Inc. (Wood Dale, IL, U.S.A.) or Calbiochem-Novabiochem Corp. (San Diego, CA, U.S.A.) unless otherwise noted herein.

In general, these methods comprise the sequential addition of one or more amino acids or suitably protected amino acids to a growing peptide chain. Normally, either the amino or carboxyl group of the first amino acid is protected by a suitable protecting group. The protected or derivatized amino acid can then be either attached to an inert solid support or utilized in solution by adding the next amino acid in the sequence having the complimentary (amino or carboxyl) group suitably protected, under conditions suitable for forming the amide linkage. The protecting group is then removed from this newly added amino acid residue and the next amino acid (suitably protected) is then added, and so forth. After all the desired amino acids have been linked in the proper sequence, any remaining protecting groups (and any solid support) are removed sequentially or concurrently, to afford the final polypeptide. By simple modification of this general procedure, it is possible to add more than one amino acid at a time to a growing chain, for example, by coupling (under conditions which do not racemize chiral centers) a protected tripeptide with a properly protected dipeptide to form, after deprotection, a pentapeptide.

A particularly preferred method of preparing compounds of the present invention involves solid phase peptide synthesis.

In this particularly preferred method the alpha-amino function is protected by an acid or base sensitive group. Such protecting groups should have the properties of being stable to the conditions of peptide linkage formation, while being readily removable without destruction of the growing peptide chain or racemization of any of the chiral centers contained therein. Suitable protecting groups are 9-fluorenylmethyloxycarbonyl (Fmoc), *t*-butyloxycarbonyl (Boc), benzyloxycarbonyl (Cbz), biphenylisopropyl-oxycarbonyl, *t*-amyloxycarbonyl, isobornyloxycarbonyl, (α,α)-dimethyl-3,5-dimethoxybenzyloxycarbonyl, o-nitrophenylsulfenyl, 2-cyano-t-butyloxycarbonyl, and the like. The 9-fluorenylmethyloxycarbonyl (Fmoc) protecting group is preferred.

Particularly preferred side chain protecting groups are, for side chain amino groups as in lysine and arginine: 2,2,5,7,8-pentamethylchroman-6-sulfonyl (pmc), nitro, p-toluenesulfonyl, 4-methoxybenzenesulfonyl, Cbz, Boc, and adamantyloxycarbonyl; for tyrosine: benzyl, o-bromobenzyloxycarbonyl, 2,6-dichlorobenzyl, isopropyl, t-butyl (*t*-Bu), cyclohexyl, cyclopenyl and acetyl (Ac); for serine: *t*-butyl, benzyl and tetrahydropyranyl; for histidine: trityl, benzyl, Cbz, p-toluenesulfonyl and 2,4-dinitrophenyl; for tryptophan: formyl and Boc.

In the solid phase peptide synthesis method, the C-terminal amino acid is attached to a suitable solid support or resin. Suitable solid supports useful for the above synthesis are those materials which are inert to the reagents and reaction conditions of the stepwise condensation-deprotection reactions, as well as being insoluble in the media used. The preferred solid support for synthesis of C-terminal carboxy peptides is 4-hydroxymethylphenoxymethyl-copoly(styrene-1% divinylbenzene). The preferred solid support for C-terminal amide peptides is 4-(2,4-dimethoxyphenyl-Fmoc-aminomethyl)phenoxy-acetamidoethyl resin available from Applied Biosystems.

The C-terminal amino acid is coupled to the resin by means of N,N'-dicyclohexylcarbodiimide (DCC), N,N -diisopropylcarbodiimide (DIC) or O-benzotriazol-1-yl-N,N,N,N-tetramethyluroniumhexafluorophosphate (HBTU), with or without 4-dimethylaminopyridine (DMAP), 1-hydroxybenzotriazole (HOBT), benzotriazol-1-yloxy-tris(dimethylamino)phosphoniumhexafluorophosphate (BOP) or bis(2-oxo-3-oxazolidinyl)phosphine chloride (BOPC1), mediated coupling for from about 1 to about 24 hours at a temperature of between 10 ° and 50 °C in a solvent such as dichloromethane or DMF. When the solid support is 4-(2,4-dimethoxyphenyl-Fmoc-aminomethyl)-phenoxyacetamidoethyl resin, the Fmoc group is cleaved with a secondary amine, preferably piperidine, prior to coupling with the C-terminal amino acid as described above. The preferred method for coupling to the deprotected 4-(2,4-dimethoxyphenyl-Fmoc-aminomethyl)phenoxyacetamidoethyl resin is is O-benzotriazol-1-yl-N,N,N,N-tetramethyluroniumhexafluorophosphate (HBTU, 1 equiv.) and 1-hydroxybenzotriazole (HOBT, 1 equiv.) in DMF.

The coupling of successive protected amino acids can be carried out in an automatic polypeptide synthesizer as is well known in the art. In a preferred embodiment, the α-amino function in the amino acids of the growing peptide chain are protected with Fmoc. The removal of the Fmoc protecting group from the N-terminal side of the growing peptide is accomplished by treatment with a secondary amine, preferably piperidine. Each protected amino acid is then introduced in about 3-fold molar excess and the coupling is preferably carried out in DMF. The coupling agent is normally O-benzotriazol-1-yl-N,N,N,N'-tetramethyluroniumhexafluorophosphate (HBTU, 1 equiv.) and 1-hydroxybenzotriazole (HOBT, 1 equiv.).

At the end of the solid phase synthesis, the polypeptide is removed from the resin and deprotected, either in succession or in a single operation. Removal of the polypeptide and deprotection can be accomplished in a single operation by treating the resin-bound polypeptide with a cleavage reagent, for example thianisole, water, ethanedithiol and trifluoroacetic acid.

In cases wherein the C-terminus of the polypeptide is an alkylamide, the resin is cleaved by aminolysis with an alkylamine. Alternatively, the peptide may be removed by transesterification, e.g. with methanol, followed by aminolysis or by direct transamidation. The protected peptide may be purified at this point or taken to the next step directly. The removal of the side chain protecting groups is accomplished using the cleavage cocktail described above.

The fully deprotected peptide is purified by a sequence of chromatographic steps employing any or all of the following types: ion exchange on a weakly basic resin in the acetate form; hydrophobic adsorption chromatography on underivitized polystyrene-divinylbenzene (for example, AMBERLITE® XAD); silica gel adsorption chromatography; ion exchange chromatography on carboxymethylcellulose; partition chromatography, e.g. on SEPHADEX® G-25, LH-20 or countercurrent distribution; high performance liquid chromatography (HPLC.), especially reverse-phase HPLC on octyl- or octadecylsilyl-silica bonded phase column packing.

The following examples will serve to further illustrate the preparation of the novel compounds of the invention.

### Preparation of the Cleavage Reagent

The cleavage reagent (2 mL) is prepared by mixing, in the following order, thioanisole (100 µL), water (50 µL), ethanedithiol (50 µL) and trifluoroacetic acid (1.8 mL). The freshly-prepared mixture is cooled to -5 °C to -10°C and used as described below.

### Cleavage and Deprotection Procedure

A mixture of resin-bound polypeptide and cleavage reagent is stirred at 0 °C for 10-15 minutes and then at ambient temperature for a further 1.75 hours. The amount of time is increased by 0.5 hours for each additional arginine up to a total of three hours. The amount of cleavage reagent used is determined using the following formula:

| weight of resin (mg) | amount of cleavage reagent (µL) |
|---|---|
| 0-10 | 100 |
| 10-25 | 200 |
| 25-50 | 400 |
| 50-100 | 700 |
| 100-200 | 1200 |

The resin is then filtered off and rinsed with neat trifluoroacetic acid. The filtrate is then added in 0.5 mL portions to a centrifuge tube containing about 8 mL of cold diethyl ether. The suspension is then centrifuged and the supernatant is decanted off. The pellet is re-suspended in about 8 mL of ether, another 0.5 mL of the filtrate is added, and the process is repeated until all of the peptide is precipitated. The precipitated filtrate is then washed with ether, dried and lyophilized.

If the peptide does not precipitate upon addition to ether, the mixture is shaken with aqueous 30% acetic acid. The organic phase is then extracted twice with aqueous 30% acetic acid and the combined aqueous extracts are lyophilized.

### Example 1

### N-Ac-Sar-Gly-Val-D-Ile-Thr-Nva-Ile-Arg-ProNHCH₂CH₃

In the peptide synthesis column position of a Perkin- Elmer/Applied Biosynthesis SYNERGY® peptide synthesizer is placed an Pro(2-ClTrt) peptide synthesis column (25 µM amino acid; Nova Biochem). Amino acids are added sequentially according to the following synthetic cycle:
(1) Solvating the resin using DMF for about 5 minutes;
(2) Washing with DMF for about 5 minutes;
(3) Activating the incoming Fmoc protected amino acid (75 µM) using a 0.2 M solution of HBTU (75 µM) and HOBT (75 µM) in DMSO-NMP (N-methylpyrrolidone);
(4) Coupling using a solution in DMF of the activated Fmoc protected amino acid prepared in step 3 above for about 30 minutes;
(5) Washing with DMF for 5 minutes; and
(6) For peptides capped with acetyl at the N-terminus, substituting acetic acid (87 µM) for an Fmoc protected amino acid and using 87 µM each of HBTU and HOBT.
(7) For peptides capped with ethylamide at the C-terminus, adding DMF to the resin followed by ByProp (1.1 equivalents) and ethylamine (20 equivalents) in THF.

The amino acids were coupled to the resin in the following order using the conditions indicated.

| # Amino Acid | | Coupling |
|---|---|---|
| 1. | Fmoc-Arg(Pmc) | 30 minutes |
| 2. | Fmoc-Ile | 30 minutes |
| 3. | Fmoc-Nva | 30 minutes |
| 4. | Fmoc-Thr(*t*-Bu) | 30 minutes |
| 5. | Fmoc-D-Ile | 30 minutes |
| 6. | Fmoc-Val | 30 minutes |
| 7. | Fmoc-Gly | 30 minutes |
| 8. | Fmoc-Sar | 30 minutes |

Upon completion of the synthesis, the resin was washed with THF for about 5 minutes to remove DMF and shrink the resin. The resin was then gas dried with argon for about 10 minutes and nitrogen for a further 10 minutes to provide the resin-bound peptide (85 mg). Cleavage and deprotection are accomplished using the procedure described above (40 mg of dry resin-bound peptide, 700 µL of cleavage reagent, cleavage time 2.5 hours) to give the crude peptide (14 mg). Purification by HPLC using a 7µm Symmetry Prep C18 column (7.8x300 mm) with solvent mixtures varying in a gradient from 5% to 100% acetonitrile-water over a period of 50 minutes followed by lyophilization provided the desired peptide.

The pure fractions were lyophilized to yield N-Ac-Sar-Gly-Val-D-Ile-Thr-Nva-Ile-Arg-ProNHCH₂CH₃ as the trifluoroacetate salt: Rₜ = 26.5 min (10% to 40% acetonitrile in water containing 0.01 % of TFA, over 30 min period); MS (ESI), m/e 994 (M+H)⁺.

### Example 3.

### N-Ac-Sar-Gly-Val-D-IIe-Thr-Nva-lle-Arg-ProNHCH₃

The procedure described in Example 1 was used but substituting methylamine (2.0 M solution in THF) for ethylamine. After cleavage of the peptide from the resin and removal of the protecting groups the crude product was purified by C-18 column chromatography using a solvent mixture varying in a gradient of 10% to 50% acetonitrile-water containing 0.01 % TFA. The pure fractions were lyophilized to yield N-Ac-Sar-Gly-Val-D-Ile-Thr-Nva-Ile-Arg-ProNHCH₃ as the trifluoroacetate salt: Rₜ = 3.224 min , (gradient of 20% to 95% acetonitrile in water containing 0.01 M NH₄Ac over 10 min period); MS (ESI) m/e 930 (M+H)⁺; Amino Acid Anal.: 1.09 Sar; 1.03 Gly; 0.98 Val; 0.98 Ile; 0.54 Thr; 1.72 Nva; 1.01 Arg; 1.08 Pro.

### Example 4

### N-Ac-Sar-Gly-Val-D-Ile-Thr-Nva-Ile-Arg-ProNHCH₂(CH₃)

The procedure described in Example 1 was used but substituting isopropylamine for ethylamine. After cleavage of the peptide from the resin and removal of the protecting groups the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-Ile-Thr-Nva-Ile-Arg-ProNHIsopropyl as the trifluoroacetate salt: Rₜ = 3.648 min (gradient of 20% to 95% acetonitrile in water containing 0.01 M NH₄Ac over 10 min period); MS (ESI) m/e 1008 (M+H)⁺; Amino Acid Anal.: 1.10 Sar; 0.99 Gly; 0.96 Val; 1.88 Ile; 0.56 Thr; 1.67 Nva; 0.96 Arg; 1.09 Pro.

### Example 5

### N-Ac-Sar-Gly-Val-D-Ile-Thr-Nva-Ile-Arg-ProNHethyl-(1-pyrrolidine)

### Resin Preparation

4-(4-Formyl-3-methoxyphenoxy)butyryl AM resin (0.5 g, 0.54 mmol/g substitution) was placed in a solid phase synthesis reaction vessel containing (9: 1) DMA/acetic acid (4 mL). The mixture was shaken for 5 min. The resin was drained and this process was repeated three times. To the swollen resin were added 10-15 grains of activated 4A molecular sieves and (9:1) DMA/acetic acid (4mL) and 10 molar equivalents of 1-(2-aminoethyl)pyrrolidine. The slurry was shaken for 1h at rt and to it was added 10 molar equivalents of sodium triacetoxyborohydride. The slurry was shaken for 2 h at rt. The resin was drained and washed three times with DMA, three times with methanol, three times with dichloromethane, three times with diethyl ether and dried in vacuo at rt overnight. The dry resin was swollen in DMA (4 mL) and shaken for 5 min. This process was repeated twice.

### Coupling of Fmoc-Pro

To the swollen resin in the reaction vessel were added sequentially the following chemicals: DMA (4 mL), one equivalent of DIEA, a DMA solution containing 3.0 equivalents of Fmoc-Pro, 3.0 equivalents of HATU, and 3.0 equivalents of DIEA. The slurry was shaken overnight. The resin was drained and washed three times with DMA, three times with methanol, three times with dichloromethane, three times with diethyl ether and dried in vacuo at rt overnight. A small portion of the resin was used to determine the Fmoc-Pro loading. The rest of the resin was shaken with DMA (4 mL) three times for 5 min and then for 1 h at rt with a solution of (8:1:1) DMA/pyridine/acetic anhydride (5 mL). The resin was drained and washed three times with DMA, three times with methanol, three times with dichloromethane, and three times with diethyl ether. The resin was dried in vacuo at rt overnight and then used in the subsequent solid phase peptide synthesis.

### Synthesis of above peptide

In the synthesis of the above peptide the amino acids, the coupling conditions and the synthetic protocol used were the identical to as those described in Example 1. Upon completion of the synthesis the peptide and the protecting groups were cleaved at rt using (95:5) TFA/anisole (3 mL) for 3h. The resin was filtered and washed three times with methanol. The combined filtrates were concentrated in vacuo and to the residue was added diethylether. The solid precipitate was filtered. The crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-Ile-Thr-Nva-Ile-Arg-ProNHethyl(1-pyrrolidine) as the bis-trifluoroacetate salt: Rₜ = 4.40 min (gradient of 20% to 95% acetonitrile in water containing 0.01 M NH₄Ac over 10 min period); MS (ESI) m/e 1063 (M+H)⁺; Amino Acid Anal.: 0.95 Sar; 1.0 Gly; 0.86 Val; 1.63 Ile; 0.56 Thr; 1.38 Nva; 0.88 Arg; 1.07 Pro.

### Example 6

### N-Ac-Sar-Gly-Val-D-Ile-Thr-Nva-Ile-Arg-ProNHethyl(1-piperidine)

The procedure described in Example 5 was used but substituting 1-(2-aminoethyl)piperidine for 1-(2-aminoethyl)pyrrolidine in the reductive alkylation step. After cleavage of the peptide from the resin and removal of the protecting groups the crude product was purified by C-18 column chromatography using a solvent mixture varying in a gradient of 10% to 50% acetonitrile-water containing 0.01 % TFA. The pure fractions were lyophilized to yield N-Ac-Sar-Gly-Val-D-Ile-Thr-Nva-Ile-Arg-ProNHethyl-(1-piperidine) as the bis-trifluoroacetate salt: Rₜ = 4.437 min (gradient of 20% to 95% acetonitrile in water containing 0.01 M NH₄Ac over 10 min period); MS (ESI) m/e 1077 (M+H)⁺; Amino Acid Anal.: 1.11 Sar; 1.04 Gly; 0.99 Val; 1.77 Ile; 0.61 Thr; 1.61 Nva; 0.97 Arg; 1.10 Pro.

### Example 7

### N-Ac-Sar-Gly-Val-D-Ile-Thr-Nva-Ile-Arg-ProNHmethylcyclopropyl

The procedure described in Example 1 was used but substituting (aminoethyl)cyclopropane for 1-(2-aminoethylpyrrolidine). After cleavage of the peptide from the resin and removal of the protecting groups the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-Ile-Thr-Nva-Ile-Arg-ProNHmethylcyclopropyl as the trifluoroacetate salt: Rₜ = 3.815 min (gradient of 20% to 95% acetonitrile in water containing 0.01 M NH₄Ac over 10 min period); MS (ESI) m/e 1020 (M+H)⁺; Amino Acid Anal.: 1.01 Sar; 0.96 Gly: 0.96 Val; 1.66 Ile; 0.53 Thr; 1.65 Nva; 1.08 Arg; 1.09 Pro.

### Example 8

### N-Ac-Sar-Gly-V al-D-Ile-Thr-Nva-Ile-Arg-ProNHethyl-1-(R)-cyclohexyl

The procedure described in Example 5 was used but substituting (R)-1-cycloxylethylamine for 1-(2-aminoethylpyrrolidine). After cleavage of the peptide from the resin and removal of the protecting groups the crude product was purified by C-18. column chromatography using solvent mixture varying in a gradient of 10% to 50% acetonitrile-water containing 0.01% TFA. The pure fractions were lyophilized to yield N-Ac-Sar-Gly-Val-D-Ile-Thr-Nva-Ile-Arg-ProNHethyl-1-(R)-cyclohexyl as the trifluoroacetate salt: Rₜ = 5.196 min (gradient of 20% to 95% acetonitrile in water containing 0.01 M NH₄Ac over 10 min period); MS (ESI) m/e 1076 (M+H)⁺; Amino Acid Anal.: 1.19 Sar; 0.99 Gly; 0.62 Val; 1.47 Ile; 0.48 Thr; 1.57 Nva; 1.01 Arg; 0.83 Pro.

### Example 9

### N-Ac-Sar-Gly-Val-D-Ile-Thr-Nva-Ile-Arg-ProNH(2-hydroxyethyl)

The procedure described in Example 5 was used but substituting O-TBDMS-ethanolamine for 1-(2-aminoethylpyrrolidine). After cleavage of the peptide from the resin and removal of the protecting groups the crude product was purified by C-18 column chromatography using solvent mixture varying in a gradient of 10% to 50% acetonitrile-water containing 0.01 % TFA. The pure fractions were lyophilized to yield N-Ac-Sar-Gly-Val-D-Ile-Thr-Nva-Ile-Arg-ProNH(2-hydroxyethyl) as the trifluoroacetate salt: Rₜ = 4.04 min (gradient of 20% to 95% acetonitrile in water containing 0.01 M NH₄Ac over 10 min period); MS (ESI) m/e 1010 (M+H)⁺; Amino Acid Anal.: 1.04 Sar; 1.01 Gly; 0.98 Val; 1.59 Ile; 0.44 Thr; 1.45 Nva; 0.99 Arg; 1.06 Pro.

### Example 10

### N-Ac-Sar-Gly-Val-D-Ile-Thr-Nva-Ile-Arg-ProNH₂

The procedure described in Example 1 was used but substituting Fmoc-Pro-Sieber amide resin for H-Pro-2-CITrt resin. After cleavage of the peptide from the resin and removal of the protecting groups using (9:1) TFA/anisole (3 mL), the crude product was purified by C-18 column chromatography using a solvent mixture varying in a gradient of 10% to 50% acetonitrile-water containing 0.0 1 % TFA. The pure fractions were lyophilized to yield N-Ac-Sar-Gly-Val-D-Ile-Thr-Nva-Ile-Arg-ProNH₂ as the trifluoroacetate salt: Rₜ = 4.063 min (gradient of 20% to 95% acetonitrile in water containing 0.01 M NH₄Ac over 10 min period); MS (ESI) m/e 966 (M+H)⁺; Amino Acid Anal.: 0.87 Sar; 0.98 Gly: 0.94 Val; 1.73 Ile; 0.47 Thr; 1.35 Nva; 1.02 Arg; 1.05 Pro.

### Example 11

### N-Ac-Sar-Gly-Val-D-Ile-Thr-Nva-Ile-Arg-ProNHCH₂CH₂OCH₃

The procedure described in Example 5 was used but substituting 2-methoxyethylamine for 1-(2-aminoethylpyrrolidine). After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield K-Ac-Sar-Gly-Val-D-Ile-Thr-Nva-Ile-Arg-ProNHCH₂CH₂-OCH₃ as the trifluoroacetate salt: Rₜ = 3.40 min (gradient of 20% to 95% acetonitrile in water containing 0.01 M NH₄Ac over 10 min period); MS (ESI) m/e 1024 (M+H)⁺; Amino Acid Anal.: 1.02 Sar; 1.06 Gly; 0.97 Val; 1.54 Ile; 0.47 Thr; 1.81 Nva; 0.97 Arg; 1.25 Pro.

### Example 12

### N-Ac-Sar-Gly-Val-D-Ile-Thr-Nva-Ile-Arg-ProNHCH₂CH₂-cyclohexyl

The procedure described in Example 5 was used but substituting cyclohexylethylamine for 1-(2-aminoethylpyrrolidine). After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-Ile-Thr-Nva-Ile-Arg-ProNHCH₂CH₂-cyclohexyl as the trifluoroacetate salt: Rₜ = 4.97 min (gradient of 20% to 95% acetonitrile in water containing 0.01 M NH₄Ac over 10 min period); MS (ESI) m/e 1076 (M+H)⁺; Amino Acid Anal.: 0.87 Sar; 1.00 Gly; 0.88 Val; 1.34 Ile; 0.44 Thr; 1.61 Nva; 1.07 Arg; 1.05 Pro.

### Example 13

### N-Ac-Sar-Gly-Val-D-Ile-Thr-Nva-Ile-Arg-ProNHCH₂CH₂CH₃

The procedure described in Example 1 was used but substituting propylamine for ethylamine. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-Ile-Thr-Nva-Ile-Arg-ProNHCH₂CH₂CH₃ as the trifluoroacetate salt: Rₜ = 3.68 min (gradient of 20% to 95% acetonitrile in water containing 0.01 M NH₄Ac over 10 min period); MS (ESI) m/e 1008 (M+H)⁺; Amino Acid Anal.: 0.94 Sar; 1.09 Gly; 0.96 Val; 1.58 Ile; 0.51 Thr: 1.78 Nva; 0.96 Arg; 1.23 Pro.

### Example 14

### N-Ac-Sar-Gly-Val-D-alloIle-Thr-Nva-Ile-Arg-PrONHCH₂CH₃

The procedure described in Example 1 was used but substituting Fmoc-D-alloIle for Fmoc-D-Ile. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-alloIle-Thr-Nva-Ile-Arg-ProNHCH₂CH₃ as the trifluoroacetate salt: Rₜ = 22.5 min (gradient of 10% to 30% acetonitrile in water containing 0.01 % TFA over 30 min period); MS (ESI) m/e 994 (M+H)⁺; Amino Acid Anal.: 0.95 Sar; 0.96 Gly: 0.97 Val; 0.99 Ile; 0.54 Thr; 1.66 Nva; 1.14 Arg; 1.08 Pro.

### Example 15

### N-Ac-Sar-Gly-V al-D-Leu-Thr-Nva-Ile-Arg-ProNHCH₂CH₃

The procedure described in Example 1 was used but substituting Fmoc-D-Leu for Fmoc-D-Ile. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-Leu-Thr-Nva-Ile-Arg-ProNHCH₂CH₃ as the trifluoroacetate salt: Rₜ = 3.54 min (gradient of 10% to 30% acetonitrile in water containing 0.01 % TFA over 30 min period); MS (ESI) m/e 994 (M+H)⁺; Amino Acid Anal.: 1.00 Sar; 0.93 Gly; 0:96 Val; 1.02 Leu; 0.58 Thr: 1.50 Nva; 0.99 Ile; 1.14 Arg; 1.08 Pro.

### Example 16

### N-Ac-Sar-Gly-Val-Ile-Thr-Nva-Ile-Arg-ProNHCH₂CH₃

The procedure described in Example 1 was used but substituting Fmoc-Ile for Fmoc-D-Ile. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-Ile-Thr-Nva-Ile-Arg-ProNHCH₂CH₃ as the trifluoroacetate salt: Rₜ = 3.28 min (gradient of 10% to 30% acetonitrile in water containing 0.01 % TFA over 30 min period); MS (ESI) m/e 994 (M+H)⁺; Amino Acid Anal.: 0.95 Sar; 0.94 Gly; 0.89 Val; 1.70 Ile; 0.52 Thr; 1.67 Nva; 0.99 Ile; 1.27 Arg; 1.06 Pro.

### Example 17

### N-Ac-Sar-Gly-Val-Gly-Thr-Nva-Ile-Arg-ProNHCH₂CH₃

The procedure described in Example 1 was used but substituting Fmoc-Gly for Fmoc-D-Ile. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-Gly-Thr-Nva-Ile-Arg-ProNHCH₂CH₃ as the trifluoroacetate salt: Rₜ = 2.47 min (gradient of 10% to 30% acetonitrile in water containing 0.01% TFA over 30 min period); MS (ESI) m/e 938 (M+H)⁺; Amino Acid Anal.: 1.10 Sar; 1.94 Gly; 1.03 Val; 0.98 Ile; 0.54 Thr; 1.61 Nva; 1.28 Arg; 1.05 Pro.

### Example 18

### N-Ac-Sar-Gly-Val-D-V al-Thr-Nva-Ile-Arg-ProNHCH₂CH₃

The procedure described in Example I was used but substituting Fmoc-D-Val for Fmoc-D-Ile. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01 % TFA. The pure fractions were lyophilized to yield N-Ac-Sar-Gly-Val-D-Val-Thr-Nva-Ile-Arg-ProNHCH₂CH₃ as the trifluoroacetate salt: Rₜ = 3.13 min (gradient of 10% to 30% acetonitrile in water containing 0.01 % TFA over 30 min period); MS (ESI) m/e 980 (M+H)⁻: Amino Acid Anal.: 1.07 Sar; 1.0 Gly; 2.01 Val; 0.99 Ile; 0.62 Thr; 1.54Nva; 1.49 Arg: 1.11 Pro.

### Example 19

### N-Ac-Sar-Gly-Val-alloIle-Thr-Nva-Ile-Mg-PrONHCH₂CH₃

The procedure described in Example 1 was used but substituting Fmoc-allolle for Fmoc-D-Ile. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01 % TFA. The pure fractions were lyophilized to yield N-Ac-Sar-Gly-Val-alloIle-Thr-Nva-Ile-Arg-ProNHCH₂CH₃ as the trifluoroacetate salt: Rₜ = 4.174 min (gradient of 10% to 30% acetonitrile in water containing 0.0 1 % TFA over 30 min period); MS (ESI) m/e 994 (M+H)⁻: Amino Acid Anal.: 1.02 Sar; 0.99 Gly; 0.95 Val; 1.29 Ile; 0.45 Thr; 1.52 Nva; 1.54 Arg; 1.07 Pro.

### Example 20

### N-Ac-Sar-Gly-Val-D-Ala-Thr-Nva-Ile-Arg-ProNHCH₂CH₃

The procedure described in Example 1 was used but substituting Fmoc-D-Ala for Fmoc-D-Ile. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01 % TFA. The pure fractions were lyophilized to yield N-Ac-Sar-Gly-Val-D-Ala-Thr-Nva-Ile0-Arg-ProNHCH₂CH₃ as the trifluoroacetate salt: Rₜ = 3.826 min (gradient of 10% to 30% acetonitrile in water containing 0.01% TFA over 30 min period); MS (APCI) m/e 952 (M)⁺ and 908 (M-44)⁺.

### Example 21

### N-Ac-Sar-Gly-Val-D-Lys-Thr-Nva-Ile-Arg-ProNHCH₂CH₃

The procedure described in Example 1 was used but substituting Fmoc-Lys(Boc) for Fmoc-D-Ile. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01 % TFA. The pure fractions were lyophilized to yield N-Ac-Sar-Gly-Val-D-Lys-Thr-Nva-Ile-Arg-ProNHCH₂CH₃ as the trifluoroacetate salt: Rₜ = 3.544 min (gradient of 10% to 30% acetonitrile in water containing 0.01 % TFA over 30 min period); MS (APCI) m/e 1009 (M)⁺ and 965 (M-44)⁺.

### Example 22

### N-Ac-Sar-Gly-Val-D-Met-Thr-Nva-Ile-Arg-ProNHCH₂CH₃

The procedure described in Example 1 was used but substituting Fmoc-D-Met for Fmoc-D-Ile. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01 % TFA. The pure fractions were lyophilized to yield N-Ac-Sar-Gly-Val-D-Met-Thr-Nva-Ile-Arg-ProNHCH₂CH₃ as the trifluoroacetate salt: Rₜ = 4.141 min (gradient of 10% to 30% acetonitrile in water containing 0.01% TFA over 30 min period); MS (APCI) m/e 1012 (M)⁺ .

### Example 23

### N-Ac-Sar-Gly-Val-D-Nle-Thr-Nva-Ile-Arg-ProNHCH₂CH₃

The procedure described in Example 1 was used but substituting Fmoc-D-Nle for Fmoc-D-Ile. After cleavage of the peptide from the resin and removal of the protecting groups using (9: 1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-Nle-Thr-Nva-Ile-Arg-ProNHCH₂CH₃ as the trifluoroacetate salt: Rₜ = 4.383 min (gradient of 10% to 30% acetonitrile in water containing 0.0 1 % TFA over 30 min period); MS (APCI) m/e 994 (M)⁺.

### Example 24

### N-Ac-Sar-Gly-Val-D-Phe-Thr-Nva-Ile-Arg-ProNHCH₂CH₃

The procedure described in Example 1 was used but substituting Fmoc-D-Phe for Fmoc-D-Ile. After cleavage of the peptide from the resin and removal of the protecting groups using (9: 1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-VaI-D-Phe-Thr-Nva-Ile-Arg-ProNHCH₂CH₃ as the trifluoroacetate salt: Rₜ = 4.476 min (gradient of 10% to 30% acetonitrile in water containing 0.01 % TFA over 30 min period); MS (APCI) m/e 1028 (M)⁺.

### Example 25

### N-Ac-Sar-Gly-Val-D-Trp-Thr-Nva-Ile-Arg-ProNHCH₂CH₃

The procedure described in Example 1 was used but substituting Fmoc=D-Trp(Boc) for Fmoc-D-Ile. After cleavage of the peptide from the resin and removal of the protecting groups using (9: 1) TFA/anisole (3 mL) the crude product was purified by C-18 column chromatography using a solvent mixture varying in a gradient of 10% to 50% acetonitrile-water containing 0.01 % TFA. The pure fractions were lyophilized to yield N-Ac-Sar-Gly-Val-D-Trp-Thr-Nva-Ile-Arg-ProNHCH₂CH₃ as the trifluoroacetate salt: Rₜ = 4.430 min (gradient of 10% to 30% acetonitrile in water containing 0.01 % TFA over 30 min period); MS (APCI) m/e 1024 (M)⁺.

### Example 26

### N-Ac-Sar-Gly-Val-D-Tyr-Thr-Nva-Ile-Mg-PrONHCH₂CH₃

The procedure described in Example 1 was used but substituting Fmoc-D-Tyr(2-ClTrt) for Fmoc-D-Ile. After cleavage of the peptide from the resin and removal of the protecting groups using (9:1) TFAlanisole (3 mL) the crude product was purified by C-18 column chromatography using a solvent mixture varying in a gradient of 10% to 50% acetonitrile-water containing 0.01 % TFA. The pure fractions were lyophilized to yield N-Ac-Sar-Gly-Val-D-Tyr-Thr-Nva-Ile-Arg-ProNHCH₂CH₃ as the trifluoroacetate salt: Rₜ= 3.964 min (gradient of 10% to 30% acetonitrile in water containing 0.0 1 % TFA over 30 min period); MS (APCI) m/e 1045 (M)⁺.

### Example 27

### N-Ac-Sar-Gly-Val-D-4,4 -Biphenylala-Thr-Nva-Ile-Arg-ProNHCH₂CH₃

The procedure described in Example 1 was used but substituting Fmoc-D-4,4-Biphenylala for Fmoc-D-Ile. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-4,4-Biphenylala-Thr-Nva-Ile-Arg-ProNHCH₂CH₃ as the trifluoroacetate salt: Rₜ = 5.005 min (gradient of 10% to 30% acetonitrile in water containing 0.0 1 % TFA over 30 min period); MS (APCI) m/e 1104 (M)⁺ .

### Example 28

### N-Ac-Sar-Gly-Val-D-Cha-Thr-Nva-Ile-Arg-ProNHCH₂CH₃

The procedure described in Example 1 was used but substituting Fmoc-D-Cha for Fmoc-D-Ile. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-Cha-Thr-Nva-Ile-Arg-ProNHCH₂CH₃ as the trifluoroacetate salt: Rₜ = 5.005 min (gradient of 10% to 30% acetonitrile in water containing 0.01% TFA over 30 min period); MS (APCI) m/e 1034 (M)⁻.

### Example 29

### N-Ac-Sar-Gly-Val-D-Chg-Thr-Nva-Ile-Arg-ProNHCH₂CH₃

The procedure described in Example 1 was used but substituting Fmoc-D-Chg for Fmoc-D-Ile. After cleavage of the peptide from the resin and removal of the protecting groups using (9: 1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-Chg-Thr-Nva-Ile-Arg-ProNHCH₂CH₃ as the trifluoroacetate salt: Rₜ = 4.377 min (gradient of 10% to 30% acetonitrile in water containing 0.01 % TFA over 30 min period); MS (APCI) m/e 977 (M)⁺.

### Example 30

### N-Ac-Sar-Gly-Val-D-4-ClPhe-Thr-Nva-Ile-Arg-ProNHCH₂CH₃

The procedure described in Example 1 was used but substituting Fmoc-D-4-ClPhe for Fmoc-D-Ile. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-4-ClPhe-Thr-Nva-Ile-Arg-ProNHCH₂CH₃ as the trifluoroacetate salt: Rₜ = 4.674 min (gradient of 10% to 30% acetonitrile in water containing 0.01% TFA over 30 min period); MS (APCI) m/e 1018 (M)⁺.

### Example 31

### N-Ac-Sar-Gly-Val-D-Hphe-Thr-Nva-Ile-Arg-ProNHCH₂(CH₃)₂

The procedure described in Example 1 was used but substituting Fmoc-D-Hphe for Fmoc-D-Ile. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-Hphe-Thr-Nva-Ile-Arg-ProNHCH₂CH₃ as the trifluoroacetate salt: Rₜ = 4.597 min (gradient of 10% to 30% acetonitrile in water containing 0.01 % TFA over 30 min period); MS (APCI) m/e 1042 (M)⁺ and 998 (M-44)⁺.

### Example 32

### N-Ac-Sar-Gly-Val-Dehydroleu-Thr-Nva-Ile-Arg-ProNHCH₂CH₃

The procedure described in Example 1 was used but substituting Fmoc-Dehydroleu for Fmoc-D-Ile. After cleavage of the peptide from the resin and removal of the protecting groups using (9: 1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-Dehydroleu-Thr-Nva-Ile-Arg-ProNHCH₂CH₃ as the trifluoroacetate salt: Rₜ = 4.1707 min (gradient of 10% to 30% acetonitrile in water containing 0.01% TFA over 30 min period); MS (APCI) m/e 992 (M)⁺ and 949 (M-4.4)⁺.

### Example 33

### N-Ac-Sar-GIy-Val-D-3-CF₃Phe-Thr-Nva-Ile-Arg-ProNHCH₂CH₃

The procedure described in Example 1 was used but substituting Fmoc-D-3-CF₃Phe for Fmoc-D-Ile. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-3-CF₃Phe-Thr-Nva-Ile-Arg-ProNHCH₂CH₃ as the trifluoroacetate salt: Rₜ = 4.825 min (gradient of 10% to 30% acetonitrile in water containing 0.01% TFA over 30 min period); MS (APCI) m/e 1097 (M)⁺ and 1053 (M-44)⁺.

### Example 34

### N-Ac-Sar-Gly-Val-D-pentaFPhe-Thr-Nva-Ile-Arg-ProNHCH₂CH₃

The procedure described in Example 1 was used but substituting Fmoc-D-pentaFPhe for Fmoc-D-Ile. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-pentaFPhe-Thr-Nva-Ile-Arg-ProNHCH₂CH₃ as the trifluoroacetate salt: Rₜ = 4.810 min (gradient of 10% to 30% acetonitrile in water containing 0.01 % TFA over 30 min period); MS (APCI) m/e 1118 (M)⁺ and 1075 (M-44)⁺.

### Example 35

### N-Ac-Sar-Gly-Val-D-3,4-diClPhe-Thr-Nva-Ile-Arg-ProNHCH₂CH₃

The procedure described in Example 1 was used but substituting Fmoc-D-3,4-diClPhe for Fmoc-D-Ile. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-3,4-diClPhe-Thr-Nva-Ile-Arg-ProNHCH2CH3 as the trifluoroacetate salt: Rₜ = 4.911 min (gradient of 10% to 30% acetonitrile in water containing 0.0 1 % TFA over 30 min period); MS (APCI) m/e 1100 (M+3)⁺.

### Example 36

### N-Ac-Sar-Gly-Val-D-3-ClPhe-Thr-Nva-Ile-Arg-ProNHCH₂CH₃

The procedure described in Example 1 was used but substituting Fmoc-D-3-ClPhe for Fmoc-D-Ile. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-3-ClPhe-Thr-Nva-Ile-Arg-ProNHCH₂CH₃ as the trifluoroacetate salt: Rₜ = 4.689 min (gradient of 10% to 30% acetonitrile in water containing 0.01% TFA over 30 min period); MS (APCI) m/e 1062 (M)⁺.

### Example 37

### N-Ac-Sar-Gly-Val-D-2-Thienylala-Thr-Nva-Ile-Arg-ProNHCH₂CH₃

The procedure described in Example 1 was used but substituting Fmoc-D-2-Thienylala for Fmoc-D-Ile. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Glv-Val-D-2-Thienylala-Thr-Nva-Ile-Arg-ProNHCH₂)CH₃ as the trifluoroacetate salt: Rₜ = 4.388 min (gradient of 10% to 30% acetonitrile in water containing 0.01 % TFA over 30 min period); MS (APCI) m/e 1034 (M)⁺.

### Example 38

### N-Ac-Sar-Gly-Val-D-3-CNPhe-Thr-Nva-Ile-Arg-ProNHCH₂CH₃

The procedure described in Example 1 was used but substituting Fmoc-D-3-CN-Phe for Fmoc-D-Ile. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.0 1 % TFA. The pure fractions were lyophilized to yield N-Ac-Sar-Gly-Val-D-3-CNPhe-Thr-Nva-Ile-Arg-ProNHCH₂CH₃ as the trifluoroacetate salt: Rₜ = 4.361 min (gradient of 10% to 30% acetonitrile in water containing 0.01% TFA over 30 min period); MS (APCI) m/e 1009 (M)⁺.

### Example 40

### N-Ac-Sar-Gly-Val-D-3-Benzothienylala-Thr-Nva-Ile-Arg-ProNHCH₂CH₃

The procedure described in Example 1 was used but substituting Fmoc-D-3-Benzothienylala for Fmoc-D-Ile. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-3-Benzothienylala-Thr-Nva-Ile-Arg-ProNHCH₂CH₃ as the trifluoroacetate salt: Rₜ = 4.797 min (gradient of 10% to 30% acetonitrile in water containing 0.0 1 % TFA over 30 min period); MS (APCI) m/e 1084 (M)⁺.

### Example 41

### N-Ac-Sar-Gly-Val-D-3,4-diF-Phe-Thr-Nva-Ile-Arg-ProNHCH₂CH₃

The procedure described in Example 1 was used but substituting Fmoc-D-3,4-diF-Phe for Fmoc-D-Ile. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-3,4-diF-Phe-Thr-Nva-Ile-Arg-ProNHCH₂CH₃ as the trifluoroacetate salt: Rₜ = 4.608 min (gradient of 10% to 30% acetonitrile in water containing 0.01 % TFA over 30 min period); MS (APCI) m/e 1064 (M)⁺.

### Example 42

### N-Ac-Sar-Gly-Val-D-Ile-Thr-DNva-Ile-Arg-PrONHCH₂CH₃

The procedure described in Example 1 was used but substituting Fmoc-DNva for Fmoc-Nva. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01 % TFA. The pure fractions were lyophilized to yield N-Ac-Sar-Gly-Val-D-Ile-Thr-DNva-Ile-Arg-ProNHCH₂CH₃ as the trifluoroacetate salt: Rₜ = 3.75 min (gradient of 10% to 30% acetonitrile in water containing 0.01 % TFA over 30 min period); MS (ESI) m/e 994 (M+H)⁺; Amino Acid Anal.: 1.08 Sar; 0.96 Gly; 0.95 Val; 1.74 Ile; 0.50 Thr; 1.69 Nva; 1.26 Arg; 1.09 Pro.

### Example 43

### N-Ac-Sar-Gly-Val-D-Ile-Thr-Gln-Ile-Arg-ProNHCH₂CH₃

The procedure described in Example 1 was used but substituting Fmoc-Gln(Trt) for Fmoc-Nva. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-Ile-Thr-Gln-Ile-Arg-ProNHCH₂CH₃ as the trifluoroacetate salt: Rₜ = 3.047 min (gradient of 10% to 30% acetonitrile in water containing 0.01 % TFA over 30 min period); MS (ESI) m/e 1023 (M+H)⁺; Amino Acid Anal.: 1.15 Sar; 0.96 Gly; 0.63 Val; 1.7 Ile; 0.46 Thr; 0.65 Glu; 1.45 Arg; 1.04 Pro.

### Example 44

### N-Ac-Sar-Gly-Val-D-Ile-Thr-Cha-Ile-Arg-ProNHCH₂CH₃

The procedure described in Example 1 was used but substituting Fmoc-Cha for Fmoc-Nva. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-Ile-Thr-Cha-Ile-Arg-ProNHCH₂CH₃ as the trifluoroacetate salt: Rₜ = 4.503 min (gradient of 10% to 30% acetonitrile in water containing 0.01% TFA over 30 min period); MS (ESI) m/e 1048 (M+H)⁺; Amino Acid Anal.: 1.18 Sar; 0.94 Gly; 0.59 Val; 1.65 Ile; 0.45 Thr; 0.37 Cha; 1.45 Arg; 1.06 Pro.

### Example 45

### N-Ac-Sar-Gly-Val-D-Ile-Thr-Gly-Ile-Arg-ProNHCH₂CH₃

The procedure described in Example 1 was used but substituting Fmoc-Gly for Fmoc-Nva. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-Ile-Thr-Gly-Ile-Arg-ProNHCH₂CH₃ as the trifluoroacetate salt: Rₜ = 3.11 min (gradient of 10% to 30% acetonitrile in water containing 0.01% TFA over 30 min period); MS (ESI) m/e 952 (M+H)⁻.

### Example 46

### N-Ac-Sar-Gly-Val-D-Ile-Thr-Ala-Ile-Arg-ProNHCH₂CH₃

The procedure described in Example 1 was used but substituting Fmoc-Ala for Fmoc-Nva. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01 % TFA. The pure fractions were lyophilized to yield N-Ac-Sar-Gly-Val-D-Ile-Thr-Ala-Ile-Mg-PrONHCH₂CH₃ as the trifluoroacetate salt: Rₜ = 3.16 min (gradient of 10% to 30% acetonitrile in water containing 0.01 % TFA over 30 min period); MS (ESI) m/e 966 (M+H)⁻.

### Example 47

### N-Ac-Sar-Gly=Val-D-Ile-Thr-Val-Ile-Arg-ProNHCH₂CH₃

The procedure described in Example 1 was used but substituting Fmoc-Val for Fmoc-Nva. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-Ile-Thr-Val-Ile-Arg-ProNHCH₂CH₃ as the trifluoroacetate salt: Rₜ = 3.36 min (gradient of 10% to 30% acetonitrile in water containing 0.01 % TFA over 30 min period); MS (ESI) m/e 994 (M+H)⁻.

### Example 48

### N-Ac-Sar-Gly-Val-D-Ile-Thr-Abu-Ile-Arg-ProNHCH₂CH₃

The procedure described in Example 1 was used but substituting Fmoc-Abu for Fmoc-Nva. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01 % TFA. The pure fractions were lyophilized to yield N-Ac-Sar-Gly-Val-D-Ile-Thr-Abu-Ile-Arg-ProNHCH₂CH₃ as the trifluoroacetate salt: Rₜ = 3.23 min (gradient of 10% to 30% acetonitrile in water containing 0.01% TFA over 30 min period); MS (ESI) m/e 980 (M+H)⁺.

### Example 49

### N-Ac-Sar-Gly-Val-D-Ile-Thr-Allylgly-Ile-Arg-ProNHCH₂CH₃

The procedure described in Example 1 was used but substituting Fmoc-Allylgly for Fmoc-Nva. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01 % TFA. The pure fractions were lyophilized to yield N-Ac-Sar-Gly-Val-D-Ile-Thr-Allylgly-Ile-Arg-ProNHCH₂CH₃ as the trifluoroacetate salt: Rₜ = 3.40 min (gradient of 10% to 30% acetonitrile in water containing 0.01% TFA over 30 min period); MS (ESI) m/e 992 (M+H)⁺.

### Example 50

### N-Ac-Sar-Gly-Val-D-Ile-Thr-Octylgly-Ile-Arg-ProNHCH₂CH₃

The procedure described in Example 1 was used but substituting Fmoc-Octylgly for Fmoc-Nva. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-Ile-Thr-Octylgly-Ile-Arg-ProNHCH₂CH₃ as the trifluoroacetate salt: Rₜ = 5.30 min (gradient of 10% to 30% acetonitrile in water containing 0.01% TFA over 30 min period); MS (ESI) m/e 1064 (M+H)⁺.

### Example 51

### N-Ac-Sar-Gly-Val-D-Ile-Thr-Met-Ile-Arg-ProNHCH₂CH₃

The procedure described in Example 1 was used but substituting Fmoc-Met for Fmoc-Nva. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01 % TFA. The pure fractions were lyophilized to yield N-Ac-Sar-Gly-Val-D-Ile-Thr-Met-Ile-Arg-ProNHCH₂CH₃ as the trifluoroacetate salt: Rₜ = 3.48 min (gradient of 10% to 30% acetonitrile in water containing 0.01% TFA over 30 min period); MS (ESI) m/e 1027 (M+H)⁺)⁺.

### Example 52

### N-Cyclohexylacetyl-Sar-Gly-Val-D-Ile-Tlu-Nva-Ile-Arg-ProNHCH₂CH₃

The procedure described in Example I was used but substituting cyclohexylacetic acid for acetic acid. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Cyclohexylacetyl-Sar-Gly-Val-D-Ile-Thr-Nva-Ile-Ar-ProNHCH₂CH₃ as the trifluoroacetate salt: Rₜ = 5. 11 min (gradient of 10% to 30% acetonitrile in water containing 0.01% TFA over 30 min period); MS (ESI) m/e 1076 (M+H⁺)⁺; Amino Acid Anal.: 1.15 Sar; 0.97 Gly; 0.95 Val; 1.79 Ile; 0.54 Thr; 1.66 Nva; 1.28 Arg; 1.08 Pro.

### Example 53

### N-(2-Me-Nicotinyl)-Sar-Gly-Val-D-Ile-Thr-Nva-Ile-Arg-ProNHCH₂CH₃

The procedure described in Example 1 was used but substituting 2-Me-nicotinic acid for acetic acid. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-(2-Me-Nicotinyl)-Sar-Gly-Val-D-Ile-Thr-Nva-Ile-Arg-ProNHCH₂CH₃ as the trifluoroacetate salt: Rₜ = 5.11 min (gradient of 10% to 30% acetonitrile in water containing 0.0 1 % TFA over 30 min period); MS (ESI) m/e 1071 (M+H)⁺; Amino Acid Anal.: 1.19 Sar; 1.01 Gly; 0.99 Val; 1.79 Ile; 0.57 Thr; 1.70 Nva; 1.59 Arg; 1.17 Pro.

### Example 54

### N-Succinyl-Sar-Gly-Val-D-Ile-Thr-Nva-Ile-Arg-ProNHCH₂CH₃

The procedure described in Example 1 was used but acylating the peptide resin (after the Fmoc-Sar coupling and deprotection) with a (1:1) succinic anhydride/pyridine mixture (2 mL) overnight. After washing the resin and cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Succinyl-Sar-Gly-Val-D-Ile-Thr-Nva-Ile-Arg-ProNHCH₂CH₃ as the trifluoroacetate salt: Rₜ = 2.72 min (gradient of 10% to 30% acetonitrile in water containing 0.01% TFA over 30 min period); MS (ESI) m/e 1052 (M+H)⁺; Amino Acid Anal.: 1.16 Sar; 1.05 Gly; 0.95 Val; 1.85 Ile; 0.57 Thr; 1.70 Nva; 1.59 Arg; 1.17 Pro.

### Example 55

### N-Nicotinyl-Sar-Gly-Val-D-Ile-Thr-Nva-Ile-Arg-ProNHCH₂CH₃

The procedure described in Example 1 was used but substituting nicotinic acid for acetic acid at the last coupling. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Nicotinyl-Sar-Gly-Val-D-Ile-Thr-Nva-He-Arg-ProNHCH₂CH₃ as the trifluoroacetate salt: Rₜ = 3.6 min (gradient of 10% to 30% acetonitrile in water containing 0.01% TFA over 30 min period); MS (ESI) m/e 1057 (M+H)⁺; Amino Acid Anal.: 1.03 Sar; 0.89 Gly; 0.81 Val; 1.48 Ile; 0.40 Thr; 1.46 Nva; 1.07 Arg; 1.04 Pro.

### Example 56

### N-Propionyl-Sar-Gly-Val-D-Ile-Thr-Nva-Ile-Arg-ProNHCH₂CH₃

The procedure described in Example 1 was used but substituting propionic acid for acetic acid at the last coupling. After cleavage of the peptide from the resin and removal of the protecting groups using (9: 1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Propionyl-Sar-Gly-Val-D-Ile-Thr-Nva-Ile-Arg-ProNHCH₂CH₃ as the trifluoroacetate salt: Rₜ = 3.7 min (gradient of 10% to 30% acetonitrile in water containing 0.01 % TFA over 30 min period); MS (ESI) m/e 1008 (M+H)⁺; Amino Acid Anal.: 0.93 Sar; 0.97 Gly; 0.88 Val; 1.60 Ile; 0.44 Thr; 1.58 Nva; 1.17 Arg; 1.10 Pro.

### Example 57

### N-MeOacetyl-Sar-Gly-Val-D-Ile-Thr-Nva-Ile-Arg-ProNHCH₂CH₃

The procedure described in Example 1 was used but substituting methoxyacetic acid for acetic acid at the last coupling. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-MeOacetyl-Sar-Gly-Val-D-Ile-Thr-Nva-Ile-Arg-ProNHCH₂CH₃ as the trifluoroacetate salt: Rₜ = 3.45 min (gradient of 10% to 30% acetonitrile in water containing 0.01% TFA over 30 min period); MS (ESI) m/e 1024 (M+H)⁺; Amino Acid Anal.: 1.12 Sar; 1.06 Gly; 0.94 Val; 1.62 Ile; 0.48 Thr; 1.91 Nva; 1.40 Arg; 1.27 Pro.

### Example 58

### N-Shikimyl-Sar-Gly-Val-D-Ile-Thr-Nva-Ile-Arg-ProNHCH₂CH₃

The procedure described in Example 1 was used but substituting shikimic acid for acetic acid at the last coupling. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Shikimyl-Sar-Gly-Val-D-Ile-Thr-Nva-Ile-Arg-ProNHCH₂CH₃ as the trifluoroacetate salt: Rₜ = 3.0 min (gradient of 10% to 30% acetonitrile in water containing 0.01% TFA over 30 min period); MS (ESI) m/e 1108 (M+H)⁺; Amino Acid Anal.: 1.22 Sar; 1.06 Gly; 0.94 Val; 1.80 Ile; 0.55 Thr; 1.70 Nva; 1.28 Arg; 1.26 Pro.

### Example 59

### N-(2-Furoyl)-Sar-Gly-Val-D-Ile-Thr-Nva-Ile-Arg-ProNHCH₂CH₃

The procedure described in Example 1 was used but substituting 2-furoic acid for acetic acid at the last coupling. After cleavage of the peptide from the resin and removal of the protecting groups using (9: 1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-(2-Furoyl)-Sar-Gly-Val-D-Ile-Thr-Nva-Ile-Arg-ProNHCH₂CH₃ as the trifluoroacetate salt: Rₜ = 4.0 min (gradient of 10% to 30% acetonitrile in water containing 0.01 % TFA over 30 min period); MS (ESI) m/e 1046 (M+H)⁺; Amino Acid Anal.: 1.02 Sar; 1.00 Gly; 0.99 Val; 1.66 Ile; 0.45 Thr; 1.75 Nva; 1.45 Arg; 1.21 Pro.

### Example 60

### N-Butyryl-Sar-Gly-V al-D-I le-Thr-Nva-Ile-Arg-ProNHCH₂CH₃

The procedure described in Example 1 was used but substituting butyric acid for acetic acid at the last coupling. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.0 1 % TFA. The pure fractions were lyophilized to yield N-Butyryl-Sar-Gly-Val-D-Ile-Thr-Nva-Ile-Arg-ProNHCH₂CH₃ as the trifluoroacetate salt: Rₜ = 4.03 min (gradient of 10% to 30% acetonitrile in water containing 0.01% TFA over 30 min period); MS (ESI) m/e 1022 (M+H)⁺; Amino Acid Anal.: 1.13 Sar; 0.99 Gly; 1.01 Val; 1.93 Ile; 0.67 Thr; 1.61 Nva; 1.45 Arg; 1.08 Pro.

### Example 61

### N-(Tetrahydro-2-furoyl)-Sar-Gly-Val-D-Ile-Thr-Nva-Ile-Arg-ProNHCH₂CH₃

The procedure described in Example 1 was used but substituting tetrahydro-2-furoic acid for acetic acid at the last coupling. After cleavage of the peptide from the resin and removal of the protecting groups using (9:1) TFA/anisole (3 mL) the crude product was purified by C-18 column chromatography a solvent mixture varying in a gradient of 10% to 50% acetonitrile-water containing 0.0 1 % TFA. The pure fractions were lyophilized to yield N-(tetrahydro-2furoyl)-Sar-Gly-Val-D-Ile-Thr-Nva-Ile-Arg-ProNHCH₂CH₃ as the trifluoroacetate salt: Rₜ = 3.91 min (gradient of 10% to 30% acetonitrile in water containing 0.01% TFA over 30 min period); MS (ESI) m/e 1050 (M+H)⁺; Amino Acid Anal.: 1.12 Sar; 0.97 Gly; 0.88 Val; 1.41 Ile; 0.42 Thr; 1.60 Nva; 1.43 Arg; 1.03 Pro.

### Example 62

### N-[CH₃C(O)NH-(CH₂)₂-O(CH₂)₂-O-CH₂-C(O)]-Sar-Gly-Val-D-Ile-Thr-Nva-Ile-Arg-ProNHCH₂CH₃

The procedure described in Example 1 was used but coupling with Fmoc-8-amino-3,6-dioxo-octanoic acid after the Fmoc-Sar coupling, after removal of the terminal Fmoc the peptide resin was coupled with acetic acid as described above. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-[CH₃C(O)NH-(CH₂)₂-O-(CH₂)₂-O-CH₂-C(O)]-Sar-Gly-Val-D-Ile-Thr-Nva-Ile-Arg-ProNHCH₂CH₃ as the trifluoroacetate salt: Rₜ = 3.32 min (gradient of 10% to 30% acetonitrile in water containing 0.01 % TFA over 30 min period); MS (ESI) m/e 1139 (M+H)⁺; Amino Acid Anal.: 1.04 Sar; 1.01 Gly; 0.91 Val; 1.67 Ile; 0.53 Thr; 1.77 Nva; 1.39 Arg; 1.02 Pro.

### Example 63

### N-[6-N'-Acetyl-(CH₂)₅C(O)]-Sar-Gly-Val-D-Ile-Thr-Nva-Ile-Arg-ProNHCH₂CH₃

The procedure described in Example 1 was used but coupling with Fmoc-6-aminohexanoic acid after the Fmoc-Sar coupling, after removal of the terminal Fmoc the peptide resin was coupled with acetic acid as described above. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-[6-N'-Acetyl-(CH₂)₅C(O)]-Sar-Gly-Val-D-Ile-Thr-Nva-Ile-Arg-ProNHCH₂CH₃ as the trifluoroacetate salt: Rₜ = 3.60 min (gradient of 10% to 30% acetonitrile in water containing 0.01% TFA over 30 min period); MS (ESI) m/e 1107 (M+H)⁺; Amino Acid Anal.: 1.13 Sar; 0.96 Gly; 0.89 Val; 1.42 Ile; 0.43 Thr; 1.68 Nva; 1.44 Arg; 1.04 Pro.

### Example 65

### N-[4-N'-Acetyl-butyryl]-Sar-Gly-Val-D-Ile-Thr-Nva-Ile-Arg-ProNHCH₂CH₃

The procedure described in Example 1 was used but coupling with Fmoc-4-aminobutyric acid after the Fmoc-Sar coupling, after removal of the terminal Fmoc the peptide resin was coupled with acetic acid as described above. After cleavage of the peptide from the resin and removal of the protecting groups using (9:1) TFA/anisole (3 mL) the crude product was purified by C-18 column chromatography a solvent mixture varying in a gradient of 10% to 50% acetonitrile-water containing 0.01% TFA. The pure fractions were lyophilized to yield N-[4-N-Acetyl-butyryl]-Sar-Gly-Val-D-Ile-Thr-Nva-Ile-Arg-ProNHCH₂CH₃ as the trifluoroacetate salt: Rₜ = 4.09 min (gradient of 10% to 30% acetonitrile in water containing 0.01% TFA over 30 min period); MS (ESI) m/e 1079 (M+H)⁺; Amino Acid Anal.: 1.03 Gaba; 1.07 Sar; 0.93 Gly; 1.00 Val; 1.90 Ile; 0.54 Thr; 1.30 Nva; 1.54 Arg; 1.06 Pro.

### Example 66

### H-Sar-GIy-Val-D-Ile-Thr-Nva-Ile-Arg-ProNHCH₂CH₃

The procedure described in Example 1 was used but omitting' the acetic acid coupling at the end. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield H-Sar-Gly-Val-D-Ile-Thr-Nva-Ile-Arg-ProNHCH₂CH₃ as the bistrifluoroacetate salt: Rₜ = 3.65 min (gradient of 10% to 30% acetonitrile in water containing 0.01 % TFA over 30 min period); MS (ESI) m/e 952 (M+H)⁺; Amino Acid Anal.: 1.00 Sar; 1.00 Gly; 0.99 Val; 1.67 Ile; 0.50 Thr; 1.76 Nva; 1.47 Arg; 1.22 Pro.

### Example 73

### N-Ac-Sar-Gly-Val-D-Ile-Thr-Leu-Ile-Arg-ProNHCH₂CH₃

The procedure described in Example 1 was used but substituting Fmoc-Leu for Fmoc-Nva. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01 % TFA. The pure fractions were lyophilized to yield N-Ac-Sar-Gly-Val-D-Ile-Thr-Leu-Ile-Arg-ProNHCH₂CH₃ as the trifluoroacetate salt: Rₜ = 4.348 min (gradient of 10% to 30% acetonitrile in water containing 0.01% TFA over 30 min period); MS (ESI) m/c 1008 (M+H)⁻; Amino Acid Anal.: 0.88 Sar; 0.99 Gly; 0.95 Val; 1.03 Ile; 0.55 Thr; 1.12 Leu: 1.53 Arg; 1.07 Pro.

### Example 74

### N-Ac-Sar-Gly-Val-D-Ile-Thr-Ser-Ile-Arg-ProNHCH₂CH₃

The procedure described in Example 1 was used but substituting Fmoc-Ser(tBu) for Fmoc-Nva. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.0 1 % TFA. The pure fractions were lyophilized to yield N-Ac-Sar-Gly-Val-D-Ile-Thr-Ser-Ile-Arg-ProNHCH₂CH₃ as the trifluoroacetate salt: Rₜ = 3.963 min (gradient of 10% to 30% acetonitrile in water containing 0.01 % TFA over 30 min period); MS (ESI) m/e 982 (M+H)⁺; Amino Acid Anal.: 0.91 Sar; 0.97 Gly; 1.00 Val; 1.03 Ile; 0.56 Thr; 0.23 Ser; 1.52 Arg; 1.08 Pro.

### Example 75

### N-Ac-Sar-Gly-Val-D-Ile-Thr-Nva-Ile-Arg-Pro-D-AlaNH₂

The procedure described in Example 10 was used but substituting Fmoc-D-Ala-Sieber amide resin for Fmoc-Pro-Sieber amide resin. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-VaI-D-IIe-Thr-Nva-Ile-Arg-Pro-D-AlaNH₂ as the trifluoroacetate salt: Rₜ = 4.117 min (gradient of 10% to 30% acetonitrile in water containing 0.01% TFA over 30 min period); MS (ESI) m/e 1037 (M+H)⁺; Amino Acid Anal.: 0.85 Sar; 0.94 Gly; 0.92 Val; 1.83 Ile; 0.54 Thr; 1.18 Nva; 1.01 Arg; 1.04 Pro; 1.01 Ala.

### Example 84

### N-Ac-Sar-Gly-Val-D-Tyr(Et)-Thr-NvA-Ile-Arg-ProNHCH₂CH₃

The procedure described in Example 1 was used but substituting Fmoc-D-Tyr(Et) for Fmoc-D-Ile. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-At-Sar-Gly-Val-D-Ty-r(Et)-Tbr-Nva-Ile-Arg-ProNHCH₂CH₃ as the trifluoroacetate salt: Rₜ = 6.01 min (gradient of 10% to 30% acetonitrile in water containing 0.01% TFA over 30 min period); MS (APCI) m/e 1072 (M)⁺.

### Example 85

### N-Ac-Sar-Gly-Val-D-Cys(tBu)-Thr-Nva-Ile-Arg-PrONHCH₂CH₃

The procedure described in Example 1 was used but substituting Fmoc-D-Cys(tBu) for Fmoc-D-Ile. After cleavage of the peptide from the resin and removal of the protecting groups using (9: 1) TFA/anisole- (3 mL) the crude product was purified by C-18-column chromatography using a solvent mixture varying in a gradient of 10% to 50% acetonitrile-water containing 0.01% TFA. The pure fractions were lyophilized to yield N-Ac-Sar-Gly-Val-D-Cys(tBu)-Thr-Nva-Ile-Arg-ProNHCH₂CH₃ as the trifluoroacetate salt: Rₜ =5.96 min (gradient of 10% to 30% acetonitrile in water containing 0.01% TFA over 30 min period); MS (APCI) m/e 1040 (M)⁺.

### Example 87

### Example 87

### N-Ac-Sar-Gly-Val-D-Tyr(Bzl)-Thr-Nva-Ile-Arg-ProNHCH₂CH₃

The procedure described in Example 1 was used but substituting Fmoc-D-Tyr(Bzl) for Fmoc-D-Ile. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01 % TFA. The pure fractions were lyophilized to yield N-Ac-Sar-Gly-Val-D-Tyr(Bzl)-Thr-Nva-Ile-Arg-ProNHCH₂CH₃ as the trifluoroacetate salt: Rₜ = 6.74 min (gradient of 10% to 30% acetonitrile in water containing 0.01 % TFA over 30 min period); MS (APCI) m/e 1135 (M+H)⁺.

### Example 88

### N-Ac-Sar-Gly-Val-D-Ser(Bzl)-Thr-Nva-Ile-Arg-ProNHCH₂CH₃

The procedure described in Example 1 was used but substituting Fmoc-D-Ser(Bzl) for Fmoc-D-Ile. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-Ser(Bzl)-Thr-Nva-Ile-Arg-ProNHCH₂CH₃ as the trifluoroacetate salt: Rₜ = 5.95 min (gradient of 10% to 30% acetonitrile in water containing 0.01% TFA over 30 min period); MS (APCI) m/e 1058 (M)⁺.

### Example 89

### N-Ac-Sar-Gly-Val-D-1Nal-Thr-Nva-Ile-Arg-ProNHCH₂CH₃

The procedure described in Example 1 was used but substituting Fmoc-D-1Nal for Fmoc-D-Ile. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-lNa1-Thr-Nva-Ile-Arg-ProNHCH₂CH3 as the trifluoroacetate salt: Rₜ = 6.30 min (gradient of 10% to 30% acetonitrile in water containing 0.01% TFA over 30 min period); MS (APCI) m/e 1081 (M+3)⁺.

### Example 90

### N-Ac-Sar-Gly-Val-D-tButylgly-Thr-Nva-Ile-Arg-ProNHCH₂CH₃

The procedure described in Example 1 was used but substituting Fmoc-D-tButylgly for Fmoc-D-Ile. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01 % TFA. The pure fractions were lyophilized to yield N-Ac-Sar-Gly-Val-D-tButylgly-Thr-Nva-Ile-Arg-ProNHCH₂CH₃ as the trifluoroacetate salt: Rₜ = 5.46 min (gradient of 10% to 30% acetonitrile in water containing 0.01 % TFA over 30 min period); MS (APCI) m/e 994 (M)⁺.

### Example 91

### N-Ac-Sar-Gly-Val-D-Orn-Thr-Nva-Ile-Arg-ProNHCH₂CH₃

The procedure described in Example 1 was used but substituting Fmoc-D-Orn(Boc) for Fmoc-D-Ile. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-Orn-Thr-Nva-Ile-Arg-ProNHCH₂CH₃ as the trifluoroacetate salt: Rₜ= 1.69 min (gradient of 10% to 30% acetonitrile in water containing 0.01% TFA over 30 min period); MS (APCI) m/e 995 (M)⁺.

### Example 92

### N-Ac-Sar-Gly-Val-D-Thr(Bzl)-Thr-Nva-Ile-Arg-ProNHCH₂CH₃

The procedure described in Example 1 was used but substituting Fmoc-D-Thr(Bzl) for Fmoc-D-Ile. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-Thr(Bzl)-Thr-Nva-Ile-Arg-ProNHCH₂)CH₃ as the trifluoroacetate salt: Rₜ = 6.10 min (gradient of 10% to 30% acetonitrile in water containing 0.0 1 % TFA over 30 min period); MS (APCI) m/e 1072 (M)⁺.

### Example 93

### N-Ac-Sar-Gly-V al-D-2Nal-Thr-Nva-Ile-Arg-ProNHCH₂CH₃

The procedure described in Example 1 was used but substituting Fmoc-D-2Nal for Fmoc-D-Ile. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-2Nal-Thr-Nva-Ile-Arg-ProNHCH₂CH₃ as the trifluoroacetate salt: Rₜ = 6.33 min (gradient of 10% to 30% acetonitrile in water containing 0.01% TFA over 30 min period); MS (APCI) m/e 1078 (M)⁻.

### Example 94

### N-Ac-Sar-Gly-V al-D-Phe(4-Me)-Thr-Nva-Ile-Arg-ProNHCH₂CH₃

The procedure described in Example 1 was used but substituting Fmoc-D-Phe(4-Me) for Fmoc-D-Ile. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01 % TFA. The pure fractions were lyophilized to yield N-Ac-Sar-Gly-Val-D-Phe(4-Me)-Thr-Nva-Ile-Arg-ProNHCH₂CH₃ as the trifluoroacetate salt: Rₜ = 3.654 min (gradient of 10% to 30% acetonitrile in water containing 0.01% TFA over 30 min period); MS (ESI) m/e 1042 (M)⁺.

### Example 95

### N-Ac-Sar-Gly-Val-D-Phe(3,4-diMeO)-Thr-Nva-Ile-Arg-ProNHCH₂CH₃

The procedure described in Example 1 was used but substituting Fmoc-D-Phe(3,4-diMeO) for Fmoc-D-Ile. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-Phe(3.4-diMeO)-Thr-Nva-Ile-Arg-ProNHCH₂CH₃ as the trifluoroacetate salt: Rₜ = 3.006 min (gradient of 10% to 30% acetonitrile in water containing 0.01% TFA over 30 min period); MS (ESI) m/e 1088 (M)⁺.

### Example 96

### N-Ac-Sar-Gly-Val-D-Phe(3,4,5-triF)-Thr-Nva-Ile-Arg-PrONHCH₂CH₃

The procedure described in Example 1 was used but substituting Fmoc-D-Phe(3,4,5-triF) for Fmoc-D-Ile. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-Phe(3,4,5-triF)-Thr-Nva-Ile-Arg-PrONHCH₂CH₃ as the trifluoroacetate salt: Rₜ = 3.848 min (gradient of 10% to 30% acetonitrile in water containing 0.01% TFA over 30 min period); MS (ESI) m/e 1082 (M)⁺.

### Example 97

### N-Ac-Sar-Gly-Val-D-Phe(4-NO₂)-Thr-Nva-Ile-Arg-ProNHCH₂CH₃

The procedure described in Example 1 was used but substituting Fmoc-D-Phe(4-NO₂) for Fmoc-D-Ile. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01 % TFA. The pure fractions were lyophilized to yield N-Ac-Sar-Gly-Val-D-Phe(4-NO₂)-Thr-Nva-Ile-Arg-ProNHCH₂CH₃ as the trifluoroacetate salt: Rₜ = 3.483 min (gradient of 10% to 30% acetonitrile in water containing 0.01 % TFA over 30 min period); MS (ESI) m/e 1073 (M)⁺.

### Example 98

### N-Ac-Sar-Gly-Val-D-Pen-Thr-Nva-Ile-Arg-ProNHCH₂CH₃

The procedure described in Example 1 was used but substituting Fmoc-D-Pen(Trt) for Fmoc-D-Ile. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-Pen-Thr-Nva-Ile-Arg-ProNHCH₂CH₃ as the trifluoroacetate salt: Rₜ = 2.928 min (gradient of 10% to 30% acetonitrile in water containing 0.01 % TFA over 30 min period); MS (ESI) m/e 1012 (M)⁺.

### Example 99

### N-Ac-Sar-Gly-Val-D-Pen(Acm)-Thr-Nva-Ile-Arg-ProNHCH₂CH₃

The procedure described in Example 1 was used but substituting Fmoc-D-Pen(Acm) for Fmoc-D-Ile. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-Pen(Acm)-Thr-Nva-Ile-Arg-ProNHCH₂CH₃ as the trifluoroacetate salt: Rₜ = 2.415 min (gradient of 10% to 30% acetonitrile in water containing 0.01% TFA over 30 min period); MS (ESI) m/e 1083 (M)⁺.

### Example 100

### N-Ac-Sar-Gly-Val-D-Pen(Bzl)-Thr-Nva-lle-Arg-PrONHCH₂CH₃

The procedure described in Example 1 was used but substituting Fmoc-D-Pen(Bzl) for Fmoc-D-Ile. After cleavage of the peptide from the resin and removal of the protecting groups using (9: 1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-Pen(Bzl)-Thr-Nva-Ile-Arg-ProNHCH₂CH₃ as the trifluoroacetate salt: Rₜ =4.124 min (gradient of 10% to 30% acetonitrile in water containing 0.01 % TFA over 30 min period); MS (ESI) m/e 1102 (M)⁺.

### Example 101

### N-Ac-Sar-Gly-Val-D-Abu-Thr-Nva-Ile-Arg-ProNHCH₂CH₃

The procedure described in Example 1 was used but substituting Fmoc-D-Abu for Fmoc-D-Ile. After cleavage of the peptide from the resin and removal of the protecting groups using (9: 1) TFA/anisole (3 mL) the crude product was purified by C-18 column chromatography using a solvent mixture varying in a gradient of 10% to 50% acetonitrile-water containing 0.01 % TFA. The pure fractions were lyophilized to yield N-Ac-Sar-Gly-Val-D-Abu-Thr-Nva-Ile-Arg-ProNHCH₂CH₃ as the trifluoroacetate salt: Rₜ = 2.533 min (gradient of 10% to 30% acetonitrile in water containing 0.01% TFA over 30 min period); MS (ESI) m/e 966 (M)⁺.

### Example 102

### N-Ac-Sar-Gly-Val-D-Phe(4-NH₂)-Thr-Nva-Ile-Arg-ProNHCH₂CH₃

The procedure described in Example 1 was used but substituting Fmoc-D-Phe(4-Boc-NH₂) for Fmoc-D-Ile. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01 % TFA. The pure fractions were lyophilized to yield N-Ac-Sar-Gly-Val-D-Phe(4-NH₂)-Thr-Nva-Ile-Arg-ProNHCH₂CH₃ as the trifluoroacetate salt: R₁ = 2.545 min (gradient of 10% to 30% acetonitrile in water containing 0.01 % TFA over 30 min period); MS (ESI) m/e 1043 (M)⁺.

### Example 120

### N-Ac-Sar-Gly-Val-D-Leu-Ala-Nva-Ile-Arg-ProNHCH₂CH₃

The procedure described in Example 15 was used but substituting Fmoc-Ala for Fmoc-Thr(tBu). After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-Leu-Ala-Nva-Ile-Arg-ProNHCH₂CH₃ as the trifluoroacetate salt: Rₜ = 3.481 min (gradient of 10% to 30% acetonitrile in water containing 0.01% TFA over 30 min period); MS (ESI) m/e 964 (M)⁺.

### Example 121

### N-Ac-Sar-Gly-Val-D-Leu-Pro-Nva-Ile-Arg-ProNHCH₂CH₃

The procedure described in Example 15 was used but substituting Fmoc-Pro for Fmoc-Thr(tBu). After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-Leu-Pro-Nva-Ile-Arg-ProNHCH₂CH₃ as the trifluoroacetate salt: Rₜ = 3.621 min (gradient of 10% to 30% acetonitrile in water containing 0.01 % TFA over 30 min period); MS (ESI) m/e 990 (M)⁺.

### Example 122

### N-Ac-Sar-Gly-Val-D-Leu-Trp-Nva-He-Arg-ProNHCH₂CH₃

The procedure described in Example 15 was used but substituting Fmoc-Trp(Boc) for Fmoc-Thr(tBu). After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-Leu-Trp-Nva-Ile-Arg-ProNHCH₂CH3 as the trifluoroacetate salt: Rₜ = 4.378 min (gradient of 10% to 30% acetonitrile in water containing 0.01% TFA over 30 min period); MS (ESI) m/e 1079 (M)⁺.

### Example 123

### N-Ac-Sar-Gly-Val-D-Leu-Tyr-Nva-Ile-Arg-ProNHCH₂CH₃

The procedure described in Example 15 was used but substituting Fmoc-Tyr(tBu) for Fmoc-Thr(tBu). After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-Leu-Tyr-Nva-Ile-Arg-ProNHCH₂CH₃ as the trifluoroacetate salt: Rₜ = 3.606 min (gradient of 10% to 30% acetonitrile in water containing 0.01% TFA over 30 min period); MS (ESI) m/e 1056 (M)⁺.

### Example 124

### N-Ac-Sar-Gly-Val-D-Leu-Nva-Nva-Ile-Arg-ProNHCH₂CH₃

The procedure described in Example 15 was used but substituting Fmoc-Nva for Fmoc-Thr(tBu). After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-Leu-Nva-Nva-Ile-Arg-ProNHCli₂CH₃ as the trifluoroacetate salt: Rₜ = 3.870 min (gradient of 10% to 30% acetonitrile in water containing 0.01 % TFA over 30 min period); MS (ESI) m/e 992 (M)⁺.

### Example 125

### N-Ac-Sar-Gly-Val-D-Leu-Gly-Nva-Ile-Arg-ProNHCH₂CH₃

The procedure described in Example 15 was used but substituting Fmoc-Gly for Fmoc-Thr(tBu). After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-Leu-Gly-Nva-Ile-Arg-ProNHCH₂CH₃ as the trifluoroacetate salt: Rₜ = 3.397 min (gradient of 10% to 30% acetonitrile in water containing 0.01 % TFA over 30 min period); MS (ESI) m/e 950 (M)⁺.

### Example 126

### N-Ac-Sar-Gly-Val-D-Leu-Lys(Ac)-Nva-Ile-Arg-ProNHCH₂CH₃

The procedure described in Example 15 was used but substituting Fmoc-Lys(Ac) for Fmoc-Thr(tBu). After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-Leu-Lys(Ac)-Nva-Ile-Arg-ProNHCH₂CH₃ as the trifluoroacetate salt: Rₜ = 3.365 min (gradient of 10% to 30% acetonitrile in water containing 0.0 1 % TFA over 30 min period); MS (ESI) m/e 1063 (M)⁺.

### Example 127

### N-Ac-Sar-Gly-Val-D-Leu-2Nal-Mva-Me-Arg-ProNHCH₂CH₃

The procedure described in Example 15 was used but substituting Fmoc-2Nal for Fmoc-Thr(tBu). After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-Leu-2Nal-Nva-Ile-Arg-ProNHCH₂CH₃ as the trifluoroacetate salt: Rₜ = 4.992 min (gradient of 10% to 30% acetonitrile in water containing 0.01% TFA over 30 min period); MS (ESI) m/e 1090 (M)⁺.

### Example 128

### N-Ac-Sar-Gly-Val-D-Leu-1Nal-Nva-Ile-Arg-ProNHCH₂CH₃

The procedure described in Example 15 was used but substituting Fmoc-lNa1 for Fmoc-Thr(tBu). After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-Leu-1Nal-Nva-Ile-Arg-ProNHCH₂CH₃ as the trifluoroacetate salt: Rₜ= 5.032 min (gradient of 10% to 30% acetonitrile in water containing 0.01 % TFA over 30 min period); MS (ESI) m/e 1090 (M)⁺.

### Example 129

### N-Ac-Sar-Gly-Val-D-Leu-Octylgly-Nva-Ile-Arg-ProNHCH₂CH₃.

The procedure described in Example 15 was used but substituting Fmoc-Octylgly for Fmoc-Thr(tBu). After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-Leu-Octylgly-Nva-Ile-Arg-ProNHCH₂CH₃ as the trifluoroacetate salt: Rₜ = 5.90 min (gradient of 10% to 30% acetonitrile in water containing 0.01% TFA over 30 min period); MS (ESI) m/e 1062 (M)⁺.

### Example 130

### N-Ac-Sar-Gly-Val-D-Leu-Gin-Nva-Ile-Arg-ProNHCH₂CH₃

The procedure described in Example 15 was used but substituting Fmoc-Gln(Trt) for Fmoc-Thr(tBu). After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-Leu-Gln-Nva-Ile-Arg-ProNUCH₂CH₃ as the trifluoroacetate salt: Rₜ = 3.323 min (gradient of 10% to 30% acetonitrile in water containing 0.01% TFA over 30 min period); MS (ESI) m/e 1021 (M)⁺.

### Example 131

### N-Ac-Sar-Gly-Val-D-Leu-Met-Nva-Ile-Arg-ProNHCH₂CH₃

The procedure described in Example 15 was used but substituting Fmoc-Met for Fmoc-Thr(tBu). After cleavage of the peptide from the resin and removal of the protecting groups using (9: 1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-Leu-Met-Nva-lie-Arg-ProNECH₂CH₃ as the trifluoroacetate salt: Rₜ = 3.901 min (gradient of 10% to 30% acetonitrile in water containing 0.01% TFA over 30 min period); MS (ESI) m/e 1024 (M)⁺.

### Example 132

### N-Ac-Sar-Gly-Val-D-Leu-Ser-Nva-Ile-Arg-ProNHCH₂CH₃

The procedure described in Example 15 was used but substituting Fmoc-Ser(tBu) for Fmoc-Thr(tBu). After cleavage of the peptide from the resin and removal of the protecting groups using (9: 1) TFA/anisole (3 mL) the crude product was purified by C-18 column chromatography using a solvent mixture varying in a gradient of 10% to 50% acetonitrile-water containing 0.01 % TFA. The pure fractions were lyophilized to yield N-Ac-Sar-Gly-Val-D-Leu-Ser-Nva-Ile-Arg-ProNHCH₂CH₃ as the trifluoroacetate salt: Rₜ = 3.414 min (gradient of 10% to 30% acetonitrile in water containing 0.01% TFA over 30 min period); MS (ESI) m/e 980 (M)⁺.

### Example 133

### N-Ac-Sar-Gly-Val-D-Leu-Allylgly-Nva-Ile-Arg-ProNHCH₂CH₃

The procedure described in Example 15 was used but substituting Fmoc-Allylgly for Fmoc-Thr(tBu). After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-Leu-Allylgly-Nva-Ile-Arg-ProNHCH₂CH₃ as the trifluoroacetate salt: Rₜ = 3.801 min (gradient of 10% to 30% acetonitrile in water containing 0.01% TFA over 30 min period); MS (ESI) m/e 990 (M)⁺.

### Example 134 '

### N-Ac-Sar-Gly-Val-D-Leu-Ile-Nva-Ile-Arg-ProNHCH₂CH₃

The procedure described in Example 15 was used but substituting Fmoc-Ile for Fmoc-Thr(tBu). After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-Leu-Ile-Nva-IIe-Arg-ProNHCH₂CH₃ as the trifluoroacetate salt: Rₜ= 4.028 min (gradient of 10% to 30% acetonitrile in water containing 0.01 % TFA over 30 min period): MS (ESI) m/e 1006 (M)⁺.

### Example 135

### N-Ac-Sar-Gly-Val-D-Leu-D-Thr-Nva-IIe-Arg-ProNHCH₂CH₃

The procedure described in Example 15 was used but substituting Fmoc-D-Thr(tBu) for Fmoc-Thr(tBu). After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.0 1 % TFA. The pure fractions were lyophilized to yield N-Ac-Sar-Gly-Val-D-Leu-D-Thr-Nva-Ile-Arg-ProNHCH₂CH₃ as the trifluoroacetate salt: Rₜ = 3.437 min (gradient of 10% to 30% acetonitrile in water containing 0.01% TFA over 30 min period); MS (ESI) m/e 994 (M)⁺.

### Example 136

### N-Ac-Sar-Gly-V al-D-Ile-Thr-Ile-Ile-Arg-ProNHCH₂CH₃

The procedure described in Example 1 was used but substituting Fmoc-Ile for Fmoc-Nva. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-Ile-Thr-Ile-Ile-Arg-ProNHCH₂CH₃ as the trifluoroacetate salt: Rₜ = 3.54 min (gradient of 10% to 30% acetonitrile in water containing 0.01% TFA over 30 min period); MS (ESI) m/e 1008 (M)⁺; Amino Acid Anal.: 1.07 Sar; 0.94 Gly; 0.91 Val; 3.02 Ile; 0.47 Thr; 1.24 Arg; 1.04 Pro.

### Example 137

### N-Ac-Sar-Gly-Val-D-Ile-Thr-Nle-Ile-Arg-ProNHCH₂CH₃

The procedure described in Example 1 was used but substituting Fmoc-Nle for Fmoc-Nva. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-Ile-Thr-Nle-Ile-Arg-ProNHCH₂CH₃ as the trifluoroacetate salt: Rₜ = 3.80 min (gradient of 10% to 30% acetonitrile in water containing 0.01% TFA over 30 min period); MS (ESI) m/e 1006 (M)⁺.

### Example 138

### N-Ac-Sar-Gly-Val-D-Ile-Thr-Cit-Ile-Arg-ProNHCH₂CH₃

The procedure described in Example 1 was used but substituting Fmoc-Cit for Fmoc-Nva. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01 % TFA. The pure fractions were lyophilized to yield N-Ac-Sar-Gly-Val-D-Ile-Thr-Cit-Ile-Arg-ProNHCH₂CH₃ as the trifluoroacetate salt: Rₜ = 2.83 min (gradient of 10% to 30% acetonitrile in water containing 0.01% TFA over 30 min period); MS (ESI) m/e 1052 (M)⁺; Acid Anal.: 1.05 Sar; 1.00 Gly; 1.00 Val; 2.13 Ile; 0.65 Thr; 1.11 Cit; 1.49 Arg; 1.10 Pro.

### Example 139

### N-Ac-Sar-Gly-Val-D-Ile-Thr-Met(O₂)-Ile-Arg-ProNHCH₂CH₃

The procedure described in Example 1 was used but substituting Fmoc-Met(O₂) for Fmoc-Nva. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.0 1 % TFA. The pure fractions were lyophilized to yield N-Ac-Sar-Gly-Val-D-Ile-Thr-Met(O₂)-Ile-Arg-ProNHCH₂CH₃ as the trifluoroacetate salt: Rₜ =2.701 min (gradient of 10% to 30% acetonitrile in water containing 0.01% TFA over 30 min period); MS (ESI) m/e 1058 (M)⁺; Acid Anal.: 1.36 Sar; 0.94 Gly; 0.62 Val; 2.06 Ile; 0.13 Thr; 0.66 Met(O2); 1.50 Arg; 0.68 Pro.

### Example 140

### N-Ac-Sar-Gly-Val-D-Ile-Thr-Arg-Ile-Arg-ProNHCH₂CH₃

The procedure described in Example 1 was used but substituting Fmoc-Arg(Pmc) for Fmoc-Nva. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-Ile-Thr-Arg-Ile-Arg-ProNHCH₂CH₃ as the trifluoroacetate salt: Rₜ = 0.54 min (gradient of 10% to 30% acetonitrile in water containing 0.01% TFA over 30 min period); MS (ESI) m/e 1049 (M)⁺; Acid Anal.: 0.92 Sar; 0.74 Gly; 0.86 Val; 2.00 Ile; 0.49 Thr; 2.67 Arg; 1.00 Pro.

### Example 141

### N-Ac-Sar-Gly-Val-D-Ile-Thr-Tyr-Ile-Arg-ProNHCH₂CH₃

The procedure described in Example 1 was used but substituting Fmoc-Tyr(tBu) for Fmoc-Nva. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-Ile-Tlu-Tyr-Ile-Arg-ProNHCH₂CH₃ as the trifluoroacetate salt: Rₜ = 3.048 min (gradient of 10% to 30% acetonitrile in water containing 0.01% TFA over 30 min period); MS (ESI) m/e 1058 (M)⁺; Acid Anal.: 0.88 Sar; 0.99 Gly; 0.97 Val; 1.97 Ile; 0.52 Thr; 0.92 Tyr; 1.58Arg; 1.08 Pro.

### Example 142

### N-Ac-Sar-Gly-V al-D-Ile-Thr-Glu-Ile-Arg-ProNHCH₂CH₃

The procedure described in Example 1 was used but substituting Fmoc-Glu(OtBu)-OH for Fmoc-Nva. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01 % TFA. The pure fractions were lyophilized to yield N-Ac-Sar-Gly-Val-D-Ile-Thr-Glu-Ile-Arg-ProNHCH₂CH₃ as the trifluoroacetate salt: Rₜ = 2.348 min (gradient of 10% to 30% acetonitrile in water containing 0.01% TFA over 30 min period); MS (ESI) m/e 1024 (M)⁺; Acid Anal.: 1.05 Sar; 1.024 Gly; 0.94 Val; 2.67 Ile; 0.47 Thr; 0.94 Glu; 2.20 Arg; 1.09 Pro.

### Example 143

### N-Ac-Sar-Gly-Val-D-Ile-Thr-Lys(Ac)-Ile-Arg-ProNHCH₂CH₃

The procedure described in Example 1 was used but substituting Fmoc-Lys(Ac) for Fmoc-Nva. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-Ile-Thr-Lys(Ac)-Ile-Ar;-ProNHCH₂CH₃ as the trifluoroacetate salt: Rₜ = 2.744 min (gradient of 10% to 30% acetonitrile in water containing 0.01% TFA over 30 min period); MS (ESI) m/e 1065 (M)⁺; Acid Anal.: 1.03 Sar; 0.99 Gly; 0.95 Val; 2.04 Ile; 0.66 Thr; 1.05 Lys; 1.41 Arg; 1.02 Pro.

### Example 144

### N-Ac-Sar-Gly-Val-D-Ile-Thr-Propargylgly-Ile-Arg-ProNHCH₂CH₃

The procedure described in Example 1 was used but substituting Fmoc-Propargylgly for Fmoc-Nva. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.0 1 % TFA. The pure fractions were lyophilized to yield N-Ac-Sar-Gly-Val-D-Ile-Thr-Propargylgly-Ile-Arg-ProNHCH₂CH₃ as the trifluoroacetate salt: Rₜ = 3.003 min (gradient of 10% to 30% acetonitrile in water containing 0.01% TFA over 30 min period); MS (ESI) m/e 990 (M)⁺; Acid Anal.: 1.05 Sar; 1.00 Gly; 0.93 Val; 2.10 Ile; 0.54 Thr; 1.71 Arg; 0.97 Pro.

### Example 145

### N-Ac-Sar-Gly-Val-D-alloIle-Thr-Gln-Ile-Arg-ProNHCH₂CH₃

The procedure described in Example 1 was used but substituting Fmoc-D-alloIle for Fmoc-D-Ile and Fmoc-Gln(Trt) for Fmoc-Nva. After cleavage of the peptide from the resin and removal of the protecting groups using (9: 1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-alloIle-Thr-Gln-Ile-Arg-ProNHCH₂CH₃ as the trifluoroacetate salt: Rₜ = 2.704 min (gradient of 10% to 30% acetonitrile in water containing 0.01% TFA over 30 min period); MS (ESI) m/e 1023 (M)⁺; Acid Anal.: 0.93 Sar; 0.94 Gly; 0.94 Val; 2.10 Ile; 0.51 Thr; 0.87 Glu; 1.45 Arg; 1.03 Pro.

### Example 146

### N-Ac-Sar-Gly-V al-D-Leu-Thr-Gln-Ile-Arg-ProNHCH₂CH₃

The procedure described in Example 15 was used but substituting Fmoc-Gln(Trt) for Fmoc-Nva. After cleavage of the peptide from the resin and removal of the protecting groups using (9: 1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-Leu-Thr-Gln-Ile-Arg-ProNHCH₂CH₃ as the trifluoroacetate salt: Rₜ = 2.685 min (gradient of 10% to 30% acetonitrile in water containing 0.01% TFA over 30 min period); MS (ESI) m/e 1023 (M)⁺; Acid Anal.: 0.98 Sar; 0.74 Gly; 0.95 Val; 1.04 Ile; 0.49 Thr; 1.04 Leu; 0.94 Glu; 1.63 Arg; 0.97 Pro.

### Example 147

### N-Ac-Bala-Sar-Gly-Val-D-Ile-Thr-Nva-Ile-Arg-ProNHCH₂CH₃

The procedure described in Example 65 was used but substituting Fmoc-beta-alanine for Fmoc-4-amino-butyric acid. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Bala-Sar-Gly-Val-D-Ile-Thr-Nva-Ile-Arg-ProNHCH₂CH₃ as the trifluoroacetate salt: Rₜ = 2.92 min (gradient of 10% to 30% acetonitrile in water containing 0.01% TFA over 30 min period); MS (ESI) m/e 1065 (M)⁺; Acid Anal.: 0.99 Sar; 0.99 Gly; 1.00 Val; 1.86Ile; 0.49 Thr; 1.07 Nva; 1.5 1 Arg; 1.02 Pro.

### Example 148

### N-Phenylacetyl-Sar-Gly-Val-D-Ile-Thr-Nva-Ile-Arg-ProNHCH₂CH₃

The procedure described in Example 60 was used but substituting phenylacetic acid for butyric acid. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Phenylacetyl-Sar-Gly-Val-D-Ile-Thr-Nva-Ile-Arg-ProNHCH₂CH₃ as the trifluoroacetate salt: Rₜ = 3.83 min (gradient of 10% to 30% acetonitrile in water containing 0.01% TFA over 30 min period); MS (ESI) m/e 1070 (M)⁺; Acid Anal.: 1.04 Sar; 0.979 Gly; 1.01 Val; 1.90 Ile; 0.59 Thr; 1.09 Nva; 1.53 Arg; 1.03 Pro.

### Example 149

### N-Ac-Sar-Gly-Val-D-Ile-Thr-Nva-Ile-Arg-Pro-Azagly-NH₂

To a solution of N-Ac-Sar-Gly-Val-D-Ile-Thr(tBu)-Nva-Ile-Arg(Pmc)-Pro-OH (0.1288 g) in DMF was added semicarbazide hydrochloride (0.222 g) followed by DIEA (0.346 ml) and PyBrop (0.0513 g). The solution was stirred at rt for 36 hr. The solvent was removed in vacuo and the residue was treated with diethyl ether. The solid was filtered and then treated with (9: 1) TFA/anisole (3 mL) at rt for 4 hr. The solvent was again removed in vacuo and the residue was treated with diethyl ether. The precipitate was filtered to give the crude product as a solid. This was purified by C-18 column chromatography using a solvent mixture varying in a gradient of 10% to 50% acetonitrile-water containing 0.01% TFA. The pure fractions were lyophilized to yield N-Ac-Sar-Gly-Val-D-Ile-Thi-Nva-Ile-Arg-Pro-Azagly-NH₂ as the trifluoroacetate salt: Rₜ = 2:67 min (gradient of 10% to 30% acetonitrile in water containing 0.01% TFA over 30 min period); MS (ESI) m/e 1024 (M)⁺ Acid Anal.: 0.99 Sar; 0.98 Gly; 1.00 Val; 2.13 Ile; 0.56 Thr; 1.09 Nva; 0.92 Arg; 1-02 Pro.

### Example 151

### N-Ac-Sar-Gly-Val-D-Ile-Thr-Nva-Ile-Arg-Pro-SerNH₂

The procedure described in Example 75 was used but substituting Fmoc-Ser(tBu)-Sieber amide resin for Fmoc-D-Ala-Sieber amide resin. After cleavage of the peptide from the resin and removal of the protecting groups using (9:1) TfAlanisole (3 mL) the crude product was purified by C-18 column chromatography using a solvent mixture varying in a gradient of 10% to 50% acetonitrile-water containing 0.0 1 % TFA. The pure fractions were lyophilized to yield N-Ac-Sar-Gly-Val-D-Ile-Thr-Nva-Ile-Arg-Pro-SerNH₂ as the trifluoroacetate salt: Rₜ = 2.65 min (gradient of 10% to 30% acetonitrile in water containing 0.01% TFA over 30 min period); MS (ESI) m/e 1053 (M)⁺; Acid Anal.: 0.99 Sar; 0.95 Gly; 1.00 Val; 1.96 Ile; 0.57 Thr; 1.12 Nva; 1.03 Arg; 1.03 Pro; 0.27 Ser.

### Example 152

### N-Succinyl-S ar-Gly-V al-D-Leu-Thr-Nva-Ile-Arg-ProNHCH₂CH₃

The procedure described in Example 54 was used but substituting Fmoc-D-Leu for Fmoc-D-IIe. Alter cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Succinyl-Sar-Gly-Val-D-Leu-Thr-Nva-Ile-Arg-ProNHCH₂CH₃ as the trifluoroacetate salt: Rₜ = 2.85 min (gradient of 10% to 3 0% acetonitrile in water containing 0.01 % TFA over 30 min period); MS (ESI) m/e 1052 (M)⁺; Acid Anal.: 1.01 Sar; 0.93 Gly; 0.95Val; 1.16 Leu; 1.10 Ile; 0.51 Thr; 1.04 Nva; 1.67 Arg; 0.96 Pro.

### Example 171

### N-Ac-Sar-Gly-Val-D-Ile-Thr-Gln-Ile-Arg-ProNHCH₂(CH₃)₂

The procedure described in Example 4 was used but substituting Fmoc-Gln(Trt) for Fmoc-Nva. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-He-Thr-Gln-He-Arg-ProNHCH2(CH3)2 as the trifluoroacetate salt: Rₜ = 2.80 min (gradient of 10% to 30% acetonitrile in water containing 0.01% TFA over 30 min period); MS (ESI) m/e 1037 (M)⁺; Acid Anal.: 0.98 Sar; 0.94 Gly; 0.97 Val; 2.23 Ile; 0.51 Thr; 0.90 Glu; 1.16 Arg; 1.03 Pro.

### Example 172

### N-Ac-Sar-Gly-Val-D-Leu-Thr-Gln-Ile-Arg-ProNHCH₂(CH₃)₂

The procedure described in Example 4 was used but substituting Fmoc-D-Leu for Fmoc-D-Ile and Fmoc-Gln(Trt) for Fmoc-Nva. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01 % TFA. The pure fractions were lyophilized to yield N-Ac-Sar-Gly-Val-D-Leu-Tur-GIn-Ile-Arg-ProNHCH₂(CH₃)₂ as the trifluoroacetate salt: Rₜ = 2.90 min (gradient of 10% to 30% acetonitrile in water containing 0.01% TFA over 30 min period); MS (ESI) m/e 1037 (M)⁺; Acid Anal.: 1.05 Sar; 0.97 Gly; 0.99 Val; 1.30 Leu 1.11 Ile; 0.52 Thr; 0.89 Glu; 1.20 Arg; 1.04 Pro.

### Example 173

### H-Sar-Gly-Val-D-Leu-Thr-Gln-Ile-Arg-ProNHCH₂(CH₃)₂

The procedure described in Example 172 was used but omitting the last coupling with acetic acid. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield H-Sar-Gly-Val-D-Leu-Thr-Gln-Ile-Arg-ProNHCH₂(CH₃)₂ as the trifluoroacetate salt: Rₜ = 2.55 min (gradient of 10% to 30% acetonitrile in water containing 0.01% TFA over 30 min period); MS (ESI) m/e 981 (M)⁺; Acid Anal.: 1.02 Sar; 0.93 Gly; 1.02 Val; 1.05 Leu; 1.02 Ile; 0.55 Thr; 0.84 Gln; 1.31 Arg; 1.03 Pro.

### Example 174

### N-Succinyl-Sar-Gly-Val-D-Ile-Thr-Gln-Ile-Arg-ProNHCH₂CH₃

The procedure described in Example 54 was used but substituting Fmoc-Gln(Trt) for Fmoc-Nva. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Succinyl-Sar-Gly-Val-D-Ile-Thr-Gln-Ile-Arg-ProNHCH₂CH₃ as the trifluoroacetate salt: Rₜ = 2.02 min (gradient of 10% to 30% acetonitrile in water containing 0.01% TFA over 30 min period); MS (ESI) m/e 1081 (M) ; Acid Anal.: 1.00 Sar, 0.94 Gly; 1.00 Val; 2.00 Ile; 0.52 Thr; 0.87 Gln; 1.37 Arg; 1.05 Pro.

### Example 175

### N-Succinyl-Sar-Gly-Val-D-Leu-Thr-Gln-Ile-Arg-ProNHCH₂CH₃

The procedure described in Example 174 was used but substituting Fmoc-D-Leu for Fmoc-D-Ile. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01 % TFA. The pure fractions were lyophilized to yield N-Succinyl-Sar-Gly-Val-D-Leu-Thr-Gln-Ile-Arg-ProNHCH₂CH₃ as the trifluoroacetate salt: Rₜ = 2.284 min (gradient of 10% to 30% acetonitrile in water containing 0.01% TFA over 30 min period); MS (ESI) m/e 1081 (M)⁺.

### Example 176

### N-Succinyl-Sar-Gly-Val-D-Leu-Thr-Gln-Ile-Arg-ProNHCH₂(CH₃)₂

The procedure described in Example 4 was used but substituting Fmoc-D-Leu for Fmoc-D-Ile and Fmoc-Gln(Trt) for Fmoc-Nva. Following the coupling with Fmoc-Sar and protection the resin was treated with succinic anhydride/pyridine as described in Example 54. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Succinyl-Sar-Gly-Val-D-Leu-Thr-Gln-Ile-Arg-ProNHCH₂(CH₃)₂ as the trifluoroacetate salt: Rₜ = 2.56 min (gradient of 10% to 30% acetonitrile in water containing 0.01% TFA over 30 min period); MS (ESI) m/e 1095 (M)⁺; Acid Anal.: 0.95 Sar; 0.94 Gly; 1.02 Val; 1.02 Leu; 1.05 Ile; 0.56 Thr; 0.86 Gln; 1.00 Arg; 1.07 Pro.

### Example 177

### N-Ac-Sar-Gly-Val-D-Leu-Thr-Asp-Ile-Arg-ProNHCH₂CH₃

The procedure described in Example 146 was used but substituting Fmoc-Asp(OtBu)-OH for Fmoc-Gln(Trt). After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-Leu-Thr-Asp-Ile-Arg-ProNHCH₂CH₃ as the trifluoroacetate salt: Rₜ = 2.53 min (gradient of 10% to 30% acetonitrile in water containing 0.0 1 % TFA over 30 min period); MS (ESI) m/e 1010 (M)⁺; Acid Anal.: 1.00 Sar; 0.95 Gly; 1.01 Val; 1.02 Leu; 1.00 Ile; 0.56 Thr; 0.99 Asp; 1.43 Arg; 1.03 Pro.

### Example 178

### N₇Ac-Sar-Gly-Val-D-Ile-Thr-Asp-Ile-Arg-ProNHCH₂CH₃

The procedure described in Example 142 was used but substituting Fmoc-Asp(OtBu)-OH for Fmoc-Glu(OtBu)-OH. After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-Ile-Thr-Asp-Ile-Arg-ProNHCH₂CH₃ as the trifluoroacetate salt: Rₜ = 2.455 min (gradient of 10% to 30% acetonitrile in water containing 0.01% TFA over 30 min period); MS (ESI) m/e 1010 (M)⁺.

### Example 179

### N-Ac-Sar-Gly-V al-D-Ile-Thr-Asn-Ile-Arg-ProNHCH₂CH₃

The procedure described in Example 43 was used but substituting Fmoc-Asn(Trt) for Fmoc-Gln(Trt). After cleavage of the peptide from the resin and removal of the protecting groups using (9: 1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-Ile-Thr-Asn-Ile-Arg-ProNHCH₂CH₃ as the trifluoroacetate salt: Rₜ = 2.68 min (gradient of 10% to 30% acetonitrile in water containing 0.01% TFA over 30 min period); MS (ESI) m/e 1009 (M)⁺.

### Example 180

### N-Ac-Sar-Gly-Val-D-Ile-Thr-Met(O)-Ile-Arg-PrONHCH₂CH₃

The procedure described in Example 139 was used but substituting Fmoc-Met(O) for Fmoc-Met(O₂) After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-Ile-Thr-Met(O)-Ile-Arg-PrONHCH₂CH₃ as the trifluoroacetate salt: Rₜ = 2.713 min (gradient of 10% to 30% acetonitrile in water containing 0.01 % TFA over 30 min period); MS (ESI) m/e 1042 (M)⁺.

### Example 181

### N-Ac-Sar-Gly-V al-D-Leu-Thr-Asn-Ile-Arg-ProNHCH₂CH₃

The procedure described in Example 146 was used but substituting Fmoc-Asn(Trt) for Fmoc-Gln(Trt). After cleavage of the peptide from the resin and removal of the protecting groups using (9:1) TFA/anisole (3 mL) the crude product was purified by C-18 column chromatography using a solvent mixture varying in a gradient of 10% to 50% acetonitrile-water containing 0.01% TFA. The pure fractions were lyophilized to yield N-Ac-Sar-Gly-Val-D-Leu-Thr-Asn-Ile-Arg-ProNHCH₂CH₃ as the trifluoroacetate salt: Rₜ = 2.752 min (gradient of 10% to 30% acetonitrile in water containing 0.01% TFA over 30 min period); MS (ESI) m/e 1009 (M)⁺.

### Example 182

The procedure described in Example 1 is used but separately substituting in the syntheses Fmoc-D-Ile with the following amino acids: Fmoc-D-Thr(tBu), Fmoc-D-Ser(tBu), Fmoc-D-Hser(tBu), Fmoc-D-Gln(Trt), Fmoc-D-Asn(Trt), Fmoc-D-Cit, Fmoc-D-Hcit, Fmoc-D-Hle, Fmoc-D-Neopentylgly. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01 % TFA. The pure fractions are lyophilized to yield the trifluoroacetate salt of the following peptides:
N-Ac-Sar-Gly-Val-D-Thr-Thr-Nva-Ile-Arg-ProNECH₂CH₃,
N-Ac-Sar-Gly-Val-D-Ser-Thr-Nva-Ile-Arg-ProNHCH₂CH₃,
N-Ac-Sar-Gly--Val-D-Hser-Thr-Nva-Ile-Arg-ProNHCH₂CH₃,
N-Ac-Sar-Gly-Val-D-Gln-Thr-Nva-Ile-Arg-ProNHCH₂CH₃,
N-Ac-Sar-Gly-V al-D-Asn-Thr-Nva-Ile-Arg-ProNHCH₂CH₃,
N-Ac-Sar-Gly-Val-D-Cit-Thr-Nva-Ile-Arg-ProNHCH₂CH₃,
N-Ac-Sar-Gly-Val-D-Hcit-Thr-Nva-Ile-Arg-ProNHCH₂CH₃,
N-Ac-Sar-Gly-Val-D-Hle-Thr-Nva-Ile-Arg-ProNHCH₂CH₃, and
N-Ac-Sar-Gly-Val-D Neopentylgly-Thr-Nva-Ile-Arg-ProNHCH₂CH₃..

### Example 183

### N-Ac-Sar-Gly-Val-D-Ile-Thr-Phe(4-CONH2)-Ile-Arg-ProNHCH₂CH₃

The procedure described in Example 43 is used but substituting Fmoc-Phe[4-CONH(Trt)] for Fmoc-Gln(Trt). After cleavage of the peptide from the resin and removal of the protecting groups using (9: 1) TFA/anisole (3 mL) the crude product is purified by C-18 column chromatography using a solvent mixture varying in a gradient of 10% to 50% acetonitrile-water containing 0.01 % TFA. The pure fractions are lyophilized to yield N-Ac-Sar-Gly-Val-D-Ile-Thr-Phe(4-CONH₂)-Ile-Arg-PrONHCH₂CH₃.

### Example 191

### N-Ac-Sar-Gly-Val-D-Ile-Phe(4-CH₂OH)-Nva-Ile-Arg-ProNHCH₂CH₃

The procedure described in Example 1 is used but substituting Fmoc-Phe[4-CH₂O(Trt)] for Fmoc-Thr(Trt). After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Ac-Sar-Gly-Val-D-Ile-Phe(4-CH₂OH)-Nva-Ile-Arg-PrONHCH₂CH₃.

### Example 200

### N-Succinyl-Sar-Gly-Val-D-alloIle-Thr-Nva-Ile-Arg-ProNHCH₂CH₃

The procedure described in Example 54 is used but substituting Fmoc-D-alloIle for Fmoc-D-Ile. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Succinyl-Sar-Gly-Val-D-allolle-Thr-Nva Ile-Arg-ProNHCH₂CH₃ as the trifluoroacetate salt.

### Example 201

### N-Succinyl-Sar-Gly-Val-D-Ile-Thr-Gln-Ile-Arg-ProNHCH₂CH₃

The procedure described in Example 54 is used but substituting Fmoc-Gln(Trt) for Fmoc-Nva. After cleavage of the peptide from the resin and removal of the protecting groups using (9: 1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Succinyl-Sar-Gly-Val-D-lie-Thr-Gln-Ile-Arg-ProNHCH₂CH₃ as the trifluoroacetate salt.

### Example 202

### N-Succinyl-Sar-Gly-V al-D-Ile-Thr-Gln-Ile-Arg-Pro-D-AlaNH₂

The procedure described in Example 75 is used but substituting Fmoc-Gln(Trt) for Fmoc-Nva and, after the coupling with Fmoc-Sar, acylating the peptide resin with succinic anhydride as described in Example 54. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Succinyl-Sar-Gly-Val-D-Ile-Thr-Gln-Ile-Arg-Pro-D-AlaNH₂ as the trifluoroacetate salt.

### Example.203

### N-Succinyl-Sar-Gly-Val-D-alloIle-Tb.r-Gln-Ile-Arg-ProNHCH₂CH₃

The procedure described in Example 201 is used but substituting Fmoc-D-alloIle for Fmoc-D-Ile. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Succinyl-Sar-Gly-Val-D-alloIle-Thr-Gln-Ile-Arg-ProNHCH₂CH₃ as the trifluoroacetate salt.

### Example 204

### N-Succinyl-Sar-Gly-Val-D-alloIle-Thr-Gln-Ile-Arg-Pro-D-AlaNH₂

The procedure described in Example 202 is used but substituting Fmoc-D-allolle for Fmoc-D-Ile. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01 % TFA. The pure fractions are lyophilized to yield N-Succinyl-Sar-Gly-Val-D-alloIle-Thr-Gln-Ile-Arg-Pro-D-AlaNH₂ as the trifluoroacetate salt.

### Example 205

### N-Succinyl-Sar-Gly-Val-D-alloIle-Thr-Gin-Ile-Arg-ProNHCH₂(CH₃)₂

The procedure described in Example 175 is used but substituting Fmoc-D-allolle for Fmoc-D-Leu. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Succinyl-Sar-Gly-Val-D-allolle-Thr-Gln-Ile-Arg-ProNHCH₂(CH₃)₂ as the trifluoroacetate salt.

### Example 206

### N-Succinyl-Sar-Gly-Val-D-Ile-Thr-Gln-Ile-Arg-ProNHCH₂(CH₃)₂

The procedure described in Example 205 is used but substituting Fmoc-D-Ile for Fmoc-D-allolle. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Succinyl-Sar-Gly-Val-D-Ile-Thr-Gln-Ile-Arg-ProNHCH₂(CH₃)₂ as the trifluoroacetate salt.

### Example 207

### N-Ac-Sar-Gly-Val-D-alloIle-Thr-Nva-Ile-Arg-Pro-D-AlaNH₂

The procedure described in Example 75 is used but substituting Fmoc-D-alloIle for Fmoc-D-Ile. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Ac-Sar-Gly-Val-D-allolle-Thr-Nva-Ile-Arg-Pro-D-Ala-NH₂ as the trifluoroacetate salt.

### Example 208

### N-Ac-Sar-Gly-Val-D-alloIle-Thr-Nva-Ile-Arg-ProNHCH₂(CH₃)₂

The procedure described in Example 4 is used but substituting Fmoc-D-alloIle for Fmoc-D-Ile. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Ac-Sar-Gly-Val-D-alloIle-Thr-Gln-Ile-Arg-ProNHCH₂(CH₃)₂ as the trifluoroacetate salt.

### Example 209

### N-Ac-Sar-Gly-Val-D-Ile-Thr-Gln-Ile-Arg-Pro-D-AlaNH₂

The procedure described in Example 75 is used but substituting Fmoc-Gln(Trt) for Fmoc-Nva. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Ac-Sar-Gly-Val-D-Ile-Thr-Gln-Ile-Arg-Pro-D-AlaNH₂ as the trifluoroacetate salt.

### Example 210

### N-Ac-Sar-Gly-Val-D-Ile-Thr-Gln-Ile-Arg-ProNHCH₂(CH₃)₂

The procedure described in Example 4 is used but substituting Fmoc-Gln(Trt) for Fmoc-Nva. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Ac-Sar-Gly-Val-D-Ile-Thr-Gln-Ile-Arg-ProNHCH₂(CH₃)₂ as the trifluoroacetate salt.

### Example 211

### N-Ac-Sar-Gly-Val-D-allolle-Thr-Gln-Ile-Arg-Pro-D-AlaNH₂

The procedure described in Example 209 is used but substituting Fmoc-D-allolle for Fmoc-D-Ile. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Ac-Sar-Gly-Val-D-alloIle-Thr-Gln-Ile-Arg-Pro-D-AlaNH₂ as the trifluoroacetate salt.

### Example 212

### N-Ac-Sar-Gly-Val-D-alloIle-Thr-Gln-Ile-Arg-ProNHCH₂(CH₃)₂

The procedure described in Example 210 is used but substituting Fmoc-D-alloIle for Fmoc-D-Ile. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01 % TFA. The pure fractions are lyophilized to yield N-Ac-Sar-Gly-Val-D-allolle-Thr-Gln-Ile-Arg-PrONHCH₂(CH₃)₂ as the trifluoroacetate salt.

### Example 213

### N-Ac-Sar-Gly-Val-D-Ile-Thr-Nva-Ile-Arg-Pro-SarNH2

The procedure described in Example 75 is used but substituting Fmoc-Sar-Seiberamide-resin for Fmoc-D-Ala-Seiberamide-resin. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Ac-Sar-Gly-Val-D-Ile-Thr-Nva-Ile-Arg-Pro-SarNH₂ as the trifluoroacetate salt.

### Example 214

### N-Ac-Sar-Gly-Val-D-alloIle-Thr-Nva-Ile-Arg-Pro-SarNH₂

The procedure described in Example 213 is used but substituting Fmoc-D-alloIle for Fmoc-D-Ile. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Ac-Sar-Gly-Val-D-alloIle-Thr-Nva-Ile-Arg-Pro-SarNH₂ as the trifluoroacetate salt.

### Example 215

### N-Ac-Sar-Gly-V al-D-Ile-Thr-Gln-Ile-Arg-Pro-SarNH₂

The procedure described in Example 213 is used but substituting Fmoc-Gln(Trt) for Fmoc-Nva. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01 % TFA. The pure fractions are lyophilized to yield N-Ac-Sar-Gly-Val-D-Ile-Thr-GIn-Ile-Arg-Pro-SarNH₂ as the trifluoroacetate salt.

### Example 216

### N-Ac-Sar-Gly-Val-D-alloIle-Thr-Gln-Ile-Arg-Pro-SarNH₂

The procedure described in Example 215 is used but substituting Fmoc-D-alloIle for Fmoc-D-Ile. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01 % TFA. The pure fractions are lyophilized to yield N-Ac-Sar-Gly-Val-D-alloIle-Thr-Gln-Ile-Arg-Pro-SarNH₂ as the trifluoroacetate salt.

### Example 217

### N-Ac-Sar-Gly-Val-D-alloIle-Thr-Ser-Ile-Arg-Pro-D-AlaNH₂

The procedure described in Example 207 is used but substituting Fmoc-Ser(tBu) for Fmoc-Nva. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Ac-Sar-Gly-Val-D-allolle-Thr-Ser-Ile-Arg-Pro-D-AlaNH₂ as the trifluoroacetate salt.

### Example 218

### N-Ac-Sar-Gly-Val-D-allolle-Thr-Ser-Ile-Arg-ProNHCH₂(CH₃)₂

The procedure described in Example 208 is used but substituting Fmoc-Ser(tBu) for Fmoc-Nva. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Ac-Sar-Gly-Val-D-allolle-Thr-Ser-Ile-Arg-PrONHCH₂(CH₃)₂ as the trifluoroacetate salt.

### Example 219

### N-Ac-Sar-Gly-Val-D-allolle-Thr-Ser-Ile-Arg-ProNHCH₂CH₃

The procedure described in Example 15 is used but substituting Fmoc-Ser(tBu) for Fmoc-Nva. After cleavage of the peptide from the resin and removal of the protecting groups using (9: 1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Ac-Sar-Gly-Val-D-alloIle-Thr-Ser-Ile-Arg-ProNHCH₂CH₃ as the trifluoroacetate salt.

### Example 220

### N-Ac-Sar-Gly-Val-D-Ile-Thr-Orn(Ac)Ile-Arg-ProNHCH₂CH₃

The procedure described in Example 1 is used but substituting Finoc-Orn(Ac) for Fmoc-Nva. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01 % TFA. The pure fractions are lyophilized to yield N-Ac-Sar-Gly-Val-D-Ile-Thr-O.rn(Ac)-Ile-Arg-ProNHCH₂CH₃ as the trifluoroacetate salt.

### Example 221

### N-Ac-Sar-Gly-Val-D-Ile-Thr-Gln-Ile-Arg-Pro-AzaglyNH₂

The procedure described in Example 149 is used but substituting Fmoc-Gln(Trt) for Fmoc-Nva. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Ac-Sar-Gly-Val-D-Ile-Thr-Gln-Ile-Arg-Pro-AzaglyNH₂ as the trifluoroacetate salt.

### Example 222

### N-Ac-Sar-Gly-Val-D-alloIle-Thr Nva-Ile-Arg-Pro-AzaglyNH₂

The procedure described in Example 149 is used but substituting Fmoc-D-alloIle for Fmoc-D-Ile. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Ac-Sar-Gly-Val-D-alloIle-Thr-Nva-Ile-Arg-Pro-AzaglyNH₂ as the trifluoroacetate salt.

### Example 223

### N-Ac-Sar-Gly-Val-D-alloIle-Thr-Gln-Ile-Arg-Pro-AzaglyNH₂

The procedure described in Example 222 is used but substituting Fmoc-Gln(Trt) for Fmoc-Nva. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Ac-Sar-Gly-Val-D-alloIle-Thr-Gln-Ile-Arg-Pro-AzaglyNH₂ as the trifluoroacetate salt.

### Example 224

### N-(2-THFcarbonyl)-Sar-Gly-Val-D-alloIle-Thr-Nva-Ile-Arg-ProNHCH₂CH₃

The procedure described in Example 61 is used but substituting tetrahydro-2-furoic acid for acetic acid. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-(2-THFcarbonyl)-Sar-Gly-Val-D-alloIle-Thr-Nva-Ile-Arg-ProNHCH₂CH₃ as the trifluoroacetate salt.

### Example 225

### N-(2-THFcarbonyl)-Sar-Gly-V al-D-Ile-Thr-Gln-Ile-Arg-ProNHCH₂CH₃

The procedure described in Example 61 is used but substituting Fmoc-Gln(Trt) for Fmoc-Nva. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.0 1 % TFA. The pure fractions are lyophilized to yield N-(2-THFcarbonyl)-Sar-Gly-Val-D-Ile-Thr-Gln-Ile-Arg-ProNHCH₂CH₃ as the trifluoroacetate salt.

### Example 226

### N-(2-THFcarbonyl)-Sar-Gly-Val-D-alloIle-Thr-Gln-Ile-Arg-ProNHCH₂CH₃

The procedure described in Example 225 is used but substituting Fmoc-D-alloIle for Fmoc-D-Ile. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-(2-THFcarbonyl)-Sar-Gly-Val-D-alloIle-Thr-Gln-Ile-Arg-ProNHCH₂CH₃ as the trifluoroacetate salt.

### Example 227

### N-(2-THFcarbonyl)-Sar-Gly-Val-D-Ile-Thr-Gln-Ile-Arg-Pro-D-AlaNH₂

The procedure described in Example 209 is used but substituting tetrahydro-2-furoic acid for acetic acid. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-(2-THFcarbonyl)-Sar-Gly-Val-D-Ile-Thr-Gln-Ile-Arg-Pro-D-AlaNH₂ as the trifluoroacetate salt.

### Example 228

### N-(2-THFcarbonyl)-Sar-Gly-Val-D-alloIle-Thr-Gln-Ile-Arg-Pro-D-AlaNH₂

The procedure described in Example 227 is used but substituting Fmoc-D-allolle for Fmoc-D-Ile. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized-to yield N-(2-THFcarbonyl)-Sar-Gly-Val-D-alloIle-Thr-Gln-Ile-Arg-Pro-D-AlaNH2 as the trifluoroacetate salt.

### Example 229

### N-(2-THFcarbonyl)-Sar-Gly-Val-D-alloIle-Thr-Gln-Ile-Arg-ProNHCH₂(CH₃)₂

The procedure described in Example 4 is used but substituting Fmoc-D-allolle for Fmoc-D-Ile, Fmoc-Gln(Trt) for Fmoc-Nva and tetRahydro-2-furoic acid for acetic acid. After cleavage of the peptide from the resin and removal of the protecting groups using (9: 1) TFA/anisole (3 mL) the crude product is purified by C-18 column chromatography using a solvent mixture varying in a gradient of 10% to 50% acetonitrile-water containing 0.01 % TFA. The pure fractions are lyophilized to yield N-(2-THFcarbonyl)-Sar-Gly-Val-D-alloIle-Thr-Gin-Ile-Ara ProNHCH₂(CH₃)₂ as the trifluoroacetate salt.

### Example 230

The procedures described in Examples 224, 225, 226, 227, 228, and 229 are used but substituting N-acetyl-6-aminocaproic acid (6-Ac-Aca) instead of tetrahydro-2-furoyl. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01 % TFA. The pure fractions are lyophilized to yield the following peptides as trifluoroacetate salt:
N-(6-Ac-Aca)-Sar-Gly-Val-D-alloIle-Thr-Nva-Ile.-Arg-ProNHCH₂CH₃,
N-(6-Ac-Aca)-Sar-Gly-Val-D-Ile-Thr-Gln-Ile-Arg-ProNHCH₂CH₃,
N-(6-Ac-Aca)-Sar-Gly-Val-D-alloIle-Thr-Gln-Ile-Arg-ProNHCH₂CH₃,
N-(6-Ac-Aca)-Sar-Gly-Val-D-Ile-Thr-GIn-Ile-Arg-Pro-D-AlaNH₂,
N-(6-Ac-Aca)-Sar-Gly-Val-D-allolle-Thr-Gln-Ile-Arg-Pro-D-AIaNH₂, and
N-(6-Ac-Aca)-Sar-Gly-Val-D-alloIle-Thr-Gln-Ile-Arg-ProNHCH₂(CH₃)₂.

### Example 231

The procedures described in Examples 224, 225, 226, 227, 228, and 229 are used but substituting N-acetyl-4-aminobutyric acid (4-Ac-Gaba) instead of N-acetyl-6-aminocaproic acid. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield the following peptides as trifluoroacetate salt:
N-(4-Ac-Gaba)-Sar-Gly-Val-D-allolle-Thr-Nva-Ile-Arg-ProNHCH₂CH₃,
N-(4-Ac-Gaba)-Sar-Gly-Val-D-Ile-Thr-Gln-Ile-Arg-ProNHCH₂CH₃,
N-(4-Ac-Gaba)-Sar-Gly-Val-D-allolle-Thr-Gln-Ile-Arg-ProNHCH₂CH₃,
N-(4-Ac-Gaba)-Sar-Gly-Val-D-Ile-Thr-Gln-Ile-Arg-Pro-D-AlaNH₂,
N-(4-Ac-Gaba)-Sar-Gly-Val-D-alloIle-Thr-Gln-Ile-Arg-Pro-D-AlaNH₂, and
N-(4-Ac-Gaba)-Sar-Gly-Val-D-allolle-Thr-GIn-Ile-Arg-PrONHCH₂(CH₃)₂.

### Example 232

The procedures described in Examples 224, 225, 226, 227, 228, and 229 are used but substituting 2-furoic acid instead of tetrahydro-2-furoic acid. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield the following peptides as trifluoroacetate salt:
N-(2-Furoyl)-Sar-Gly-V al-D-alloIle-Thr-Nva-Ile-Arg-ProNHCH₂CH₃,
N-(2-Furoyl)-Sar-Gly-Val-D-Ile-Thr-Gln-Ile-Arg-ProNHCH₂CH₃,
N-(2-Furoyl)-Sar-Gly-Val-D-alIoIle-Thr-Gln-Ile-Arg-ProNHCH₂CH₃,
N-(2-Furoyl)-Sar-Gly-Val-D-Ile-Thr-Gln-Ile-Arg-Pro-D-AlaNH₂,
N -(2-Furoyl)-Sar-Gly-Val-D-alloIle-Thr-Gln-Ile-Arg-Pro-D-AlaNH₂, and
N-(2-Furoyl)-Sar-Gly-Val-D-alloIle-Thr-Gln-Ile-Arg-ProNHCH₂(CH₃)₂.

### Example 233

The procedures described in Examples 224, 225, 226, 227, 228, and 229 are used but substituting shikimic acid instead of tetrahydro-2-furoic acid. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield the following peptides as trifluoroacetate salt:
N-(Shikimyl)-Sar-Gly-Val-D-alloIle-Thr-Nva-Ile-Arg-ProNHCH₂CH₃,
N-(Shikimyl)-Sar-Gly-Val-D-Ile-Thr-Gln-Ile-Arg-ProNHCH₂CH₃,
N-(Shikimyl)-Sar-Gly-Val-D-alloIle-Thr-Gln-Ile-Arg-ProNHCH₂CH₃,
N-(Shikimyl)-Sar-Gly-Val-D-Ile-Thr-Gln-Ile-Arg-Pro-D-AlaNH₂,
N -(Shikimyl)-Sar-Gly-Val-D-alloIle-Thr-Gln-Ile-Arg-Pro-D-AlaNH₂, and
N-(Shikimyl)-Sar-Gly-Val-D-allolle-Thr-Gln-Ile-Arg-ProNHCH₂(CH₃)₂.

### Example 234

The procedures described in Examples 224, 225, 226, 227, 228, and 229 are used but substituting 2-methyl-nicotinic acid instead of tetrahydro-2-furoic acid. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield the following peptides:
N-(2-Me-Nicotinyl)-Sar-Gly-Val-D-alloIle-Thr-Nva-Ile-Arg-ProNHCH₂CH₃,
N-(2-Me-Nicotinyl)-Sar-Gly-Val-D-Ile-Thr-GIn-Ile-Arg-PrONHCH₂CH₃,
N-(2-Me-Nicotinyl)-Sar-Gly-Val-D-allolle-Thr-Gln-Ile-Arg-proNHCH₂CH3,
N-(2-Me-Nicotinyl)-Sar-Gly-Val-D-Ile-Thr-Gln-Ile-Arg-Pro-D-AlaNH₂,
N-(2-Me-Nicotinyl)-Sar-Gly-Val-D-allolle-Thr-Gln-Ile-Arg-Pro-D-AlaNH₂ and
N-(2-Me-Nicotinyl)-Sar-Gly-Val-D-allolle-Thr-Gln-Ile-Arg-PrONHCH₂(CH₃)₂.

### Example 235

### N-Ac-Sar-Gly-Val-D-allolle-Thr-Leu-Ile-Arg-Pro-D-AlaNH,

The procedure described in Example 75 is used but substituting Fmoc-D-allolle for Fmoc-D-Ile and Fmoc-Leu for Fmoc-Nva. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Ac-Sar-Gly-Val-D-allolle-Thr-Leu-Ile-Arg-Pro-D-AlaNH₂ as the trifluoroacetate salt.

### Example 236

### N-Ac-Sar-Gly-Val-D-Ile-Thr-Leu-Ile-Arg-ProNHCH₂(CH₃)₂

The procedure described in Example 4 is used but substituting Fmoc-Leu for Fmoc-Nva. After cleavage of the peptide from the resin and removal of the protecting groups using (9: 1) TFA/anisole (3 mL) the crude product is purified by C-18 column chromatography using a solvent mixture varying in a gradient of 10% to 50% acetonitrile-water containing 0.01 % TFA. The pure fractions are lyophilized to yield N-Ac-Sar-Gly-Val-D-Ile-Thr-Leu-Ile-Arg-ProNHCH₂(CH₃)₂ as the trifluoroacetate salt.

### Example 237

### N-Ac-Sar-Gly-V al-D-alloIle-Thr-Leu-Ile-Arg-ProNHCH₂CH₃

The procedure described in Example 73 is used but substituting Fmoc-D-alloIle for Fmoc-D-Ile. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01 % TFA. The pure fractions are lyophilized to yield N-Ac-Sar-Gly-Val-D-alloIle-Thr-Leu-Ile-Arg-ProNHCH₂CH₃ as the trifluoroacetate salt.

### Example 238

### N-Ac-Sar-Gly-Val-D-Ile-Thr-Leu-Ile-Arg-Pro-D-AlaNH₂

The procedure described in Example 75 is used but substituting Fmoc-Leu for Fmoc-Nva. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Ac-Sar-Gly-Val-D-Ile-Thr-Leu-He-Arg-Pro-D-AlaNH₂ as the trifluoroacetate salt.

### Example 239

### N-Succinyl-Sar-Gly-Val-D-Ile-Thr-Leu-Ile-Arg-Pro-D-AlaNH₂

The procedure described in Example 75 is used but substituting Fmoc-Leu for Fmoc-Nva and acylating with succinic anhydride after the coupling with Fmoc-Sar and deprotection as described in Example 54. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Succinyl-Sar-Gly-Val-D-Ile-Thr-Leu-Ile-Arg-Pro-D-AlaNH₂ as the trifluoroacetate salt.

### Example 240

### N-Succinyl-Sar-Gly-Val-D-Ile-Thr-Leu-Ile-Arg-ProNHCH₂(CH₃)₂

The procedure described in Example 206 is used but substituting Fmoc-Leu for Fmoc-Gln(Trt) and acylating with succinic anhydride after the coupling with Fmoc-Sar and deprotection as described in Example 54. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Succinyl-Sar-Gly-Val-D-Ile-Thr-Leu-Ile-Arg-ProNHCH₂(CH₃)₂ as the trifluoroacetate salt.

### Example 241

The procedures described in Examples 201, 202 and 203 are used but substituting Fmoc-Leu instead of Fmoc-Gln(Trt). After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield the following peptides:
N-Succinyl-Sar-Gly-Val-D-Ile-Thr-Leu-Ile-Arg-ProNHCH₂CH₃,
N-Succinyl-Sar-Gly-Val-D-alloIle-Thr-Leu-Ile-Arg-ProNHCH₂CH₃, and
N-Succinyl-Sar-Gly-Val-D-alloIle-Thr-Leu-Ile-Arg-Pro-D-AlaNH₂

### Example 242

### N-Succinyl-Sar-Gly-Val-D-Ile-Thr-Leu-Ile-Arg-Pro-AzaglyNH₂

The procedure described in Example 149 is used but substituting Fmoc-Leu for Fmoc-Nva and acylating with succinic anhydride after the coupling with Fmoc-Sar and deprotection as described in Example 54. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Succinyl-Sar-Gly-Val-D-Ile-Thr-Leu-Ile-Arg-Pro-AzaglyNH2 as the trifluoroacetate salt.

### Example 243

### N-Ac-Sar-Gly-Val-D-alloIle-Thr-Nva-Ile-Arg-ProNHethyl-(1 -pyrrolidine)

The procedure described in Example 5 is used but substituting Fmoc-D-alloIle for Fmoc-D-Ile. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.0 1 % TFA. The pure fractions are lyophilized to yield N-Ac-Sar-Gly-Val-D-alloIle-Thr-Nva-Ile-Arg-ProNHethyl-(1-pyrrolidine) as the trifluoroacetate salt.

### Example 244

### N-Ac-Sar-Gly-Val-D-alloIle-Thr-Nva-Ile-Arg-ProNH(ethyl-1 -cyclohexyl)

The procedure described in Example 8 is used but substituting Fmoc-D-alloIle for Fmoc-D-Ile. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Ac-Sar-Gly-Val-D-alloIle-Thr-Nva-Ile-Arg-ProNH(ethyl-1-cyclohexyl) as the trifluoroacetate salt.

### Example 245

### N-Ac-Sar-Gly-Val-D-Ile-Thr-Gln-Ile-Arg-ProNHethyl-(1-pyrrolidine)

The procedure described in Example 5 is used but substituting Fmoc-Gln(Trt) for Fmoc-Ivva. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01 % TFA. The pure fractions are lyophilized to yield N-Ac-Sar-Gly-Val-D-Ile-Thr-Gln-Ile-Arg-ProNHethyl-(1-pyrrolidine) as the trifluoroacetate salt.

### Example 246

### N-Ac-Sar-Gly-Val-D-Ile-Thr-Gln-Ile-Arg-ProNH(ethyl-1-cyclohexyl)

The procedure described in Example 8 is used but substituting Fmoc-Gln(Trt) for Fmoc-Nva. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.0 1 % TFA. The pure fractions are lyophilized to yield N-Ac-Sar-Gly-Val-D-Ile-Thr-Gln-Ile-Arg-ProNH(ethyl-1-cyclohexyl) as the trifluoroacetate salt.

### Example 247

### N-Succinyl-Sar-Gly-Val-D-Ile-Thr-Gln-Ile-Arg-ProNH(ethyl-1-cyclohexyl)

The procedure described in Example 246 is used but acylating the peptide resin with succinic anhydride after the coupling with Fmoc-Sar and deprotection as described in Example 54. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Succinyl-Sar-Gly-Val-D-Ile-Thr-Gln-Ile-Arg-ProNH(ethyl-1-cyclohexyl) as the trifluoroacetate salt.

### Example 248

The procedures described in Examples 11 is used but substituting the appropriate protected amino acids as described in Examples 14, 43, 74, 73, 54, 174, and 132 respectively. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01 % TFA. The pure fractions are lyophilized to yield the following peptides as trifluoroacetate salt:
N-Ac-Sar-Gly-Val-D-alloIle-Thr-Nva-Ile-Arg-ProNHCH₂CH₂OCH₃,
N-Ac-Sar-Gly-Val-D-Ile-Thr-Gln-Ile-Arg-ProNHCH₂CH₂OCH₃,
N-Ac-Sar-Gly-Val-D-Ile-Thr-Ser-Ile-Arg-ProNl4CH₂CH,OCH₃,
N-Ac-Sar-Gly-Val-D-Ile-Thr-Leu-Ile-Arg-ProNHCH₂CH₂OCH₃,
N-Succinyl-Sar-Gly-Val-D-Ile-Thr-Nva-Ile-Arg-ProNHCH₂CH₂OCH₃,
N-Succinyl-Sar-Gly-Val-D-Ile-Thr-Gln-Ile-Arg-ProNHCH₂CH₂OCH₃,
N-Succinyl-Sar-Gly-Val-D-allolle-Thr-GIn-Ile-Arg-PrONHCH₂CH₂OCH₃,
N-Ac-Sar-Gly-Val-D-Ile-Ser-Nva-Ile-Arg-ProNHCH₂CH₂OCH₃, and
N-Ac-Sar-Gly-Val-D-Leu-Ser-Nva-Ile-Arg-ProNHCH₂CH₂OCH₃,

### Example 249

The procedures described in Examples 49 is used but substituting the appropriate protected amino acids as described in Examples 14, 4, 75, 54 and 132 respectively. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield the following peptides as trifluoroacetate salt:
N -Ac-Sar-Gly-Val-D-alloIle-Thr-Allygly-Ile-Arg-ProNHCH₂CH₃,
N -Ac-Sar-Gly-Val-D-Ile-Thr-Allygly-Ile- Arg-ProNHCH₂(CH₃)₂,
N-Ac-Sar-Gly-Val-D-Ile-Thr-Allygly-Ile-Arg-Pro-D-AlakH₂,
N-Ac-Sar-Gly- Val- D-alloIle- Thr-Allygly-Ile-Arg-Pro-D-AlaNH₂,
N-Succinyl-Sar-Gly-Val-D-Ile-Thr-Allygly-Ile-Arg-Pro-D-AlaNH₂,
N-Ac-Sar-Gly-Val-D-Ile-Ser-Allygly-Ile-Arg-Pro-ProNHCH₂CH₃, and
N-Ac-Sar-Gly-Val-D-Leu-Ser-Allygly-Ile-Arg-Pro-ProNHCH₂CH₃.

### Example 250

### N-Ac-Sar-Gly-Val-D-Ile-Thr-Nva-Ile-Arg-Pro-D-SerNH₂

The procedure described in Example 75 is used but substituting Fmoc-D-Ser(tBu)-Sieber amide resin for Fmoc-D-Ala-Sieber amide resin. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Ac-Sar-Gly-Val-D-Ile-Thr-Nva-Ile-Arg-Pro-D-SerNH₂ as the trifluoroacetate salt.

### Example 251

### N-Ac-Sar-Gly-Val-D-Ile- Thr-Nva-Ile-Arg-ProNHOH

The procedure described in Example 149 is used but hydroxylamine hydrochloride for semicarbazide hydrochloride. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Ac-Sar-Gly-Val-D-Ile-Thr-Nva-Ile-Arg-ProNHOH as the trifluoroacetate salt.

### Example 252

### N-Ac-Sar-Gly-Val-D-Ile-Ser-Nva-Ile-Arg-ProNHCH₂CH₃

The procedure described in Example 132 is used but substituting Fmoc-D-Ile for Fmoc-D-Leu. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Ac-Sar-Gly-Val-D-Ile-Ser-Nva-Ile-Arg-ProNHCH₂CH₃ as the trifluoroacetate salt.

### Example 253

### N-Ac-Sar-Gly-Val-D-alloIle-Ser-Nva-Ile-Arg-ProNHCH₂CH₃

The procedure described in Example 132 is used but substituting Fmoc-D-alloIle for Fmoc-D-Leu. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01 % TFA. The pure fractions are lyophilized to yield N-Ac-Sar-Gly-Val-D-allolle-Ser-Nva-Ile-Arg-ProNHCH₂CH₃ as the trifluoroacetate salt.

### Example 254

### N-Ac-Sar-Gly-Val-D-Leu-Hser-Nva-Ile-Arg-ProNHCH₂CH₃

The procedure described in Example 132 is used but substituting Fmoc-Hser(tBu) for Fmoc-Ser(tBu). After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Ac-Sar-Gly-Val-D-Leu-Hser-Nva-Ile-Arg-ProNHCH₂CH₃ as the trifluoroacetate salt.

### Example 262

The procedures described in Example 46 is used but substituting the appropriate protected amino acids as describes in Examples 75, 4, 54, and 132. After cleavage of the peptide from the resin and removal of the protecting groups using (9: 1) TFA/anisole (3 mL) the crude product is purified by C-18 column chromatography using a solvent mixture varying in a gradient of 10% to 50% acetonitrile-water containing 0.0.1 % TFA: The pure fractions are lyophilized to yield the following peptides as trifluoroacetate salt:
N-Ac-Sar-Gly-Val-D-allolle-Thr-Ala-Ile-Arg-PrONHCH₂CH₃,
N-Ac-Sar-Gly-Val-D-lie-Thr-Ala-Ile-Arg-ProNHCH(CH₃)₂,
N-Ac-Sar-Gly-Val-D-Ile-Thr-Ala-Ile-Arg-Pro-D-AlaNH₂,
N-Ac-Sar-Gly-Va.D-allolle-Thr-Ala-IIe-Arg-Pro-D-AIaNH₂,
N-Succinyl-Sar-Gly-Val-D-lle-Thr-Ala-Ile-Arg-Pro-D-AlaNH₂,
N-Ac-Sar-Gly-Val-D-Ile-Ser-Ala-Ile-Arg-ProNHCH₂CH₃, and
N-Ac-Sar-Gly-Val-D-Leu-Ser-Ala-Ile-Arg-ProNHCH₂CH₃.

### Example 263

The procedures described in Example 262 is used but substituting Fmoc-Val for Fmoc-Ala. After cleavage of the peptide from the resin and removal of the protecting groups using (9:1) TFA/anisole (3 mL) the crude product is purified by C-18. column chromatography using a solvent mixture varying in a gradient of 10% to 50% acetonitrile-water containing 0.41% TFA. The pure fractions are lyophilized to yield the following peptides as trifluoroacetate salt:
N-Ac-Sar-Gly-Val-D-allolle-Thr-Val-Ile- Arg-ProNHCH₂CH₃,
N-Ac-Sar-Gly-Val-D-Ile-Thr-Val-Ile-Arg-ProNHCH₂(CH₃)₂,
N-Ac-Sar-Gly-Val-D-Ile-Thr-Val-Ile-Arg-Pro-D-AlaNH₂,
N-Ac-Sar-Gly-Val-D-allolle-Thr-Val-Ile-Arg-Pro-D-AlaNH₂,
N-Succinyl-Sar-Gly-Val-D-Ile-Thr-Val-Ile-Arg-Pro-D-AlaNH₂,
N-Ac-Sar-Gly-Val-D-Ile-Ser-Val-Ile-Arg-ProNECH₂CH₃, and
N-Ac-Sar-Gly- Val-D-Leu-Ser- Val-Ile-Arg-ProNHCH₂CH₃.

### Example 264

The procedures described in Example 263 is used but substituting Fmoc-DNva for Fmoc-Nva. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield the following peptides as trifluoroacetate salt:
N-Ac-Sar-Gly-Val-D-allolle-Thr-D-Nva-Ile-Arg-ProNHCH₂CH₃,
N-Ac-Sar-Gly-Val-D-Ile-Thr-D-Nva-Ile-Arg-ProNHCH₂(CH₃)₂,
N-Ac-Sar-Gly-Val-D-Ile-Thr-D-Nva-Ile-Arg-Pro-D-AlaNH₂,
N-Ac-Sar-Gly-Val-D-allolle-Thr-D-Nva-Ile-Arg-Pro-D-AlaNH₂,
N-Succinyl-Sar-Gly-Val-D-Ile-Thr-D-Nva-Ile-Arg-Pro-D-AlaNH₂,
N-Ac-Sar-Gly-Val-D-Ile-Ser-D-Nva-Ile-Arg-Pro-ProNHCH₂CH₃, and
N-Ac-Sar-Gly-Val-D-Leu-Ser-D-Nva-Ile-Arg-Pro-ProNHCH₂CH₃.

### Example 265

### N-Ac-Sar-Gly-Val-D-Ile-Ser-Gln-Ile-Arg-ProNHCH₂CH₃

The procedure described in Example 132 is used but substituting Fmoc-D-Ile for Fmoc-D-Leu and Fmoc-Gln(Trt) for Fmoc-Nva. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01 % TFA. The pure fractions are lyophilized to yield N-Ac-Sar-Gly-Val-D-Ile-Ser-Gln-Ile-Arg-ProNHCH₂CH₃ as the trifluoroacetate salt.

### Example 266

### N-Ac-Sar-Gly-V al-D-Leu-Ser-Gln-Ile-Arg-ProNHCH₂CH₃

The procedure described in Example 132 is used but substituting Fmoc-Gln(Trt) for Fmoc-Nva. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01 % TFA. The pure fractions are lyophilized to yield N-Ac-Sar-Gly-Val-D-Leu-Ser-Gln-Ile-Arg-ProNHCH₂CH₃ as the trifluoroacetate salt.

### Example 267

### N-Ac-Sar-Gly-Val-D-Leu-Ser-Nva-Ile-Arg-Pro-D-AlaNH₂

The procedure described in Example 75 is used but substituting Fmoc-D-Leu for Fmoc-D-Ile and Fmoc-Ser(tBu) for Fmoc-Thr(tBu). After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Ac-Sar-Gly-Val-D-Leu-Ser-Nva-Ile-Arg-Pro-D-AlaNH₂ as the trifluoroacetate salt.

### Example 268.

### N-Ac-Sar-Gly-Val-D-Ile-Ser-Nva-Ile-Arg-Pro-D-AlaNH₂

The procedure described in Example 267 is used but substituting Fmoc-D-Ile for Fmoc-D-Leu and Fmoc-Ser(tBu) for Fmoc-Thr(tBu). After cleavage of the peptide from the resin and removal of the protecting groups using (9: 1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Ac-Sar-Gly-Val-D-Ile-Ser-Nva-Ile-Arg-Pro-D-AlaNH₂ as the trifluoroacetate salt.

### Example 269

### N-Succinyl-Sar-Gly-Val-D-Leu-Ser-Nva-Ile-Arg-PrONHCH₂CH₃

The procedure described in Example 54 is used but substituting Fmoc-D-Leu for Fmoc-D-Ile and Fmoc-Ser(tBu) for Fmoc-Thr(tBu). After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Succinyl-Sar-Gly- Val-D-Leu-Ser-Nva-Ile-Arg-ProNHCH₂CH₃ as the trifluoroacetate salt.

### Example 270

### N-Succinyl-Sar-Gly-Val-D-Ile-Ser-Nva-Ile-Arg-ProNHCH₂CH₃

The procedure described in Example 269 is used but substituting Fmoc-D-Ile for Fmoc-D-Leu. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Succinyl-Sar-Gly-Val-D-Ile-Ser-Nva-Ile-Arg-ProNHCH₂CH₃ as the trifluoroacetate salt.

### Example 271

### N-Succinyl-Sar-Gly-Val-D-Leu-Ser-Gln-Ile-Arg-ProNHCH₂CH₃

The procedure described in Example 270 is used but substituting Fmoc-D-Leu for Fmoc-D-Ile and Fmoc-Gln(Trt) for Fmoc-Nva. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01 % TFA. The pure fractions are lyophilized to yield N-Succinyl-Sar-Gly-Val-D-Leu-Ser-Gln-Ile-Arg-ProNHCH₂CH₃ as the trifluoroacetate salt.

### Example 272

### N-Succinyl-Sar-Gly-Val-D-Ile-Ser-Gln-Ile-Arg-ProNHCH₂CH₃

The procedure described in Example 270 is used but substituting Fmoc-Gln(Trt) for Fmoc-Nva. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01 % TFA. The pure fractions are lyophilized to yield N-Succinyl-Sar-Gly-Val-D-Ile-Ser-Gln-Ile-Arg-ProNHCH₂CH₃ as the trifluoroacetate salt.

### Example 273

### N-Ac-Sar-Gly-Val-D-Ile-Ser-Ser-Ile-Arg-ProNHCH₂CH₃

The procedure described in Example 265 is used but substituting Fmoc-Ser(tBu) for Fmoc-Nva. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Ac-Sar-Gly-Val-D-Ile-Ser-Ser-Ile-Arg-ProNHCH₂CH₃ as the trifluoroacetate salt.

### Example 274

### N-Ac-Sar-GIy-Val-D-Leu-Ser-Ser-Ile-Arg-ProNHCH₂CH₃

The procedure described in Example 266 is used but substituting Fmoc-Ser(tBu) for Fmoc-Nva. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Ac-Sar-Gly-Val-D-Leu-Ser-Ser-Ile-Arg-ProNHCH₂CH₃ as the trifluoroacetate salt.

### Example 275

### N-Ac-Sar-Gly-Val-D-Leu-Ser-Nva-Ile-Arg-ProNHCH₂(CH₃)₂

The procedure described in Example 13 is used but substituting Fmoc-D-Leu for Fmoc-D-Ile and Fmoc-Ser(tBu) for Fmoc-Thr(tBu). After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Ac-Sar-Gly-Val-D-Leu-Ser-Nva-Ile-Arg-ProNHCH₂(CH₃)₂ as the trifluoroacetate salt.

### Example 276

### N-Ac-Sar-Gly-Val-D-Ile-Ser-Nva-Ile-Arg-ProNHCH₂(CH₃)₂

The procedure described in Example 13 is used but substituting Fmoc-Ser(tBu) for Fmoc-Thr(tBu). After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Ac-Sar-Gly-Val-D-Ile-Ser-Nva-Ile-Arg-ProNHCH₂(CH₃)₂ as the trifluoroacetate salt.

### Example 277

### N-Ac-Sar-Gly-V al-D-Leu-Ser-Leu-Ile-Arg-ProNHCH₂CH₃

The procedure described in Example 132 is used but substituting Fmoc-Leu for Fmoc-Nva. After cleavage of the peptide from the resin and removal of the protecting groups using (9;1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Ac-Sar-Gly-Val-D-Leu-Ser-Leu-Ile-Arg-ProNHCH₂CH₃ as the trifluoroacetate salt.

### Example 278

### N-Ac-Sar-Gly-Val-D-Ile-Ser-Leu-Ile-Arg-ProNHCH₂CH₃

The procedure described in Example 277 is used but substituting Fmoc-D-Ile for Fmoc-D-Leu. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01 % TFA. The pure fractions are lyophilized to yield N-Ac-Sar-Gly-Val-D-Ile-Ser-Leu-Ile-Arg-ProNHCH₂CH₃ as the trifluoroacetate salt.

### Example 279

### N-Ac-Sar-Gly-Val-D-alloIle-Ser-Nva-Ile-Arg-ProNHCH₂CH₃

The procedure described in Example 132 is used but substituting Fmoc-D-alloIle for Fmoc-D-Leu. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Ac-Sar-Gly-Val-D-allolle-Ser-Nva-Ile-Arg-ProNHCH₂CH₃ as the trifluoroacetate salt.

### Example 280

### N-Ac-Sar-Gly-Val-D-alloIle-Ser-Gln-Ile-Arg-ProNHCH₂CH₃

The procedure described in Example 265 is used but substituting Fmoc-D-allolle for Fmoc-D-Ile. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Ac-Sar-Gly-Val-D-alloIle-Ser-Gln-Ile-Arg-ProNHCH₂CH₃ as the trifluoroacetate salt.

### Example 281

### N-Succinyl-Sar-Gly-Val-D-alloIle-Ser-Nva-Ile-Arg-ProNHCH₂CH₃

The procedure described in Example 270 is used but substituting Fmoc-D-alloIle for Fmoc-D-Ile. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Succinyl-Sar-Gly-Val-D-alloIle-Ser-Nva-Ile-Arg-ProNHCH₂CH₃ as the trifluoroacetate.

### Example 282

### N-Ac-Sar-Gly-Val-D-allolle-Ser-Nva-Ile-Arg-ProNHCH₂(CH₃)₂

The procedure described in Example 276 is used but substituting Fmoc-D-allolle for Fmoc-D-Ile. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01 % TFA. The pure fractions are lyophilized to yield N-Ac-Sar-Gly-Val-D-alloIle-Ser-Nva-Ile-Arg-ProNHCH₂(CH₃)₃ as the trifluoroacetate.

### Example 283

### N-Ac-Sar-Gly-Val-D-alloIle-Ser-Nva-Ile-Arg-Pro-D-AlaNH₂

The procedure described in Example 268 is used but substituting Fmoc-D-alloIle for Fmoc-D-Ile. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to N-Ac-Sar-Gly-Val-D-alloIle-Ser-Nva-Ile-Arg-Pro-D-AlaNH₂, as the trifluoroacetate.

### Example 284

### N-Ac-Sar-Gly-V al-D-alloIle-Ser-Leu-Ile-Arg-ProNHCH₂CH₃

The procedure described in Example 265 is used but substituting Fmoc-D-alloIle for Fmoc-D-Ile and Fmoc-Leu for Fmoc-Gln(Trt). After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Ac-Sar-Gly-Val-D-allolle-Ser-Leu-Ile-Arg-PrONHCH₂CH₃ as the trifluoroacetate.

### Example 285

### N-Ac-Sar-G!y-VaI-D-aIloIIe-Ser-Ser-IIe-Arg-ProNHCH₂CH₃

The procedure described in Example 276 is used but substituting Fmoc-D-allolle for Fmoc-D-Ile. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Ac-Sar-Gly-Val-D-alloIle-Ser-Ser-Ile-Arg-ProNHCH₂CH₃ as the trifluoroacetate.

### Example 286

The procedure described in Example 125 is used but separately substituting Fmoc-D-Ile and Fmoc-D-alloIle, respectively, for Fmoc-D-Leu. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to the the following peptides as trifluoroacetate salt:
N-Ac-Sar-Gly-Val-D-Ile-Gly-Nva-Ile-Arg-ProNHCH₂CH₃ and
N-Ac-Sar-Gly-V al-D-alloIle-Gly-Nva-Ile-Arg-ProNHCH₂CH₃.

### Example 287

The procedure described in Example 125 and 286 is used but separately substituting Fmoc-D-Ile and Fmoc-D-alloIle, respectively, for Fmoc-D-Leu and substituting Fmoc-Gln(Trt) for Fmoc-Nva. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield the trifluoroacetate salt of:
N-Ac-Sar-Gly-Val-D-Leu-Gly-Gln-Ile-Arg-ProNHCH₂CH₃,
N-Ac-Sar-Gly-Val-D-Ile-Gly-Gln-Ile-Arg-ProNHCH₂CH₃, and
N-Ac-Sar-Gly-Val-D-alloIle-Gly-Gln-Ile-Arg-ProNHCH₂CH₃.

### Example 288

The procedure described in Example 123 is used but separately substituting Fmoc-D-Ile and Fmoc-D-allolle, respectively, for Fmoc-D-Leu. After cleavage of the peptide from the resin and removal of the protecting groups using (9: 1) TFA/anisole (3 mL) the crude product is purified by C-18 column chromatography using a solvent mixture varying in a gradient of 10% to 50% acetonitrile-water containing 0.01% TFA. The pure fractions are lyophilized to yield the trifluoroacetate salt of:
N-Ac-Sar-Gly-Val-D-Ile-Tyr-Nva-Ile-Arg-ProNHCH₂CH₃ and
N-Ac-Sar-Gly-Val-D-allolle-Tyr-Nva-Ile-Arg-PrONHCH₂CH₃.

### Example 289

The procedure described in Example 123 and 288 is used but separately substituting Emoc-D-Ile and Fmoc-D-allolle, respectively, for Fmoc-D-Leu and substituting Fmoc-Gln(Trt) for Fmoc-Nva . After cleavage of the peptide from the resin and removal of the protecting groups using (9:1) TFA/anisole (3 mL) the crude product is purified by C-18 column chromatography using a solvent mixture varying in a gradient of . 10% to 50% acetonitrile-water containing 0.01% TFA. The pure fractions are lyophilized to yield the trifluoroacetate salt of:
N-Ac-Sar-Gly-Val-D-Leu-Tyr-Gln-Ile-Arg-ProNHCH₂CH₃,
N-Ac-Sar-Gly-Val-D-Ile-Tyr-GIn-Ile-Arg-PrONHCH₂CH₃, and
N-Ac-Sar-Gly-Val-D-alloIle-Tyr-Gln-Ile-Arg-ProNHCH₂CH₃.

### Example 290

### N-Ac-Sar-Gly-Val-D-Ser-Thr-Nva-Ile-Arg-ProNHCH₂CH₃

The procedure described in Example 1 is used but substituting Fmoc-D-Ser(tBu) for Fmoc-D-Ile. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Ac-Sar-Gly-Val-D-Ser-Thr-Nva-Ile-Arg-ProNHCH₂CH₃ as the trifluoroacetate.

### Example 291

### N-Ac-Sar-Gly-Val-D-Thr-Thr-Nva-Ile-Arg-ProNHCH₂CH₃

The procedure described in Example 1 is used but substituting Fmoc-D-Thr(tBu) for Fmoc-D-Ile. After cleavage of the peptide from the resin and removal of the protecting groups using (9: 1) TFA/anisole (3 mL) the crude product is purified by C-18 column chromatography using a solvent mixture varying in a gradient of 10% to 50% acetonitrile-water containing 0.01 % TFA. The pure fractions are lyophilized to yield N-Ac-Sar-Gly-Val-D-Thr-Thr-Nva-Ile-Arg-ProNHCH₂CH₃ as the trifluoroacetate.

### Example 292

### N-Ac-Sar-Gly-Val-D-Gln-Thr-Nva-Ile-Arg-ProNHCH₂CH₃

The procedure described in Example 1 is used but substituting Fmoc-D-Gln(Trt) for Fmoc-D-Ile. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01 % TFA. The pure fractions are lyophilized to yield N-Ac-Sar-Gly-Val-D-Gln-Thr-Nva-Ile-Arg-ProNHCH₂CH₃ as the trifluoroacetate.

### Example 293

### N-Ac-Sar-Gly-Va1-D-Asn-Thr-Nva-Ile-Arg-ProNHCH₂CH₃

The procedure described in Example 1 is used but substituting Fmoc-D-Asn(Trt) for Fmoc-D-Ile. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Ac-Sar-Gly-Val-D-Asn-Thr-Nva-Ile-Arg-ProNHCH₂CH₃ as the trifluoroacetate.

### Example 294

### N-Ac-Sar-Gly-Val-D-Arg-Thr-Nva-Ile-Arg-ProNHCH₂CH₃

The procedure described in Example 1 is used but substituting Fmoc-D-Arg(Pmc) for Fmoc-D-Ile. After cleavage of the peptide from the resin and removal of the protecting groups using (9: 1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Ac-Sar-Gly-Val-D-Arg-Thr-Nva-Ile-Arg-ProNHCH₂CH₃ as the trifluoroacetate.

### Example 295

### N-Ac-Sar-Gly-Val-D-3-Pal-Thr-Nva-Ile-Arg-ProNHCH₂CH₃

The procedure described in Example 1 is used but substituting Fmoc-D-3-Pal for Fmoc-D-Ile. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Ac-Sar-Gly-Val-D-3-Pal-Thr-Nva-lle-Arg-Pr_{O}NHCH₂CH₃ as the trifluoroacetate.

### Example 296

### N-Ac-Sar-Gly-Val-D-Glu-Thr-Nva-Ile-Arg-ProNHCH₂CH₃

The procedure described in Example 1 is used but substituting Fmoc-D-Glu(OtBu)-OH for Fmoc-D-Ile. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Ac-Sar-Gly-Val-D-Glu-Thr-Nva-Ile-Arg-ProNHCH₂CH₃ as the trifluoroacetate.

### Example 297

### N-Ac-Sar-Gly-Val-D-Asp-Thr-Nva-Ile-Arg-PrONHCH₂CH₃

The procedure described in Example 1 is used but substituting Fmoc-D-Asp(OtBu)-OH for Fmoc-D-Ile. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Ac-Sar-Gly-Val-D-Asp-Thr-Nva-Ile-Arg-ProNHCH₂CH₃ as the trifluoroacetate.

### Example 298

### N-Ac-Sar-Gly-Val-D-His-Thr-Nva-Ile-Arg-ProNHCH₂CH₃

The procedure described in Example 1 is used but substituting Fmoc-D-His(Boc)-OH for Fmoc-D-Ile. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Ac-Sar-Gly-Val-D-His-Thr-Nva-Ile-Arg-ProNHCH₂CH₃ as the trifluoroacetate.

### Example 299

### N-Ac-Sar-Gly-Val-D-Hser-Thr-Nva-Ile-Arg-ProNHCH₂CH₃

The procedure described in Example 1 is used but substituting Fmoc-D-Hser(tBu) for Fmoc-D-Ile. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01 % TFA. The pure fractions are lyophilized to yield N-Ac-Sar-Gly-Val-D-Hser-Thr-Nva-Ile-Arg-ProNHCH₂CH₃ as the trifluoroacetate.

### Example 300

### N-Ac-Sar-Gly-Val-D-alloThr-Thr-Nva-Ile-Arg-ProNHCH₂CH₃

The procedure described in Example 1 is used but substituting Fmoc-D-alloThr(tBu) for Fmoc-D-Ile. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Ac-Sar-Gly-Val-D-alloThr-Thr-Nva-Ile-Arg-ProNHCH₂CH₃ as the trifluoroacetate.

### Example 301 1

### N-Ac-Sar-Gly-Val-D-Ile-Thr-Nva-D-Ile-Arg-ProNHCH₂CH₃

The procedure described in Example 1 is used but substituting Fmoc-D-Ile for Fmoc-Ile. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Ac-Sar-Gly-Val-D-Ile-Thr-Nva-D-Ile-Arg-ProNHCH₂CH₃ as the trifluoroacetate.

### Example 302

### N-Ac-Sar-Gly-Val-D-Ser-Thr-Gln-Ile-Arg-ProNHCH₂CH₃

The procedure described in Example 290 is used but substituting Fmoc-Gln(Trt) for Fmoc-Nva. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Ac-Sar-Gly-Val-D-Ser-Thr-Gln-Ile-Arg-ProNHCH₂CH₃ as the trifluoroacetate.

### Example 303

### N-Ac-Sar-Gly-Val-D-Thr-Thr-Gln-Ile-Arg-ProNHCH₂CH₃

The procedure described in Example 291 is used but substituting Fmoc-Gln(Trt) for Fmoc-Nva. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Ac-Sar-Gly-Val-D-Thr-Thr-Gin-Ile-Arg-ProNHCH₂CH₃ as the trifluoroacetate.

### Example 304

### N-Ac-Sar-Gly-Val-D-alloThr-Thr-Gln-Ile-Arg-ProNHCH₂CH₃

The procedure described in Example 300 is used but substituting Fmoc-Gln(Trt) for Fmoc-Nva. After cleavage of the peptide from the resin and removal of the protecting groups using (9:1) TFA/anisole (3 mL) the crude product is purified by C-1 8 column chromatography using a solvent mixture varying in a gradient of 10% to 50% acetonitrile-water containing 0.01% TFA. The pure fractions are lyophilized to yield N-Ac-Sar-Gly-V al-D-alloThr- Thr-Gln-Ile-Arg-ProNHCH₂CH₃ as the trifluoroacetate.

### Example 305

N-Ac-Sar-Gly-Val-D-Ser-Ser-Nva-Ile-Arg-ProNHCH₂CH₃.

The procedure described in Example 290 is used but substituting Fmoc-Ser(tBu) for Fmoc-Thr(tBu). After cleavage of the peptide from the resin and removal of the protecting groups using (9: 1) TFA/anisole (3 mL) the crude product is purified by C-18 column chromatography using a solvent mixture varying in a gradient of 10% to 50% acetonitrile-water containing 0.01% TFA. The pure fractions are lyophilized to N-Ac-Sar-Gly-Val-D-Ser-Ser-Nva-Ile-Arg-ProNHCH₂CH₃ as the trifluoroacetate.

### Example 306

### N-Ac-Sar-Gly-V al-D-Thr-Ser-Nva-Ile-Arg-ProNHCH₂CH₃

The procedure described in Example 291 is used but substituting Fmoc-Ser(tBu) for Fmoc-Thr(tBu). After cleavage of the peptide from the resin and removal of the protecting groups using (9:1) TFA/anisole (3 mL) the crude product is purified by C- 18 column chromatography using a solvent mixture varying in a gradient of 10% to 50% acetonitrile-water containing 0.01% TFA. The pure fractions are lyophilized to yield N-Ac-Sar-Gly-Val-D-Thr-Ser-Nva-Ile-Arg-ProNHCH₂CH₃ as the trifluoroacetate.

### Example 307

### N-Ac-Sar-Gly-Val-D-alloThr-Ser-Nva-Ile-Arg-ProNHCH₂CH₃

The procedure described in Example 300 is used but substituting Fmoc-Ser(tBu) for Fmoc-Thr(tBu). After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Ac-Sar-Gly-Val-D-alloThr-Ser-Nva-Ile-Arg-ProNHCH₂CH₃ as the trifluoroacetate.

### Example 308

### N-Ac-Sar-Gly-Val-D-alloThr-Ser-Gln-Ile-Arg-ProNHCH₂CH₃

The procedure described in Example 304 is used but substituting Fmoc-Gln(Trt) for Fmoc-Nva. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01 % TFA. The pure fractions are lyophilized to yield N-Ac-Sar-Gly-Val-D-alloThr-Ser-Gln-Ile-Arg-ProNHCH₂CH₃ as the trifluoroacetate.

### Example 309

### N-Ac-Sar-Gly-Val-D-Thr-Ser-Gln-Ile-Arg-ProNHCH₂CH₃

The procedure described in Example 303 is used but substituting Fmoc-Gln(Trt) for Fmoc-Nva. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01 % TFA. The pure fractions are lyophilized to yield N-Ac-Sar-Gly-Val-D-Thr-Ser-Gln-Ile-Arg-ProNHCH₂CH₃ as the trifluoroacetate.

### Example 310

The procedure described in Examples 132 and 266 is used but substituting N-acetyl-6-aminocaproic acid (6-Ac-Aca) for acetic acid. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield the following peptides as trifluoroacetate salt:
N-(6-Ac-Aca)-Sar-Gly-Val-D-Leu-Ser-Gln-Ile-Arg-ProN'HCH₂(CH₃)₂, and
N-(6-Ac-Aca)-Sar-Gly-Val-D-Leu-Ser-Nva-Ile-Arg-ProNHCH₂(CH₃)₂.

### Example 311

The procedure described in Examples 310 is used but substituting N-acetyl-gamma-aminobutyric acid (4-Ac-Gaba) instead of N-acetyl-6-aminocaproic acid. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield the following peptides as trifluoroacetate salt:
N-(4-Ac-Gaba)-Sar-Gly-Val-D-Leu-Ser-Gln-Ile-Arg-ProNHCH₂(CH₃)₂, and
N-(4-Ac-Gaba)-Sar-Gly-Val-D-Leu-Ser-Nva-Ile-Arg-ProNHCH₂(CH₃)₂.

### Example 312

The procedure described in Examples 311 is used but substituting 2-furoic acid instead of N-acetyl-gamma-aminobutyric acid. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield the following peptides as trifluoroacetate salt:
N-(2-Furoyl)-Sar-Gly-Val-D-Leu-Ser-Gln-lie-Arg-ProNHCH₂(CH₃)₂, and
N-(2-Furoyl)-Sar-Gly-V al-D-Leu-Ser-Nva-Ile-Arg-ProNHCH₂(CH₃)₂.

### Example 313

The procedure described in Examples 311 is used but substituting shikimic acid instead of 2-furoic acid. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield the following peptides as trifluoroacetate salt:
N-(Shikimyl)-Sar-Gly-Val-D-Leu-Ser-Gln-Ile-Arg-ProNHCH₂(CH₃)₂, and
N-(Shikimyl)-Sar-Gly-Val-D-Leu-Ser-Nva-Ile-Arg-ProNHCH₂(CH₃)₂.

### Example 314

The procedure described in Examples 311 is substituting shikimic acid instead of 2-furoic acid. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield the following peptides as trifluoroacetate salt:
N-(Shikimyl)-Sar-Gly-Val-D-Leu-Ser-Gln-Ile-Arg-ProNHCH₂(CH₃)₂, and
N-(Shikimyl)-Sar-Gly-Val-D-Leu-Ser-Nva-Ile-Arg-ProNHCH₂(CH₃)₂.

### Example 315

The procedure described in Examples 312 is used but substituting 2-methyl-nicotinic acid instead of 2-furoic acid. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield the following peptides as trifluoroacetate salt:
N-(2-Me-nicotinyl)-Sar-Gly-Val-D-Leu-Ser-Gln-Ile-Arg- ProNHCH₂(CH₃)₂, and
N-(2-Me-nicotinyl)-Sar-Gly-Val-D-Leu-Ser-Nva-Ile-Arg-ProNHCH₂(CH₃)₂.

### Example 316

### N-Ac-Sar-Gly-Val-D-Leu-Ser-Nva-Ile-Arg-ProNHethyl-l-(R)-cyc1ohexyl

The procedure described in Example 8 is used but substituting Fmoc-DLeu for Fmoc-DIIe and Fmoc-Ser(tBu) for Fmoc-Thr(tBu). After cleavage of the peptide from the resin and removal of the protecting groups using (9: 1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Ac-Sar-Gly-Val-D-Leu-Ser-Nva-Ile-Arg-ProNHethyl-1-(R)-cyclohexyl as the trifluoroacetate.

### Example 317

### N-Ac-Sar-Gly-Val-DIIe-Thr-Ser-Ile-Arg-ProNHethyl-1-(R)-cyclohexyl

The procedure described in Example 8 is used but substituting Fmoc-Ser(tBu) for Fmoc-Nva. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Ac-Sar-Gly-Val-Dlle-Thr-Ser-Ile-Are-ProNHethyl-1-(R)-cyclohexyl as the trifluoroacetate.

### Example 318

### N-Ac-Sar-Gly-Val-DIle-Thr-Leu-Ile-Arg-ProNHethyl-1-(R)-cyclohexyl

The procedure described in Example 8 is used but substituting Fmoc-Leu for Fmoc-Nva. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Ac-Sar-Gly-Val-DIle-Thr-Leu-Ile-Arg-ProNHethyl-1-(R)-cyclohexyl as the trifluoroacetate.

### Example 319

### N-Ac-Sar-Gly-Val-D-Leu-Thr-Nva-Ile-Arg-ProNHethyl-1-(R)-cyclohexyl

The procedure described in Example 8 is used but substituting Fmoc-D-Leu for Fmoc-DIIe. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Ac-Sar-Gly-Val-D-Leu-Thr-Nva-Ile-Arg-ProNHethyl-1-(R)-cyclohexyl as the trifluoroacetate.

### Example 320

### N-Ac-Sar-Gly-Val-D-Leu-Ser-Ser-Ile-Arg-ProNHethyl-1-(R)-cyclohexyl

The procedure described in Example 316 is used but substituting Fmoc-Ser(tBu) for Fmoc-Nva. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01 % TFA. The pure fractions are lyophilized to yield N-Ac-Sar-Gly-Val-D-Leu-Ser-Ser-Ile-Arg-ProNHethyl-1-(R)-cyclohexyl as the trifluoroacetate.

### Example 321

### N-Ac-Sar-Gly-Val-D-Leu-Ser-Gln-Ile-Arg-ProNHethyl-1-(R)-cyclohexyl

The procedure described in Example 316 is used but substituting Fmoc-Gln(Trt) for Fmoc-Nva. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-Ac-Sar-Gly-Val-D-Leu-Ser-Gln-Ile-Arg-ProNHethyl-1-(R)-cyclohexyl as the trifluoroacetate.

### Example 322

### N-Ac-Sar-Gly-Val-Dlle-Thr-Nva-Ile-Arg-ProNHethyl-1-(S)-cyclohexyl

The procedure described in Example 8 is used but substituting (S)-1-cycloxylethylamine for (R)-1-cycloxylethylamine. After cleavage of the peptide from the resin and removal of the protecting groups the crude product was purified by C-18 column chromatography using solvent mixture varying in a gradient of 10% to 50% acetonitrile-water containing 0.01% TFA. The pure fractions were lyophilized to yield N-Ac-Sar-Gly-Val-Dlle-Thr-Nva-Ile-Arg-ProNHethyl-1-(S)-cyclohexyl as the trifluoroacetate salt.

### Example 323

The procedures described in Example 98 is used but substituting the appropriate protected amino acids as describes in Examples 132, 43, 54, and 75. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield the following peptides as trifluoroacetate salt:
N -Ac-Sar-Gly- Val-D- Pen-Ser-Nva-Ile-Arg-ProNHCH₂CH₃,
N-Ac-Sar-Gly-Val-D-Pen-Gly-Nva-Ile-Arg-ProNHCH₂CH₃,
N-Ac-Sar-Gly-Val-D-Pen-Thr-Gln-Ile-Arg-ProNHCH₂CH₃,
N-Ac-Sar-Gly-Val-D-Pen-Ser-Nva-Ile-Arg-ProNHCH₂(CH₃)2,
N-Succinyl-Sar-Gly-Val-D-Pen-Ser-Nva-Ile-Arg-ProNHCH₂CH₃,
N-Ac-Sar-Gly-Val-D-Pen-Ser-Nva-Ile-Arg-Pro-D-AlaNH₂,
N-Ac-Sar-Gly-V al-D-Pen-Ser-Gln-Ile-Arg-ProNHCH₂CH₃,
N-Ac-Sar-Gly-Val-D-Pen-Gly-Gln-Ile-Arg-ProNHCH₂CH₃,
N-Ac-Sar-Gly-Val-D-Pen-Ser-Ser-Ile-Arg-ProNHCH₂CH₃,
N-Ac-Sar-G!y-Val-0-Pen-Thr-Ser-He-Arg-ProNHCH₂CH₃,
N-Ac-Sar-Gly-Val-D-Pen-Thr-Leu-Ile-Arg-ProNHC H₂CH₃,
N-Ac-Sar-Gly-Val-D-Pen-Ser-Leu-Ile-Arg-ProNHCH₂CH₃,
N-Succinyl-Sar-Gly-Val-D-Pen-Ser-Ser-Ile-Arg-ProNHCH₂CH₃,
N-Succinyl-Sar-Gly-Val-D-Pen-Ser-Leu-Ile-Arg-ProNHCH₂CH₃, and
N-Succinyl-Sar-Gly-Val-D-Pen-Thr-Gln-Ile-Arg-ProNHCH₂(CH₃)₂.

### Example 324

### N-Ac-Sar-Gly-Val-D-Cys-Thr-Nva-Ile-Arg-ProNHCH₂CH₃

The procedure described in Example 98 is used but substituting Fmoc-D-Cys(Trt) for Fmoc-D-Pen(Trt). After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield N-N-Ac-Sar-Gly-Val-D-Cys-Thr-Nva-Ile-Arg-ProNHCH₂CH₃ as the trifluoroacetate.

### Example 325

The procedures described in Example 324 is used but substituting the appropriate protected amino acids as describes in Examples 132, 43, 54, and 75. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01 % TFA. The pure fractions are lyophilized to yield the following peptides as trifluoroacetate salt:
N-Ac-Sar-Gly-Val-D-Cys-Ser-Nva-Ile-Arg-ProNHCH₂CH₃,
N-Ac-Sar-Gly-Val-D-Cys-Gly-Nva-Ile-Arg-ProNHCH₂CH₃,
N-Ac-Sar-GIy-Val- D-Cys-Thr-Gln-Ile-Arg-ProNHCH₂CH₃,
N-Ac-Sar-Gly-Val-D-Cys-Ser-Nva-Ile-Arg-ProNHCH₂(CH₃)₂,
N-Succinyl-Sar-Gly-Val-D-Cys-Ser-Nva-Ile-Arg-ProNHCH₂CH₃,
N-Ac-Sar-Gly-Val-D-Cys-Ser-Nva-Ile-Arg-Pro-D-AlaNH₂,
N-Ac-Sar-Gly-Val-D-Cys-Ser-Gln-Ile-Arg-ProNHCH₂CH₃,
N-Ac-Sar-Gly-Val-D-Cys-Gly-Gln-Ile-Arg-ProNHCH₂CH₃
N-Ac-Sar-Gly-Val-D-Cys-Ser-Ser-Ile-Arg-ProNHCH₂CH₃,
N-Ac-Sar-Gly-Val-D-Cys-Thr-Ser-Ile-Arg-ProNHCH₂CH₃,
N-Ac-Sar-Gly-Val-D-Cys-Thr-Leu-Ile-Arg-ProNHCH₂CH₃,
N-Ac-Sar-Gly- Val- D-Cys-Ser-Leu-Ile-Arg-ProNHCH₂CH₃,
N-Succinyl-Sar-Gly-Val-D-Cys-Ser-Ser-Ile-Arg-ProNHCH₂CH₃, and
N-Succinyl-Sar-Gly-Val-D-Cys-Ser-Leu-Ile-Arg-ProNHCH₂CH₃,

### Example 329

### N-Ac-Sar-Gly-Val-D-Leu-Pen-Nva-Ile-Arg-ProNHCH₂CH₃

The procedure described in Example 120 is used but substituting Fmoc-Pen(Trt) for Fmoc-Ala. After cleavage of the peptide from the resin and removal of the protecting groups the crude product was purified by C-18 column chromatography using solvent mixture varying in.a gradient of 10% to 50% acetonitrile-water containing 0.01 % TFA. The pure fractions were lyophilized to yield N-Ac-Sar-Gly-Val-D-Leu-Pen-Nva-Ile-Arg-ProNHCH₂CH₃ as the trifluoroacetate salt.

### Example 330

The procedures described in Example 329 is used but substituting the appropriate protected amino acids as describes in Examples 14, 15,132,43, 54, and 75. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield the following peptides as trifluoroacetate salt:
N-Ac-Sar-Gly-Val-D-Ile-Pen-Nva-Ile-Arg-ProNHCH₂CH₃,
N-Ac-Sar-Gly-Val-D-alloIle-Pen -Nva-Ile-Arg-ProNHCH₂CH₃,
N-Ac-Sar-Gly-Val-D-Ile-Pen-Gln-Ile-Arg-ProNHCH₂CH₃,
N-Ac-Sar-Gly-Val-D-Ile-Pen-Ser-Ile-Arg-ProNHCH₂CH₃,
N-Ac-Sar-Gly-Val-D-Ile-Pen-Leu-Ile-Arg-ProNHCH₂CH₃,
N-Ac-Sar-Gly-Val-D-Ile-Pen-Nva-Ile-Arg-ProNHCH (CH₃)₂,
N-Ac-Sar-Gly- Val-D-Ile-Pen-Nva-Ile-Arg-Pro-D-AlaNH₂,
N-Succinyl-Sar-Gly-Val-D-Ile-Pen-Nva-Ile-Arg-ProNHCH₂CH₃,
N-Succinyl-Sar-Gly-Val-D-Ile-Pen-Gln-Ile-Arg-ProNHCH₂CH₃, and
N-Succinyl-Sar-Gly-Val-D-Ile-Pen-Gln-Ile-Arg-ProNHCH (CH₃)₂.

### Example 331

### N-Ac-Sar-Gly-Val-D-Ile-Thr-Pen-Ile-Arg-ProNHCH₂CH₂OCH₃

The procedure described in Example 11 is used but substituting Fmoc-Pen(Trt) for Fmoc-Nva. After cleavage of the peptide from the resin and removal of the protecting groups the crude product was purified by C-18 column chromatography using solvent mixture varying in a gradient of 10% to 50% acetonitrile-water containing 0.01% TFA. The pure fractions were lyophilized to yield N-Ac-Sar-Gly-Val-D-Ile-Thr-Pen-Ile-Arg-ProNHCH₂CH₂OCH₃ as the trifluoroacetate salt.

### Example 332

The procedures described in Example 331 is used but substituting the appropriate protected amino acids as describes in Examples 14, 15, 132,43, 54, and 75. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield the following peptides as trifluoroacetate salt:
N-Ac-Sar-Gly-Val-D-aflolle-Thr-Pen-Ile-Arg-ProNHCH₂CH₃,
N-Ac-Sar-Gly-V al-D-Leu-Thr-Pen-Ile-Arg-ProNHCH₂CH₃,
N-Ac-Sar-Gly- V al-D-Ile- Thr-Pen-IIe-Arg-Pro-D-AlaNH₂.
N-Succinyl-Sar-Gly-Val-D-Ile-Thr-Pen-Ile-Arg-ProNHCH₂CH₃,
N-Ac-Sar-Gly-Val-D-Ile-Thr-Pen-Ile-Arg-ProNHCH (CH₃)₂,
N-Ac-Sar-Gly- V al-D-Leu-Ser-Pen-Ile-Arg-ProNHCH₂CH₃,
N-Ac-Sar-Gly-Val-D-Leu-Gly-Pen-Ile-Arg-PrONHCH₂CH₃, and
N-Succinyl-Sar-Gly-Val-D-Leu-Ser-Pen-Ile-Arg-ProNHCH₂CH₃.

### Example 333

### N-Ac-Sar-Gly-Val-D-Phe(3,4,5-triF)-Thr-Gln-Ile-Arg-ProNhCH₂CH₃

The procedure described in Example 96 is used but substituting Fmoc-Gln(Trt) for Fmoc-Nva. After cleavage of the peptide from the resin and removal of the protecting groups the crude product was purified by C-18 column chromatography using solvent mixture varying in a gradient of 10% to 50% acetonitrile-water containing 0.01% TFA. The pure fractions were lyophilized to yield N-Ac-Sar-Gly-Val-D-Phe(3,4,5-triF)-Thr-Gln-Ile-Arg-ProNHCH₂CH₃ as the trifluoroacetate salt.

### Example 334

The procedures described in Example 333 is used but substituting the appropriate protected amino acids as describes in Examples 132, 43, 54, and 75. After cleavage of the peptide from the resin and removal of the protecting groups using (9: 1) TFA/anisole (3 mL) the crude product is purified by C-18 column chromatography using a solvent mixture varying in a gradient of 10% to 50% acetonitrile-water containing 0.01% TFA. The pure fractions are lyophilized to yield the following peptides as trifluoroacetate salt:
N-Ac-Sar-Gly-Val-D.Phe(3,4,5-triF)-Ser-Nva-Ile-Arg-PrONHCH₂CH₃,
N-Ac-Sar-Gly-Val-D-Phe(3,4,5-triF)-Gly-Nva-Ile-Arg-ProNHCH₂CH₃,
N-Ac-Sar-Gly-Val-D-Phe(3,4,5-triF)-Ser-Leu-Ile-Arg-ProNHCH₂CH₃,
N-Ac-Sar-Gly-Val-D-Phe(3,4,5-triF)-Ser-Nva-Ile-Arg-Pro-D-AlaNH₂,
N-Succinyl-Sar-Gly-Val-D-Phe(3,4,5-triF)-Thr-Gln-Ile-Arg-ProNHCH₂CH₃,
N-Succinyl-Sar-Gly-Val-D-Phe(3,4,5-triF)-Ser-Gln-Ile-Arg-ProNHCH₂CH₃,
N-Succinyl-Sar-Gly-Val-D-Phe(3,4,5-triF)-Thr-Gln-Ile-Arg-ProNHCH (CH₃)₂,
N-Ac-Sar-Gly-Val-D-Phe(3,4,5-triF)-Ser-GIn-Ile-Arg-ProNHCH₂CH₃, and
N-Ac-Sar-Gly-Val-D-Phe(3,4,5-triF)-Ser-Ser-Ile-Arg-ProNHCH₂CH₃.

### Example 337

The procedure described in Example 231 used but substituting N-acetyl-beta-alanine (3-Ac-Bala) for N-acetyl-4-aminobutyric acid. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield the following peptides as trifluoroacetate salt:
N-(3-Ac-Bala)-Sar-Gly-Val-D-allolle-Thr-Nva-Ile-Arg-PrONHCH₂CH₃;
N-(3-Ac-Bala)-Sar-Gly-Val-D-Ile-Tur-GIn-Ile-Arg-ProNHCH₂CH₃,
N-(3-Ac-Bala)-Sar-Gly-Val-D-allolle-Thr-Gln-Ile-Arg-ProNHCH₂CH₃,
N-(3-Ac-Bala)-Sar-Gly-Val-D-Ile-Thr-Gln-Ile-Arg-Pro-D-AlaNH₂.
N-(3-Ac-Bala)-Sar-Gly-Val-D-alloIle-Thr-Gln-Ile-Arg-Pro-DAlaNH₂
N-(3-Ac-Bala)-Sar-Gly-Val-D-alloIle-Thr-Gln-Ile-Arg-ProNHCH₂(CH₃)₂.
N-(3-Ac-Bala)-Sar-Gly-Val-D-Leu-Ser-Nva-Ile-Arg-PrONHCH₂CH₃,
N-(3-Ac-Bala)-Sar-Gly-Val-D-Leu-Thr-Nva-Ile-Arg-ProNHCH₂CH₃,
N-(3-Ac-Bala)-Sar-Gly- V al- D-Pen- Thr-Nva- Ile-Arg- ProNHCH₂CH₃,
N-(3-Ac-Bala)-Sar-Gly-Val-D-Ile-Ser-Nva-Ile-Arg-PrONHCH₂CH₃,

### Example 338

### N-Ac-Sar-Gly-V al-D-Ile-Thr-Nva-Ile-Arg-Pro-OH

The procedure described in Example 1 is used but substituting omitting the coupling with ethylamine. After cleavage of the peptide from the resin and removal of the protecting groups the crude product was purified by C-18 column chromatography using solvent mixture varying in a gradient of 10% to 50% acetonitrile-water containing 0.01 % TFA. The pure fractions were lyophilized to yield N-Ac-Sar-Gly-Val-D-Ile-Thr-Nva-Ile-Arg-Pro-OH as the trifluoroacetate salt.

### Example 339

The procedures described in Example 338 is used but substituting the appropriate protected amino acids as described in Examples 14, 15,132, 43, 54, and 75. After cleavage of the peptide from the resin and removal of the protecting groups using (9:1) TFA/anisole (3 mL) the crude product is purified by C-18 column chromatography using a solvent mixture varying in a gradient of 10% to 50% acetonitrile-water containing 0.01% TFA. The pure fractions are lyophilized to yield the following peptides as trifluoroacetate salt:
N-Ac-Sar-Gly-Val-D-alloIle-Thr-Nva-Ile-Arg-Pro-OH,
N-Ac-Sar-Gly-V al-D-Leu-Thr-Nva-Ile-Arg-Pro-OH,
N-Ac-Sar-Gly-Val-D-Pen-Thr-Nva-Ile-Arg-Pro-OH,
N-Ac-Sar-Gly-Val-D-Phe(3 ,4,5-trif)-Thr-Nva-Ile-Arg-Pro-OH,
N-Ac-Sar-Gly-V al-D-Ile-Thr-Gln-Ile-Arg-Pro-OH,
N-Ac-Sar-Gly-Val-D-Leu-Ser-Nva-Ile-Arg-Pro-OH,
N-Ac-Sar-Gly-Val-D-Ile-Ser-GIn-Ile-Arg-Pro-OH,
N-Succinyl-Sar-Gly-Val-D-Ile-Thr-Nva-Ile-Arg-Pro-OH, and
N-S uccinyl-Sar-Gly- Val-D-Leu- Thr-Gln-Ile-Arg-Pro-OH.

### In Vitro Assay for Angiogenic Activity

The human microvascular endothelial (HMVEC) migration assay was run according to the procedure of S. S. Tolsma, O. V. Volpert, D. J. Good, W. F. Frazier, P. J. Polverini and N. Bouck, J. Cell Biol. 122, 497-511 (1993).

The HMVEC migration assay was carried out using Human Microvascular Endothelial Cells-Dermal (single donor) and Human Microvascular Endothelial Cells, (neonatal). The BCE or HMVEC cells were starved overnight in DME containing 0.1 % bovine serum albumin (BSA). Cells were then harvested with trypsin and resuspended in DME with 0.1 % BSA at a concentration of 1.5 X 10⁶ cells per ml. Cells were added to the bottom of a 48 well modified Boyden chamber (Nucleopore Corporation, Cabin John, MD). The chamber was assembled and inverted, and cells were allowed to attach for 2 hours at 37 °C to polycarbonate chemotaxis membranes (5 µm pore size) that had been soaked in 0.1% gelatin overnight and dried. The chamber was then reinverted, and test substances (total volume of 50 µl), including activators, 15 ng/ml bFGF/VEGF, were added to the wells of the upper chamber. The apparatus was incubated for 4 hours at 37 °C. Membranes were recovered, fixed and stained (Diff Quick, Fisher Scientific) and the number of cells that had migrated to the upper chamber per 3 high power fields counted. Background migration to DME + 0.1 BSA was subtracted and the data reported as the number of cells migrated per 10 high power fields (400X) or, when results from multiple experiments were combined, as the percent inhibition of migration compared to a positive control.

The compounds described in Examples 1 to 339 inhibited human endothelial cell migration in the above assay from about 30% to about 95% inhibition when tested at concentrations of 10 nM or 20 nM, as reported below in Table 3.

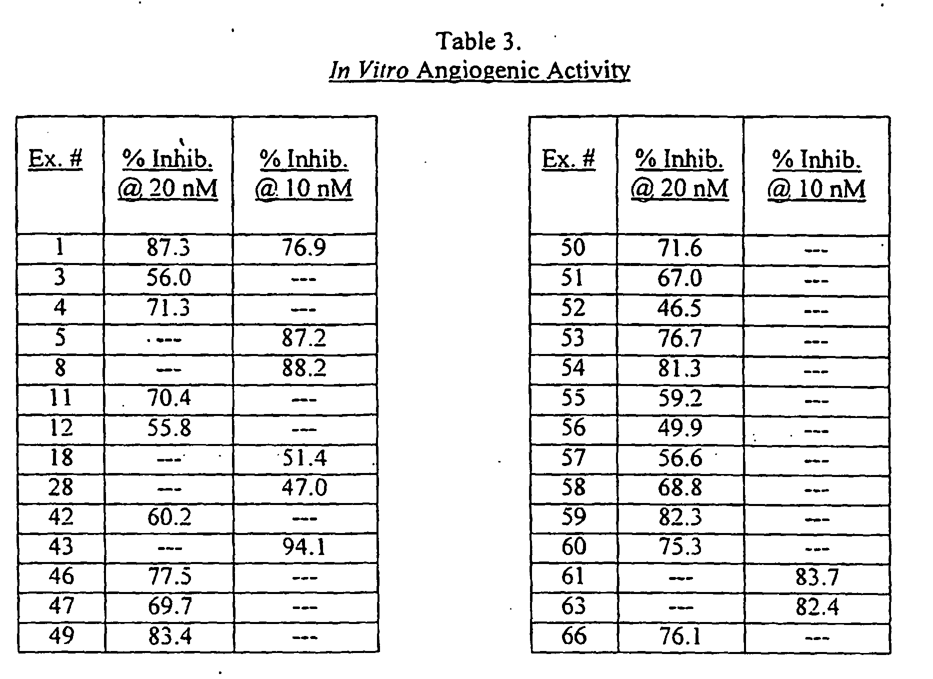

## Claims

1. A compound of the formula:
A₀-A₁-A₂-A₃-A₄-A₅-A₆-A₇-A₈-A₉-A₁₀
or a pharmaceutically acceptable salt, ester, solvate or prodrug thereof, wherein:
A₁ is sarcosyl, A₂ is glycyl, A₃ is valyl, A₇ is isoleucyl, A₈ is arginyl, A₉ is prolyl, and A₀, A₄, A₅, A₆, and A₁₀ are as follows:
A₀ is hydrogen or an acyl group selected from:
(1) R-(CH₂)ₙ-C(O)-; wherein n is an integer selected from 0, 1, 2, 3 and 5 and R is selected from methyl; N-acetylamino; methoxyl; carboxyl; cyclohexyl; cyclohexyl containing one double bond and substituted with three hydroxyl groups; and a 5- or 6-membered aromatic or nonaromatic ring ooptionally containing one heteroatom selected from nitrogen and oxygen, wherein the ring is optionally substituted with alkyl; and
(2) R¹-CH₂CH₂-(OCH₂CH₂O)p-CH₂-C(O)-; wherein R¹ is N-acetylamino, and p is 1;
A₄ is an amino acyl residue of L or D configuration selected from:
(1) allo-isoleucyl,
(2) glycyl,
(3) isoleucyl,
(5) dehydroleucyl.
(6) D-alanyl,
(7) D-3-(naphth-1-yl)alanyl,
(8) D-3-(naphth-2-yl)alanyl,
(9) D-(3-pyridyl)-alanyl,
(10) D-2-aminobutyryl,
(11) D-allo-isoleucyl,
(12) D-allo-threonyl,
(14) D-asparaginyl,
(15) D-aspartyl,
(16) D-3-(3-benzothienyl)alanyl
(17) D-3-(4,4-biphenyl)alanyl,
(18) D-3-(3-chlorophenyl)alanyl
(19) D-3-(3-trifluoromethylphenyl)alanyl,
(20) D-3-(3-cyanophenyl)alanyl,
(21) D-3-(3,4-difluorophenyl)alanyl,
(22) D-citrullyl,
(23) D-cyclohexylalanyl,
(24) D-cyclohexyglycyl,
(25) D-cystyl,
(26) D-cystyl(S-t-butyl),
(27) D-glutaminyl,
(28) D-glutamyl,
(29) D-histidyl,
(31) D-homophenylalanyl,
(32) D-homoseryl,
(33) D-isoleucyl,
(34) D-leucyl,
(36) D-lysyl,
(37) D-methionyl,
(38) D-neopentylglycyl,
(39) D-norleucyl,
(41) D-omithyl,
(42) D-penicillaminyl,
(43) D-penicillaminyl(acetamidomethyl),
(44) D-penicillaminyl(S-benzyl),
(45) D-phenylalanyl,
(46) D-3-(4-aminophenyl)alanyl,
(47) D-3-(4-methylphenyl)alanyl,
(48) D-3-(4-nitrophenyl)alanyl,
(49) D-3-(3,4-dimethoxyphenyl)alanyl,
(50) D-3-(3,4,5-trifluorophenyl)alanyl,
(52) D-seryl,
(53) D-seryl(O-benzyl),
(54) D-*t*-butylglycyl,
(55) D-thienylalanyl,
(56) D-threonyl,
(57) D-threonyl (O-benzyl),
(58) D-tryptyl,
(59) D-tyrosyl(*O*-benzyl),
(60) D-tyrosyl(O-ethyl),
(61) D-tyrosyl,
(62) D-valyl; and
(63) D-3-(4-chlorophenyl)alanyl;
A₅ is an amino acyl residue selected from:
(1) alanyl,
(3) 3-(naphth-1-yl)alanyl,
(4) 3-(naphth-2-yl)alanyl,
(6) allylglycyl,
(7) glutaminyl,
(8) glycyl,
(10) homoseryl,
(11) isoleucyl,
(12) lysyl(N-epsilon-acetyl),
(13) methionyl,
(14) norvalyl,
(15) octylglycyl,
(17) 3-(4-hydroxymethylphenyl)alanyl,
(18) prolyl,
(19) seryl,
(20) threonyl,
(21)tryptyl,
(22) tyrosyl,
(26) D-threonyl, and
(27) penicillaminyl;
A₆ is an amino acyl residue selected from:
(1) alanyl,
(5) 2-aminobutyryl,
(6) allylglycyl,
(7) arginyl,
(8) aspartyl,
(9) aspartyl,
(10) citrullyl,
(11) 3-(cyclohexyl)alanyl,
(12) glutaminyl,
(13) glutamyl,
(14) glycyl,
(19) isoleucyl,
(20) leucyl,
(21) lysyl(N-epsilon-acetyl),
(23) methionyl(sulfone),
(24) methionyl(sulfoxide),
(25) methionyl,
(26) norleucyl,
(27) norvalyl,
(28) octylglycyl,
(30) 3-(4-carboxyamidephenyl)alanyl,
(31) propargylglycyl,
(32) seryl,
(35) tyrosyl,
(36) valyl,
(42) D-norvalyl, and
(44) penicillaminyl;
A₁₀ is selected from:
(1) hydroxyl,
(2) azaglycylamide,
(3) D-alanylamide,
(7) sarcosylamide,
(8) serylamide,
(9) D-serylamide,
(10) a group represented by the formula 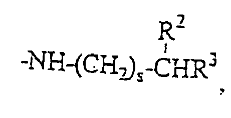 and
(11) a group represented by the formula-NH-R⁴;
wherein:
s is an integer selected from 0 and 1,
R² is selected from hydrogen, alkyl, and a 6-membered cycloalkyl ring;
R³ is selected from hydrogen, hydroxy, alkyl, alkoxy, and a 6-membered ring containing one nitrogen, provided that s is not zero when R³ is hydroxy or alkoxy; and
R⁴ is selected from hydrogen and hydroxy.

2. A compound according to Claim 1, wherein A₄ is an amino acyl residue selected from:
(1) D-alanyl,
(2) D-3-(naphth-1-yl)alanyl,
(3) D-3-(naphth-2-yl)alanyl,
(4) D-(3-pyridyl)-alanyl,
(5) D-2-aminobutyryl,
(6) D-allo-isoleucyl,
(7) D-allo-threonyl,
(9) D-asparaginyl,
(10) D-aspartyl,
(11) D-3-(3-chlorophenyl)alanyl,
(12) D-3-(3-trifluoromethylphenyl)alanyl,
(13) D-3-(3-cyanophenyl)alanyl,
(14) D-3-(3,4-difluorophenyl)alanyl,
(15) D-cyclohexylalanyl,
(16) D-cylohexylglycyl,
(17) D-cystyl,
(18) D-glutaminyl,
(19)D-glutamyl,
(20) D-histidyl,
(22) D-homophenylalanyl,
(23) D-homoseryl,
(24) D-isoleucyl,
(25) D-leucyl,
(27) D-methionyl,
(28) D-neopentylglycyl,
(29) D-norleucyl,
(31) D-penicillaminyl,
(32) D-penicillaminyl(acetamidomethyl),
(33) D-penicillaminyl(S-benzyl),
(34) D-phenylalanyl,
(35) D-3-(4-aminophenyl)alanyl,
(36) D-3-(4-methylphenyl)alanyl,
(37) D-3-(4-nitrophenyl)alanyl,
(38) D-3-(3,4-dimethoxyphenyl)alanyl,
(39) D-3-(3,4,5-trifluorophenyl)alanyl,
(41) D-seryl,
(42) D-seryl(O-benzyl),
(43) D-t-butylglycyl,
(44) D-thienylalanyl,
(45) D-threonyl,
(46) D-threonyl(O-benzyl),
(47) D-tyrosyl(O-ethyl),
(48) D-tyrosyl,
(49) D-valyl, and
(50) D-3(4-chlorophenyl)alanyl.

3. A compound according to Claim 2, wherein A₄ is an amino acyl residue selected from:
(1) D-allo-isoleucyl,
(3) D-3- (3-cyanophenyl) alanyl,
(4) D-cystyl,
(5) D-isoleucyl,
(6) D-leucyl,
(7) D-penicillaminyl,
(8) D-phenylalanyl,
(9) D-3- (3,4,5-trifluorophenyl) alanyl, and
(10) D-3- (4-aminophenyl) alanyl.

4. A compound according to Claim 1, wherein A₅ is selected from:
(1) glycyl,
(2) octylglycyl,
(3) penicillaminyl,
(4) seryl,
(5) threonyl, and
(6) tyrosyl.

5. A compound according to Claim 1, wherein A₆ is selected from:
(1) glutaminyl,
(2) leucyl,
(3) norvalyl, and
(4) seryl.

6. A compound according to Claim 2, wherein A₀ is selected from:
(1) acetyl,
(2) butyryl,
(5) N-acetyl-beta-alanyl,
(6) (6-N-acetylamino)caproyl,
(8) cyclohexylacetyl,
(9)furoyl,
(10) gamma-aminobutyryl,
(11) 2-methoxyacetyl,
(12) methylnicotinyl,
(13) nicotinyl,
*(15)* phenylacetyl,
(16) propionyl,
(17) shikimyl,
(18) succinyl, and
(19) tetrahydrofuroyl.

7. A compound according to Claim 2, wherein A₁₀ is selected from:
(1) D-alanylamide,
(2) azaglycylamide,
(3) serylamide,
(4) ethylamide,
(5) hydroxylamide,
(6) isopropylamide,
(7) propylamide,
(8) 2-(cyclohexyl)ethylamide,
(9) 2-(1-pyrrolidine)ethylamide,
(10) 1-(cyclohexyl)ethylamide,
(11) 2-(methoxy)ethylamide, and
(12) 2- (hydroxy)ethylamide.

8. A compound according to Claim 1, wherein A₄ is an amino acyl residue selected from:
(1) D-allo-isoleucyl,
(3) D-3- (3-cyanophenyl) alanyl,
(4) D-cystyl,
(5) D-isoleucyl,
(6) D-leucyl,
(7) D-penicillaminyl,
(8) D-phenylalanyl,
(9) D-3- (3,4,5-trifluorophenyl) alanyl, and
(10) D-3- (4-aminophenyl) alanyl;
A₅ is an amino acyl residue selected from:
(1) octylglycyl,
(2) glycyl,
(3) penicillaminyl,
(4) seryl,
(5) threonyl, and
(6) tyrosyl; and
A₆ is an amino acyl residue selected from:
(1) glutaminyl,
(2) leucyl,
(3) norvalyl, and
(4) seryl.

9. A compound according to Claim 8, wherein A₀ is selected from:
(1) acetyl,
(2) butyryl,
(5) N-acetyl-beta-alanyl,
(6) (6-N-acetylamino) caproyl,
(8) cyclohexylacetyl,
(9) furoyl,
(10) gamma-aminobutyryl,
(11) 2-methoxyacetyl,
(12) methylnicotinyl,
(13) nicotinyl,
(15) phenylacetyl,
(16) propionyl,
(17) shikimyl,
(18) succinyl, and
(19) tetrahydrofuroyl.

10. A compound according to Claim 8, wherein A₁₀ is selected from:
(1) D-alanylamide,
(2) azaglycylamide,
(3) serylamide
(4) ethylamide,
(5) hydroxylamide,
(6) isopropylamide,
(7) propylamide,
(8) 2-(cyclohexyl)ethylamide,
(9) 2-(1-pyrrolidine)ethylamide,
(10) 1-(cyclohexyl)ethylamide,
(11) 2-(methoxy)ethylamide, and
(12) 2-(hydroxy)ethylamide.

11. A compound according to claim 1, or a pharmaceutically acceptable salt, ester, solvate or prodrug thereof, selected from:
(1) N-Ac-Sar-Gly-Val-D-Ile-Thr-Nva-Ile-Arg-ProNHCH₂CH₃,
(2) pyroGlu-Gly-Val-D-Ile-Thr-Nva-Ile-Arg-ProNHCH₂CH₃,
(3) N-Ac-Sar-Gly-Val-D-Ile-Thr-Nva-Ile-Arg-ProNHCH₃,
(4) N-Ac-Sar-Gly-Val-D-Ile-Thr-Nva-Ile-Arg-ProNHCH(CH₃)₂,
(5) N-Ac-Sar-Gly- Val-D-Ile- Thr-Nva-Ile-Arg-ProNHCH₂CH₂-(1-pyrrolidine),
(6) N-Ac-Sar-Gly-Val-D-Ile-Thr-Nva-Ile-Arg-ProNHethyl(1-piperidine),
(7) N-Ac-Sar-Gly-Val-D-Ile-Thr-Nva-Ile-Arg-ProNHmethylcyclopropyl,
(8) N-Ac-Sar-Gly-Val-D-Ile-Thr-Nva-Ile-Arg-ProNH (ethyl-1-(R)-cyclohexyl),
(9) N-Ac-Sar-Gly-Val-D-Ile-Thr-Nva-Ile-Arg-ProNH₂,
(10) N-Ac-Sar-Gly-Val-D-Ile-Thr-Nva-Ile-Arg-ProNHCH₂CH₂OCH₃,
(11) N-Ac-Sar-Gly-Val-D-Ile-Thr-Nva-Ile-Arg-ProNHCH₂CH₂cyclohexyl,
(12) N-Ac-Sar-Gly-Val-D-Ile-Thr-Nva-Ile-Arg-ProNHCH₂CH₂CH₃,
(13) N-Ac-Sar-Gly- Val-D-alloIle- Thr-Nva-Ile-Arg-ProNHCH₂CH₃,
(14) N-Ac-Sar-Gly-Val-D-Leu-Thr-Nva-Ile-Arg-ProNHCH₂CH₃,
(15) N-Ac-Sar-Gty-Val-I!e-Thr-Nva-He-Arg-ProNHCH₂CH₃,
(16) N-Ac-Sar-Gly-Val-Gly-Thr-Nva-Ile-Arg-ProNHCH₂CH₃,
(17) N-Ac-Sar-Gly-Val-D-Val-Thr-Nva-Ile-Arg-ProNHCH₂CH₃,
(18) N-Ac-Sar-Gly-Val-D-Ala-Thr-Nva-Ile-Arg-ProNHCH₂CH₃,
(19) N-Ac-Sar-Gly-Val-D-Met-Thr-Nva-Ile-Arg-ProNHCH₂CH₃,
(20) N-Ac-Sar-Gly-Val-D-Me-Thr-Nva-Ile-Arg-PrONHCH₂CH₃,
(21) N-Ac-Sar-Gly-Val-D-Phe-Thr-Nva-Ile-Arg-ProNHCH₂CH₃,
(22) N-Ac-Sar-Gly-Val-D-Tyr-Thr-Nva-Ile-Arg-ProNHCH₂CH₃,
(23) N-Ac-Sar-Gly-Val-D-4,4-Biphenylala-Thr-Nva-Ile-Arg-ProNHCH₂CH₃,
(24) N-Ac-Sar-Gly-Val-D-Cha-Thr-Nva-Ile-Arg-ProNHCH₂CH₃,
(25) N-Ac-Sar-Gly-Val-D-Chg-Thr-Nva-Ile-Arg-ProNHCH₂CH₃,
(26) N-Ac-Sar-Gly-Val-D-4-ClPhe-Thr-Nva-Ile-Arg-ProNHCH₂CH₃,
(27) N-Ac-Sar-Gly-Val-D-Hphe-Thr-Nva-Ile-Arg-ProNHCH₂CH₃,
(28) N-Ac-Sar-Gly- Val-Dehydroleu- Thr-Nva-Ile-Arg-ProNHCH₂CH₃,
(29) N-Ac-Sar-Gly-Val-D-3-CF3Phe-Thr-Nva-Ile-Arg-ProNHCH₂CH₃.
(32) N-Ac-Sar-Gly-Val-D-3-ClPhe-Thr-Nva-Ile-Arg-ProNHCH₂CH₃,
(33) N-Ac-Sar-Gly-Val-D-2-Thienylala-Thr-Nva-Ile-Arg-ProNHCH₂CH₃,
(34) N-Ac-Sar-Gly-Val-D-3-CNPhe-Thr-Nva-Ile-Arg-ProNHCH₂CH₃,
(35) N-Ac-Sar-Gly-Val-D-Ile-Thr-DNva-Ile-Arg-ProNHCH₂CH₃,
(36) N-Ac-Sar-Gly-Val-D-Ile-Thr-Gln-Ile-Arg-ProNHCH₂CH₃,
(37) N-Ac-Sar-Gly-Val-D-Ile-Thr-Cha-Ile-Arg-ProNHCH₂CH₃,
(38) N-Ac-Sar-Gly-Val-D-Ile-Thr-Gly-Ile-Arg-ProNHCH₂CH₃,
(39) N-Ac-Sar-Gly-Val-D-Ile-Thr-Ala-Ile-Arg-ProNHCH₂CH₃,
(40) N-Ac-Sar-Gly-Val-D-Ile-Thr-Val-Ile-Arg-ProNHCH₂CH₃,
(41) N-Ac-Sar-Gly-Val-D-Ile-Thr-Abu-Ile-Arg-ProNHCH₂CH₃,
(42) N-Ac-Sar-Gly-Val-D-Ile-Thr-Allylgly-Ile-Arg-ProNHCH₂CH₃,
(43) N-Ac-Sar-Gly-Val-D-Ile-Thr-Octylgly-Ile-Arg-ProNHCH₂CH₃,
(44) N-Ac-Sar-Gly-Val-D-Ile-Thr-Met-Ile-Arg-ProNHCH₂CH₃,
(45) N-Cyclohexylacetyl-Sar-Gly-Val-D-Ile-Thr-Nva-Ile-Arg-ProNHCH₂CH₃,
(46) N- (2-Me-Nicotinyl)-Sar-Gly-Val-D-Ile-Thr-Nva-Ile-Arg-ProNHCH₂CH₃,
(47) N-Succinyl-Sar-Gly-Val-D-Ile-Thr-Nva-Ile-Arg-ProNHCH₂CH₃,
(48) N-Nicotinyl-Sar-Gly-Val-D-Ile-T'hr-Nva-Ile-Arg-ProNHCH₂CH₃,
(49) N-Propionyl-Sar-Gly- V al-D-Ile- Thr-Nva-Ile-Arg-ProNHCH₂CH₃,
(50) N-(MeO) acetyl-Sar-Gly-Val-D-Ile-Thr-Nva-Ile-Arg-ProNHCH₂CH₃,
(51) N-(Shikimyl)-Sar-Gly-Val-D-Ile-Thr-Nva-Ile-Arg-ProNHCH₂CH₃,
(52) N-(2-Furoyl)-Sar-Gly-Val-D-Ile-Thr-Nva-Ile-Arg-ProNHCH₂CH₃,
(53) N-Butyryl-Sar-Gly-Val-D-Ile-Thr-Nva-Ile-Arg-ProNHCH₂CH₃,
(54) N-[2-THFcarbonyl]-Sar-Gly-Val-D-Ile-Thr-Nva-Ile-Arg-ProNHCH₂CH₃,
(55) N-[CH₃C(O)NH-(CH₂)₂-O-(CH₂)₂-O-CH₂-C(O)]-Sar-Gly-Val-D-Ile-Thr-Nva-Ile-Arg-ProNHCH₂CH₃,
(56) N-[6-N-acetyl-(CH₂)₅C(O)₅-Sar-Gly-Val-D-Ile-Thr-Nva-Ile-Arg-ProNHCH₂CH₃,
(58) N- [4-N-Acetylaminobutyryl]-Sar-Gly-Val-D-Ile-Thr-Nva-Ile-Arg-ProNHCH₂CH₃,
(59) H-Sar-Gly-Val-D-Ile-Thr-Nva-Ile-Arg-ProNHCH₂CH₃,
(66) N-Ac-Sar-Gly-Val-D-Ile-Thr-Leu-Ile-Arg-ProNHCH₂CH₃,
(67) N-Ac-Sar-Gly-Val-D-Ile-Thr-Ser-Ile-Arg-ProNHCH₂CH₃,
(68) N-Ac-Sar-Gly-Val-D-Ile-Thr-Nva-Ile-Arg-Pro-D-AlaNH₂,
(77) N-Ac-Sar-Gly-Val-D-Tyr (Et)-Thr-Nva-Ile-Arg-ProNHCH₂CH₃,
(78) N-Ac-Sar-Gly-Val-D-Cys (tBu)-Thr-Nva-Ile-Arg-ProNHCH₂CH₃,
(79) N-Ac-Sar-Gly-Val-D-Cys-Thr-Nva-Ile-Arg-ProNHCH₂CH₃,
(80) N-Ac-Sar-Gly-Val-D-Tyr (Bzl)-Thr-Nva-Ile-Arg-ProNHCH₂CH₃,
(81) N-Ac-Sar-Gly-Val-D-Ser (Bzl)-Thr-Nva-Ile-Arg-ProNHCH₂CH₃,
(82) N-Ac-Sar-Gly-Val-D-1Nal-Thr-Nva-Ile-Arg-ProNHCH₂CH₃,
(83) N-Ac-Sar-Gly-Val-D-tButylgly-Thr-Nva-Ile-Arg-ProNHCH₂CH₃,
(84) N-Ac-Sar-Gly-Val-D-Orn-Thr-Nva-Ile-Arg-ProNHCH₂CH₃,
(85) N-Ac-Sar-Gly-Val-D-Thr (Bzl)-Thr-Nva-Ile-Arg-ProNHCH₂CH₃,
(86) N-Ac-Sar-Gly-Val-D-2Nal-Thr-Nva-Ile-Arg-ProNHCH₂CH₃,
(87) N-Ac-Sar-Gly-Val-D-Phe (4-Me)-Tbr-Nva-Ile-Arg-ProNHCH₂CH₃,
(88) N-Ac-Sar-Gly-Val-D-Phe (3,4-diMeO)-Thr-Nva-Ile-Arg-ProNHCH₂CH₃,
(89) N-Ac-Sar-Gly-Val-D-Phe (3,4,5-triF)-Thr-Nva-Ile-Arg-ProNHCH₂CH₃,
(90) N-Ac-Sar-Gly-Val-D-Phe (4-NO₂)-Thr-Nva-Ile-Arg-ProNHCH₂CH₃,
(91) N-Ac-Sar-Gly-Val-D-Pen-Thr-Nva-Ile-Arg-ProNHCH₂CH₃,
(92) N-Ac-Sar-Gly-Val-D-Pen (Acm)-Thr-Nva-Ile-Arg-ProNHCH₂CH₃,
(93) N-Ac-Sar-Gly-Val-D-Abu-Thr-Nva-Ile-Arg-ProNHCH₂CH₃,
(94) N-Ac-Sar-Gly-Val-D-Phe (4-NH₂)-Thr-Nva-Ile-Arg-ProNHCH₂CH₃,
(108) N-Ac-Sar-Gly-Val-D-Leu-Ala-Nva-Ile-Arg-ProNHCH₂CH₃,
(109) N-Ac-Sar-Gly-Val-D-Leu-Pro-Nva-Ile-Arg-ProNHCH₂CH₃,
(110) N-Ac-Sar-Gly-Val-D-Leu-Trp-Nva-Ile-Arg-ProNHCH₂CH₃,
(111) N-Ac-Sar-Gly-Val-D-Leu-Tyr-Nva-Ile-Arg-ProNHCH₂CH₃,
(112) N-Ac-Sar-Gly-Val-D-Leu-Nva-Nva-Ile-Arg-ProNHCH₂CH₃,
(113) N-Ac-Sar-Gly-Val-D-Leu-Gly-Nva-Ile-Arg-ProNHCH₂CH₃,
(114) N-Ac-Sar-Gly-Val-D-Leu-Lys (Ac)-Nva-Ile-Arg-ProNHCH₂CH₃,
(115) N-Ac-Sar-Gly-Val-D-Leu-2Nal-Nva-Ile-Arg-ProNHCH₂CH₃,
(116) N-Ac-Sar-Gly-Val-D-Leu-INal-Nva-Ile-Arg-ProNHCH₂CH₃,
(117) N-Ac-Sar-Gly-Val-D-Leu-Octylgly-Nva-Ile-Arg-ProNHCH₂CH₃,
(118) N-Ac-Sar-Gly-Val-D-Leu-Gln-Nva-Ile-Arg-ProNHCH₂CH₃,
(119) N-Ac-Sar-Gly-Val-D-Leu-Met-Nva-Ile-Arg-ProNHCH₂CH₃,
(120) N-Ac-Sar-Gly-Val-D-Leu-Ser-Nva-Ile-Arg-ProNHCH₂CH₃,
(121) N-Ac-Sar-Gly-Val-D-Leu-Allylgly-Nva-Ile-Arg-ProNHCH₂CH₃,
(122) N-Ac-Sar-Gly-Val-D-Leu-Ile-Nva-Ile-Arg-ProNHCH₂CH₃
(123) N-Ac-Sar-Gly-Val-D-Leu-D-Thr-Nva-Ile-Arg-ProNHCH₂CH₃,
(124) N-Ac-Sar-Gly-Val-D-Ile-Thr-Ile-Ile-Arg-ProNHCH₂CH₃,
(125) N-Ac-Sar-Gly-Val-D-Ile-Thr-Nle-Ile-Arg ProNHCH₂CH₃,
(126) N-Ac-Sar-Gly-Val-D-Ile-Thr-Cit-Ile-Arg-ProNHCH₂CH₃,
(127) N-Ac-Sar-Gly-Val-D-Ile-Thr-Met (O₂)-Ile-Arg-ProNHCH₂CH₃,
(128) N-Ac-Sar-Gly-VaI-D-Ile-Thr-Arg-Ile-Arg-ProNHCH₂CH₃,
(129) N-Ac-Sar-Gly-Val-D-Ile-Tk-Tyr-Ile-Arg-ProNHCH₂CH₃,
(130) N-Ac-Sar-Gly-Val-D-Ile-Thr-Glu-Ile-Arg-ProNHCH₂CH₃,
(131) N-Ac-Sar-Gly-Val-D-Ile-Thr-Lys (Ac)-Ile-Arg-ProNHCH₂CH₃,
(132) N-Ac-Sar-Gly-Val-D-Ile-Thr-Propargylgly-Ile-Arg-ProNHCH₂CH₃,
(133) N-Ac-Sar-Gly-Val-D-alloIle-Thr-Gln-Ile-Arg-ProNHCH₂CH₃,
(134) N-Ac-Sar-Gly-Val-D-Leu-Thr-Gln-Ile-Arg-ProNHCH₂CH₃,
(135) N-Ac-Bala-Sar-Gly-Val-D-Ile-Thr-Nva-Ile-Arg-ProNHCH₂CH₃,
(136) N-Phenylacetyl-Sar-Gly-Val-D-Ile-Thr-Nva-Ile-Arg-ProNHCH₂CH₃,
(137) N-Ac-Sar-Gly- Val-D-Ile- Thr-Nva-Ile-Arg-Pro-AzaglyNH₂,
(139) N-Ac-Sar-Gly-Val-D-Ile-Thr-Nva-Ile-Arg-Pro-SerNH₂,
(140) N-Succinyl-Sar-Gly-Val-D-Leu-Thr-Nva-Ile-Arg-ProNHCH₂CH₃, (158) N-Ac-Sar-Gly-Val-D-Ile-Thr-Gln-Ile-Arg-ProNHCH(CH₃)₂,
(159) N-Succinyl-Sar-Gly-Val-D-Ile-Thr-GIn-Ile-Arg-PrONHCH₂CH₃,
(160) N-Succinyl-Sar-Gly-Val-D-Leu-Thr-Gln-Ile-Arg-ProNHCH₂CH₃,
(161) N-Succinyl-Sar-Gly-Val-D-Leu-Tbr-Gln-Ile-Arg-ProNHCH(CH₃)₂,
(162) N-Ac-Sar-Gly-Val-D-Leu-Thr-Asp-Ile-Arg ProNHCH₂CH₃,
(163) N-Ac-Sar-Gly-Val-D-Ile-Thr-Asp-Ile-Arg-ProNHCH₂CH₃,
(164) N-Ac-Sar-Gly-Val-D-lie-Thr-Asn-Ile-Arg-ProN-HCH2CH3,
(165) N-Ac-Sar-Gly-Val-D-Ile-Thr-Met(O)-Ile-Arg-ProNHCH₂CH₃,
(166) N-Ac-Sar-Gly-Val-D-Leu-Thr-Asn-Ile-Arg-ProNHGH₂CH₃,
(167) N-Ac-Sar-Gly-Val-D-Thr-Thr-Nva-Ile-Arg-ProNHCH₂CH₃,
(168) N-Ac-Sar-Gly-Val-D-Ser-Thr-Nva-Ile-Arg-ProNHCH₂CH₃,
(169) N-Ac-Sar-Gly-Val-D-Hser-Thr-Nva-De-Arg-ProNHCH₂CH₃,
(170) N-Ac-Sar-Gly-Val-D-Gln-Tbr-Nva-Ile-Arg-ProNHCH₂CH₃,
(171) N-Ac-Sar-Gly-Val-D-Asn-Thr-Nva-Ile-Arg-ProNHCH₂CH₃,
(172) N-Ac-Sar-Gly-Val-D-Cit-Thr-Nva-Ile-Arg-ProNHCH₂CH₃,
(173) N-Ac-Sar-Gly-Val-D-Hcit-Thr-Nva-Ile-Arg-ProNHCH₂CH_{3,}
(174) N-Ac-Sar-Gly-Val-D-Hle-Thr-Nva-Ile-Arg-ProNHCH₂CH₃,
(175) N-Ac-Sar-Gly-Val-D-Neopentylgly-Thr-Nva-Ile-Arg-ProNHCH₂CH₃,
(176) N-Ac-Sar-Gly-Val-D-Ile-Thr-Phe(4-CONH₂)-Ile-Arg-ProNHCH₂CH₃,
(197) N-Succinyl-Sar-Gly-Val-D-alloIle-Tbr-Nva-Ile-Arg-PrONHCH₂CH₃,
(198) N-Succinyl-Sar-Gly-Val-D-Ile-Tk-Gln-Ile-Arg-ProNHCH₂CH₃,
(199) N-Succinyl-Sar-Gly-Val-D-Ile-Tbr-Gln-Ile-Arg-Pro-D-AlaNH₂,
(200) N-Succinyl-Sar-Gly-Val-D-alloIle-Thr-Gln-Ile-Ara-ProNHCH₂CH₃,
(201) N-Succinyl-Sar-Gly- Val-D-alloIle- Thr-Gln-Ile-Arg-Pro-D-AlaNH₂,
(202) N-Succinyl-Sar-Gly-Val-D-alloIle-Thr-Gln-Ile-Arg-ProNHCH₂CH₃,
(203) N-Succinyl-Sar-Gly-Val-D-Ile-Thr-Gln-Ile-Arg-ProNHCH₂CH₃,
(204) N-Ac-Sar-Gly-Val-D-alloIle-Thr-Nva-Ile-Arg-Pro-D-AlaNH2,
(205) N-Ac-Sar-Gly-Val-D-alloIle-Thr-Nva-Ile-Arg-ProNHCH(CH₃)₂.
(206) N-Ac-Sar-Gly-Val-D-Ile-Thr-Gln-Ile-Arg-Pro-D-AlaNH₂,
(207) N-Ac-Sar-Gly-Val-D-Ile-Thr-Gln-Ile-Arg-ProNHCH(CH₃)₂,
(208) N-Ac-Sar-Gly-Val-D-alloIle-Thr-Gln-Ile-Arg-Pro-D-AlaNH₂,
(209) N-Ac-Sar-Gly-Val-D-alloIle-Thr-Gln-Ile-Arg-ProNHCH(CH₃)₂,
(210) N-Ac-Sar-Gly-Val-D-Ile-Thr-Nva-Ile-Arg-Pro-SarNH₂,
(211) N-Ac-Sar-Gly-Val-D-alloIle-Thr-Nva-Ile-Arg-Pro-SarNH₂,
(212) N-Ac-Sar-Gly-Val-D-Ile-Thr-Gln-Ile-Arg-Pro-SarNH₂,
(213) N-Ac-Sar-Gly-Val-D-alloIle-Thr-Gln-Ile-Arg-Pro-SarNH₂,
(214) N-Ac-Sar-Gly-Val-D-alloIle-Thr-Ser-Ile-Arg-Pro-D-AlaNH₂,
(215) N-Ac-Sar-Gly-Val-D-alloIle-Thr-Ser-Ile-Arg-ProNHCH(CH₃)₂, (216)N-Ac-Sar-Gly-Val-D-alloIle-Thr-Ser-Ile-Arg-ProNHCH₂CH₃,
(217) N-Ac-Sar-Gly-Val-D-Ile-Thr-Orn(Ac)-Ile-Arg-ProNHCH₂CH₃,
(218) N-Ac-Sar-Gly-Val-D-Ile-Thr-Gln-Ile-Arg-Pro-AzaglyNH₂,
(219) N-Ac-Sar-Gly-Val-D-alloIle-Thr-Nva-Ile-Arg-Pro-AzaglyNH₂,
(220) N-Ac-Sar-Gly- V al-D-alloIle- Thr-Gln-Ile-Arg-Pro-AzaglyNH₂,
(221) N-(2-THFcarbonyl)-Sar-Gly-Val-D-alloIle-Thr-Nva-Ile-Arg-ProNHCH₂CH₃,
(222) N-(2-THFcarbonyl)-Sar-Gly-Val-D-Ile-7hr-G1n-Ile-Arg-ProNHCH₂CH₃,
(223) N-(2-THFcarbonyl)-Sar-Gly-Val-D-alloIle-Thr-Gln-Ile-Arg-Pro NHCH₂CH₃.
(224) N-(2-THFcarbonyl)-Sar-Gly-Val-D-Ile-Thr-Gln-Ile-Arg-Pro-D-AlaNH₂,
(225) N-(2-THFcarbonyl)-Sar-Gly-Val-D-alloIle-Thr-Gln-Ile-Arg-Pro-D-AlaNH₂,
(226) N-(2-THFcarbonyl)-Sar-Gly-Val-D-alloIle-Thr-Gln-Ile-Arg-Pro NHCH(CH₃)₂,
(227) N-(6-Ac-Aca)-Sar-Gly-Val-D-alloIle- Thr-Nva-Ile-Arg-ProNHCH₂CH₃,
(228) N-(6-Ac-Aca)-Sar-Gly-Val-D-Ile-Thr-Gln-Ile-Arg-ProNHCH₂CH₃,
(229) N-(6-Ac-Aca)-Sar-Gly-Val-D-alloIle-Thr-Gln-Ile-Arg-ProNHCH₂CH₃,
(230) N-(6-Ac-Aca)-Sar-Gly-Val-D-Ile-Thr-Gln-Ile-Arg-Pro-D-AlaNH₂,
(231) N-(6-Ac-Aca)-Sar-Gly-Val-D-alloIle-Thr-Gln-Ile-Arg-Pro-D-AlaNH₂,
(232) N-(6-Ac-Aca)-Sar-Gly-Val-D-alloIle-Thr-Gin-Ile-Arg-ProNHCH(CH₃)₂,
(233) N-(4-Ac-Gaba)-Sar-Gly-Val-D-alloIle-Thr-Nva-Ile-Arg-ProNHCH₂CH₃,
(234) N-(4-Ac-Gaba)-Sar-Gly-Val-D-Ile-Thr-Gln-Ile-Arg-ProNHCH₂CH₃,
(235) N-(4-Ac-Gaba)-Sar-Gly-Val-D-alloIle-Thr-Gin-Ile-Arg-ProNHCH₂CH₃,
(236) N-(4-Ac-Gaba)-Sar-Gly-Val-D-Ile-Thr-Gln-Ile-Arg-Pro-D-AlaNH2,
(237) N-(4-Ac-Gaba)-Sar-Gly-Val-D-allolle-Thr-Gln-Ile-Arg-Pro-D-AlaNH₂,
(238) N-(4-Ac-Gaba)-Sar-Gly-Val-D-alloIle-Thr-Gln-Ile-Arg-Pro NHCH(CH₃)₂,
(239) N-(2-Furoyl)-Sar-Gly-Val-D-alloIle-Thr-Nva-Ile-Arg-ProNHCH₂CH₃,
(240) N-(2-Furoyl)-Sar-Gly-Val-D-Ile-Thr-Gln-Ile-Arg-ProNHCH₂CH₃,
(241) N-(2-Furoyl)-Sar-Gly- Val-D-alloIle-Thr-Gln-Ile-Arg-ProNHCH₂CH₃,
(242) N-(2-Furoyl)-Sar-Gly-Val-D-Ile-Thr-Gln-Ile-Arg-Pro-D-AlaNH₂,
(243) N-(2-Furoyl)-Sar-Gly-Val-D-alloIle-Thr-GIn-Ile-Arg-Pro-D-AlaNH₂,
(244) N-(2-Furoyl)-Sar-Gly-Val-D-alloIle-Thr-Gln-Ile-Arg-ProNHCH(CH₃)₂,
(245) N-(Shikimyl)-Sar-Gly-Val-D-allolle-Thr-Nva-Ile-Arg-ProNHCH₂CH₃,
(246) N-(Shikimyl)-Sar-Gly-Val-D-Ile-Thr-Gln-Ile-Arg-ProNHCH₂CH₃,
(247) N-(Shikimyl)-Sar-Gly-Val-D-alloIle-Thr-Gln-Ile-Arg-ProNHCH₂CH₃,
(248) N-(Shikimyl)-Sar-Gly-Val-D-Ile-Thr-Gln-Ile-Arg-Pro-D-AlaNH₂,
(249) N-(Shikimyl)-Sar-Gly-Val-D-aUoIle-Thr-Gln-Ile-Arg-Pro-D-AlaNH₂,
(250) N- (Shikimyl)-Sar-Gly-Val-D-alloIle-Thr-Gln-Ile-Arg-ProNHCH(CH₃)₂,
(251) N- (2-Me-Nicotinyl)-Sar-Gly-Val-D-alloIle-Thr-Nva-Ile-Arg-ProNHCH₂CH₃.
(252) N-(2-Me-Nicotinyl)-Sar-Gly-Val-D-Ile-Thr-Gln-Ile-Arg-ProNHCH₂CH₃,
(253) N-(2-Me-Nicotinyl)-Sar-Gly-Val-D-alloIle-Thr-Gln-Ile-Arg-Pro NHCH₂CH₃.
(254) N-(2-Me-Nicotinyl)-Sar-Gly-Val-D-Ile-Thr-Gln-Ile-Arg-Pro-D-AlaNH₂,
(255) N-(2-Me-Nicotinyl)-Sar-Gly-Val-D-alloIle-Thr-Gln-Ile-Arg-Pro-D-AlaNH₂,
(256) N-(2-Me-Nicotinyl)-Sar-Gly-Val-D-allolle-Thr-Gln-Ile-Arg-ProNHCH(CH₃)₂,
(257) N-Ac-Sar-Gly- Val-D-alloIle- Thr-Leu-Ile-Arg-Pro-D-AlaNH₂,
(258) N-Ac-Sar-Gly-Val-D-Ile-Thr-Leu-Ile-Arg-ProNHCH(CH₃)₂,
(259) N-Ac-Sar-Gly-Val-D-alloIle-Thr-Leu-Ile-Arg-ProNHCH₂CH₃,
(260) N-Ac-Sar-Gly-Val-D-Ile-Thr-Leu-Ile-Arg-Pro-D-AlaNH₂,
(261) N-Succinyl-Sar-Gly- Val-D-Ile- Thr-Leu-Ile-Arg-Pro-D-AlaNH₂,
(262) N-Succbyl-Sar-Gly-Val-D-Ile-Tk-Leu-Ile-Arg-PrONHCH₂CH₃,
(263) N-Succinyl-Sar-Gly-Val-D-Ile-Thr-Leu-Ile-Arg-ProNHCH₂CH₃,
(264) N-Succinyl-Sar-Gly-Val-D-alloIle-Thr-Leu-Ile-Arg-ProNHCH₂CH₃,
(265) N-Succinyl-Sar-Gly- Val- D-alloIle- Thr-Leu- Ile-Arg-Pro-D- AlaNH₂,
(266) N-Succinyl-Sar-Gly- Val-D-Ile- Thr-Leu-Ile-Arg-Pro-AzaglyNH₂,
(267) N-Ac-Sar-Gly-Val-D-alloIle-Thr-Nva-Ile-Arg-ProNHethyl-(1-pyrrolidine),
(268) N-Ac-Sar-Gly-Val-D-alloIle-Thr-Nva-Ile-Arg-ProNH (ethyl-1-cyclohexyl),
(269) N-Ac-Sar-Gly-Val-D-Ile-Thr-Gln-Ile-Arg-ProNHethyl- (1-pyrrolidine),
(270) N-Ac-Sar-Gly-Val-D-Ile-Thr-Gln-Ile-Arg-ProNH (ethyl-1-cyclohexyl),
(271) N-Succinyl-Sar-Gly-Val-D-Ile-Thr-Gln-Ile-Arg-ProNH (ethyl-1-cyclohexyl).
(272) N-Ac-Sar-Gly-Val-D-alloIle-Thr-Nva-Ile-Arg-ProNHCH₂CH₂OCH₃,
(273) N-Ac-Sar-Gly-Val-D-Ile-Thr-Gln-Ile-Arg-ProNHCH₂CH₂OCH₃,
(274) N-Ac-Sar-Gly-Val-D-Ile-Thr-Ser-Ile-Arg-ProNHCH2CH₂OCH₃,
(275) N-Ac-Sar-Gly-Val-D-Ile-Thr-Leu-Ile-Arg-ProNHCH₂CH₂OCH₃,
(276) N-Succinyl-Sar-Gly-Val-D-Ile-Thr-Nva-Ile-Arg-ProNHCH₂CH₂OCH₃,
(277) N-Succinyl-Sar-Gly-Val-D-Ile-Thr-Gln-Ile-Arg-ProNHCH₂CH₂OCH₃,
(278) N-Succinyl-Sar-Gly-Val-D-alloIle-Thr-Gln-Ile-Arg-ProNHCH₂CH₂OCH₃,
(279) N-Ac-Sar-Gly-Val-D-Ile-Ser-Nva-Ile-Arg-ProNHCH₂CH₂OCH₃,
(280) N-Ac-Sar-Gly-Val-D-Leu-Ser-Nva-Ile-Arg-ProNHCH₂CH₂OCH₃,
(281) N-Ac-Sar-Gly-Val-D-alloIle-Thr-Allygly-Ile-Arg-ProNHCH₂CH₃,
(282) N-Ac-Sar-Gly-Val-D-Ile-Thr-Allygly-Ile-Arg-ProNHCH(CH₃)₂,
(283) N-Ac-Sar-Gly-Val-D-Ile-Thr-Allygly-Ile-Arg-Pro-D-AlaNH₂,
(284) N-Ac-Sar-Gly-Val-D-alloIle-Thr-AHygly-Ile-Arg-Pro-D-AlaNH₂,
(285) N-Succinyl-Sar-Gly-Val-D-Ile-Thr-Allygly-Ile-Arg-Pro-D-AlaNH₂,
(286) N-Ac-Sar-Gly-Val-D-Ile-Ser-Allygly-Ile-Arg-Pro-ProNHCH₂CH₃,
(287) N-Ac-Sar-Gly-Val-D-Leu-Ser-Allygly-Ile-Arg-Pro-ProNHCH₂CH₃,
(288) N-Ac-Sar-GIy-Val-D-Ile-Thr-Nva-Ile-Arg-Pro-SarNH₂,
(289) N-Ac-Sar-Gly-Val-D-Ile-Thr-Nva-Ile-Arg-ProNHOH,
(290) N-Ac-Sar-Gly-Val-D-Ile-Ser-Nva-Ile-Arg-ProNHCH₂CH₃,
(291) N-Ac-Sar-Gly-Val-D-alloIle-Ser-Nva-Ile-Arg-ProNHCH₂CH₃,
(292) N-Ac-Sar-Gly-Val-D-Leu-Hser-Nva-Ile-Arg-ProNHCH₂CH₃,
(300) N-Ac-Sar-Gly-Val-D-alloIle-Thr-Ala-Ile-Arg-ProNHCH₂CH₃,
(301) N-Ac-Sar-Gly-Val-D-Ile-Thr-Ala-Ile-Arg-ProNHCH(CH₃)₂,
(302) N-Ac-Sar-Gly-Val-D-Ile-Thr-Ala-Ile-Arg-Pro-D-AlaNH₂,
(303) N-Ac-Sar-Gly-Val-D-alloIle-Thr-Ala-Ile-Arg-Pro-D-AlaNH₂,
(304) N-Succinyl-Sar-Gly-Val-D-Ile-Thr-Ala-Ile-Arg-Pro-D-AlaNH₂,
(305) N-Ac-Sar-Gly-Val-D-Ile-Ser-Ala-Ile-Arg-ProNHCH₂CH₃,
(306) N-Ac-Sar-Gly-Val-D-Leu-Ser-Ala-Ile-Arg-ProNHCH₂CH₃,
(307) N-Ac-Sar-Gly-Val-D-alloIle-Thr-Val-Ile-Arg-ProNHCH₂CH₃,
(308) N-Ac-Sar-Gly-Val-D-Ile-Thr-Val-Ile-Arg-ProNHCH(CH₃)₂,
(309) N-Ac-Sar-Gly-Val-D-Ile-Thr-Val-Ile-Arg-Pro-D-AlaNH₂,
(310) N-Ac-Sar-Gly-Val-D-alloIle-Thr-Val-Ile-Arg-Pro-D-AlaNH₂,
(311) N-Succinyl-Sar-Gly-Val-D-Ile-Thr-Val-Ile-Arg-Pro-D-AlaNH₂,
(312) N-Ac-Sar-Gly-Val-D-Ile-Ser-Val-Ile-Arg-ProNHCH₂CH₃,
(313) N-Ac-Sar-Gly-Val-D-Leu-Ser-Val-Ile-Arg-ProNHCH₂CH₃,
(314) N-Ac-Sar-Gly-Val-D-alloIle-Thr-D-Nva-Ile-Arg-ProNHCH₂CH₃, (315)N-Ac-Sar-Gly-Val-D-Ile-Thr-D-Nva-Ile-Arg-ProNHCH(CH₃)₂,
(316) N-Ac-Sar-Gly-Val-D-Ile-Thr-D-Nva-Ile-Arg-Pro-D-AlaNH₂,
(317) N-Ac-Sar-Gly-Val-D-alloIle-Thr-D-Nva-Ile-Arg-Pro-D-AlaNH₂,
(318) N-Suceinyl-Sar-Gly-Val-D-Ile-Thr-D-Nva-Ile-Arg-Pro-D-AlaNH₂,
(319) N-Ac-Sar-Gly-Val-D-Ile-Ser-D-Nva-Ile-Arg-ProNHCH₂CH₃,
(320) N-Ac-Sar-Gly-Val-D-Leu-Ser-D-Nva-Ile-Arg-ProNHCH₂CH₃,
(321) N-Ac-Sar-Gly-Val-D-Ile-Ser-Gln-Ile-Arg-ProNHCH₂CH₃,
(322) N-Ac-Sar-Gly-Val-D-Leu-Ser-Gln-Ile-Arg-ProNHCH₂CH₃,
(323) N-Ac-Sar-Gly-Val-D-Leu-Ser-Nva-Ile-Arg-Pro-D-AlaNH₂,
(324) N-Ac-Sar-Gly-Val-D-Ile-Ser-Nva-Ile-Arg-Pro-D-AlaNH₂,
(325) N-Succinyl-Sar-Gly-Val-D-Leu-Ser-Nva-Ile-Arg-ProNHCH₂CH₃,
(326) N-Succinyl-Sar-Gly-Val-D-Ile-Ser-Nva-Ile-Arg-ProNHCH₂CH₃,
(327) N-Succinyl-Sar-Gly-Val-D-Leu-Ser-Gln-Ile-Arg-ProNHCH₂CH₃,
(328) N-Succinyl-Sar-Gly-Val-D-Ile-Ser-Gln-Ile-Arg-ProNHCH₂CH₃,
(329) N-Ac-Sar-Gly-Val-D-Ile-Ser-Ser-Ile-Arg-ProNHCH₂CH₃,
(330) N-Ac-Sar-Gly-Val-D-Leu-Ser-Ser-Ile-Arg-ProNHCH₂CH₃,
(331) N-Ac-Sar-Gly-Val-D-Leu-Ser-Nva-Ile-Arg-ProNHCH₂CH₂CH₃,
(332) N-Ac-Sar-Gly-Val-D-Ile-Ser-Nva-Ile-Arg-ProNHCH₂CH₂CH₃,
(333) N-Ac-Sar-Gly-Val-D-Leu-Ser-Leu-Ile-Arg-ProNHCH₂CH₃,
(334) N-Ac-Sar-Gly-Val-D-Ile-Ser-Leu-Ile-Arg-ProNHCH₂CH₃,
(335) N-Ac-Sar-Gly-Val- D-alloIle-Ser-Nva- Ile-Arg-ProNHCH₂CH₃,
(336) N-Ac-Sar-Gly-Val-D-alloIle-Ser-Gln-Ile-Arg-ProNHCH₂CH₃,
(337) N-Succinyl-Sar-Gly-Val-D-alloIle-Ser-Nva-Ile-Arg-ProNHCH₂CH₃,
(338) N-Ac-Sar-Gly-Val-D-alloIle-Ser-Nva-Ile-Arg-ProNHCH₂CH₂CH₃,
(339) N-Ac-Sar-Gly-Val-D-alloIle-Ser-Nva-Ile-Arg-Pro-D-AlaNH₂,
(340) N-Ac-Sar-Gly-Val-D-alloIle-Ser-Leu-Ile-Arg-ProNHCH₂CH₃,
(341) N-Ac-Sar-Gly-Val-D-alloIle-Ser-Ser-Ile-Arg-ProNHCH₂CH₃,
(342) N-Ac-Sar-Gly-Val-D-Ile-Gly-Nva-Ile-Arg-ProNHCH₂CH₃,
(343) N-Ac-Sar-Gly-Val-D-alloIle-Gly-Nva-Ile-Arg-ProNHCH₂CH₃,
(344) N-Ac-Sar-Gly-Val-D-Leu-Gly-Gln-Ile-Arg-ProNHCH₂CH₃,
(345) N-Ac-Sar-Gly-Val-D-Ile-Gly-Gln-Ile-Arg-ProNHCH₂CH₃,
(346) N-Ac-Sar-Gly-Val-D-alloIle-Gly-Gln-Ile-Arg-ProNHCH₂CH₃,
(347) N-Ac-Sar-Gly-Val-D-Ile-Tyr-Nva-Ile-Arg-ProNHCH₂CH₃,
(348) N-Ac-Sar-Gly-Val-D-alloIle-Tyr-Nva-Ile-Arg-ProNHCH₂CH₃,
(349) N-Ac-Sar-Gly-Val-D-Leu-Tyr-Gln-Ile-Arg-ProNHCH₂CH₃,
(350) N-Ac-Sar-Gly-Val-D-Ile-Tyr-Gln-Ile-Arg-ProNHCH₂CH₃,
(351) N-Ac-Sar-Gly-Val-D-alloIle-Tyr-GIn-Ile-Arg-ProNHCH₂CH₃,
(352) N-Ac-Sar-Gly-Val-D-Ser-Thr-Nva-Ile-Arg-ProNHCH₂CH₃,
(353) N-Ac-Sar-Gly-Val-D-Thr-Thr-Nva-Ile-Arg-ProNHCH₂CH₃,
(354) N-Ac-Sar-Gly-Val-D-Gln-Thr-Nva-Ile-Arg-ProNHCH₂CH₃,
(355) N-Ac-Sar-Gly-Val-D-Asn-Thr-Nva-Ile-Arg-ProNHCH₂CH₃,
(356) N-Ac-Sar-Gly-Val-D-Arg-Thr-Nva-Ile-Arg-ProNHCH₂CH₃,
(357) N-Ac-Sar-Gly-Val-D-3-Pal-Thr-Nva-Ile-Arg-ProNHCH₂CH₃,
(358) N-Ac-Sar-Gly-Val-D-Glu-Thr-Nva-Ile-Arg-ProNHCH₂CH₃,
(359) N-Ac-Sar-Gly-Val-D-Asp-Thr-Nva-Ile-Arg-ProNHCH₂CH₃,
(360) N-Ac-Sar-Gly-Val-D-His-Thr-Nva-Ile-Arg-ProNHCH₂CH₃,
(361) N-Ac-Sar-Gly-Val-D-Hser-Thr-Nva-Ile-Arg-ProNHCH₂CH₃,
(362) N-Ac-Sar-Gly- Val-D-alloThr- Thr-Nva-Ile-Arg-ProNHCH₂CH₃,
(363) N-Ac-Sar-Gly-Val-D-Ile-Thr-Nva-D-Ile-Arg-ProNHCH₂CH₃,
(364) N-Ac-Sar-Gly-Val-D-Ser-Thr-Gln-Ile-Arg-ProNHCH₂CH₃,
(365) N-Ac-Sar-Gly-Val-D-Thr-Thr-Gln-Ile-Arg-ProNHCH₂CH₃,
(366) N-Ac-Sar-Gly-Val-D-alloThr-Thr-Gln-Ile-Arg-ProNHCH₂CH₃,
(367) N-Ac-Sar-Gly-Val-D-Ser-Ser-Nva-Ile-Arg-ProNHCH₂CH₃,
(368) N-Ac-Sar-Gly-Val-D-Thr-Ser-Nva-Ile-Arg-ProNHCH₂CH₃,
(369) N-Ac-Sar-Gly-Val-D-alloThr-Ser-Nva-Ile-Arg-ProNHCH₂CH₃,
(370) N-Ac-Sar-Gly-Val-D-alloThr-Ser-Gln-Ile-Arg-ProNHCH₂CH₃,
(371) N-Ac-Sar-Gly-Val-D-Thr-Ser-Gln-Ile-Arg-ProNHCH₂CH₃,
(372) N-(6-Ac-Aca)-Sar-Gly-Val-D-Leu-Ser-Gln-Ile-Arg-ProNHCH₂CH₃,
(373) N-(6-Ac-Aca)-Sar-Gly-Val-D-Leu-Ser-Nva-Ile-Arg-ProNHCH₂CH₃,
(374) N-(4-Ac-Gaba)-Sar-Gly-Val-D-Leu-Ser-Gln-Ile-Arg-ProNHCH₂CH₃,
(375) N-(4-Ac-Gaba)-Sar-Gly-Val-D-Leu-Ser-Nva-Ile-Arg-ProNHCH₂CH₃,
(376) N-(2-Furoyl)-Sar-Gly-Val-D-Leu-Ser-Gln-Ile-Arg-ProNHCH₂CH₃,
(377) N-(2-Furoyl)-Sar-Gly-Val-D-Leu-Ser-Nva-Ile-Arg-ProNHCH₂CH₃,
(378) N-(Shikimyl)-Sar-Gly-Val-D-Leu-Ser-Gln-Ile-Arg-ProNHCH₂CH₃,
(379) N-(Shikimyl)-Sar-Gly-Val-D-Leu-Ser-Nva-Ile-Arg-ProNHCH₂CH₃,
(382) N-(2-Me-nicotinyl)-Sar-Gly-Val-D-Leu-Ser-Gln-Ile-Arg-ProNHCH₂CH₃,
(383) N- (2-Me-nicotinyl)-Sar-Gly-Val-D-Leu-Ser-Nva-Ile-Arg-ProNHCH₂CH₃,
(384) N-Ac-Sar-Gly-Val-D-Leu-Ser-Nva-Ile-Arg-ProNHethyl-1-(R)-cyclohexyl,
(385) N-Ac-Sar-Gly-Val-D-Leu-Ser-Gln-Ile-Arg-ProNHethyl-1- (R)-cyclohexyl,
(386) N-Ac-Sar-Gly-Val-DIle-Thr-Ser-Ile-Arg-ProNHethyl-1 -(R)-cyclohexyl,
(387) N-Ac-Sar-Gly-Val-D-Leu-Thr-Nva-Ile-Arg-ProNHethyl-1-(R)-cyclohexyl,
(388) N-Ac-Sar-Gly-Val-D-Leu-Ser-Ser-Ile-Arg-ProNHethyl-1-(R)-cyclohexyl,
(389) N-Ac-Sar-Gly- Val-DIle- Thr-Nva-Ile-Arg-ProNHethyl-1-(S)-cyclohexyl,
(390) N-Ac-Sar-Gly-Val-D-Pen-Ser-Nva-Ile-Arg-ProNHCH₂CH₃,
(391) N-Ac-Sar-Gly-Val-D-Pen-Gly-Nva-Ile-Arg-ProNHCH₂CH₃,
(392) N-Ac-Sar-Gly-Val-D-Pen-Thr-Gln-Ile-Arg-ProNHCH₂CH₃,
(393) N-Ac-Sar-Gly-Val-D-Pen-Ser-Nva-Ile-Arg-ProNHCH(CH₃)₂,
(394) N-Succinyl-Sar-Gly-Val-D-Pen-Ser-Nva-Ile-Arg-ProNHCH₂CH₃,
(395) N-Ac-Sar-Gly-Val-D-Pen-Ser-Nva-Ile-Arg-Pro-D-AlaNH₂,
(396) N-Ac-Sar-Gly-Val-D-Pen-Ser-Gln-Ile-Ar,--ProNHCH₂CH₃,
(397) N-Ac-Sar-Gly-Val-D-Pen-Gly-Gln-Ile-Arg-ProNHCH₂CH₃,
(398) N-Ac-Sar-Gly-Val-D-Pen-Ser-Ser-Ile-Arg-ProNHCH₂CH₃,
(399) N-Ac-Sar-Gly-Val-D-Pen-Thr-Ser-Ile-Arg-ProNHCH₂CH₃,
(400) N-Ac-Sar-Gly-Val-D-Pen-Thr-Leu-Ile-Arg-ProNHCH₂CH₃,
(401) N-Ac-Sar-Gly-Val-D-Pen-Ser-Leu-Ile-Arg-PrONHCH₂CH₃,
(402) N-Succinyl-Sar-Gly- V al-D-Pen-Ser-Ser-Ile-Arg-ProNHCH₂CH₃,
(403) N-Succinyl-Sar-Gly-Val-D-Pen-Ser-Leu-Ile-Arg-ProNHCH₂CH₃,
(404) N-Succinyl-Sar-Gly-Val-D-Pen-Thr-Gln-Ile-Arg-ProNHCH (CH₃)₂,
(405) N-Ac-Sar-Gly-Val-D-Cys-Thr-Nva-Ile-Arg-ProNHCH₂CH₃,
(406) N-Ac-Sar-Gly-Val-D-Cys-Ser-Nva-Ile-Arg-ProNHCH₂CH₃,
(407) N-Ac-Sar-Gly-Val-D-Cys-Gly-Nva-Ile-Arg-ProNHCH₂CH₃,
(408) N-Ac-Sar-Gly- Val-D-Cys- Thr-Gln-Ile-Arg-ProNHCH₂CH₃,
(409) N-Ac-Sar-Gly-Val-D-Cys-Ser-Nva-Ile-Arg-ProNHCH (CH₃)₂,
(410) N-Succinyl-Sar-Gly-Val-D-Cys-Ser-Nva-Ile-Arg-ProNHCH₂CH₃,
(411) N-Ac-Sar-Gly-Val-D-Cys-Ser-Nva-Ile-Arg-Pro-D-AlaNH₂,
(412) N-Ac-Sar-Gly-Val-D-Cys-Ser-Gln-Ile-Arg-ProNHCH₂CH₃,
(413) N-Ac-Sar-Gly-Val-D-Cys-Gly-Gln-Ile-Arg-ProNHCH₂CH₃,
(414) N-Ac-Sar-Gly-Val-D-Cys-Ser-Ser-Ile-Arg-ProNHCH₂CH₃,
(415) N-Ac-Sar-Gly-Val-D-Cys-Thr-Ser-Ile-Arg-ProNHCH₂CH₃,
(416) N-Ac-Sar-Gly-Val-D-Cys-Thr-Leu-Ile-Arg-ProNHCH₂CH₃,
(417) N-Ac-Sar-Gly-Val-D-Cys-Ser-Leu-Ile-Arg-ProNHCH₂CH₃,
(418) N-Succmyl-Sar-Gly-Val-D-Cys-Ser-Ser-Ile-Arg-ProNHCH₂CH₃,
(419) N-Succinyl-Sar-Gly-Val-D-Cys-Ser-Leu-Ile-Arg-ProNHCH₂CH₃,
(431) N-Ac-Sar-Gly-Val-D-Leu-Pen-Nva-Ile-Arg-ProNHCH₂CH₃,
(432) N-Ac-Sar-Gly-Val-D-Ile-Pen-Nva-Ile-Arg-ProNHCH₂CH₃,
(433) N-Ac-Sar-Gly- Val-D-alloIle-Pen-Nva-Ile-Arg-ProNHCH₂CH₃,
(434) N-Ac-Sar-Gly-Val-D-Ile-Pen-Gln-Ile-Arg-ProNHCH₂CH₃,
(435) N-Ac-Sar-Gly-Val-D-Ile-Pen-Ser-Ile-Arg-ProNHCH₂CH₃,
(436) N-Ac-Sar-Gly-Val-D-Ile-Pen-Leu-Ile-Arg-ProNHCH₂CH₃,
(437) N-Ac-Sar-Gly-Val-D-Ile-Pen-Nva-Ile-Arg-ProNHCH(CH₃)₂,
(438) N-Ac-Sar-Gly-Val-D-Ile-Pen-Nva-Ile-Arg-Pro-D-AlaNH₂,
(439) N-Succinyl-Sar-Gly- Val-D-lie-Pen-Nva-Ile-Arg-ProNHCH₂CH₃,
(440) N-Succinyl-Sar-Gly-Val-D-Ile-Pen-Gln-Ile-Arg-ProNHCH₂CH₃,
(441) N-Succinyl-Sar-Gly- Val- D-Ile-Pen-Gln- Ile-Arg- ProNHCH(CH₃)₂,
(442) N-Ac-Sar-Gly-Val-D-Ile-Thr-Pen-Ile-Arg-ProNHCH₂CH₂OCH₃,
(443) N-Ac-Sar-Gly-Val-D-alloIle-Thr-Pen-Ile-Arg-ProNHCH₂CH₃,
(444) N-Ac-Sar-Gly-Val-D-Leu- Thr-Pen-Ile-Arg-ProNHCH₂CH₃,
(445) N-Ac-Sar-Gly- V al-D-Ile- Thr-Pen-Ile-Arg-Pro-D-AlaNH₂,
(446) N-Succinyl-Sar-Gly-Val-D-Ile-Thr-Pen-Ile-Arg-ProNHCH₂CH₃,
(447) N-Ac-Sar-Gly- Val-D-Ile- Thr-Pen-Ile-Arg-ProNHCH(CH₃)₂,
(448) N-Ac-Sar-Gly-Val-D-Leu-Ser-Pen-Ile-Arg-ProNHCH₂CH₃,
(449) N-Ac-Sar-Gly-Val-D-Leu-Gly-Pen-Ile-Arg-ProNHCH₂CH₃,
(450) N-Succinyl-Sar-Gly-Val-D-Leu-Ser-Pen-Ile-Arg-ProNHCH₂CH₃,
(451) N-Ac-Sar-Gly-Val-D-Phe (3,4,5-triF)-Thr-Gln-Ile-Arg-ProNHCH₂CH₃,
(452) N-Ac-Sar-Gly-Val-D-Phe (3,4,5-triF)-Ser-Nva-Ile-Arg-ProNHCH₂CH₃,
(453) N-Ac-Sar-Gly-Val-D-Phe (3,4,5-triF)-Gly-Nva-Ile-Arg-ProNHCH₂CH₃,
(454) N-Ac-Sar-Gly-Val-D-Phe (3,4,5-triF)-Ser-Leu-Ile-Arg-ProNHCH₂CH₃,
(455) N-Ac-Sar-Gly-Val-D-Phe (3,4,5-triF)-Ser-Nva-Ile-Arg-Pro-D-AlaNH₂,
(456) N-Succinyl-Sar-Gly-Val-D-Phe (3,4,5-triF)-Thr-Gln-Ile-Arg-ProNHCH₂CH₃,
(457) N-Succinyl-Sar-Gly-Val-D-Phe (3,4,5-triF)-Ser-Gln-Ile-Arg ProNHCH₂CH₃,
(458) N-Succinyl-Sar-Gly-Val-D-Phe (3,4,5-triF)-Thr-Gln-Ile-Arg-ProNHCH (CH₃)₂,
(459) N-Ac-Sar-Gly-Val-D-Phe (3,4,5-triF)-Ser-Gln-Ile-Arg-ProNHCH₂CH₃,
(460) N-Ac-Sar-Gly-Val-D-Phe (3,4,5-triF)-Ser-Ser-Ile-Arg-ProNHCH₂CH₃,
(470) N-(3-Ac-Bala)-Sar-Gly- V al-D-alloIle- Thr-Nva-Ile-Arg-ProNHCH₂CH₃,
(471) N-(3-Ac-Bala)-Sar-Gly-Val-D-Ile-Thr-Gln-Ile-Arg-ProNHCH₂CH₃,
(472) N-(3-Ac-Bala)-Sar-Gly-Val-D-alloIle-Thr-Gln-Ile-Arg-ProNHCH₂CH₃,
(473) N-(3-Ac-Bala)-Sar-Gly-Val-D-Ile-Thr-Gln-Ile-Arg-Pro-DAlaNH₂,
(474) N-(3-Ac-Bala)-Sar-Gly-Val-D-aUolle-Thr-Gln-Ile-Arg-Pro-DAlaNH₂,
(475) N-(3-Ac-Bala)-Sar-Gly-Val-D-alloIle-Thr-Gin-IIe-Arg-ProNHCH(CH₃)₂,
(476) N-(3-Ac-Bala)-Sar-Gly-Val-D-Leu-Ser-Nva-Ile-A-rg-ProNHCH₂CH₃,
(477) N-(3-Ac-Bala)-Sar-Gly-Val-D-Leu-Thr-Nva-Ile-Arg-ProNHCH₂CH₃,
(478) N-(3-Ac-Bala)-Sar-Gly- V al-D-Pen-Thr-Nva-Ile-Arg-ProNHCH₂CH₃,
(479) N-(3-Ac-Bala)-Sar-Gly-Val-D-Ile-Ser-Nva-Ile-Arg-ProNHCH₂CH₃,
(484) N-Ac-Sar-Gly-Val-D-Ile-Thr-Nva-Ile-Arg-Pro-OH,
(485) N-Ac-Sar-Gly-Val-D-alloIle-Thr-Nva-Ile-Arg-Pro-OH,
(486) N-Ac-Sar-Gly-Val-D-Leu-Thr-Nva-Ile-Arg-Pro-OH,
(487) N-Ac-Sar-Gly-Val-D-Pen-Thr-Nva-Ile-Arg-Pro-OH,
(488) N-Ac-Sar-Gly-Val-D-Phe (3,4,5-triF)-Tbr-Nva-Ile-Arg-Pro-OH,
(489) N-Ac-Sar-Gly-Val-D-Ile-Thr-Gln-Ile-Arg-Pro-OH,
(490) N-Ac-Sar-Gly-Val-D-Leu-Ser-Nva-Ile-Arg-Pro-OH,
(492) N-Ac-Sar-Gly-Val-D-Ile-Ser-Gln-Ile-Arg-Pro-OH,
(493) N-Succinyl-Sar-Gly-Val-D-Ile-Thr-Nva-Ile-Arg-Pro-OH,
(494) N-Succinyl-Sar-Gly-Val-D-Leu-Thr-Gln-Ile-Arg-Pro-OH,
(495) N-Ac-Sar-Gly- Val-allo-Ile-Thr-Nva-Ile-Arg-ProNHCH₂CH₃.
(496) N-Ac-Sar-Gly-Val- D-Lys- Thr-Nva-Ile-Arg-ProNHCH₂CH₃,
(497) N-Ac-Sar-Gly- Val-D- Trp-Thr-Nva-Ile-Arg-PruNHCH₂CH₃;
(498) N-Ac-Sar-Gly-Val-D-3.3-Dipheylala-Thr -Nva-Ile-Arg-ProNHCH₂CH₃:
(499) N-Ac-Sar-Gly- Val-D-3-Benzothienylala-Thr-Nva-Ile-Arg-ProNHCN₂CH₃,
(500) N-Ac-Sar-Gly-Val-D-3.4-diF-Phe-Thr-Nva-Ile-Arg-ProNHVH₂CH₃.
(501) N-Ac-Sar-Gly-Val-D-Pen(Bzl)-Thr-Nva-Ile-Arg-ProNHCH₂CH₃,
(502) N-Ac-Sar-Gly- Val-D-Leu-Thr-Gln-Ile-Arg-ProNHCH(CH₃)₂,
(503) H-Sar-Gly- Val-D-Leu- Thr-Gln- Ile-Arg-ProNHCH₂CH₃.
(508) N-Ac-Sar-Gly-Val-D-Ile -Thr-Nva-Ile-Arg-ProNHCH₂CH₂OH and
(510) N-Ac-Sar-Gly-Val-D-Ile -Thr-Leu-Ile-Arg-ProNH((R)-1-cyclohexylethyl)

12. A compound according to Claim 11, selected from:
(1) N-Ac-Sar-Gly- Val-D-Ile- Thr-Nva-Ile-Arg-ProNHCH₂CH₃,
(2) N-Ac-Sar-Gly-Val-D-Ile-Thr-Nva-Ile-Arg-ProNHCH₂CH₂-(1-pyrrolidine),
(3) N-Ac-Sar-Gly-Val-D-Ile-Thr-Nva-Ile-Arg-ProNH (ethyl-1- (R)-cyclohexyl),
(4) N-Ac-Sar-Gly-Val-D-Ile-lhr-Nva-Ile-Arg-ProNH₂,
(5) N-Ac-Sar-Gly-Val-D-Ile-Thr-Nva-Ile-Arg-ProNHCH(CH₃)₂,
(6) N-Ac-Sar-Gly-Val-D-alloIle-Thr-Nva-Ile-Arg-ProNHCH₂CH₃,
(7) N-Ac-Sar-Gly-Val-D-Val-Tbr.-Nva-Ile-Arg-ProNHCH₂CH3,
(8) N-Ac-Sar-Gly-Val-D-Nle-Thr-Nva-Ile-Arg-ProNHCH₂CH₃,
(9) N-Ac-Sar-Gly-Val-D-Phe-Tbr-Nva-Ile-Arg-ProNHCH₂CH₃,
(10) N-Ac-Sar-Gly-Val-D-Cha-Thr-Nva-Ile-Arg-ProNHCH₂CH₃,
(12) N-Ac-Sar-Gly-Val-D-3-ClPhe-Thr-Nva-Ile-Arg-ProNHCH₂CH₃,
(13) N-Ac-Sar-Gly-Val-D-2-Thienylala-Thr-Nva-Ile-Arg-ProNHCH₂CH₃,
(14) N-Ac-Sar-Gly-Val-D-3-CNPhe-Thr-Nva-Ile-Arg-ProNHCH₂CH₃,
(15) N-Ac-Sar-Gly-Val-D-Ile-Thr-Cha-Ile-Arg-ProNHCH₂CH₃,
(16) N[2-THF-C(O)]-Sar-Gly-Val-D-Ile-Thr-Nva-Ile-Arg-ProNHCH₂CH₃,
(17) N[6-N-acetyl-(CH₂)₅C(O)]-Sar-Gly-Val-D-Ile-Thr-Nva-Ile-Arg-ProNHCH₂CH₃,
(19) N- (4-N-Acetylaminobutyryl]-Sar-Gly-Val-D-Ile-Thr-Nva-Ile-Arg ProNHCH₂CH₃,
(23) N-Ac-Sar-Gly-Val-D-Ile-Thr-Leu-Ile-Arg-ProNHCH₂CH₃,
(24) N-Ac-Sar-Gly-Val-D-Ile-Thr-Ser-Ile-Arg-ProNHCH₂CH₃,
(25) N-Ac-Sar-Gly-Val-D-Ile-Thr-Nva-Ile-Arg-Pro-D-AlaNH₂,
(30) N-Ac-Sar-Gly-Val-D-Leu-Ala-Nva-Ile-Arg-ProNHCH₂CH₃,
(31) N-Ac-Sar-Gly-Val-D-Leu-Trp-Nva-Ile-Arg-ProNHCH₂CH₃,
(32) N-Ac-Sar-Gly-Val-D-Leu-Tyr-Nva-Ile-Arg-ProNHCH₂CH₃,
(33) N-Ac-Sar-Gly-Val-D-Leu-Gly-Nva-Ile-Arg-ProNHCH₂CH₃,
(34) N-Ac-Sar-Gly-Val-D-Leu-2Nal-Nva-Ile-Arg-ProNHCH₂CH₃,
(35) N-Ac-Sar-Gly-Val-D-Leu-lNa1-Nva-Ile-Arg-ProNHCH₂CH₃,
(36) N-Ac-Sar-Gly-Val-D-Leu-Octylgly-Nva-Ile-Arg-ProNHCH₂CH₃,
(37) N-Ac-Sar-Gly-Val-D-Leu-Ser-Nva-Ile-Arg-ProNHCH₂CH₃,
(38) N-Ac-Sar-Gly-Val-D-Leu-Allylgly-Nva-Ile-Arg-ProNHCH₂CH₃,
(39) N-Ac-Sar-Gly-Val-D-Leu-D-Thr-Nva-Ile-Arg-ProNHCH₂CH₃,
(40) N-Ac-Sar-Gly-Val-D-Ile-Thr-Tyr-Ile-Arg-ProNHCH_{Z}CH₃,
(41) N-Ac-Sar-Gly-Val-D-Ile-Thr-Glu-Ile-Arg-ProNHCH₂CH₃,
(42) N-Ac-Sar-Gly-Val-D-Ile-Thr-Propargylgly-Ile-Arg-ProNHCH₂CH₃,
(43) N-Ac-Sar-Gly- Val-D-alloIle- Thr-Gln-Ile-Arg-ProNHCH₂CH₃,
(44) N-Ac-Bala-Sar-Gly-Val-D-Ile-Thr-Nva-Ile-Arg-ProNHCH₂CH₃,
(45) N-Phenylacetyl-Sar-Gly-Val-D-Ile-Thr-Nva-Ile-Arg-ProNHCH₂CH₃,
(46) N-Ac-Sar-Gly-Val-D-Ile-Thr-Nva- Ile-Arg-Pro-AzaglyNH₂,
(47) N-Ac-Sar-Gly-Val-D-Ile-Thr-Nva-Ile-Arg-Pro-SerNH₂,
(48)N-(6-Ac-Aca)-Sar-Gly-Val-D-Leu-Ser-Gln-Ile-Arg-ProNHCH₂CH₃,
(49) N-(6-Ac-Aca)-Sar-Gly-Val-D-Leu-Ser-Nva-Ile-Arg-ProNHCH₂CH₃,
(50) N-(4-Ac-Gaba)-Sar-Gly-Val-D-Leu-Ser-Gln-Ile-Arg-ProNHCH₂CH₃,
(51) N-(4-Ac-Gaba)-Sar-Gly-Val-D-Leu-Ser-Nva-Ile-Arg-ProNHCH₂CH₃,
(52) N-(2-Furoyl)-Sar-Gly-Val-D-Leu-Ser-Gln-Ile-Arg-ProNHCH₂CH₃,
(53) N-(2-Furoyl)-Sar-Gly-Val-D-Leu-Ser-Nva-Ile-Arg-ProNHCH₂CH₃,
(54) N -(Shikimyl)-Sar-Gly- V al-D-Leu-Ser-Gln- Ile-Arg- ProNHCH₂CH₃,
(55) N-(Shikimyl)-Sar-Gly-Val-D-Leu-Ser-Nva-Ile-Arg-ProNHCH₂CH₃,
(58) N-(2-Me-nicotinyl)-Sar-Gly- Val- D-Leu-Ser-Gln- Ile-Arg ProNHCH₂CH₃,
(59) N- (2-Me-nicotinyl)-Sar-Gly-Val-D-Leu-Ser-Nva-Ile-Arg-ProNHCH₂CH₃,
(60) N-Ac-Sar-Gly-Val-D-Ile-Thr-Nva-Ile-Arg-Pro-OH,
(62) N-Ac-Sar-Gly-Val-D-Pen-Thr-Nva-Ile-Arg-ProNHCH₂CH₃,
(63) N-Ac-Sar-Gly-Val-D-Phe (3,4,5-triF)-Thr-Nva-Ile-Arg-ProNHCH₂CH₃, and
(64) N-Ac-Sar-Gly-Val-D-Phe (4-NH₂)-Thr-Nva-Ile-Arg-ProNHCH₂CH₃.

13. A pharmaceutical composition comprising a compound of Claim 1 and a pharmaceutically acceptable carrier.

14. Use of a therapeutically effective amount of a compound in Claim 1 for manufacturing a medicament for treating a patient in need of anti-angiogenesis therapy.

15. A composition for the treatment of a disease selected from cancer, arthritis, psoriasis, angiogenesis of the eye associated with infection or surgical intervention, macular degeneration and diabetic retinopathy comprising a peptide as defined in Claim 1 in combination with a pharmaceutically acceptable carrier.

16. A compound according to Claim 11, selected from:
N-Ac-Sar-Gly-Val-D-Ile-Thr-Nva-Ile-Arg-ProNH₂CH₃
N-Ac-Syr-Gly-Val-D-allolle-Thr-Nva-Ile-Arg-Pro-NHCH₂CH₃
N- Ac-Sar -Gly-Val-D-Ile-Thr-Gln-Ile-Arg-ProNHCH₂CH₃and
N-Ac-Sar-Gly-Val-D-allolle-Ser-Ser-Arg-Arg-ProNHCH₂CH₃,

17. A compound according to Claim 16, which is N-Ac-Sar-Gly-Val-D-Ile-Thr-Nva-Ile-ArK-ProNHCH₂CH₃

18. A compound according to Claim 16, which is N-Ac-Sar-Gly-Val-D-alloIle-Thr-Nva-Ile-Arg-proNHCH₂-CH₃.

19. A compound according to Claim 16, which is N-Ac-Sar-Gly-Val-D-Ile-Thr-Gln-Ile-Arg-ProNHCH₂CH₃.

20. A compound according to Claim 16 , which is N-Ac-Sar-Gly-Val-D-alloIle-Ser -Ser-Ile-Arg-ProNHCH₂CH₃.

21. The composition according to Claim 13, comprising a compound or a pharmaceutically acceptable salt ester solvate, or prodrug thereof, selected from the group consisting of
N-Ac-Sar-Gly-Val-D-Ile-Thr-Nva-Ile-Arg-ProNHCH₂CH₃,
N-Ac-Sar-Gly-Val-D-alloIle-Thr-Nva-Ile-Arg-ProNHCH₂CH₃,
N-Ac-Sar-Gly-Val-D-Ile-Thr-Glr-Ile-Arg-ProNHCH₂CH₂, and
N-Ac-Sar-Gly-Val-D-alloIle-Ser-Ser-Ile-Arg-ProNHCH₂CH₃.
and a pharmaceutically acceptable carrier.

22. The composition according to Claim 21, comprising the compound N-Ac-Sar-Gly-Val-D-Ile-Thr-Nva-Ile-Arg-ProNHCH₂CH₃ or a pharmaceutically acceptable salt, ester, solvate, or prodrug thereof and a pharmaceutically acceptable carrier.

23. The composition according to Claim 21, comprising the compound N-Ac-Sar-Gly-Val-D-allolle-Thr-Nva-Ile-Pro-NHCH₂CH₃, or a pharmaceutically acceptable salt, ester, solvate, or prodrug thereof, and a pharmaceutically acceptable carrier.

24. The composition according to Claim 21, comprising the compound N-Ac-Sar-Gly-Val-D-Ile-Thr-Gln-Ile-Arg-ProNHCH₂CH₃, or a pharmaceutically acceptable salt, ester, solvate, or prodrug thereof, and a pharmaceutically acceptable carrier.

25. The composition according to Claim 21, comprising the compound N-Ac-Sar-Gly-Val-D-alloIle-Ser-Ser-Ile-Arg-ProNHCH₂CH₃, or a pharmaceutically acceptable salt, ester solvate, or prodrug thereof and a pharmaceutically acceptable carrier.

26. The composition according to Claim 15 comprising a compound, or a pharmaceutically acceptable salt, ester solvate or prodrug thereof, selected from the group consisting of
N-Ac-Sar-Gly-Val-D-Ile-Thr-Nva-Ile-Arg-ProNHCH₂CH₃.
N-Ac-Sar-Gly-Val-D-alloIle-Thr-Nya-Ile-Arg-ProNHCH₂CH₃.
N-Ac-Sar-Gly-Val- D-Tle- Thr-Gln- Ile-Arg- ProNHCH₂CH₃. and
N-Ac-Sar-Gly-Val-D-alloIle-Ser-Ser-Ile-Arg-ProNHCH₂CH₃.

27. The composition according to Claim 26 comprising the compound N-Ac-Sar-Gly-Val-D-Tle-Thr-Nva-Ile-Arg-ProNHCH₂CH₃ or a pharmaceutically acceptable salt, ester solvate, or prodrug thereof

28. The composition according, to Claim 26 comprising the compound N-Ac-Sar-Gly-Val-D-alloIle-Thr-Nva-Ile-Arg-ProNHCH₂CH₃ or a pharmaceutically acceptable salt, ester, solvate or prodrug thereof

29. The composition according to Claim 2.6 comprising, the compound N-Ac-Sar-Glv-Val-D-Ile-Thr-Gln-Ile-agr-ProNHCH₂CH₃ or a pharmaceutically acceptable salt, ester, solvate or prodrug thereof

30. The composition according to Claim 26 comprising the compound N-Ac-Sar-Gly-Val-D-allolle-Ser-Ser-Ile-Arg-ProNHCH₂CH₃ or a pharmaceutically acceptable salt ester solvate, or prodrug thereof.

31. The use of Claim 14 wherein the patient is a non-human animal.

32. The use of Claim 14 wherein the patient is a human.

33. The use of Claim 14 comprising a compound of any of claims 17, 18, 19 or 20.

34. The use of Claim 33 wherein the patient is a non-human animal.

35. The use of Claim 33 wherein the patient is a human.

## Patentansprüche

1. Eine Verbindung der Formel:
A₀-A₁-A₂-A₃-A₄-A₅-A₆-A₇-A₈-A₉-A₁₀
oder ein pharmazeutisch verträgliches Salz, Ester, Solvat oder Prodrug davon, worin:
A₁ Sarcosyl ist, A₂ Glycyl ist, A₃ Valyl ist, A₇ Isoleucyl ist, A₈ Arginyl ist, A₉ Prolyl ist und A₀, A₄, A₅, A₆ und A₁₀ sind wie folgt:
A₀ ist Wasserstoff oder eine Acylgruppe, gewählt aus:
(1) R-(CH₂)ₙ-C(O)-; worin n eine ganze Zahl ist, gewählt aus 0, 1, 2, 3 und 5, und R ist gewählt aus Methyl; N-Acetylamino; Methoxyl; Carboxyl; Cyclohexyl; Cyclohexyl, enthaltend eine Doppelbindung und substiuiert mit drei Hydroxylgruppen; und ein 5- oder 6-gliedriger aromatischer oder nicht aromatischer Ring, wahlweise enthaltend ein Heteroatom, gewählt aus Stickstoff und Sauerstoff, worin der Ring wahlweise substituiert ist mit Alkyl; und
(2) R¹-CH₂CH₂-(OCH₂CH₂₀)ₚ-CH₂-C(O)-; worin R¹ N-Acetylamino ist und p ist 1;
A₄ ist ein Aminoacylrest der L oder D Konfiguration, gewählt aus:
(1) Allo-isoleucyl,
(2) Glycyl,
(3) Isoleucyl,
(5) Dehydroleucyl,
(6) D-Alanyl,
(7) D-3-(Naphth-1-yl)alanyl,
(8) D-3-(Naphth-2-yl)alanyl,
(9) D-(3-Pyridyl)-alanyl,
(10) D-2-Aminobutyryl,
(11) D-Allo-isoleucyl,
(12) D-Allo-threonyl,
(14) D-Asparaginyl,
(15) D-Aspartyl,
(16) D-3-(3-Benzothienyl)alanyl,
(17) D-3-(4,4-Biphenyl)alanyl,
(18) D-3-(3-Chlorphenyl)alanyl,
(19) D-3-(3-Trifluormethylphenyl)alanyl,
(20) D-3-(3-Cyanophenyl)alanyl,
(21) D-3-(3,4-Difluorphenyl)alanyl,
(22) D-Citrullyl,
(23) D-Cyclohexylalanyl,
(24) D-Cyclohexyglycyl,
(25) D-Cystyl,
(26) D-Cystyl(S-t-butyl),
(27) D-Glutaminyl,
(28) D-Glutamyl,
(29) D-Histidyl,
(31) D-Homophenylalanyl,
(32) D-Homoseryl,
(33) D-Isoleucyl,
(34) D-Leucyl,
(36) D-Lysyl,
(37) D-Methionyl,
(38) D-Neopentylglycyl,
(39) D-Norleucyl,
(41) D-Ornithyl,
(42) D-Penicillaminyl,
(43) D-Penicillaminyl(acetamidomethyl),
(44) D-Penicillaminyl(S-benzyl),
(45) D-Phenylalanyl,
(46) D-3-(4-Aminophenyl)alanyl,
(47) D-3-(4-Methylphenyl)alanyl,
(48) D-3-(4-Nitrophenyl)alanyl,
(49) D-3-(3,4-Dimethoxyphenyl)alanyl,
(50) D-3-(3,4,5-Trifluorphenyl)alanyl,
(52) D-Seryl,
(53) D-Seryl(*O*-benzyl)
(54) D-t-Butylglycyl,
(55) D-Thienylalanyl,
(56) D-Threonyl,
(57) D-Threonyl(*O*-Benzyl),
(58) D-Tryptyl,
(59) D-Tyrosyl(O-benzyl),
(60) D-Tyrosyl(O-ethyl),
(61) D-Tyrosyl,
(62) D-Valyl; und
(63) D-3-(4-Chlorphenyl)alanyl;
A₅ ist ein Aminoacylrest, gewählt aus:
(1) Alanyl,
(3) 3-(Naphth-1-yl)alanyl,
(4) 3-(Naphth-2-yl)alanyl,
(6) Allylglycyl,
(7) Glutaminyl,
(8) Glycyl,
(10) Homoseryl,
(11) Isoleucyl,
(12) Lysyl(N-epsilon-acetyl),
(13) Methionyl,
(14) Norvalyl,
(15) Octylglycyl,
(17) 3-(4-Hydroxymethylphenyl)alanyl,
(18) Prolyl,
(19) Seryl,
(20) Threonyl,
(21) Tryptyl,
(22) Tyrosyl,
(26) D-Thronyl, und
(27) Penicillaminyl;
A₆ ist ein Aminoacylrest, gewählt aus:
(1) Alanyl,
(5) 2-Aminobutyryl,
(6) Allylglycyl,
(7) Arginyl,
(8) Asparaginyl,
(9) Aspartyl,
(10) Citrullyl,
(11) 3-(Cyclohexyl)alanyl,
(12) Glutaminyl,
(13) Glutamyl,
(14) Glycyl,
(19) Isoleucyl,
(20) Leucyl,
(21) Lysyl(N-epsilon-acetyl),
(23) Methionyl(sulfon),
(24) Methionyl(sulfoxid),
(25) Methionyl,
(26) Norleucyl,
(27) Norvalyl,
(28) Octylglycyl,
(30) 3-(4-Carboxyamidphenyl)alanyl,
(31) Propargylglycyl,
(32) Seryl,
(35) Tyrosyl,
(36) Valyl,
(42) D-Norvalyl, und
(44) Penicillaminyl;
A₁₀ ist gewählt aus:
(1) Hydroxyl,
(2) Azaglycylamid,
(3) D-Alanylamid,
(7) Sarcosylamid,
(8) Serylamid,
(9) D-Serylamid,
(10) einer Gruppe dargestellt durch die Formel 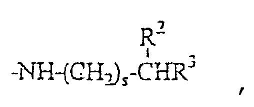 und
(11) einer Gruppe, dargestellt durch die Formel -NH-R⁴;
worin:
s eine ganze Zahl ist, gewählt aus 0 und 1,
R² gewählt ist aus Wasserstoff, Alkyl und einem 6-gliedrigen Cycloalkylring;
R₃ gewählt ist aus Wasserstoff, Hydroxy, Alkyl, Alkoxy und einem 6-gliedrigen Ring, enthaltend einen Stickstoff, vorausgesetzt, daß s nicht null ist, wenn R³ Hydroxy oder Alkoxy ist, und
R⁴ ist gewählt aus Wasserstoff und Hydroxy.

2. Eine Verbindung gemäß Anspruch 1, worin A₄ ein Aminoacylrest ist, gewählt aus:
(1) D-Alanyl,
(2) D-3-(Naphth-1-yl)alanyl,
(3) D-3-(Naphth-2-yl)alanyl,
(4) D-(3-Pyridyl)-alanyl,
(5) D-2-Aminobutyryl,
(6) D-Allo-isoleucyl,
(7) D-Allo-threonyl,
(9) D-Asparaginyl,
(10) D-Aspartyl,
(11) D-3-(3-Chlorphenyl)alanyl,
(12) D-3-(3-Trifluormethylphenyl)alanyl,
(13) D-3-(3-Cyanophenyl)alanyl,
(14) D-3-(3,4-Difluorphenyl)alanyl,
(15) D-Cyclohexylalanyl,
(16) D-Cyclohexylglycyl,
(17) D-Cystyl,
(18) D-Glutaminyl,
(19) D-Glutamyl,
(20) D-Histidyl,
(22) D-Homophenylalanyl,
(23) D-Homoseryl,
(24) D-Isoleucyl,
(25) D-Leucyl,
(27) D-Methionyl,
(28) D-Neopentylglycyl,
(29) D-Norleucyl,
(31) D-Penicillaminyl,
(32) D-Penicillaminyl(acetamidomethyl),
(33) D-Penicillaminyl(S-benzyl),
(34) D-Phenylalanyl,
(35) D-3-(4-Aminophenyl)alanyl,
(36) D-3-(4-Methylphenyl)alanyl,
(37) D-3-(4-Nitrophenyl)alanyl,
(38) D-3-(3,4-Dimethoxyphenyl)alanyl,
(39) D-3-(3,4,5-Trifluorphenyl)alanyl,
(41) D-Seryl,
(42) D-Seryl(O-benzyl),
(43) D-t-Butylglycyl,
(44) D-Thienylalanyl,
(45) D-Threonyl,
(46) D-Threonyl(O-benzyl)
(47) D-Tyrosyl(O-ethyl),
(48) D-Tyrosyl,
(49) D-Valyl, und
(50) D-3(4-Chlorphenyl)alanyl.

3. Eine Verbindung gemäß Anspruch 2, worin A₄ ein Aminoacylrest ist, gewählt aus:
(1) D-Allo-isoleucyl,
(3) D-3-(3-Cyanophenyl)alanyl,
(4) D-Cystyl,
(5) D-Isoleucyl,
(6) D-Leucyl,
(7) D-Penicillaminyl,
(8) D-Phenylalanyl,
(9) D-3-(3,4,5-Trifluorphenyl)alanyl, und
(10) D-3-(4-Aminophenyl)alanyl.

4. Eine Verbindung gemäß Anspruch 1, worin A₅ gewählt ist aus:
(1) Glycyl,
(2) Octylglycyl,
(3) Penicillaminyl,
(4) Seryl,
(5) Threonyl, und
(6) Tyrosyl.

5. Eine Verbindung gemäß Anspruch 1, worin A₆ gewählt ist aus:
(1) Glutaminyl,
(2) Leucyl,
(3) Norvalyl, und
(4) Seryl.

6. Eine Verbindung gemäß Anspruch 2, worin A₀ gewählt ist aus:
(1) Acetyl,
(2) Butyryl,
(5) N-Acetyl-beta-alanyl,
(6) (6-N-Acetylamino)caproyl,
(8) Cyclohexylacetyl,
(9) Furoyl,
(10) Gamma-Aminobutyryl,
(11) 2-Methoxyacetyl,
(12) Methylnicotinyl,
(13) Nicotinyl,
(15) Phenylacetyl,
(16) Propionyl,
(17) Shikimyl,
(18) Succinyl, und
(19) Tetrahydrofuroyl.

7. Eine Verbindung gemäß Anspruch 2, worin A₁₀ gewählt ist aus:
(1) D-Alanylamid,
(2) Azaglycylamid,
(3) Serylamid,
(4) Ethylamid,
(5) Hydroxylamid,
(6) Isopropylamid,
(7) Propylamid,
(8) 2-(Cyclohexyl)ethylamid,
(9) 2-(1-Pyrrolidin)ethylamid,
(10) 1-(Cyclohexyl)ethylamid,
(11) 2-(Methoxy)ethylamid, und
(12) 2-(Hydroxy)ethylamid.

8. Eine Verbindung gemäß Anspruch 1, worin A₄ ein Aminoacylrest ist, gewählt aus:
(1) D-Allo-isoleucyl,
(3) D-3-(3-Cyanophenyl)alanyl,
(4) D-Cystyl,
(5) D-Isoleucyl,
(6) D-Leucyl,
(7) D-Penicillaminyl,
(8) D-Phenylalanyl,
(9) D-3-(3,4,5-trifluorphenyl)alanyl, und
(10) D-3-(4-Aminophenyl)alanyl;
A₅ ist ein Aminoacylrest gewählt aus:
(1) Octylglycyl,
(2) Glycyl,
(3) Penicillaminyl,
(4) Seryl,
(5) Threonyl, und
(6) Tyrosyl; und
A₆ ist ein Aminoacylrest gewählt aus:
(1) Glutaminyl,
(2) Leucyl,
(3) Norvalyl, und
(4) Seryl.

9. Eine Verbindung gemäß Anspruch 8, worin A₀ gewählt ist aus:
(1) Acetyl,
(2) Butyryl,
(5) N-Acetyl-beta-alanyl,
(6) (6-N-Acetylamino)caproyl,
(8) Cyclohexylacetyl,
(9) Furoyl,
(10) Gamma-Aminobutyryl,
(11) 2-Methoxyacetyl,
(12) Methylnicotinyl,
(13) Nicotinyl,
(15) Phenylacetyl,
(16) Propionyl,
(17) Shikimyl,
(18) Succinyl, und
(19) Tetrahydrofuroyl.

10. Eine Verbindung gemäß Anspruch 8, worin A₁₀ gewählt ist aus
(1) D-Alanylamid,
(2) Azaglycylamid,
(3) Serylamid,
(4) Ethylamid,
(5) Hydroxylamid,
(6) Isopropylamid,
(7) Propylamid,
(8) 2-(Cyclohexyl)ethylamid,
(9) 2-(1-Pyrrolidin)ethylamid,
(10) 1-(Cyclohexyl)ethylamid,
(11) 2-(Methoxy)ethylamid, und
(12) 2-(Hydroxy)ethylamid.

11. Eine Verbindung gemäß Anspruch 1, oder ein pharmazeutisch verträgliches Salz, Ester, Solvat oder Prodrug davon, gewählt aus:
(1) N-Ac-Sar-Gly-Val-D-Ile-Thr-Nva-Ile-Arg-ProNHCH₂CH₃,
(2) pyroGlu-Gly-Val-D-Ile-Thr-Nva-Ile-Arg-ProNHCH₂CH₃,
(3) N-Ac-Sar-Gly-Val-D-Ile-Thr-Nva-Ile-Arg-ProNHCH₃,
(4) N-Ac-Sar-Gly-Val-D-Ile-Thr-Nva-Ile-Arg-ProNHCH(CH₃)₂,
(5) N-Ac-Sar-Gly-Val-D-Ile-Thr-Nva-Ile-Arg-ProNHCH₂CH₂-(1-pyrrolidine),
(6) N-Ac-Sar-Gly-Val-D-Ile-Thr-,Nva-Ile-Arg-ProNHethyl(1-piperidine),
(7) N-Ac-Sar-Gly-Val-D-Ile-Thr-Nva-Ile-Arg-ProNHmethylcyclopropyl,
(8) N-Ac-Sar-Gly-Val-D-Ile-Thr-Nva-Ile-Arg-ProNH (ethyl-1-(R)-cyclohexyl),
(9) N-Ac-Sar-Gly-Val-D-Ile-Thr-Nva-Ile-Arg-ProNH₂,
(10) N-Ac-Sar-Gly-Val-D-Ile-Thr-Nva-Ile-Arg-ProNHCH₂CH₂OCH₃,
(11) N-Ac-Sar-Gly-Val-D-Ile-Thr-Nva-Ile-Arg-ProNHCH₂CH₂cyclohexyl,
(12) N-Ac-Sar-Gly-Val-D-Ile-Thr-Nva-Ile-Arg-ProNHCH₂CH₂CH₃,
(13) N-Ac-Sar-Gly-Val-D-alloIle-Thr-Nva-Ile-Arg-ProNHCH₂CH₃,
(14) N-Ac-Sar-Gly-Val-D-Leu-Thr-Nva-Ile-Arg-ProNHCH₂CH₃,
(15) N-Ac-Sar-Gly-Val-Ile-Thr-Nva-Ile-Arg-ProNHCH₂CH₃,
(16) N-Ac-Sar-Gly-Val-Gly-Thr-Nva-Ile-Arg-ProNHCH₂CH₃,
(17) N-Ac-Sar-Gly-Val-D-VaI-Thr-Nva-Ile-Arg-ProNHCH₂CH₃,
(18) N-Ac-Sar-Gly-Val-D-Ala-Thr-Nva-Ile-Arg-ProNHCH₂CH₃,
(19) N-Ac-Sar-Gly-Val-D-Met-Thr-Nva-Ile-Arg-ProNHCH₂CH₃,
(20) N-Ac-Sar-Gly-Val-D-Nle-Thr-Nva-11e-Arg-ProNHCH₂CH₃,
(21) N-Ac-Sar-Gly- Val-D-Phe- Thr-Nva-Ile-Arg-ProNHCH₂CH₃,
(22) N-Ac-Sar-Gly-Val-D-Tyr-Thr-Nva-Ile-Arg-ProNHCH₂CH₃,
(23) N-Ac-Sar-Gly-VaI-D-4,4-BiphenylaIa-Thr-Nva-Ile-Arg-ProNHCH₂CH₃,
(24) N-Ac-Sar-Gly- Val-D-Cha-Thr-Nva- He-Arg- ProNHCH₂CH₃,
(25) N-Ac-Sar-Gly-Val-D-Chg-Thr-Nva-Ile-Arg-ProNHCH₂CH₃,
(26) N-Ac-Sar-Gly-Val-D-4-ClPhe-Thr-Nva-Ile-Arg-ProNHCH₂CH₃,
(27) N-Ac-Sar-Gly- Val-D-Hphe- Thr-Nva-Ile-Arg-ProNHCH₂CH₃,
(28) N-Ac-Sar-Gly-Val-Dehydroleu-Thr-Nva-Ile-Arg-ProNHCH₂CH₃,
(29) N-Ac-Sar-Gly-Val-D-3-CF3Phe-Thr-Nva-Ile-Arg-ProNHCH₂CH₃,
(32) N-Ac-Sar-Gly-Val-D-3-ClPhe-Thr-Nva-Ile-Arg-ProNHCH₂CH₃,
(33) N-Ac-Sar-Gly-Val-D-2-Thienylala-Thr-Nva-Ile-Arg-ProNHCH₂CH₃,
(34) N-Ac-Sar-Gly- Val-D-3-CNPhe- Thr-Nva-Ile-Arg-ProNHCH₂CH₃,
(35) N-Ac-Sar-Gly-Val-D-Ile-Thr-DNva-Ile-Arg-ProNHCH₂CH₃,
(36) N-Ac-Sar-Gly-Val-D-Ile-Thr-Gln-Ile-Arg-ProNHCH₂CH₃,
(37) N-Ac-Sar-Gly-Val-D-Ile-Thr-Cha-Ile-Arg-ProNHCH₂CH₃,
(38) N-Ac-Sar-Gly-Val-D-Ile-Thr-Gly-Ile-Arg-ProNHCH₂CH₃,
(39) N-Ac-Sar-Gly-Val-D-Ile-Thr-Ala-Ile-Arg-ProNHCH₂CH₃,
(40) N-Ac-Sar-Gly- Val-D-Ile-Thr- Val-Ile-Arg-ProNHCH₂CH₃.
(41) N-Ac-Sar-Gly-Val-D-Ile-Thr-Abu-Ile-Arg-ProNHCH₂CH₃,
(42) N-Ac-Sar-Gly-Val-D-Ile-Thr-Allylgly-Ile-Arg-ProNHCH₂CH₃,
(43) N-Ac-Sar-Gly- Val-D-Ile- Thr-Octylgly-Ile-Arg-ProNHCH₂CH₃,
(44) N-Ac-Sar-Gly- Val-D-Ile- Thr-Met-Ile-Arg-ProNHCH₂CH₃,
(45) N-Cyclohexylacetyl-Sar-Gly-Val-D-Ile-Thr-Nva-Ile-Arg-ProNHCH₂CH,
(46) N- (2-Me-Nicotinyl)-Sar-Gly-Val-D-Ile-Thr-Nva-Ile-Arg-ProNHCH₂CH₃,
(47) N-Succinyl-Sar-Gly-Val-D-Ile-Thr-Nva-Ile-Arg-ProNHCH₂CH₃,
(48) N-Nicotinyl-Sar-Gly-Val-D-Ile-Thr-Nva-Ile-Arg-ProNHCH₂CH₃,
(49) N-Propionyl-Sar-Gly-Val-D-Ile-Thr-Nva-Ile-Arg-ProNHCH₂CH₃,
(50) N-(MeO) acetyl-Sar-Gly-Val-D-Ile-Thr-Nva-Ile-Arg-ProNHCH₂CH₃,
(51) N-(Shikimyl)-Sar-Gly-Val-D-Ile-Thr-Nva-Ile-Arg-ProNHCH₂CH₃,
(52) N-(2-Furoyl)-Sar-Gly-Val-D-Ile-Thr-Nva-Ile-Arg-ProNHCH₂CH₃,
(53) N-Butyryl-Sar-Gly-Val-D-Ile-Thr-Nva-Ile-Arg-ProNHCH₂CH₃,
(54) N-[2-THFcarbonyl]-Sar-Gly-Val-D-Ile-Thr-Nva-Ile-Arg-ProNHCH₂CH₃,
(55) N-[CH₃C(O)NH-(CH₂)₂-O-(CH₂)₂-O-CH₂ (O)]-Sar-Gly-Val-D-Ile-Thr-Nva-Ile-Arg-ProNHCH₂CH₃,
(56) N-[6-N-acetyl-(CH₂)₅C(O)]-Sar-Gly-Val-D-Ile-Thr-Nva-Ile-Arg-ProNHCH₂CH₃,
(58) N- [4-N-Acetylaminobutyryl]-Sar-Gly-Val-D-Ile-Thr-Nva-Ile-Arg-ProNHCH₂CH₃,
(59) H-Sar-Gly-Val-D-Ile-Thr-Nva-Ile-Arg-PrONHCH₂CH₃,
(66) N-Ac-Sar-Gly-Val-D-Ile-Thr-Leu-Ile-Arg-ProNHCH₂CH₃,
(67) N-Ac-Sar-Gly-Val-D-Ile-Thr-Ser-lle-Arg-ProNHCH₂CH₃,
(68) N-Ac-Sar-Gly-Val-D-Ile-Thr-Nva-Ile-Arg-Pro-D-AlaNH₂,
(77) N-Ac-Sar-Gly-Val-D-Tyr (Et)-Thr-Nva-Ile-Arg-ProNHCH₂CH₃,
(78) N-Ac-Sar-Gly-Val-D-Cys (tBu)-Thr-Nva-Ile-Arg-ProNHCH₂CH₃,
(79) N-Ac-Sar-Gly- Val-D-Cys- Thr-Nva-Ile-Arg-ProNHCH₂CH₃,
(80) N-Ac-Sar-Gly-Val-D-Tyr (Bzl)-Thr-Nva-Ile-Arg-ProNHCH₂CH₃,
(81) N-Ac-Sar-Gly-Val-D-Ser (Bzl)-Thr-Nva-Ile-Arg-ProNHCH₂CH₃,
(82) N-Ac-Sar-Gly-Val-D-lNa1-Thr-Nva-Ile-Arg-ProNHCH₂CH₃,
(83) N-Ac-Sar-Gly-Val-D-tButylgly-Thr-Nva-Ile-Arg-ProNHCH₂CH₃,
(84) N-Ac-Sar-Gly-Val-D-Orn-Thr-Nva-Ile-Arg-ProNHCH₂CH₃,
(85) N-Ac-Sar-Gly-Val-D-Thr (Bzl)-Thr-Nva-Ile-Arg-ProNHCH₂CH_{3,}
(86) N-Ac-Sar-Gly-Val-D-2Nal-Thr-Nva-Ile-Arg-ProNHCH₂CH₃,
(87) N-Ac-Sar-Gly-Val-D-Phe (4-Me)-Thr-Nva-lle-Arg-ProNHCH₂CH₃,
(88) N-Ac-Sar-Gly-Val-D-Phe (3,4-diMeO)-Trhr-Nva-Ile-Arg-ProNHCH₂CH₃,
(89) N-Ac-Sar-Gly-Val-D-Phe (3,4,5-triF)-Thr-Nva-Ile-Arg-ProNHCH₂CH₃,
(90) N-Ac-Sar-Gly-Val-D-Phe (4-NO₂)-Thr-Nva-Ile-Arg-ProNHCH₂CH₃,
(91) N-Ac-Sar-Gly-Val-D-Pen-Thr-Nva-Ile-Arg-ProNHCH₂CH₃,
(92) N-Ac-Sar-Gly-Val-D-Pen (Acm)- Thr-Nva-Ile-Arg-ProNHCH₂CH₃,
(93) N-Ac-Sar-Gly-Val-D-Abu-Thr-Nva-Ile-Arg-ProNHCH₂CH₃,
(94) N-Ac-Sar-Gly-Val-D-Phe (4-NH₂)-Thr-Nva-Ile-Arg-ProNHCH₂CH₃,
(108) N-Ac-Sar-Gly-Val-D-Leu-Ala-Nva-Ile-Arg-ProNHCH₂CH₃,
(109) N-Ac-Sar-Gly- Val-D-Leu-Pro-Nva-Ile-Arg-ProNHCH₂CH₃,
(110) N-Ac-Sar-Gly-Val-D-Leu-Trp-Nva-Ile-Arg-ProNHCH₂CH₃,
(111) N-Ac-Sar-Gly-Val-D-Leu-Tyr-Nva-Ile-Arg-ProNHCH₂CH₃,
(112) N-Ac-Sar-Gly-Val-D-Leu-Nva-Nva-Ile-Arg-ProNHCH₂CH₃,
(113) N-Ac-Sar-Gly-Val-D-Leu-Gly-Nva-Ile-Arg-ProNHCH₂CH₃,
(114) N-Ac-Sar-Gly-Val-D-Leu-Lys (Ac)-Nva-Ile-Arg-ProNHCH₂CH₃,
(115) N-Ac-Sar-Gly-Val-D-Leu-2Nal-Nva-IIe-Arg-ProNHCH₂CH₃,
(116) N-Ac-Sar-Gly-Val-D-Leu-INal-Nva-Ile-Arg-ProNHCH-₂CH₃,
(117) N-Ac-Sar-Gly-Val-D-Leu-Octylgly-Nva-Ile-Arg-ProNHCH₂CH₃,
(118) N-Ac-Sar-G ly-Val.D-Leu-Gln-Nva-Ile-Arg-ProNHCH₂CH₃,
(119) N-Ac-Sar-Gly-Val-D-Leu-Met-Nva-Ile-Arg-ProNHCH₂CH₃,
(120) N-Ac-Sar-Gly-Val-D-Leu-Ser-Nva-Ile-ATg-ProNHCH₂CH₃,
(121) N-Ac-Sar-Gly-Val-D-Leu-Aliylgly-Nva-Ile-Arg-ProNHCH₂CH₃,
(122) N-Ac-Sar-Gly-Val-D-Leu-Ile-Nva-Ile-Arg-ProNHCH₂CH₃,
(123) N-Ac-Sar-Gly-Val-D-Leu-D-Thr-Nva-Ile-Arg-ProNHCH₂CH₃,
(124) N-Ac- Sar-Gly-Val-D-Ile-Thr-Ile-Ile-Arg-ProNHCH₂CH₃,
(125) N-Ac-Sar-Gly- Val-D-Ile- Thr-Nle-Ile-Arg-ProNHCH₂CH₃,
(126) N-Ac-Sar-Gly-Val-D-Ile-Thr-Cit-Ile-Arg-ProNHCH₂CH₃,
(127) N-Ac-Sar-Gly-Val-D-Ile-Thr-Met (O₂)-Ile-Arg-ProNHCH₂CH₃,
(128) N-Ac-Sar-Gly-Val-D-Ile-Thr-Arg-lle-Ar-g-ProNHCH₂CH₃,
(129) N-Ac-Sar-Gly-Val-D-Ile-Thr-Tyr-Ile-Arg-ProNHCH₂CH₃,
(130) N-Ac-Sar-Gly-Val-D-Ile-Ihr-Glu-Ile-Arg-ProNHCH₂CH₃,
(131) N-Ac-Sar-Gly-Val-D-Ile-Thr-Lys (Ac)-Ile-Arg-ProNHCH₂CH₃,
(132) N-Ac-Sar-Gly-Val-D-Ile-Thr-Propargylgly-Ile-Axg-ProNHCH₂CH₂
(133) N-Ac-Sar-Gly-Val-D-alloIle-Thr-Gln-Ile-Arg-ProNHCH₂CH₃,
(134) N-Ac-Sar-Gly-Val-D-Leu-Thr-Gln-Ile-Arg-ProNHCH₂CH₃,
(135) N-Ac-Bala-Sar-Gly-Val-D-Ile-Thr-Nva-Ile-Arg-ProNHCH₂CH₃,
(136) N-Phenylacetyl-Sar-Gly-Val--D-Ile-Thr-Nva-Ile-Arg-ProNHCH₂CH₃,
(137) N-Ac-Sar-Gly-Val-D-Ile-Thr-Nva-Ile-Arg-Pro-AzaglyNH₂,
(139) N-Ac-Sar-Gly-Val-D-Ile-Thr-Nva-Ile-Arg-Pro-SerNH₂,
(140) N-Succinyl-Sar-Gly-Val-D-Leu-Thr-Nva-Ile-Arg-ProNHCHCH₃,
(158) N-Ac-Sar-Gly-Val-D-Ile-Thr-GIn-Ile-Arg-ProNHCH(CH₃)₂
(159) N-Succinyl-Sar-Gly-Val-D-Ile-Thr-GIn-Ile-Arg-ProNHCH₂CH₃,
(160) N-Succinyl-Sar,-Gly- Val-D-Leu- Thr-Gln-Ile-Arg-ProNHCH2CH₃,
(161) N-Succinyl-Sar-Gly- Val-D-Leu- Thr-Gln-Ile-Arg-ProNHCH(CH₃)₂,
(162) N-Ac-Sar-Gly-Val-D-Leu-Thr-Asp-Ile-Arg-ProNHCH₂CH₃,
(163) N-Ac-Sar-Gly- Val-D-Ile-Thr-Asp-Ile-Arg-ProNHCH₂CH₃,
(164) N-Ac-Sar-Gly-Val-D-Ile-Thr-Asn-Ile-Arg-ProNHCH₂CH₃,
(165) N-Ac-Sar-Gly-Val-D-Ile-Thr-Met(O)-Ile-Arg-ProNHCH₂CH₃,
(166) N-Ac-Sar-Gly-Val-D-Leu-Thr-Arg-Ile-Arg-ProNHCH₂CH₃,
(167) N-Ac-sar-Gly-val-D-Thr-Thr-Nva-Ile-Arg-PraNHCH₂CH₃,
(168) N-Ac-Sar-Gly-Val-D-Ser-Thr-Nva-Ile-Arg-ProNHCH₂CH₃,
(169) N-Ac-Sar-Gly- Val-D-Hser-Thr-Nva-Ile-Arg-ProNHCH₂CH₃,
(170) N-Ac-Sar-Gly- Val-D-Gln- Thr-Nva-Ile-Arg-ProNHCH₂CH₃.
(171) N-Ac-Sar-Gly- Val-D-Arg-Thr-Nva-Ile-Arg-ProNHCH₂CH₃,
(172) N-Ac-Sar-Gly-Val-D-Cit-Ther-Nva-Ile-Arg-ProNHCH₂CH₃,
(173) N-Ac-Sar-Gly- Val-D-Hcit- Thr-Nva-Ile-Arg-ProNHCH₂CH₃,
(174) N-Ac-Sar-Gly-Val-D-Hle-Thr-Nva-Ile-Arg-ProNHCH₂CH₃,
(175) N-Ac-Sar-Gly-Val-D-Neopentylgly-Thr-Nva-Ile-Arg-ProNHCH₂CH₃,
(176) N-Ac-Sar-Gly-Val-D-Ile-Th-Phe(4-CONH₂)-Ile-Arg-ProNHCH₂CH₃,
(197) N-Succinyl-Sar-Gly-Val-D-alloIle-Thr-Nva-Ile-Arg-ProNHCH₂CH₃,
(198) N-Succinyl-Sar-Gly-Val-D-Ile-Thr-Gln-Ile-Arg-ProNHCH₂CH₃,
(199) N-Succinyl-Sar-Gly-Val-D-Ile-Thr-Gln-Ile-Arg-Pro-D-AlaNH₂,
(200) N-Succinyl-Sar-Gly-Val-D-alloIle-Thr-Gln-Ile-Arg-ProNHCH₂CH₃,
(201) N-Succinyl-Sar-Gly- Val-D-alloIle-Thr-Gln-De-Arg-Pro-D-AlaNH₂,
(202) N-Succinyl-Sar-Gly-Val-D-alloIle-Thr-Gln-Ile-Arg-ProNHCH₂CH₃,
(203) N-Succinyl-Sar-Gly-Val-D-Ile-Thr-Gln-Ile-Arg-ProNHCH₂CH₃,
(204) N-Ac-Sar-Gly- Val-D-alloIle- Thr-Nva-Ile-Arg-Pro-D-AlaNH₂,
(205) N-Ac-Sar-Gly-Val-D-alloIle- Thr-Nva-Ile-Arg-ProNHCH(CH₃)₂,
(206) N-Ac-Sar-Gly-Val-D-Ile-Thr-Gln-Ile-Arg-Pro-D-AlaNH₂,
(207) N-Ac-Sar-Gly-Val-D-Ile-Thr-Gln-Ile-Arg-ProNHCH(CH₃)₂,
(208) N-Ac-Sar-Gly-Val-D-allolle-Thr-Gln-Ile-Arg-Pro-D-AlaNH₂,
(209) N-Ac-Sar-Gly-Val-D-alloIle-Thr-GLn-Ile-Arg-ProNHCH(CH₃)₂,
(210) N-Ac-Sar-Gly-Val-D-Ile-Thr-Nva-Ile-Arg-Pro-SarNH₂,
(211) N-Ac-Sar-Gly-Val-D-alloIle-Thr-Nva-Ile-Arg-Pro-SarNH₂,
(212) N-Ac-S ar-Gly-Val-D-Ile-Thr-GIn-Ile-Arg-Pro-SarNH₂,
(213) N-Ac-Sar-Gly- Val-D-alloIle- Thr-Gln-Ile-Arg-Pro-SarNH₂,
(214) N-Ac-Sar-Gly-Val-D-alloIle-Thr-Ser-Ile-Arg-Pro-D-AlaNH₂,
(215) N-Ac-Sar-Gly-Val-D-alloIle-Ther-Ser-Ile-Mg-ProNHCH(CH₃)₂,
(216)N-Ac-Sar-Gly-Val-D-allolle-Thr-Ser-Ile-Arg-ProNHCH₂CH₃,
(217) N-Ac-Sar-Gly-Val-D-Ile-Thr-Om (Ac)-Ile-Arg-ProNHCH₂CH₃,
(218) N-Ac-Sar-Gly-VaI-D-Ile-Thr-Gln-Ile-Arg-Pro-AzaglyNH₂,
(219) N-Ac-Sar-Gly- Val-D-allolle-Thr-Nva-Ile-Arg-Pro-AzaglyNH₂,
(220) N-Ac-Sar-Gly-Val-D-allolle-Thr-GIn-Ile-Arg-Pro-AzaglyNH₂,
(221) N-(2-carbonyl)-Sar-Gly-Val-D-alloIle-Thr-Nva-Ile-Arg-Pro-NHCH₂CH₃,
(222) N-(2-THFcarbonyl)-Sar-Gly-Val-D-He-Thr-Gln-Ile-Arg-ProNHCH₂CH₃,
(223) N-(2-THFcarbonyl)-Sar-Gly-Val-D-alloIle-Thr-Gln-Ile-Arg-Pro NHCH₂CH₃.
(224) N-(2-THF carbonyl)-Sar-Gly-Val-D-Ile-Thr-Gln-Ile-Arg-Pro-D-AlaNH₂,
(225) N-(2-THF carbonyl)-Sar-Gly- Val-D-alloIle- Thr-Gln- Ile-Arg-Pro-D-AlaNH₂,
(226) N-(2-THFcarbonyl)-Sar-Gly-Val-D-alloIle-Thr-Gln-Ile-Arg-Pro NHCH(CH₃)₂,
(227) N-(6-Ac-Aca)-Sar-Gly-Val-D-alloIle-Thr-Nva-Ile-Arg-ProNHCH₂CH₃,
(228) N- (6-Ac-Aca)-Sar-Gly-Val-D-Ile-Tk-Gln-Ile-Arg-ProNHCH₂CH₃,
(229) N-(6-Ac-Aca)-Sar-Gly-Val-D-alloIle. Tbr-Gln-Ile-Arg-ProNHCH₂CH₃,
(230) N-(6-Ac-Aca)-Sar-Gly-Val-D-Ile-Thr-Gln-Ile-Arg-Pro-D-AlaNH₂,
(231) N-(6-Ac-Aca)-Sar-Gly-Val-D-alloIle.Thr-Gln-lle-Arg-Pro-D-AlaNH₂,
(232) N-(6-Ac-Aca)-Sar-Gly-Val-D-alloIle-Thr-Gln-Ile-Arg-ProNHCH(CH₃)₂,
(233) N-{4-.Ac-Gaba)-Sar-Gly-Val-D-alloIle-Tbr-Nva-Ile-Arg-ProNHCH₂CH₃,
(234) N-(4-Ac-Gaba)-Sar-Gly-Val-D-Ile-Thr-Gln-Ile-Arg-ProNHCH₂CH₃,
(235) N-(4-Ac-Gaba)-Sar-Gly-Val-D-alloIle-Thr-Gln-Ile-Arg-ProNHCH₂CH₃,
(236) N-(4-Ac-Gaba)-Sar-Gly-Val-D-Ile-Thr--Gln-Ile-Arg-Pro-D-AlaNH₂,
(237) N-(4-Ac-Gaha)-Sar-Gly-Val-D-alloIle-Thr-Gln-Ile-Arg-Pro-D-AlaNH₂,
(238) N-(4-Ac-Gaba)-Sar-Gly-Val-D-alloIle-Thr-Gln-Ile-Arg-Pro NHCH(CH₃)₂,
(239) N-(2-Furoyl)-Sar-Gly-Val-D-alloIle-Thr-Nva-Ile-Arg-ProNHCH₂CH₃,
(240) N-(2-Furoyl)-Sar-GIy-Val-D-Ile-Thr-Gln-Ile-Arg-ProNHCH₂CH₃,
(241) N-(2-Furoyl)-Sar-Gly-Val-D-aIloIle-Thr-GIn-Ile-Arg-ProNHCH₂CH₃,
(242) N-(2-Furoyl)-Sar-Gly-Val-D-Ile-Thr-Gln-Tle-Arg-Pro-D-AlaNH₂,
(243) N-(2-Furoyl)-Sar-Gly-Val-D-allolle-Thr-Gln-Ile-Arg-Pro-D-AlaNH₂,
(244) N-(2-Furoyl)-Sar-Gly-Val-D-alioIle-Thr-Gln-Ile-Arg-ProNHCH(CH₃)₂,
(245) N-(Shikimyl)-Sar-Gly-Val-D-allolle-Thr-Nva-Ile-Arg-ProNHCH₂CH₃,
(246) N- (Shikimyl)-Sar-Gly--Val-D-Ile-Thr-Gln-Ile-Arg-ProNECH₂CH₃,
(247) N-(Shikimyl)-Sar-Gly- Val-D-allolle- Thr-Gln-Ile-Arg-ProNHCH₂CH₃,
(248) N-(Shikimyl)-Sar-Gly-Val-D-Ile-Thr-Gln-Ile-Arg-Pro-D-AlaNH₂,
(249) N-(SNkünyl)-Sar-Gly-Val-D-allolle-Thr-Gln-Ile-Arg-Pro-D-AlaNH₂,
(250) N- (Shikirnyl)-Sar-Gly-Val-D-allolle-Thr-Gln-Ile-Arg-ProNHCH(CH₃)₂,
(251) N- (2-Me-Nicotinyl)-Sar-Gly-Val-D-allolle-Thr-Nva-Ile-Arg-ProNHCH₂CH₃,
(252) N-(2-Me-Nicotinyl)-Sar-Gly-Val-D-Ile-Thr-Gln-Ile-Arg-ProNHCH₂CH₃,
(253) N-(2-Me-Nicotinyl)-Sar-Gly-Val-D-alloIle-Thr-GLn-Ile-Arg-Pro NECH₂CH₃.
(254) N-(2-Me-Nicotinyl)-Sar-Gly-Val-D-Ile-Thr-Gln-Ile-Arg-Pro-D-AlaNH₂,
(255) N-(2-Me-Nicotinyl)-Sar-Gly-Val-D-alloIle-Thr-Gln-Ile-Arg-Pro-D-AlaNH₂,
(256) N-(2-Me-Nicotinyl)-Sar-Gly-Val-D-alloIle-Thr-Gln-Ile-Arg-ProNHCH(CH₃)₂,
(257) N-Ac-Sar-Gly-Val-D-alloIle-Thr-Leu-Ile-Arg-Pro-D-AlaNH₂,
(258) N-Ac-Sar-Gly- V al- D-Ile- Thr-Leu-Ile-Arg- ProNHCH(CH₃)₂,
(259) N-Ac-Sar-Gly- Val-D-aJloIle- Tbr-Leu-Ile-Arg-ProNHCH₂CH₃,
(260) N-Ac-Sar-Gly--Val-D-Ile-Thr-Leu-Ile-Arg-Pra-D-AlaNH₂,
(261) N-Succinyl-Sar-Gly-Val-D-Ile-Thr-Leu-IIe-Arg-Pro-D-AlaNH₃,
(262) N-Succinyl-Sar-Gly-Val-D-Ile-Ibr-Leu-Ile-Arg-ProNHCH-₂CH₃,
(263) N-Succinyl-Sar-Gly-Val-D-Ile-Thr-Leu-Ile-Arg-ProNHCH₂CH₃,
(264) N-Succinyl-Sar-Gly-Val-D-alloIle-Thr-Leu-Ile-Arg-ProNHCH₂CH₃,
(265) N-Succinyl-Sar-Gly-Val-D-alloIle-Thr-Leu-lle-Arg-Pro-D-AlaNH₂,
(266) N-Succinyl-Sar-Gly-Val-D-Ile-Thr-Leu-Ile-Arg-Pro-AzaglyNH₂,
(267) N-Ac-Sar-Gly-Val-D-allolle-Th-r-Nva-Ile-Arg-ProNHethyl-(1-pyrrolidine),
(268) N-A.c-Sar-Gly-Val-D-alloIle-Thr-Nva-Ile-Arg-ProNH (ethyl-1-cyclohexyl),
(269) N-Ac-Sar-Gly-Val-D-IIe-Thr-Gln-Ile-Arg-ProNHethyl- (1-pyrrolidine),
(270) N-Ac-Sar-GJy-Val-D-Ile-Thr-Gln-Ile-Arg-ProNH (ethyl-1-cyclohexyl),
(271) N-Succinyl-Sar-Gly-Val-D-Ile-Thr-Gln-Ile-Arg-ProNH (ethyl-1-cyclohexyl).
(272) N-Ac-Sar-Gly-Val-D-alloIle-Thr-Nva-Ile-Arg-ProNHCH₂CH₂OCH₃,
(273) N-Ac-Sar-Gly-Val-D-Ile-Thr-Gln-lle-Arg-PrONHCH₂CH₂0CH₃,
(274) N-Ac-Sar-Gly-Va1-D-Ile-Thr-Ser-Ile-Arg-ProNHCH2CH₂0CH₃,
(275) N-Ac-Sar-GIy-Val-D-lie-Thr-Leu-Ile-Arg-ProNHCH2CH₂0CH₃,
(276) N-Succinyl-Sar-Gly-Val-D-Ile-Thr-Nva-Ile-Arg-ProNHCH₂CH₂0CH₃,
(277) N-Succinyt-Sar-Gly-Val-D-Ile-Thr-Gln-Ile-Arg-ProNHCH₂CH₂OCH₃,
(278) N-Succinyl-Sar-Gly-Val-D-alloIle-D-Gln-Ile-Arg-ProNHCH₂CH₂OCH₃,
(279) N-Ac-Sar-Gly-Val-D-Ile-Ser-Nva-Ile-Arg-ProNHCH₂CH₂OCH₃,
(280) N-Ac-Sar-Gly-Val-D-Leu-Ser-Nva-Ile-Arg-ProNHCH₂CH₂OCH₃,
(281) N-Ac-Sar-Gly- V al-D-alloIle- Thr-Allygly-Ile-Arg-ProNHCH₂CH₃,
(282) N-Ac-Sar-Gly-Val-D-Ile-Thr-Allygly-Ile-Arg-ProNHCH(CH₃)₂,
(283) N-Ac-Sar-Gly- Val-D-Ile-Thr-Allygly-Ile-Arg-Pro-D-AlaNH₂,
(284) N-Ac-Sar-Gly-Val-D-alloIle- Thr-Allygly-Ile-Arg-Pro-D-AlaNH₂,
(285) N-Succinyl-Sar-Gly-Val-D-Ile-Ihr-Allygly-lle-Arg-Pro-D-AlaNH₂,
(286) N-Ac-Sar-Gly-Val-D-Ile-Ser-Allygly-Ile-Arg-Pro-ProNHCH₂CH₃,
(287) N-Ac-Sar-Gly-Val-D-Leu-Ser-Allygly-Ile-Arg-Pro-ProNHCH₂CH₃,
(288) N-Ac-Sar-Gly-Val-D-Ile-Thr-Nva-Ile-Arg-Pro-SarNH₂,
(289) N-Ac-Sar-Gly- Val-D-Ile- Thr-Nva-Ile-Arg-ProNHOH,
(290) N-Ac-Sar-Gly-Val-D-Ile-Ser-Nva-Ile-Arg-PrONHCH₂CH₃,
(291) N-Ac-Sar-GIy-Val-D-allolle-Ser-Nva-Ile--Arg-ProNHCH₂CH₃,
(292) N-Ac-Sar-Gly-Val-D-Leu-Hser-Nva-Ile-Arg-ProNHCH₂CH₂-CH₃,
(300) N-Ac-Sar-Gly-Val-D-allolle-Thr-Ala-Ile-Arg-ProNHCH₂CH₃,
(301) N-Ac-Sar-Gly-Val-D-Ile-lhr-Ala-Ile-Arg-ProNHCH(CH₃)₂,
(302) N-Ac-S ar-Gly-Va1-D-Ile-Thr-Ala-Ile-Arg-Pro-D-AlaNH₂,
(303) N-Ac-Sar-Gly- Val-D-allolle- Thr-Ala-Ile-Arg-Pro-D-AlaNH₂,
(304) N-SuccinyL-Sar-Gly- Val-D-Ile- Thr-Ala-Ile-Arg-Pro-D-AlaNH₂,
(305) N-Ac-Sar-Gly-Val-D-Ile-Ser-Ala-Ile-Arg-ProNHCH₂CH₃,
(306) N-Ac-Sar-Gly-Val-D-Leu-Ser-Ala-Ile-Arg-ProNHCH₂CH₃,
(307) N-Ac-Sar-Gly-Val-D-alloIle-Thr-Val-Ile-Arg-ProNHCH₂CH₃,
(308) N-Ac-Sar-Gly-Val-D-Ile-Thr-Val-Ile-Arg-ProNHCH(CH₃)₂,
(309) N-Ac-Sar-Gly-Val-D-Ile-Tbr-Val-Ile-Arg-Pro-D-AlaNH₂,
(310) N-Ac-Sar-Gly-Val-D-alloIle-Thr-Val-Ile-Arg-Pro-D-AlaNH₂,
(311) N-Succinyl-Sar-Gly-Val-D-Ile-Thr-Val-Ile-Ara,-Pro-D-AlaNH₂,
(312) N-Ac-Sar-Gly-Val-D-Ile-Ser-Val-Ile-Arg-ProNHCH₂CH₃,
(313) N-Ac-Sar-Gly-Val-D-Leu-Ser-Val-Ile-Arg-ProNHCH₂CH₃,
(314) N-Ac-Sar-Gly-Val-D-allolle-Tlar-D-Nva-Ile-Arg-ProNHCH₂CH₃,
(315)N-Ac-Sar-Gly- V al-D-Ile- Thr-D-Nva-Ile-Arg-ProNHCH(CH₃)₂,
(316) N-Ac-Sar-Gly-VaI-D-Ile-Thr-D-Nva-Ile-Arg-Pro-D-AlaNH₂,
(317) N-Ac-Sar-Gly-Val-D-alloIle-Thr-D-Nva-Ile-Arg-Pro-D-AlaNH₂,
(318) N-Succinyl-Sar-Gly-Val-D-Ile-Fhr-D-Nva-Ile-Arg-Pro-D-AlaNH₂,
(319) N-Ac-Sar-Gly-Val-D-Ile-Ser-D-Nva-Ile-Arg-ProNHCH₂CH₃,
(320) N-Ac-Sar-Gly-Val-D-Leu-Ser-D-Nva-Ile-Arg-ProNHCH₂CH₃,
(321) N-Ac-Sar-Gly-Val-D-Ile-Ser-Gln-Ile-Arg-ProNHCH₂CH₃,
(322) N-Ac-Sar-Gly-Val-D-Leu-Ser-Gln-Ile-Arg-ProNHCH₂CH₃,
(323) N-Ac-Sar-Gly-Val-D-Leu-Ser-Nva-Ile-Arg-Pro-D-AlaNH₂,
(324) N-Ac-Sar-Gly-Val-D-Ile-Ser-Nva-Ile-Arg-Pro-D-AlaNH₂,
(325) N-Succinyl-Sar-Gly-Val-D-Leu-Ser-Nva-Ile-Arg-ProNHCH₂CH₃,
(326) N-Succinyl-Sar-Gly- V al-D-Ile-Ser-Nva-Ile-Arg-ProNHCH₂CH₃,
(327) N-Succinyl-Sar-Gly- Val-D- Leu-Ser-Gln-Ile-Arg-ProNHCH₂CH₃,
(328) N-Succinyl-Sar-Gly-Val-D-Ile-Ser-Gln-Ile-Arg-PrONHCH₂CH₃,
(329) N-Ac-Sar-Gly-Val-D-Ile-Ser-Ser-Ile-Arg-ProNHCH₂CH₃,
(330) N-Ac-Sar-Gly-Val-D-Leu-Ser-Ser- Ile-Arg-ProNHCH₂CH₃,
(331) N-Ac-Sar-Gly-Val-D-Leu-Ser-Nva-Ile-Arg-ProNHCH₂CH₂CH₃,
(332) N-Ac-Sar-Gly-Val-D-Ile-Ser-Nva-Ile-Arg-ProNHCH₂CH₂CH₃,
(333) N-Ac-Sar-Gly-Val-D-Leu-Ser-Leu-Ile-Arg-ProNHCH₂CH₃,
(334) N-Ac-Sar-Gly-Val-D-Ile-Ser-Leu-lle-Arg-ProNHCH₂CH₃,
(335) N-Ac-Sar-Gly-Val-D-allolle-Ser-Nva-Ile-Arg- ProNHCH₂CH₃,
(336) N-Ac-Sar-Gly- Val-D-alloIle-Ser-Gln-Ile-Arg-ProNHCH₂CH₃.
(337) N-Succinyl-Sar-Gly- Val-D-alloIle-Ser-Nva-Ile-Arg- ProNHCH₂CH₃,
(338) N-Ac-Sar-Gly-Val-D-allolle-Ser-Nva-Ile-Arg-ProNHCH₂CH₂CH₃,
(339) N-Ac-Sar-Gly- Val-O-alloIle-Ser-Nva-Ile-Arg-Pro-D-AlaNH₂.
(340) N-Ac-Sar-Gly-Val-D-alloIle-Ser-Leu-Ile-Arp ProNHCH₂CH₃,
(341) N-Ac-Sar-Gly-Val-D-alloIle-Ser-Ser-Ile-Arg-ProNHCH₂CH₃,
(342) N-Ac-Sar-Gly-Val-D-Ile-Gly-Nva-Ile-Arg-ProNHCH₂CH₃,
(343) N-Ac-Sar-Gly-Val-D-allolle-Gly-Nva-lle-Arg-ProNHCH₂CH₃,
(344) N-Ac-Sar-Gly-Val-D-Leu-Gly-Gln-Ile-Arg-ProNHCH₂CH₃,
(345) N-Ac-Sar-Gly- V al-D- Ile-Gly-Gln- Ile-Arg..ProNHCH₂CH₃,
(346) N-Ac-Sar-Gly-Val-D-alloIle-Gly-Gln-Ile-Arg-ProNHCH₂CH₃,
(347) N-Ac-Sax-Gly-Val-D-Ile-Tyr-Nva-Ile-Arg-PrnNHCH₂CH₃,
(348) N-Ac-Sar-Gly-Val-D-alloIle-Tyr-Nva-Ile-Arg-ProNHCH₂CH₃,
(349) N-Ac-Sar-Gly-Val-D-Leu-Tyr-Gln-Ile-Arg-ProNHCH₂CH₃,
(350) N-Ac-Sar-Gly-Val-D-Ile-Tyr-Gln-Ile-Arg-ProNHCH₂CH₃,
(351) N-Ac-Sar-Gly-Val-D-allolle-Tyr-Gln-Ile-Arg-ProNHCH-₂CH₃,
(352) N-Ac-Sar-Gly-Val-D-Ser-Thr-Nva-Ile-Arg-ProNHCH₂CH₃,
(353) N-Ac-Sar-Gly-Val-D-Thr-Thr-Nva-Ile-Arg-ProNHCH₂CH₃,
(354) N-Ac-Sar-Gly-Val-D-Gln-Tbr-Nva-Ile-Arg-ProNHCH₂CH₃,
(355) N-Ac-Sar-Gly-Val-D-Asn-Thr-Nva-Ile-Arg ProNHCH₂CH₃,
(356) N-Ac-Sar-Gly-Val-D-Arg-Thr-Nva-Ile-Arg-ProNHCH₂CH₃,
(357) N-Ac-Sar-Gly-Val-D-3-Pal-Thr-Nva-Ile-Mg-PrONHCH₂CH₃,
(358) N-Ac-Sar-Gly-Val-D-Glu-7br-Nva-Ile-Arg-ProNHCH₂CH₃,
(359) N-Ac-Sar-Gly-Val-D-Asp-Thr-Nva-Ile-Arg-ProNHCH₂CH₃,
(360) N-Ac-Sar-Gly-Val-D-His- Thr-Nva-Ile-Arg-ProNHCH₂CH₃,
(361) N-Ac-Sar-Gly-Val-D-Hser-Thr-Nva-Ile-Arg-ProNHCH₂CH₃,
(362) N-Ac-Sar-Gly-Val-D-alloThr-Thr-Nva-Ile-Arg-ProNHCH₂CH₃,
(363) N-Ac-Sar-Gly-Val-D-Ile-Thr-Nva-D-Ile-Arg-ProNHCH₂CH₃,
(364) N-Ac-Sar-Gly-Val-D-Ser-Thr-Gln-Ile-Arg-ProNHCH₂CH₃,
(365) N-Ac-Sar-Gly- Val-D-Thr- Thr-Gln-Ile-Arg-ProNHCH₂CH₃,
(366) N-Ac-Sar-Gly-Val-D-alloThr-Thr-GIn-Ile-Arg-ProNHCH₂CH₃,
(367) N-Ac-Sar-Gly-Val-D-Ser-Ser-Nva-Ile-Arg-ProNHCH₂CH₃,
(368) N-Ac-Sar-Gly-Val-D-Thr-Ser-Nva-Ile-Arg-ProNHCH₂CH₃,
(369) N-Ac-Sar-Gly-Val-D-alloThr-Ser-Nva-Ile-Arg-ProNHCH₂CH₃,
(370) N-Ac-Sar-Gly-Val-D-alloThr-Ser-Gln-Ile-Arg-ProNHCH₂CH₃,
(371) N-Ac-Sar-Gly-Val-D-Thr-Ser-Gln-Ile-Arg-ProNHCH₂CH₃,
(372) N-(6-Ac-Aca)-Sar-Gly-Val-D-Leu-Ser-Gln-Ile-Arg-ProNHCH₂CH₃,
(373) N-(6-Ac-Aca)-Sar-Gly-Val-D-Leu-Ser-Nva-Ile-Arg-ProNHCH₂CH₃,
(374) N-(4-Ac-Gaba)-Sar-Gly-Val-D-Leu-Ser-Gln-Ile-Arg-ProNHCH₂CH₃,
(375) N-(4-Ac-Gaba)-Sar-Gly-Val-D-Leu-Ser-Nva-Ile-Arg-ProNHCH₂CH₃,
(376) N-(2-Furoyl)-Sar-Gly-Val-D-Leu-Ser-Gln-Ile-Arg-ProNHCH₂CH₃,
(377) N-(2-Furoyl)-Sar-Gly-Val-D-Leu-Ser-Nva-Ile-Arg-ProNHCH₂CH₃,
(378) N-(Shikimyl)-Sar-Gly-Val-D-Leu-Ser-Gln-Ile-Arg-ProNHCH₂CH₃,
(379) N-(Shikimyl)-Sar-Gly-Val-D-Leu-Ser-Nva-Ile-Arg-ProNHCH₂CH₃,
(382) N-(2-Me-nicotinyl)-Sar-Gly-Val-D-Leu-Ser-Gln-Ile-Arg-ProNHCH₂CH₃,
(383) N- (2-Me-nicotinyl)-Sar-Gly-Val-D-Leu-Ser-Nva-Ile-Arg-ProNHCH₂CH₃,
(384) N-Ac-Sar-Gly-Val-D-Leu-Ser-Nva-Ile-Arg-ProNHethyl-1-(R)-cyclohexyl,
(385) N-Ac-Sar-Gly-Val-D-Leu-Ser-Gln-Ile-Arg--ProNHethyl- 1- (R)-cyclohexyl,
(386) N-Ac-Sar-Gly-Val-DIle-Thr-ser-fle-Arg-ProNHethyl-1-(R)-cyclohexyl,
(387) N-Ac-Sar-Gly- Val-D-Leu- Thr-Nva-Ile-Arg-ProNHethyl-1-(R)-cyclohexyl,
(388) N-Ac-Sar-Gly- Val-D-Leu-Ser-Ser-Ile-Arg-ProNHethyl-1-(R)-cyclohexyl,
(389) N-Ac-Sar-Gly-Val-DIle-Thr-Nva-Ile-Arg-ProNHethyl-1-(S)-cyclohexyl,
(390) N-Ac-Sar-Gly-Val-D-Pen-Ser-Nva-Ile-Arg-ProNHCH₂CH₃,
(391) N-Ac-Sar-Gly-Val-D-Pen-Gly-Nva-Ile-Arg-ProNHCH₂CH₃,
(392) N-Ac-Sar-Gly-Val-D-Pen-Thr-Gln-Ile-Arg-ProNHCH₂CH₃,
(393) N-Ac-Sar-Gly- Val-D-Pen-Ser-Nva-Ile-Arg-ProNHCH(CH₃)₂,
(394) N-Succinyl-Sar-Gly-Val-D-Pen-Ser-Nva-Ile-Arg-ProNHCH₂CH₃,
(395) N-Ac-Sar-Gly- Val-D-Pen-Ser-Nva-Ile-Arg-Pro-D-AlaNH₂,
(396) N-Ac-Sar-Gly- Val-D-Pen-Ser-Gln- Ile-Arg-ProNHCH₂CH₃,
(397) N- Ac-Sar-Gly- Val-D-Pen-Gly-Gln-Ile-Arg-ProNHCH₂CH₃,
(398) N-Ac-Sar-Gly- Val-D-Pen-Ser-Ser-Ile-Arg-ProNHCH₂CH₃,
(399) N-Ac-Sar-Gly-Val-D-Pen-Thr-Ser-Ile-Arg-ProNHCH₂CH₃,
(400) N-Ac-Sar-Gly- Val-D-Pen- Thr-Leu-Ile-Arg-ProNHCH₂CH₃,
(401) N-Ac-Sar-Gly-Val-D-Pen-Ser-Leu-Ile-Arg-ProNHCH₂CH₃,
(402) N-Succinyl-Sar-Gly-Val-D-Pen-Ser-Ser-Ile-Arg-ProNHCH₂CH₃,
(403) N-Succinyl-Sar-Gly- Val-D-Pen-Ser-Leu-Ile-Arg- ProNHCH₂CH₃,
(404) N-Succinyl-Sar-Gly-Val-D-Pen-Thr-Gln-Ile-Arg-ProNHCH (CH₃)₂,
(405) N-Ac-Sar-Gly-Val-D-Cys-Thr-Nva-Ile-Arg-ProNHCH₂CH₃,
(406) N-Ac-Sar-Gly-Val-D-Cys-Ser-Nva-Ile-Arg-ProNHCH₂CH₃,
(407) N-Ac-Sar-Gly-Val-D-Cys-Gly-Nva-Ile-Arg-ProNHCH₂CH₃,
(408) N-Ac-Sar-Gly-Val-D-Cys-Thr-Gln-ne-Arg-ProNHCH₂CH₃,
(409) N-Ac-Sar-Gly-Val-D-Cys-Ser-Nva-Ile-Arg-ProNHCH (CH₃)₂,
(410) N-Succinyl-Sar-Gly- Val-D-Cys-Ser-Nva-Ile-Arg-ProNHCH₂CH₃,
(411) N-Ac-Sar-Gly-Val-D-Cys-Ser-Nva-Ile-Arg-Pro-D-AlaNH₂,
(412) N-Ac-Sar-Gly-Val-D-Cys-Ser-Gln-Ile-Arg-ProNHCH₂CH₃,
(413) N-Ac-Sar-Gly-Val-D-Cys-Gly-Gln-Ile-Arg-ProNHCH₂CH₃,
(414) N-Ac-Sar-Gly-Val-D-Cys-Ser-Ser-Ile-Arg ProNHCH₂CH₃,
(415) N-'Ac-Sar-Gly-VaL-D-Cys-Thr-Ser-He-Arg-ProNHCH₂CH₃
(416) N-Ac-Sar-Gly-Val-D-Cys-Thr-Leu-Ile-Arg-ProNHCH₂CH₃,
(417) N-Ac-Sar-Gly-Val-D-Cys-Ser-Leu-Ile-Arg-ProNHCH₂CH₃,
(418) N-Succinyl-Sar-Gly-Val-D-Cys-Ser-Ser-Ile-Arg-ProNHCH₂CH₃,
(419) N-Succinyl-Sar-GIy-Val-D-Cys-Ser-Leu-Ile-Arg-ProNHCH₂CH₃,
(431) N-Ac-Sar-Gly-Val-D-Leu-Pen-Nva-Ile-Arg-ProNHCH₂CH₃,
(432) N-Ac-Sar-Gly-Val-D-Ile-Pen-Nva-Ile-Arg-ProNHCH₂CH₃,
(433) N-Ac-Sar-Gly-Val-D-alloIle-Pen-Nva-Ile-Arg-ProNHCH₂CH₃,
(434) N-Ac-Sar-Gly-Val-D-Ile-Pen-Gln-Ile-Arg-ProNHCH₂CH₃,
(435) N-Ac-Sar-Gly-Val-D-Ile-Pen-Ser-Ile-Arg-ProNHCH₂CH₃,
(436) N-Ac-Sar-Gly-Val-D-Ile-Pen-Leu-Ile-Arg-ProNHCH₂CH₃,
(437) N-Ac-Sar-Gly-Val-D-Ile-Pen-Nva-Ile-Arg-ProNHCH(CH₃)₂,
(438) N-Ac-Sar-Gly-Val-D-Ile-Pen-Nva-Ile-Arg-Pro-D-AlaNH₂,
(439) N-Succinyl-Sar-Gly-Val-D-Ile-Pen-Nva- Ile-Arg-ProNHCH₂CH₃,
(440) N-Succinyl-Sar-Gly-Val-D-Ile-Pen-Gln-Ile-Arg-ProNHCH₂CH₃,
(441) N-Succinyl-Sar-Gly-Val-D-Ile-Pen-Gln-Ile-Arg-ProNHCH(CH₃)₂,
(442) N-Ac-Sar-Gly-Val-D-Ile-Thr-Pen-Ile-Arg-ProNHCH₂CH₂OCH₃,
(443) N-Ac-Sar-Gly-Val-D-alloIle-Thr-Pen-Ile-Arg-ProNHCH₂CH₃,
(444) N-Ac-Sar-Gly- Val-D-Leu- Thr-Pen-Ile-Arg-ProNHCH₂CH₃,
(445) N-Ac-Sar-Gly-Val-D-Ile-Thr-Pen-Ile-Arg-Pro-D-AlaNH₂,
(446) N-Succinyl-Sar-Gly-Val-D-Ile-Thr-Pen-Ile-Arg-ProNHCH₂CH₃,
(447) N-Ac-Sar-Gly-Val-D-Ile-Thr-Pen-Ile-Arg-ProNHCH(CH₃)₂,
(448) N-Ac-Sar-Gly-Val-D-Leu-Ser-Pen-Ile-Arg-ProNHCH₂CH₃,
(449) N-Ac-Sar-Gly-Val-D-Leu-Gly-Pen-Ile-Arg-ProNHCH₂CH₃,
(450) N-Succinyl-Sar-Gly-Val-D-Leu-Ser-Pen-Ile-Arg-ProNHCH₂CH₃,
(451) N-Ac-Sar-Gly-Val-D-Phe (3,4,5-triF)-Thr-Gln-Ile-Arg-ProNHCH₂CH₃,
(452) N-Ac-Sar-Gly-Val-D-Phe (3,4,5-triF)-Ser-Nva-Ile-Arg-ProNHCH₂CH₃,
(453) N-Ac-Sar-Gly-Val-D-Phe (3,4,5-triF)-Gly-Nva-Ile-Arg-ProNHCH₂CH₃,
(454) N-Ac-Sar-Gly-Val-D-Phe (3,4,5-triF)-Ser-Leu-Ile-Arg-ProNHCH₂CH₃,
(455) N-Ac-Sar-Gly-Val-D-Phe (3,4,5-triF)-Ser-Nva-Ile-Arg-Pro-D-AlaNH₂,
(456) N-Succinyl-Sar-Gly-Val-D-Phe (3,4,5-triF)- Thr-Gln-Ile-Arg-ProNHCH₂CH₃,
(457) N-Succinyl-Sar-Gly-Val-D-Phe (3,4,5-triF)-Ser-Gln-Ile-Arg ProNHCH₂CH₃,
(458) N-Succinyl-Sar-Gly-Val-D-Phe (3,4,5-triF)-Thr-Gin-Ile-Arg-ProNHCH (CH₃)₂,
(459) N-Ac-Sar-Gly-Val-D-Phe (3,4,5-triF)-Ser-Gln-Ile-Arg-ProNHCH₂CH₃,
(460) N-Ac-Sar-Gly-Val-D-Phe (3,4,5-triF)-Ser-Ser-Ile-Arg-ProNHCH₂CH₃,
(470) N-(3-Ac-Bala)-Sar-Gly-Val-D-alloIle-Thr-Nva-Ile-Arg-ProNHCH₂CH₃,
(471) N-(3-Ac-Bala)-Sar-Gly-Val-D-Ile-Thr-Gln-Ile-Arg-ProNHCH₂CH₃,
(472) N-(3-Ac-Bala)-Sar-Gly-Val-D-alloIle-Thr-Gln-Ile-Arg-ProNHCH₂CH₃,
(473) N-(3-Ac-B ala)-Sar-Gly-Val-D-Ile-Thr-Gh-Ile-Arg-Pro-DAlaNH₂,
(474) N-(3-Ac-Bala)-Sar-Gly-Val-D-allolle-Thr-Gln-Ile-Arg-Pro-DAlaMH₂,
(475) N-(3-Ac-Bala)-Sar-Gly-Val-D-allolle-.Tk-Gb-Ile-Arg-PTONHCH(CH₃)₂,
(476) N-(3-Ac-BaJa)-Sar-Gly- Val-D-Leu-Ser-Nva-Ile-Arg-ProNHCH₂CH)₃,
(477) N-(3-Ac,-Bala)-Sar-Gly-Val-D-Leu-Thr-Nva-Ile-Arg-ProNHCH₂CH₃,
(478) N-(3--Ac-Bala)-Sar-Gly-Val-D-Pen-Thr-Nva-Ile-Arcy-ProNHCH₂CH₃,
(479) N-(3-Ac-Bala)-Sar-Gly-Val-D-Ile-Ser-Nva-Ile-Arg-ProNHCH₂CH₃,
(484) N-Ac-Sar-Gly-Val-D-Ile-Thr-Nva-Ile-Arg-Pro-OH,
(485) N-Ac-Sar-Gly-Val-D-alloIle-Thr-Nva-Ile-Arg-Pro-OH,
(486) N-Ac-Sar-Gly-Val-D-Leu-Thr-Nva-Ile-Arg-Pro-OH,
(487) N-Ac-Sar-Gly- Val-D-Pen- Thr-Nva-Ile-Arg-Pro-OH,
(488) N-Ac-Sar-Gly-Val-D-Phe (3,4,5-triF)-Thr-Nva-Ile-Arg-Pro-OH,
(489) N-Ac-Sar-Gly-Val-D-Ile-Thr-Gln-Ile-Arg-Pro-OH,
(490) N-Ac-Sar-Gly- Val-D-Leu-Ser-Nva-Ile-Arg-Pro-OH,
(492) N-Ac-Sar-Gly-Val-D-Ile-Ser-GLn-Ile-Arg-Pro-OH,
(493) N-Succinyl-Sar-Gly-Val-D-Ile-Thr-Nva-Ile-Arg-Pro-OH,
(494) N-Succinyl-Sar-Gly-Val-D-Leu-Thr-Gln-Ile-Arg-Pro-OH,
(495) N -Ac-Sar-Gly -Val-alloIle- Thr-Nva-Ile-Arg-ProNHCH₂CH₃
(496) N-Ac-Sar-Gly-Val-D-Lys-Thr-Nva-Ile-Arg-ProNHCH₂CH₃
(497) N-Ac-Sar-Gly-Val-D-Trp-Thr-Nva-De-Arg-ProNHCH₂CH₂
(498) N-Ac-Sar-Gly-Val-D-3,3-Dipheylala-Thr-Nva-Ile-Arg-ProNHCH₂CH₃
(499) N-Ac-Sar-Gly-Val-D-3-Benzothienylala-Thr-Nva-Ile-Arg-ProNHCH₂CH₃
(500) N-Ac-Sar-Gly-Val-D-3.4-diF-Phe-Thr-Nva-Il-Arg-ProCHCH₂CH₃
(501) N-Ac-Sar-Gly- Val-D-Pen(iBzl)-Thr-Nva-Ile-Arg-ProNHCH₂CH₃
(502) N-Ac-Sar-Gly-Val-D-Leu-Thr-Gln-Ile-Arg-ProNHCH₂CH₃,
(503) H-Sar-Gly-Val-D-Leu-Thr-Gln-Ile-Arg-ProNHCH₂CH₃,
(508) N-Ac-Sar-Glv-Val-D-Ile-Thr-Nva-Ile-Arg-ProNHCH₂CH₂OH. und
(510) N-Ac-Sar-Gly-Val-D-Ile-Thr-Leu-Ile-Arg-ProNH((R)-1-cyclohexylethyl)

12. Eine Verbindung gemäß Anspruch 11, gewählt aus:
(1) -N-Ac-Sar-Gly-Vat-D-Ile-Thr-Nva-Ile-Arg-ProNHCH₂CH₃,
(2) N-Ac-Sar-Gly-Val-D-Ile-Thr-Nva-Ile-Alg-ProNHCH₂CH₂- (1-pyrrolidine),
(3) N-Ac-Sar-Gly-Val-D-Ile-Thr-Nva-IIe-Arg-ProNH (ethyl- 1- (R)-cyclohexyl),
(4) N-Ac-Sar-Gly-Val-D-Ile-Thr-Nva-Ile-Arg-ProNH₂,
(5) N-Ac-Sar-Gly-Val-D-Ile-Thr-Nva-Ile-Arg-ProNHCH (CH₃)2,
(6) N-Ac-Sar-Gly-Val-D-allolle-Thr-Nva-Ile-Arg-ProNHCH₂CH₃,
(7) N-Ac-Sar-Gly-Val-D-Val-Thr Nva-Ile-Arg-ProNHCH₂CH₃,
(8) N-Ac-Sar-Gly-Val-D-Nle-Thr-Nva-Ile-Arg-ProNHCH₂CH₃,
(9) N-Ac-Sar-Gly-Val-D-Phe-Thr-Nva-Ile-Arg-ProNHCH₂CH₃,
(10) N-Ac-Sar-Gly-Val-D-Cha-Thr-Nva-Ile-Arg-ProNHCH₂CH₃,
(12) N-Ac-Sar-Gly- Val-D-3-ClPhe- Thr-Nva-Ile-Arg-ProNHCH₂CH₃,
(13) N-Ac-Sar-Gly-Val-D-2-Thienylala-Thr-Nva-Ile-Arg-ProNHCH₂CH₃,
(14) N-Ac-Sar-Gly- Val-D-3-CNPhe- Thr-Nva-Ile-Arg-ProNHCH₂CH₃,
(15) N-Ac-Sar-Gly-Val-D-Ile-Thr-Cha-Ile-Arg-ProNHCH₂CH₃,
(16) N[2-THF-C(O)]-Sar-Gly-Val-D-Ile-Thr-Nva-Ile-Arg-ProNHCH₂CH₃,
(17) N[6-N-acetyl-(CH₂)₅C(O)]-Sar-Gly-Val-D-Ile-Thr-Nva-Ile-Arg-ProNHCH₂CH₃,
(19) N- [4-N-Acetylaminobutyryl]-Sar-Gly- Val-D-Ile- Thr-Nva-Ile-Arg ProNHCH₂CH₃,
(23) N-Ac-Sar-Gly-Val-D-Ile-Thr-Leu-Ile-Arg-ProNHCH₂CH₃,
(24) N-Ac-Sar-Gly-Val-D-Ile-Thr-Ser-Ile-Arg-ProNHCH₂CH₃,
(25) N-Ac-Sar-Gly-Val-D-Ile-Thr-Nva-Ile-Arg-Pro-D-AlaNH₂,
(30) N-Ac-Sar-Gly-Val-D-Leu-Ala-Nva-Ile-Arg-ProNHCH₂CH₃,
(31) N-Ac-Sar-Gly-Val-D-Leu-Trp-Nva-Ile-Arg-ProNHCH₂CH₃,
(32) N-Ac-Sar-Gly-Val-D-Leu-Tyr-Nva- Ile-Arg-ProNHCH₂CH₃,
(33) N-Ac-Sar-Gly-Val-D-Leu-Gly-Nva-Ile-Arg-ProNHCH₂CH₃,
(34) N-Ac-Sar-Gly-Val-D-Leu-2Nal-Nva-Ile-Arg-ProNHCH₂CH₃,
(35) N-Ac-Sar-Gly-Val-D-Leu-lNal-Nva-Ile-Arg-ProNHCH₂CH₃,
(36) N-Ac-Sar-Gly-Val-D-Leu-Octylgly-Nva-Ile-Arg-ProNHCH₂CH₃,
(37) N-Ac-Sar-Gly-Val-D-Leu-Ser-Nva-Ile-Arg-ProNHCH₂CH₃,
(3 8) N-Ac-Sar-Gly-Val-D-Leu-Allylgly-Nva-Ile-Arg-ProNHCH₂CH₃,
(39) N-Ac-Sar-Gly-Val-D-Leu-D-Thr-Nva-Ile-Arg-ProNHCH₂CH₃,
(40) N-Ac-Sar-Gly- Val-D-Ile- Thr-Tyr-IIe-Arg-ProNHCH₂CH₃,
(41) N-Ac-Sar-Gly-Val-D-Ile-Thr-Glu-Ile-Arg-ProNHCH₂CH₃,
(42) N-Ac-Sar-Gly-Val-D-Ile-Thr-Propargylgly-Ile-Arg-ProNHCH₂CH₃,
(43) N-Ac-Sar-Gly- Val-D-alloIle- Thr-Gln-Ile-Arg-ProNHCH₂CH₃,
(44) N-Ac-Bala-Sar-Gly-Val-D-Ile-Thr-Nva-Ile-Arg-ProNHCH₂CH₃,
(4.5) N-Phenylacetyl-Sar-Gly-Val-D-Ile-Thr-Nva-Ile-Arg-PrONHCH₂CH₃,
(46) N-Ac-Sar-Gly-Val-D-Ile-Thr-Nva-Ile-Arg-Pro-AzaglyNH₂,
(47) N-Ac-Sar-Gly-Val-D-Ile-Thr-Nva-Ile-Arg-Pro-SerNH₂,
(48) N-(6-Ac-Aca)-Sar-Gly-Val-D-Leu-Ser-Glu-Ile-Arg-ProNCH₂CH₃,
(49) N-(6-Ac-Aca)-Sar-Gly-Val-D-Leu-Ser-Nva-Ile-Arg-ProNHCH₂CH₃,
(50) N-(4-Ac-Gaba)-Sar-Gly-Val-D-Leu-Ser-Gln Ile-Arg-ProNHCH₂CH₃,
(51) N-(4-Ac-Gaba)-Sar-Gly-Val-D-Leu-Ser-Nva-Ile-Arg-ProNHCH₂CH₃,
(52) N-(2-Furoyl)-Sar-Gly-Val-D-Leu-Ser-Gln-Ile-Arg-ProNHCH₂CH₃,
(53) N-(2-Furoyl)-Sar-Gly-Val-D-Leu-Ser-Nva-Ile-Arg-ProNHCH₂CH₃,
(54) N-(Shikimyl)-Sar-Gly-Val-D-Leu-Ser-Gln-Ile-Arg-ProNHCH₂CH₃,
(55) N-(Shikimyl)-Sar-Gly-Val-D-Leu-Ser-Nva-Ile-Arg-ProNHCH₂CH₃,
(58) N-(2-Me-nicotinyl)-Sar=Gly-Val-D-Leu-Ser-Gln-Tle-Arg ProNHCH₂CH₃,
(59) N- (2-Me-nicotinyl)-Sar-Gly-Val-D-Leu-Ser-Nva-Ile-Asg-ProNHCH₂CH₃,
(60) N-Ac-Sar-Gly-Val-D-Ile-Thr Nva-Ile-Arg-Pro-OH,
(62) N-Ac-Sar-Gly-Val-D-Pen-Thr-Nva-Ile-Arg-ProNHCH₂CH₃,
(63) N-Ac-Sar-Gly-Val-D-Phe (3,4,5-triF)-Thr-Nva-Ile-Arg-ProNHCH₂CH₃, und
(64) N-Ac-Sar-Gly-Val-D-Phe (4-NH₂)-Thr-Nva-Ile-Arg-ProNECH₂CH₃.

13. Eine pharmazeutische Zusammensetzung, umfassend eine Verbindung von Anspruch 1 und einen pharmazeutisch verträglichen Träger.

14. Verwendung einer therapeutisch wirksamen Menge einer Verbindung in Anspruch 1 für die Herstellung eines Medikaments zur Behandlung eines Patienten, der eine Anti-Angiogenesetherapie benötigt.

15. Eine Zusammensetzung zur Behandlung einer Erkrankung, gewählt aus Krebs, Arthritis, Psoriasis. Angiogenese der Augen, assoziiert mit Infektion oder operativem Eingriff, Makuladegeneration und diabetischer Retinopathie, umfassend ein Peptid, wie in Anspruch 1 definiert, in Kombination mit einem pharmazeutisch verträglichen Träger.

16. Eine Verbindung gemäß Anspruch 11, gewählt aus:
N-Ac-Sar-Gly-Val- D-Ile-Thr-Nva-Ile-Arg-ProNHCH₂CH₃
N-Ac-Sar-Gly-Val-D-Ile-Thr-Nva-Ile-Arg-ProNHCH₂CH₃,
N-Ac-Sar-Gly-Val-D-Ile-Thr-Gln-Ile-Arg-ProNHCH₂CH₃, and
N-Ac-Sar-Gly-Val-D-alloIle-Ser-Ser-Ile- Arg-ProNHCH₂CH₃,

17. Eine Verbindung gemäß Anspruch 16, welche N-Ac-Sar-Gly-Val-D-Ile-Thr-Nva-Ile-Arg-ProNHCH₂CH₃ ist.

18. Eine Verbindung gemäß Anspruch 16, welche N-Ac-Sar-Gly-Val-D-allolle-Thr-Nva-Ile-Arg-ProNHCH₂CH₃ ist.

19. Eine Verbindung gemäß Anspruch 16, welche N-Ac-Sar-Gly-Val-D-Ile-Thr-Gln-Ile-Arg-ProNHCH₂CH₃ ist.

20. Eine Verbindung gemäß Anspruch 16, welche N-Ac-Sar-Gly-Val-D-alloIle-Ser-Ser-Ile-Arg-ProNHCH₂CH₃ ist.

21. Die Zusammensetzung gemäß Anspruch 13, umfassend eine Verbindung oder ein pharmazeutisch verträgliches Salz, Ester, Solvat oder Prodrug davon, gewählt aus der Gruppe bestehend aus
N-Ac-Sar-Gly-Val-D-Ile-Tbr-Nva-Ile-,Arg-PrONHCH₂CH₃
N-Ac-Sar-Gly-Val-D-alloIle-Thr-Nva-Ile-Arg-ProNHCH₂CH₃
N-Ac-Sar-Gly-Val-D-Ile-Thr-Gln-Ile-Arg-ProNHCH₂CH₃-and
N-Ac-Sar-Gly-Val-D-alloIle-Ser-Ser-Ile-Arg-ProNHCH₂CH₃.
und einem pharmazeutisch verträglicher Träger.

22. Die Zusammensetzung gemäß Anspruch 21, umfassend die Verbindung N-Ac-Sar-Gly-Val-D-Ile-Thr-Nva-Ile-Arg-ProNHCH₂CH₃ oder ein pharmazeutisch verträgliches Salz, Ester, Solvat oder Prodrug davon, und einen pharmazeutisch verträglichen Träger.

23. Die Zusammensetzung gemäß Anspruch 21, umfassend die Verbindung N-Ac-Sar-Gly-Val-D-alloIle-Thr-Nva-Ile-Arg-ProNHCH₂CH₃ oder ein pharmazeutisch verträgliches Salz, Ester, Solvat oder Prodrug davon, und einen pharmazeutisch verträglichen Träger.

24. Die Zusammensetzung gemäß Anspruch 21, umfassend die Verbindung N-Ac-Sar-Gly-Val-D-Ile-Thr-Gln-Arg-ProNHCH₂CH₃ oder ein pharmazeutisch verträgliches Salz, Ester, Solvat oder Prodrug davon, und einen pharmazeutisch verträglichen Träger.

25. Die Zusammensetzung gemäß Anspruch 21, umfassend die Verbindung N-Ac-Sar-Gly-Val-D-alloIle-Ser-Ser-Ile-Arg-ProNHCH₂CH₃ oder ein pharmazeutisch verträgliches Salz, Ester, Solvat oder Prodrug davon, und einen pharmazeutisch verträglichen Träger.

26. Die Zusammensetzung gemäß Anspruch 15, umfassend eine Verbindung oder ein pharmazeutisch verträgliches Salz, Ester, Solvat oder Prodrug davon, gewählt aus der Gruppe bestehend aus
N-Ac-Sar-Gly-Val-D-Ile-Thr-Nva-Ile-Arg-ProNHCH₂CH₃;
N-Ac-Sar-Gly-Val-D-alloIle-Thr-Nva-Ile-Arg-ProCHCH₂CH₃
N-Ac-Sar-Gly-Val-D-Ile-Thr-Gln-Ile-Arg-ProNHCH₂CH₃ and
N-Ac-Sar-Gly-Val-alloIle-Ser-Ser-Ile-Arg-ProNHCH₂CH₃

27. Die Zusammensetzung gemäß Anspruch 26, umfassend die Verbindung N-Ac-Sar-Gly-Val-D-Ile-Thr-Nva-Ile-Arg-ProNHCH₂CH₃ oder ein pharmazeutisch verträgliches Salz, Ester, Solvat oder Prodrug davon.

28. Die Zusammensetzung gemäß Anspruch 26, umfassend die Verbindung N-Ac-Sar-Gly-Val-D-alloIle-Thr-Nva-Ile-Arg-ProNHCH₂CH₃ oder ein pharmazeutisch verträgliches Salz, Ester, Solvat oder Prodrug davon.

29. Die Zusammensetzung gemäß Anspruch 26, umfassend die Verbindung N-Ac-Sar-Gly-Val-D-Ile-Thr-Gln-Ile-Arg-ProNHCH₂CH₃ oder ein pharmazeutisch verträgliches Salz, Ester, Solvat oder Prodrug davon.

30. Die Zusammensetzung gemäß Anspruch 26, umfassend die Verbindung N-Ac-Sar-Gly-Val-D-alloIle-Ser-Ser-Ile-Arg-ProNHCH₂CH₃ oder ein pharmazeutisch verträgliches Salz, Ester, Solvat oder Prodrug davon.

31. Die Verwendung von Anspruch 14, worin der Patient ein nicht-menschliches Tier ist.

32. Die Verwendung von Anspruch 14, worin der Patient ein Mensch ist.

33. Die Verwendung von Anspruch 14, umfassend eine Verbindung von irgendeinem der Ansprüche 17, 18, 19 oder 20.

34. Die Verwendung von Anspruch 33, worin der Patient ein nicht-menschliches Tier ist.

35. Die Verwendung von Anspruch 33, worin der Patient ein Mensch ist.

## Revendications

1. Composé de formule :
Ao- A₁-A₂-A₃-A₄-A₅-A₆-A₇-A₈-A₉-A₁₀
ou sel, ester, solvate ou précurseur médicamenteux pharmaceutiquement acceptable de celui-ci, dans lequel :
A₁ est sarcosyle, A₂ est glycyle, A₃ est valyle, A₇ est isoleucyle, A₈ est arginyle, A₉ est prolyle et A₀, A₄, A₅, A₆ et A₁₀ sont comme suit :
A₀ est un hydrogène ou un groupe acyle choisi parmi :
(1) R-(CH₂)ₙ-C(O)- ; où n est un entier choisi parmi 0, 1, 2, 3 et 5 et R est choisi parmi méthyle ; N-acétylamino ; méthoxy ; carboxyle ; cyclohexyle ; cyclohexyle contenant une double liaison et substitué par trois groupes hydroxyle ; et un cycle aromatique ou non aromatique à 5 ou 6 côtés contenant optionnellement un hétéroatome choisi parmi l'azote et l'oxygène, ce cycle étant optionnellement substitué par alkyle;
et
(2) R¹-CH₂CH₂-(OCH₂CH₂O)ₚ-CH₂-C(O)- ; où R¹ est N-acétylamino, et p est égal à 1 ;
A₄ est un résidu aminoacyle de configuration L ou D choisi parmi :
(1) allo-isoleucyle,
(2) glycyle,
(3) isoleucyle,
(5) déshydroleucyle,
(6) D-alanyle,
(7) D-3-(naphth-1-yl) alanyle,
(8) D-3-(naphth-2-yl)alanyle,
(9) D-(3-pyridyl)alanyle,
(10) D-2-aminobutyryle,
(11) D-allo-isoleucyle,
(12) D-allo-thréonyle,
(14) D-asparaginyle,
(15) D-aspartyle,
(16) D-3-(3-benzothiényl)alanyle,
(17) D-3-(4,4'-biphényl)alanyle,
(18) D-3-(3-chlorophényl)alanyle,
(19) D-3-(3-trifluorométhylphényl)alanyle,
(20) D-3-(3-cyanophényl)alanyle,
(21) D-3-(3,4-difluorophényl)alanyle,
(22) D-citrullyle,
(23) D-cyclohexylalanyle,
(24) D-cyclohexylglycyle,
(25) D-cystyle,
(26) D-cystyle (S-t-butyl),
(27) D-glutaminyle,
(28) D-glutamyle,
(29) D-histidyle,
(31) D-homophénylalanyle,
(32) D-homoséryle,
(33) D-isoleucyle,
(34) D-leucyle,
(36) D-lysyle,
(37) D-méthionyle,
(38) D-néopentylglycyle,
(39) D-norleucyle,
(41)D-omithyle,
(42) D-pénicillaminyle,
(43) D-pénicillaminyle (acétamidométhyl),
(44) D-pénicillaminyle (S-benzyl),
(45) D-phénylalanyle,
(46) D-3-(4-aminophényl)alanyle,
(47) D-3-(4-méthylphényl)alanyle,
(48) D-3-(4-nitrophényl)alanyle,
(49) D-3-(3,4-diméthoxyphényl)alanyle,
(50) D-3-(3,4,5-trifluorophényl)alanyle,
(52) D-séryle,
(53) D-séryle (O-benzyl),
(54) D-t-butylglycyle,
(55) D-thiénylalanyle,
(56) D-thréonyle,
(57) D-thréonyle (O-benzyl),
(58) D-tryptyle,
(59) D-tyrosyle (O-benzyl),
(60) D-tyrosyle (O-éthyl),
(61) D-tyrosyle,
(62) D-valyle ; et
(63) D-3-(4-chlorophényl)alanyte ;
A₅ est un résidu aminoacyle de configuration L ou D choisi parmi :
(1) alanyle,
(3) 3-(naphth-1-yl)alanyle,
(4) 3-(naphth-2-yl)alanyle,
(6) allylglycyle,
(7) glutaminyle,
(8) glycyle,
(10) homoséryle,
(11) isoleucyle,
(12) lysyle (N-epsilon-acétyl),
(13) méthionyle,
(14) norvalyle,
(15) octylglycyle,
(17) 3-(4-hydroxyméthylphényl)alanyle,
(18) prolyle,
(19) séryle,
(20) thréonyle,
(21) tryptyle,
(22) tyrosyle,
(26) D-thréonyle, et
(27) pénicillaminyle ;
A₆ est un résidu aminoacyle choisi parmi :
(1) alanyle,
(5) 2-aminobutyryle,
(6) allylglycyle,
(7) arginyle,
(8) asparaginyle,
(9) aspartyle,
(10) citrullyle,
(11) 3-(cyclohexyl)alanyle,
(12) glutaminyle,
(13) glutamyle,
(14) glycyle,
(19) isoleucyle,
(20) leucyle,
(21) lysyle (N-epsilon-acétyl),
(23) méthionyle (sulfone),
(24) méthionyle (sulfoxyde),
(25) méthionyle,
(26) norleucyle,
(27) norvalyle,
(28) octylglycyle,
(30) 3-(4-carboxyamidephényl)alanyle,
(31) propargylglycyle,
(32) séryle,
(35) tyrosyle,
(36) valyle,
(42) D-norvalyle, et
(44) pénicillaminyle ;
A₁₀ est choisi parmi:
(1) hydroxyle,
(2) azaglycylamide,
(3) D-alanylamide,
(7) sarcosylamide,
(8) sérylamide,
(9) D-sérylamide,
(10) un groupe représenté par la formule 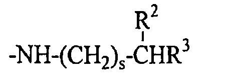 et
(11) un groupe représenté par la formule -NH-R⁴ ;
où
s est un entier choisi parmi 0 et 1,
R² est choisi parmi l'hydrogène, un alkyle et un cycle cycloalkyle à 6 côtés ;
R³ est choisi parmi l'hydrogène, les groupes hydroxy, alkyle, alcoxy, et un cycle à 6 côtés contenant un azote, dans la mesure où s n'est pas égal à 0 si R³ est un hydroxy ou un alcoxy ; et
R⁴ est choisi parmi hydrogène et hydroxy.

2. Composé selon la revendication 1, dans lequel A₄ est un résidu aminoacyle choisi parmi :
(1) D-alanyle,
(2) D-3-(naphth-1-yl)alanyle,
(3) D-3-(naphth-2-yl)alanyle,
(4) D-(3-pyridyl)alanyle,
(5) D-2-aminobutyryle,
(6) D-allo-isoleucyle,
(7) D-allo-thréonyle,
(9) D-asparaginyle,
(10) D-aspartyle,
(11) D-3-(3-chlorophényl)alanyle,
(12) D-3-(3-trifluorométhylphényl)alanyle,
(13) D-3-(3-cyanophényl)alanyle,
(14) D-3-(3,4-difluorophényl)alanyle,
(15) D-cyclohexylalanyle,
(16) D-cyclohexylglycyle,
(17) D-cystyle,
(18) D-glutaminyle,
(19) D-glutamyle,
(20) D-histidyle,
(22) D-homophénylalanyle,
(23) D-homoséryle,
(24) D-isoleucyle,
(25) D-leucyle,
(27) D-méthionyle,
(28) D-néopentylglycyle,
(29) D-norleucyle,
(31) D-pénicillaminyle,
(32) D-pénicillaminyle (acétamidométhyl),
(33) D-pénicillaminyle (S-benzyl),
(34) D-phénylalanyle,
(35) D-3-(4-aminophényl)alanyle,
(36) D-3-(4-méthylphényl)alanyle,
(37) D-3-(4-nitrophényl)alanyle,
(38) D-3-(3,4-diméthoxyphényl)alanyle,
(39) D-3-(3,4,5-trifluorophényl)alanyle,
(41) D-séryle,
(42) D-séryle (O-benzyl),
(43) D-t-butylglycyle,
(44) D-thiénylalanyle,
(45) D-thréonyle,
(46) D-thréonyle (O-benzyl),
(47) D-tyrosyle (O-éthyl),
(48) D-tyrosyle,
(49) D-valyle ; et
(50) D-3-(4-chlorophényl)alanyle.

3. Composé selon la revendication 2, dans lequel A₄ est un résidu aminoacyle choisi parmi :
(1) D-allo-isoleucyle,
(3) D-3-(3-cyanophényl)alanyle,
(4) D-cystyle,
(5) D-isoleucyle,
(6) D-leucyle,
(7) D-pénicillaminyle,
(8) D-phénylalanyle,
(9) D-3-(3,4,5-trifluorophényl)alanyle, et
(10) D-3-(4-aminophényl)alanyle.

4. Composé selon la revendication 1, dans lequel A₅ est choisi parmi :
(1) glycyle,
(2) octylglycyle,
(3) pénicillaminyle,
(4) séryle,
(5) thréonyle, et
(6)tyrosyle.

5. Composé selon la revendication 1, dans lequel A₆ est choisi parmi :
(1) glutaminyle,
(2) leucyle,
(3) norvalyle, et
(4) séryle.

6. Composé selon la revendication 2, dans lequel A₀ est choisi parmi :
(1) acétyle,
(2) butyryle,
(5) N-acétyl-β-alanyle,
(6) (6-N-acétylamino)caproyle,
(8) cyclohexylacétyle,
(9) furoyle,
(10) gamma-aminobutyryle,
(11) 2-méthoxyacétyle,
(12) méthylnicotinyle,
(13) nicotinyle,
(15) phénylacétyle,
(16) propionyle,
(17) shikimyle,
(18) succinyle, et
(19) tétrahydrofuroyle.

7. Composé selon la revendication 2, dans lequel A₁₀ est choisi parmi :
(1) D-alanylamide,
(2) azaglycylamide,
(3) sérylamide,
(4) éthylamide,
(5) hydroxylamide,
(6) isopropylamide,
(7) propylamide,
(8) 2-(cyclohexyl)éthylamide,
(9) 2-(1-pyrrolidine)éthylamide,
(10) 1-(cyclohexyl)éthylamide,
(11) 2-(méthoxy)éthylamide, et
(12) 2-(hydroxy)éthylamide.

8. Composé selon la revendication 1, dans lequel A₄ est un résidu aminoacyle choisi parmi :
(1) D-allo-isoleucyle,
(3) D-3-(3-cyanophényl)alanyle,
(4) D-cystyle,
(5) D-isoleucyle,
(6) D-leucyle,
(7) D-pénicillaminyle,
(8) D-phénylalanyle,
(9) D-3-(3,4,5-trifluorophényl)alanyle, et
(10) D-3-(4-aminophényl)alanyle,
A₅ est un résidu aminoacyle choisi parmi :
(1) octylglycyle,
(2) glycyle,
(3) pénicillaminyle,
(4) séryle,
(5) thréonyle, et
(6) tyrosyle, et
A₆ est un résidu aminoacyle choisi parmi :
(1) glutaminyle,
(2) leucyle,
(3) norvalyle, et
(4) séryle.

9. Composé selon la revendication 8, dans lequel A₀ est choisi parmi :
(1) acétyle,
(2) butyryle,
(5) N-acétyl-β-alanyle,
(6) (6-N-acétylamino)caproyle,
(8) cyclohexylacétyle,
(9) furoyle,
(10) gamma-aminobutyryle,
(11) 2-méthoxyacétyle,
(12) méthylnicotinyle,
(13) nicotinyle,
(15) phénylacétyle,
(16) propionyle,
(17) shikimyle,
(18) succinyle, et
(19) tétrahydrofuroyle.

10. Composé selon la revendication 8, dans lequel A₁₀ est choisi parmi :
(1) D-alanylamide,
(2) azaglycylamide,
(3) sérylamide,
(4) éthylamide,
(5) hydroxylamide,
(6) isopropylamide,
(7) propylamide,
(8) 2-(cyclohexyl)éthylamide,
(9) 2-(1-pyrrolidine)éthylamide,
(10) 1-(cyclohexyl)éthylamide,
(11) 2-(méthoxy)éthylamide, et
(12) 2-(hydroxy)éthylamide.

11. Composé selon la revendication 1, ou sel, ester, solvate ou précurseur médicamenteux pharmaceutiquement acceptable de celui-ci, choisi parmi :
(1) N-Ac-Sar-Gly-Val-D-Ile-Thr-Nva-Ile-Arg-ProNHCH₂CH₃,
(2) pyroGlu-Gly-Val-D-Ile-Thr-Nva-Ile-Arg-ProNHCH₂CH₃,
(3) N-Ac-Sar-Gly-Val-D-Ile-Thr-Nva-Ile-Arg-ProNHCH₃,
(4) N-Ac-Sar-Gly-Val-D-Ile-Thr-Nva-Ile-Arg-ProNHCH(CH₃)₂,
(5) N-Ac-Sar-Gly-Val-D-Ile-Thr-Nva-Ile-Arg-ProNHCH₂CH₂-(1-pyrrolidine),
(6) N-Ac-Sar-Gly-Val-D-Ile-Thr-Nva-Ile-Arg-ProNHéthyl(1-pipéridine),
(7) N-Ac-Sar-Gly-Val-D-Ile-Thr-Nva-Ile-Arg-ProNHméthylcyclopropyle,
(8) N-Ac-Sar-Gly-Val-D-Ile-Thr-Nva-Ile-Arg-ProNH (éthyl-1-(R)-cyclohexyle),
(9) N-Ac-Sar-Gly-Val-D-Ile-Thr-Nva-Ile-Arg-ProNH₂,
(10) N-Ac-Sar-Gly-Val-D-Ile-Thr-Nva-Ile-Arg-ProNHCH₂CH₂OCH₃,
(11) N-Ac-Sar-Gly-Val-D-Ile-Thr-Nva-Ile-Arg-ProNHCH₂CH₂cyclohexyle,
(12) N-Ac-Sar-Gly-Val-D-Ile-Thr-Nva-Ile-Arg-ProNHCH₂CH₂CH₃,
(13) N-Ac-Sar-Gly-Val-D-alloIle-Thr-Nva-Ile-Arg-ProNHCH₂CH₃,
(14) N-Ac-Sar-Gly-Val-D-Leu-Thr-Nva-Ile-Arg-ProNHCH₂CH₃,
(15) N-Ac-Sar-Gly-Val-Ile-Thr-Nva-Ile-Arg-ProNHCH₂CH₃,
(16) N-Ac-Sar-Gly-Val-Gly-Thr-Nva-Ile-Arg-ProNHCH₂CH₃,
(17) N-Ac-Sar-Gly-Val-D-Val-Thr-Nva-Ile-Arg-ProNHCH₂CH₃,
(18) N-Ac-Sar-Gly- Val-D-Ala- Thr-Nva-Ile-Arg-ProNHCH₂CH₃,
(19) N-Ac-Sar-Gly-Val-D-Met-Thr-Nva-Ile-Arg-ProNHCH₂CH₃,
(20) N-Ac-Sar-Gly-Val-D-Nle-Thr-Nva-Ile-Arg-ProNHCH₂CH₃,
(21) N-Ac-Sar-Gly-Val-D-Phe-Thr-Nva-Ile-Arg-ProNHCH₂CH₃,
(22) N-Ac-Sar-Gly-Val-D-Tyr-Thr-Nva-Ile-Arg-ProNHCH₂CH₃,
(23) N-Ac-Sar-Gly-Val-D-4,4-Biphénylala-Thr-Nva-Ile-Arg-ProNHCH₂CH₃,
(24) N-Ac-Sar-Gly-Val-D-Cha-Thr-Nva-Ile-Arg-ProNHCH₂CH₃,
(25) N-Ac-Sar-Gly-Val-D-Chg-Thr-Nva-Ile-Arg-ProNHCH₂CH₃,
(26) N-Ac-Sar-Gly-Val-D-4-ClPhe-Thr-Nva-Ile-Arg-ProNHCH₂CH₃,
(27) N-Ac-Sar-Gly-Val-D-Hphe-Thr-Nva-Ile-Arg-ProNHCH₂CH₃,
(28) N-Ac-Sar-Gly-Val-Déshydroleu-Thr-Nva-Ile-Arg-ProNHCH₂CH₃,
(29) N-Ac-Sar-Gly-Val-D-3-CF3Phe-Thr-Nva-Ile-Arg-ProNHCH₂CH₃,
(32) N-Ac-Sar-Gly-Val-D-3-ClPhe-Thr-Nva-Ile-Arg-ProNHCH₂CH₃,
(33) N-Ac-Sar-Gly-Val-D-2-Thiénylala-Thr-Nva-Ile-Arg-ProNHCH₂CH₃,
(34) N-Ac-Sar-Gly-Val-D-3-CNPhe-Thr-Nva-Ile-Arg-ProNHCH₂CH₃,
(35) N-Ac-Sar-Gly-Val-D-Ile-Thr-DNva-Ile-Arg-ProNHCH₂CH₃,
(36) N-Ac-Sar-Gly-Val-D-Ile-Thr-Gln-Ile-Arg-ProNHCH₂CH₃,
(37) N-Ac-Sar-Gly-Val-D-Ile-Thr-Cha-Ile-Arg-ProNHCH₂CH₃,
(38) N-Ac-Sar-Gly-Val-D-Ile-Thr-Gly-Ile-Arg-ProNHCH₂CH₃,
(39) N-Ac-Sar-Gly-Val-D-Ile-Thr-Ala-Ile-Arg-ProNHCH₂CH₃,
(40) N-Ac-Sar-Gly-Val-D-Ile-Thr-Val-Ile-Arg-ProNHCH₂CH₃,
(41) N-Ac-Sar-Gly-Val-D-Ile-Thr-Abu-Ile-Arg-ProNHCH₂CH₃,
(42) N-Ac-Sar-Gly-Val-D-Ile-Thr-Allylgly-Ile-Arg-ProNHCH₂CH₃,
(43) N-Ac-Sar-Gly-Val-D-Ile-Thr-Octylgly-Ile-Arg-ProNHCH₂CH₃,
(44) N-Ac-Sar-Gly-Val-D-Ile-Thr-Met-Ile-Arg-ProNHCH₂CH₃,
(45) N-Cyclohexylacétyl-Sar-Gly-Val-D-Ile-Thr-Nva-Ile-Arg-ProNHCH₂CH₃,
(46) N-(2-Me-Nicotinyl)-Sar-Gly-Val-D-Ile-Thr-Nva-Ile-Arg-ProNHCH₂CH₃,
(47) N-Succinyl-Sar-Gly-Val-D-Ile-Thr-Nva-Ile-Arg-ProNHCH₂CH₃,
(48) N-Nicotinyl-Sar-Gly-Val-D-Ile-Thr-Nva-Ile-Arg-ProNHCH₂CH₃,
(49) N-Propionyl-Sar-Gly-Val-D-Ile-Thr-Nva-Ile-Arg-ProNHCH₂CH₃,
(50) N-(MeO)acétyl-Sar-Gly-Val-D-Ile-Thr-Nva-Ile-Arg-ProNHCH₂CH₃,
(51) N-(Shikimyl)-Sar-Gly-Val-D-Ile-Thr-Nva-Ile-Arg-ProNHCH₂CH₃,
(52) N-(2-Furoyl)-Sar-Gly-Val-D-Ile-Thr-Nva-Ile-Arg-ProNHCH₂CH₃,
(53) N-Butyryl-Sar-Gly-Val-D-Ile-Thr-Nva-Ile-Arg-ProNHCH₂CH₃,
(54) N-[2-THFcarbonyl]-Sar-Gly-Val-D-Ile-Thr-Nva-Ile-Arg-ProNHCH₂CH₃,
(55) N-[CH₃C(O)NH-(CH₂)₂-O-(CH₂)₂-O-CH₂-C(O)]-Sar-Gly-Val-D-Ile-Thr-Nva-Ile-Arg-ProNHCH₂CH₃,
(56) N-[6-N-acétyl-(CH₂)₅C(O)]-Sar-Gly-Val-D-Ile-Thr-Nva-Ile-Arg-ProNHCH₂CH₃,
(58) N-[4-N-Acétylaminobutyryl]-Sar-Gly-Val-D-Ile-Thr-Nva-Ile-Arg-ProNHCH₂CH₃,
(59) H-Sar-Gly-Val-D-Ile-Thr-Nva-Ile-Arg-ProNHCH₂CH₃,
(66) N-Ac-Sar-Gly-Val-D-Ile-Thr-Leu-Ile-Arg-ProNHCH₂CH₃,
(67) N-Ac-Sar-Gly-Val-D-Ile-Thr-Ser-Ile-Arg-ProNHCH₂CH₃,
(68) N-Ac-Sar-Gly-Val-D-Ile-Thr-Nva-Ile-Arg-Pro-D-AlaNH₂,
(77) N-Ac-Sar-Gly-Val-D-Tyr(Et)-Thr-Nva-Ile-Arg-ProNHCH₂CH₃,
(78) N-Ac-Sar-Gly-Val-D-Cys(tBu)-Thr-Nva-Ile-Arg-ProNHCH₂CH₃,
(79) N-Ac-Sar-Gly-Val-D-Cys-Thr-Nva-Ile-Arg-ProNHCH₂CH₃,
(80) N-Ac-Sar-Gly-Val-D-Tyr(Bzl)-Thr-Nva-Ile-Arg-ProNHCH₂CH₃,
(81) N-Ac-Sar-Gly-Val-D-Ser(Bzl)-Thr-Nva-Ile-Arg-ProNHCH₂CH₃,
(82) N-Ac-Sar-Gly- Val-D-INal-Thr-Nva-Ile-Arg-ProNHCH₂CH₃,
(83) N-Ac-Sar-Gly-Val-D-tButylgly-Thr-Nva-Ile-Arg-ProNHCH₂CH₃,
(84) N-Ac-Sar-Gly-Val-D-Orn-Thr-Nva-Ile-Arg-ProNHCH₂CH₃,
(85) N-Ac-Sar-Gly-Val-D-Thr (Bzl)-Thr-Nva-Ile-Arg-ProNHCH₂CH₃,
(86) N-Ac-Sar-Gly-Val-D-2Nal-Thr-Nva-Ile-Arg-ProNHCH₂CH₃,
(87) N-Ac-Sar-Gly-Val-D-Phe(4-Me)-Thr-Nva-Ile-Arg-ProNHCH₂CH₃,
(88) N-Ac-Sar-Gly-Val-D-Phe(3,4-diMeO)-Thr-Nva-Ile-Arg-ProNHCH₂CH₃,
(89) N-Ac-Sar-Gly-Val-D-Phe(3,4,5-triF)-Thr-Nva-Ile-Arg-ProNHCH₂CH₃,
(90) N-Ac-Sar-Gly-Val-D-Phe(4-NO₂)-Thr-Nva-Ile-Arg-ProNHCH₂CH₃,
(91) N-Ac-Sar-Gly- Val-D-Pen- Thr-Nva-Ile-Arg-ProNHCH₂CH₃,
(92) N-Ac-Sar-Gly-Val-D-Pen (Acm)-Thr-Nva-Ile-Arg-ProNHCH₂CH₃,
(93) N-Ac-Sar-Gly- Val-D-Abu- Thr-Nva-Ile-Arg-ProNHCH₂CH₃,
(94) N-Ac-Sar-Gly-Val-D-Phe(4-NH₂)-Thr-Nva-Ile-Arg-ProNHCH₂CH₃,
(108) N-Ac-Sar-Gly-Val-D-Leu-Ala-Nva-Ile-Arg-ProNHCH₂CH₃,
(109) N-Ac-Sar-Gly-Val-D-Leu-Pro-Nva-Ile-Arg-ProNHCH₂CH₃,
(110) N-Ac-Sar-Gly-Val-D-Leu-Trp-Nva-Ile-Arg-ProNHCH₂CH₃,
(111) N-Ac-Sar-Gly-Val-D-Leu-Tyr-Nva-Ile-Arg-ProNHCH₂CH₃,
(112) N-Ac-Sar-Gly-Val-D-Leu-Nva-Nva-Ile-Arg-ProNHCH₂CH₃,
(113) N-Ac-Sar-Gly-Val-D-Leu-Gly-Nva-Ile-Arg-ProNHCH₂CH₃,
(114) N-Ac-Sar-Gly-Val-D-Leu-Lys(Ac)-Nva-Ile-Arg-ProNHCH₂CH₃,
(115) N-Ac-Sar-Gly-Val-D-Leu-2Na1-Nva-Ile-Arg-ProNHCH₂CH₃,
(116) N-Ac-Sar-Gly-Val-D-Leu-INal-Nva-Ile-Arg-ProNHCH₂CH₃,
(117) N-Ac-Sar-Gly-Val-D-Leu-Octylgly-Nva-Ile-Arg-ProNHCH₂CH₃,
(118) N-Ac-Sar-Gly-Val-D-Leu-Gln-Nva-Ile-Arg-ProNHCH₂CH₃,
(119) N-Ac-Sar-Gly-Val-D-Leu-Met-Nva-Ile-Arg-ProNHCH₂CH₃,
(120) N-Ac-Sar-Gly-Val-D-Leu-Ser-Nva-Ile-Arg-ProNHCH₂CH₃,
(121) N-Ac-Sar-Gly-Val-D-Leu-Allylgly-Nva-Ile-Arg-ProNHCH₂CH₃,
(122) N-Ac-Sar-Gly-Val-D-Leu-Ile-Nva-Ile-Arg-ProNHCH₂CH₃,
(123) N-Ac-Sar-Gly-Val-D-Leu-D-Thr-Nva-Ile-Arg-ProNHCH₂CH₃,
(124) N-Ac-Sar-Gly-Val-D-Ile-Thr-Ile-Ile-Arg-ProNHCH₂CH₃,
(125) N-Ac-Sar-Gly-Val-D-Ile-Thr-Nle-Ile-Arg-ProNHCH₂CH₃,
(126) N-Ac-Sar-Gly- Val-D-Ile- Thr-Cit-Ile-Arg-ProNHCH₂CH₃,
(127) N-Ac-Sar-Gly-Val-D-Ile-Thr-Met(O₂)-Ile-Arg-ProNHCH₂CH₃,
(128) N-Ac-Sar-Gly-Val-D-Ile-Thr-Arg-Ile-Arg-ProNHCH₂CH₃,
(129) N-Ac-Sar-Gly-Val-D-Ile-Thr-Tyr-Ile-Arg-ProNHCH₂CH₃,
(130) N-Ac-Sar-Gly-Val-D-Ile-Thr-Glu-Ile-Arg-ProNHCH₂CH₃,
(131) N-Ac-Sar-Gly-Val-D-Ile-Thr-Lys (Ac)-Ile-Arg-ProNHCH₂CH₃,
(132) N-Ac-Sar-Gly-Val-D-Ile-Thr-Propargylgly-Ile-Arg-ProNHCH₂CH₃,
(133) N-Ac-Sar-Gly-Val-D-alloIle-Thr-Gln-Ile-Arg-ProNHCH₂CH₃,
(134) N-Ac-Sar-Gly-Val-D-Leu-Thr-Gln-Ile-Arg-ProNHCH₂CH₃,
(135) N-Ac-Bala-Sar-Gly-Val-D-Ile-Thr-Nva-Ile-Arg-ProNHCH₂CH₃,
(136) N-Phénylacétyl-Sar-Gly-Val-D-Ile-Thr-Nva-Ile-Arg-ProNHCH₂CH₃,
(137) N-Ac-Sar-Gly-Val-D-Ile-Thr-Nva-Ile-Arg-Pro-AzaglyNH₂,
(139) N-Ac-Sar-Gly-Val-D-Ile-Thr-Nva-Ile-Arg-Pro-SerNH₂,
(140) N-Succinyl-Sar-Gly- Val-D-Leu- Thr-Nva-Ile-Arg-ProNHCH₂CH₃,
(158) N-Ac-Sar-Gly-Val-D-Ile-Thr-Gln-Ile-Arg-ProNHCH(CH₃)₂,
(159) N-Succinyl-Sar-Gly-Val-D-Ile-Thr-Gln-Ile-Arg-ProNHCH₂CH₃,
(160) N-Succinyl-Sar-Gly-Val-D-Leu-Thr-Gln-Ile-Arg-ProNHCH₂CH₃,
(161) N-Succinyl-Sar-Gly-Val-D-Leu-Thr-Gln-Ile-Arg-ProNHCH(CH₃)₂,
(162) N-Ac-Sar-Gly-Val-D-Leu-Thr-Asp-Ile-Arg-ProNHCH₂CH₃,
(163) N-Ac-Sar-Gly-Val-D-Ile-Thr-Asp-Ile-Arg-ProNHCH₂CH₃,
(164) N-Ac-Sar-Gly-Val-D-Ile-Thr-Asn-Ile-Arg-ProNHCH₂CH₃,
(165) N-Ac-Sar-Gly-Val-D-Ile-Thr-Met(O)-Ile-Arg-ProNHCH₂CH₃,
(166) N-Ac-Sar-Gly-Val-D-Leu-Thr-Asn-Ile-Arg-ProNHCH₂CH₃,
(167) N-Ac-Sar-Gly-Val-D-Thre-Thr-Nva-Ile-Arg-ProNHCH₂CH₃,
(168) N-Ac-Sar-Gly-Val-D-Ser-Thr-Nva-Ile-Arg-ProNHCH₂CH₃,
(169) N-Ac-Sar-Gly-Val-D-Hser-Thr-Nva-Ile-Arg-ProNHCH₂CH₃,
(170) N-Ac-Sar-Gly-Val-D-Gln-Thr-Nva-Ile-Arg-ProNHCH₂CH₃,
(171) N-Ac-Sar-Gly-Val-D-Asn-Thr-Nva-Ile-Arg-ProNHCH₂CH₃,
(172) N-Ac-Sar-Gly-Val-D-Cit-Thr-Nva-Ile-Arg-ProNHCH₂CH₃,
(173) N-Ac-Sar-Gly-Val-D-Hcit-Thr-Nva-Ile-Arg-ProNHCH₂CH₃,
(174) N-Ac-Sar-Gly-Val-D-Hle-Thr-Nva-Ile-Arg-ProNHCH₂CH₃,
(175) N-Ac-Sar-Gly-Val-D-Néopentylgly-Thr-Nva-Ile-Arg-ProNHCH₂CH₃,
(176) N-Ac-Sar-Gly-Val-D-Ile-Thr-Phe(4-CONH₂)-Ile-Arg-ProNHCH₂CH₃, (197) N-Succinyl-Sar-Gly-Val-D-alloIle-Thr-Nva-Ile-Arg-ProNHCH₂CH₃,
(198) N-Succinyl-Sar-Gly-Val-D-Ile-Thr-Gln-Ile-Arg-ProNHCH₂CH₃,
(199) N-Succinyl-Sar-Gly-Val-D-Ile-Thr-Gln-Ile-Arg-Pro-D-AlaNH₂,
(200) N-Succinyl-Sar-Gly- Val- D-alloIle- Thr-Gln- Ile-Arg-ProNHCH₂CH₃,
(201) N-Succinyl-Sar-Gly-Val-D-alloIle-Thr-Gln-Ile-Arg-Pro-D-AlaNH₂,
(202) N-Succinyl-Sar-Gly-Val-D-alloIle-Thr-Gln-Ile-Arg-ProNHCH₂CH₃,
(203) N-Succinyl-Sar-Gly-Val-D-Ile-Thr-Gln-Ile-Arg-ProNHCH₂CH₃,
(204) N-Ac-Sar-Gly-Val-D-alloIle-Thr-Nva-Ile-Arg-Pro-D-AlaNH₂,
(205) N-Ac-Sar-Gly-Val-D-alloIle-Thr-Nva-Ile-Arg-ProNHCH(CH₃)₂,
(206) N-Ac-Sar-Gly-Val-D-Ile-Thr-Gln-Ile-Arg-Pro-D-AlaNH₂,
(207) N-Ac-Sar-Gly-Val-D-Ile-Thr-Gln-Ile-Arg-ProNHCH(CH₃)₂,
(208) N-Ac-Sar-Gly-Val-D-alloIle-Thr-Gln-Ile-Arg-Pro-D-AlaNH₂,
(209) N-Ac-Sar-Gly-Val-D-alloIle-Thr-Gln-Ile-Arg-ProNHCH(CH₃)₂,
(210) N-Ac-Sar-Gly-Val-D-Ile-Thr-Nva-Ile-Arg-Pro-SarNH₂,
(211) N-Ac-Sar-Gly-Val-D-alloIle-Thr-Nva-Ile-Arg-Pro-SarNH₂,
(212) N-Ac-Sar-Gly-Val-D-Ile-Thr-Gln-Ile-Arg-Pro-SarNH₂,
(213) N-Ac-Sar-Gly-Val-D-alloIle-Thr-Gln-Ile-Arg-Pro-SarNH₂,
(214) N-Ac-Sar-Gly-Val-D-alloIle-Thr-Ser-Ile-Arg-Pro-D-AlaNH₂,
(215) N-Ac-Sar-Gly-Val-D-alloIle-Thr-Ser-Ile-Arg-ProNHCH(CH₃)₂,
(216) N-Ac-Sar-Gly-Val-D-alloIle-Thr-Ser-Ile-Arg-ProNHCH₂CH₃,
(217) N-Ac-Sar-Gly-Val-D-Ile-Thr-Orn (Ac)-Ile-Arg-ProNHCH₂CH₃,
(218) N-Ac-Sar-Gly-Val-D-Ile-Thr-Gln-Ile-Arg-Pro-AzaglyNH₂,
(219) N-Ac-Sar-Gly-Val-D-alloIle-Thr-Nva-Ile-Arg-Pro-AzaglyNH₂,
(220) N-Ac-Sar-Gly-Val-D-alloIle-Thr-Gln-Ile-Arg-Pro-AzaglyNH₂,
(221) N-(2-THFcarbonyl)-Sar-Gly-Val-D-alloIle-Thr-Nva-Ile-Arg-ProNHCH₂CH₃,
(222) N-(2-THFcarbonyl)-Sar-Gly-Val-D-Ile-Thr-Gln-Ile-Arg-ProNHCH₂CH₃,
(223) N-(2-THFcarbonyl)-Sar-Gly-Val-D-alloIle-Thr-Gln-Ile-Arg-ProNHCH₂CH₃,
(224) N-(2-THFcarbonyl)-Sar-Gly-Val-D-Ile-Thr-Gln-Ile-Arg-Pro-D-AlaNH₂,
(225) N-(2-THFcarbonyl)-Sar-Gly-Val-D-alloIle-Thr-Gln-Ile-Arg-Pro-D-AlaNH₂,
(226) N-(2-THFcarbonyl)-Sar-Gly-Val-D-allolle-Thr-Gln-Ile-Arg-ProNHCH(CH₃)₂,
(227) N-(6-Ac-Aca)-Sar-Gly-Val-D-allolle-Thr-Nva-Ile-Arg-ProNHCH₂CH₃,
(228) N-(6-Ac-Aca)-Sar-Gly-Val-D-Ile-Thr-Gln-Ile-Arg-ProNHCH₂CH₃,
(229) N-(6-Ac-Aca)-Sar-Gly-Val-D-allolle-Thr-Gln-Ile-Arg-ProNHCH₂CH₃,
(230) N-(6-Ac-Aca)-Sar-Gly-Val-D-Ile-Thr-Gln-Ile-Arg-Pro-D-AlaNE₂,
(231) N-(6-Ac-Aca)-Sar-Gly-Val-D-alloIle-Thr-Gln-Ile-Arg-Pro-D-AlaNH₂,
(232) N-(6-Ac-Aca)-Sar-Gly-Val-D-alloIle-Thr-Gln-Ile-Arg-ProNHCH(CH₃)₂,
(233) N-(4-Ac-Gaba)-Sar-Gly-Val-D-alloIle-Thr-Nva-Ile-Arg-ProNHCH₂CH₃,
(234) N-(4-Ac-Gaba)-Sar-Gly-Val-D-Ile-Thr-Gln-Ile-Arg-ProNHCH₂CH₃,
(235) N-(4-Ac-Gaba)-Sar-Gly-Val-D-alloIle-Thr-Gln-Ile-Arg-ProNHCH₂CH₃,
(236) N-(4-Ac-Gaba)-Sar-Gly-Val-D-Ile-Thr-Gln-Ile-Arg-Pro-D-AlaNH₂,
(237) N-(4-Ac-Gaba)-Sar-Gly-Val-D-alloIle-Thr-Gln-Ile-Arg-Pro-D-AlaNH₂,
(238) N-(4-Ac-Gaba)-Sar-Gly-Val-D-alloIle-Thr-Gln-Ile-Arg-ProNHCH(CH₃)₂,
(239) N-(2-Furoyl)-Sar-Gly-Val-D-alloIle-Thr-Nva-Ile-Arg-ProNHCH₂CH₃,
(240) N-(2-Furoyl)-Sar-Gly-Val-D-Ile-Thr-Gln-Ile-Arg-ProNHCH₂CH₃,
(241) N-(2-Furoyl)-Sar-Gly-Val-D-alloIle-Thr-Gln-Ile-Arg-ProNHCH₂CH₃,
(242) N-(2-Furoyl)-Sar-Gly-Val-D-Ile-Thr-Gln-Ile-Arg-Pro-D-AlaNH₂,
(243) N-(2-Furoyl)-Sar-Gly-Val-D-alloIle-Thr-Gln-Ile-Arg-Pro-D-AlaNH₂,
(244) N-(2-Furoyl)-Sar-Gly- V al-D-alloIle- Thr-Gln- Ile-Arg-ProNHCH(CH₃)₂,
(245) N-(Shikimyl)-Sar-Gly-Val-D-alloIle-Thr-Nva-Ile-Arg-ProNHCH₂CH₃,
(246) N-(Shikimyl)-Sar-Gly-Val-D-Ile-Thr-Gln-Ile-Arg-ProNHCH₂CH₃,
(247) N-(Shikimyl)-Sar-Gly-Val-D-allolle-Thr-Gln-Ile-Arg-ProNHCH₂CH₃,
(248) N-(Shikimyl)-Sar-Gly-Val-D-Ile-Thr-Gln-Ile-Arg-Pro-D-AlaNE₂,
(249) N-(Shikimyl)-Sar-Gly-Val-D-alloIle-Thr-Gln-Ile-Arg-Pro-D-AlaNH₂,
(250) N-(Shikimyl)-Sar-Gly-Val-D-alloIle-Thr-Gln-Ile-Arg-ProNHCH(CH₃)₂,
(251) N-(2-Me-Nicotinyl)-Sar-Gly-Val-D-alloIle-Thr-Nva-Ile-Arg-ProNHCH₂CH₃.
(252) N-(2-Me-Nicotinyl)-Sar-Gly-Val-D-Ile-Thr-Gln-Ile-Arg-ProNHCH₂CH₃,
(253) N-(2-Me-Nicotinyl)-Sar-Gly-Val-D-alloIle-Thr-Gln-Ile-Arg-ProNHCH₂CH₃.
(254) N-(2-Me-Nicotinyl)-Sar-Gly-Val-D-Ile-Thr-Gln-Ile-Arg-Pro-D-AlaNH₂,
(255) N-(2-Me-Nicotinyl)-Sar-Gly-Val-D-alloIle-Thr-Gln-Ile-Arg-Pro-D-AlaNH₂,
(256) N-(2-Me-Nicotinyl)-Sar-Gly-Val-D-alloIle-Thr-Gln-Ile-Arg-ProNHCH(CH₃)₂,
(257) N-Ac-Sar-Gly-Val-D-alloIle-Thr-Leu-Ile-Arg-Pro-D-AlaNH₂,
(258) N-Ac-Sar-Gly-Val-D-Ile-Thr-Leu-Ile-Arg-ProNHCH(CH₃)₂,
(259) N-Ac-Sar-Gly-Val-D-alloIle-Thr-Leu-Ile-Arg-ProNHCH₂CH₃,
(260) N-Ac-Sar-Gly-Val-D-Ile-Thr-Leu-Ile-Arg-Pro-D-AlaNH₂,
(261) N-Succinyl-Sar-Gly- Val-D-Ile- Thr-Leu-Ile-Arg-Pro-D-AlaNH₂,
(262) N-Succinyl-Sar-Gly-Val-D-Ile-Thr-Leu-Ile-Arg-ProNHCH₂CH₃,
(263) N-Succinyl-Sar-Gly-Val-D-Ile-Thr-Leu-Ile-Arg-ProNHCH₂CH₃,
(264) N-Succinyl-Sar-Gly-Val-D-alloIle-Thr-Leu-Ile-Arg-ProNHCH₂CH₃,
(265) N-Succinyl-Sar-Gly-Val-D-alloIle-Thr-Leu-Ile-Arg-Pro-D-AlaNH₂,
(266) N-Succinyl-Sar-Gly-Val-D-Ile-Thr-Leu-Ile-Arg-Pro-AzaglyNH₂,
(267) N-Ac-Sar-Gly-Val-D-alloIle-Thr-Nva-Ile-Arg-ProNHéthyl-(1-pyrrolidine),
(268) N-Ac-Sar-Gly-Val-D-alloIle-Thr-Nva-Ile-Arg-ProNH(éthyl-1-cyclohexyle),
(269) N-Ac-Sar-Gly-Val-D-Ile-Thr-Gln-Ile-Arg-ProNHéthyl-(1-pyrrolidine),
(270) N-Ac-Sar-Gly-Val-D-Ile-Thr-Gln-Ile-Arg-ProNH(éthyl-1-cyclohexyle),
(271) N-Succinyl-Sar-Gly-Val-D-Ile-Thr-Gln-Ile-Arg-ProNH(éthyl-1-cyclohexyle).
(272) N-Ac-Sar-Gly-Val-D-alloIle-Thr-Nva-Ile-Arg-ProNHCH₂CH₂OCH₃,
(273) N-Ac-Sar-Gly-Val-D-Ile-Thr-Gln-Ile-Arg-ProNHCH₂CH₂OCH₃,
(274) N-Ac-Sar-Gly-Val-D-Ile-Thr-Ser-Ile-Arg-ProNHCH₂CH₂OCH₃,
(275) N-Ac-Sar-Gly-Val-D-Ile-Thr-Leu-Ile-Arg-ProNHCH₂CH₂OCH₃,
(276) N-Succinyl-Sar-Gly-Val-D-Ile-Thr-Nva-Ile-Arg-ProNHCH₂CH₂OCH₃,
(277) N-Succinyl-Sar-Gly-Val-D-Ile-Thr-Gln-Ile-Arg-ProNHCH₂CH₂OCH₃,
(278) N-Succinyl-Sar-Gly-Val-D-alloIle-Thr-Gln-Ile-Arg-ProNHCH₂CH₂OCH₃,
(279) N-Ac-Sar-Gly-Val-D-Ile-Ser-Nva-Ile-Arg-ProNHCH₂CH₂OCH₃,
(280) N-Ac-Sar-Gly-Val-D-Leu-Ser-Nva-Ile-Arg-ProN-HCH₂CH₂OCH₃,
(281) N-Ac-Sar-Gly-Val-D-alloIle-Thr-Allygly-Ile-Arg-ProNHCH₂CH₃,
(282) N-Ac-Sar-Gly-Val-D-Ile-Thr-Allygly-Ile-Arg-ProNHCH(CH₃)₂,
(283) N-Ac-Sar-Gly-Val-D-Ile-Thr-Allygly-Ile-Arg-Pro-D-AlaNH₂,
(284) N-Ac-Sar-Gly- Val-D-alloIle- Thr-Allygly-Ile-Arg-Pro-D-AlaNH₂,
(285) N-Succinyl-Sar-Gly-Val-D-Ile-Thr-Allygly-Ile-Arg-Pro-D-AlaNH₂,
(286) N-Ac-Sar-Gly-Val-D-Ile-Ser-Allygly-Ile-Arg-Pro-ProNHCH₂CH₃,
(287) N-Ac-Sar-Gly-Val-D-Leu-Ser-Allygly-Ile-Arg-Pro-ProNHCH₂CH₃,
(288) N-Ac-Sar-Gly-Val-D-Ile-Thr-Nva-Ile-Arg-Pro-SarNH₂,
(289) N-Ac-Sar-Gly-Val-D-Ile-Thr-Nva-Ile-Arg-ProNHOH,
(290) N-Ac-Sar-Gly-Val-D-Ile-Ser-Nva-Ile-Arg-ProNHCH₂CH₃,
(291) N-Ac-Sar-Gly-Val-D-allolle-Ser-Nva-Ile-Arg-ProNHCH₂CH₃,
(292) N-Ac-Sar-Gly-Val-D-Leu-Hser-Nva-Ile-Arg-ProNHCH₂CH₃,
(300) N-Ac-Sar-Gly-Val-D-alloIle-Thr-Ala-Ile-Arg-ProNHCH₂CH₃,
(301) N-Ac-Sar-Gly-Val-D-Ile-Thr-Ala-Ile-Arg-ProNHCH(CH₃)₂,
(302) N-Ac-Sar-Gly-Val-D-Ile-Thr-Ala-Ile-Arg-Pro-D-AlaNH₂,
(303) N-Ac-Sar-Gly- Val-D-alloIle- Thr-Ala-Ile-Arg-Pro-D-AlaNH₂,
(304) N-Succinyl-Sar-Gly-Val-D-Ile-Thr-Ala-Ile-Arg-Pro-D-AlaNH₂,
(305) N-Ac-Sar-Gly-Val-D-Ile-Ser-Ala-Ile-Arg-ProNHCH₂CH₃,
(306) N-Ac-Sar-Gly-Val-D-Leu-Ser-Ala-Ile-Arg-ProNHCH₂CH₃,
(307) N-Ac-Sar-Gly-Val-D-alloIle-Thr-Val-Ile-Arg-ProNHCH₂CH₃,
(308) N-Ac-Sar-Gly-Val-D-Ile-Thr-Val-Ile-Arg-ProNHCH(CH₃)₂,
(309) N-Ac-Sar-Gly-Val-D-Ile-Thr-Val-Ile-Arg-Pro-D-AlaNH₂,
(310) N-Ac-Sar-Gly- Val-D-alloIle-Thr-Val-Ile-Arg-Pro-D-AlaNH₂,
(311) N-Succinyl-Sar-Gly-Val-D-Ile-Thr-Val-Ile-Arg-Pro-D-AlaNH₂,
(312) N-Ac-Sar-Gly-Val-D-Ile-Ser-Val-Ile-Arg-ProNHCH₂CH₃,
(313) N-Ac-Sar-Gly-Val-D-Leu-Ser-Val-Ile-Arg-ProNHCH₂CH₃,
(314) N-Ac-Sar-Gly- Val-D-alloIle- Thr-D-Nva-Ile-Arg-ProNHCH₂CH₃,
(315) N-Ac-Sar-Gly-Val-D-Ile-Thr-D-Nva-Ile-Arg-ProNHCH(CH₃)₂,
(316) N-Ac-Sar-Gty-Val-D-Ile-Thr-D-Nva-IIe-Arg-Pro-D-AlaNH₂,
(317) N-Ac-Sar-Gly-Val-D-alloIle-Thr-D-Nva-Ile-Arg-Pro-D-AlaNH₂,
(318) N-Succinyl-Sar-Gly-Val-D-Ile-Thr-D-Nva-Ile-Arg-Pro-D-AlaNH₂,
(319) N-Ac-Sar-Gly-Val-D-Ile-Ser-D-Nva-Ile-Arg-ProNHCH₂CH₃,
(320) N-Ac-Sar-Gly-Val-D-Leu-Ser-D-Nva-Ile-Arg-ProNHCH₂CH₃,
(321) N-Ac-Sar-Gly-Val-D-Ile-Ser-Gln-Ile-Arg-ProNHCH₂CH₃,
(322) N-Ac-Sar-Gly-Val-D-Leu-Ser-Gln-Ile-Arg-ProNHCH₂CH₃,
(323) N-Ac-Sar-Gly-Val-D-Leu-Ser-Nva-Ile-Arg-Pro-D-AlaNH₂,
(324) N-Ac-Sar-Gly-Val-D-Ile-Ser-Nva-Ile-Arg-Pro-D-AlaNH₂,
(325) N-Succinyl-Sar-Gly-Val-D-Leu-Ser-Nva-Ile-Arg-ProNHCH₂CH₃,
(326) N-Succinyl-Sar-Gly-Val-D-Ile-Ser-Nva-Ile-Arg-ProNHCH₂CH₃,
(327) N-Succinyl-Sar-Gly-Val-D-Leu-Ser-Gln-Ile-Arg-ProNHCH₂CH₃,
(328) N-Succinyl-Sar-Gly-Val-D-Ile-Ser-Gln-Ile-Arg-ProNHCH₂CH₃,
(329) N-Ac-Sar-Gly-Val-D-Ile-Ser-Ser-Ile-Arg-ProNHCH₂CH₃,
(330) N-Ac-Sar-Gly-Val-D-Leu-Ser-Ser-Ile-Arg-ProNHCH₂CH₃,
(331) N-Ac-Sar-Gly-Val-D-Leu-Ser-Nva-Ile-Arg-ProNHCH₂CH₂CH₃,
(332) N-Ac-Sar-Gly-Val-D-Ile-Ser-Nva-Ile-Arg-ProNHCH₂CH₂CH₃,
(333) N-Ac-Sar-Gly-Val-D-Leu-Ser-Leu-Ile-Arg-ProNHCH₂CH₃,
(334) N-Ac-Sar-Gly-Val-D-Ile-Ser-Leu-Ile-Arg-ProNHCH₂CH₃,
(335) N-Ac-Sar-Gly-Val-D-alloIle-Ser-Nva-Ile-Arg-ProNHCH₂CH₃,
(336) N-Ac-Sar-Gly-Val-D-alloIle-Ser-Gln-Ile-Arg-ProNHCH₂CH₃,
(337) N-Succinyl-Sar-Gly-Val-D-alloIle-Ser-Nva-Ile-Arg-ProNHCH₂CH₃,
(338) N-Ac-Sar-Gly-Val-D-alloIle-Ser-Nva-Ile-Arg-ProNHCH₂CH₂CH₃,
(339) N-Ac-Sar-Gly-Val-D-alloIle-Ser-Nva-Ile-Arg-Pro-D-AlaNH₂,
(340) N-Ac-Sar-Gly-Val-D-alloIle-Ser-Leu-Ile-Arg-ProNHCH₂CH₃,
(341) N-Ac-Sar-Gly-Val-D-alloIle-Ser-Ser-Ile-Arg-ProNHCH₂CH₃,
(342) N-Ac-Sar-Gly-Val-D-Ile-Gly-Nva-Ile-Arg-ProNHCH₂CH₃,
(343) N-Ac-Sar-Gly-Val-D-alloIle-Gly-Nva-Ile-Arg-ProNHCH₂CH₃,
(344) N-Ac-Sar-Gly-Val-D-Leu-Gly-Gln-Ile-Arg-ProNHCH₂CH₃,
(345) N-Ac-Sar-Gly-Val-D-Ile-Gly-Gln-Ile-Arg-ProNHCH₂CH₃,
(346) N-Ac-Sar-Gly-Val-D-alloIle-Gly-Gln-Ile-Arg-ProNHCH₂CH₃,
(347) N-Ac-Sar-Gly-Val-D-Ile-Tyr-Nva-Ile-Arg-ProNHCH₂CH₃,
(348) N-Ac-Sar-Gly-Val-D-alloIle-Tyr-Nva-Ile-Arg-ProNHCH₂CH₃,
(349) N-Ac-Sar-Gly-Val-D-Leu-Tyr-Gln-Ile-Arg-ProNHCH₂CH₃,
(350) N-Ac-Sar-Gly-Val-D-Ile-Tyr-Gln-Ile-Arg-ProNHCH₂CH₃,
(351) N-Ac-Sar-Gly-Val-D-alloIle-Tyr-Gln-Ile-Arg-ProNHCH₂CH₃,
(352) N-Ac-Sar-Gly-Val-D-Ser-Thr-Nva-Ile-Arg-ProNHCH₂CH₃,
(353) N-Ac-Sar-Gly-Val-D-Thr-Thr-Nva-Ile-Arg-ProNHCH₂CH₃,
(354) N-Ac-Sar-Gly-Val-D-Gln-Thr-Nva-Ile-Arg-ProNHCH₂CH₃,
(355) N-Ac-Sar-Gly-Val-D-Asn-Thr-Nva-Ile-Arg-ProNHCH₂CH₃,
(356) N-Ac-Sar-Gly-Val-D-Arg-Thr-Nva-Ile-Arg-ProNHCH₂CH₃,
(357) N-Ac-Sar-Gly-Val-D-3-Pal-Thr-Nva-Ile-Arg-ProNHCH₂CH₃,
(358) N-Ac-Sar-Gly-Val-D-Glu-Thr-Nva-Ile-Arg-ProNHCH₂CH₃,
(359) N-Ac-Sar-Gly-Val-D-Asp-Thr-Nva-Ile-Arg-ProNHCH₂CH₃,
(360) N-Ac-Sar-Gly-Val-D-His-Thr-Nva-Ile-Arg-ProNHCH₂CH₃,
(361) N-Ac-Sar-Gly-Val-D-Hser-Thr-Nva-Ile-Arg-ProNHCH₂CH₃,
(362) N-Ac-Sar-Gly-Val-D-alloThr-Thr-Nva-Ile-Arg-ProNHCH₂CH₃,
(363) N-Ac-Sar-Gly-Val-D-Ile-Thr-Nva-D-Ile-Arg-ProNHCH₂CH₃,
(364) N-Ac-Sar-Gly-Val-D-Ser-Thr-Gln-Ile-Arg-ProNHCH₂CH₃,
(365) N-Ac-Sar-Gly-Val-D-Thr-Thr-Gln-Ile-Arg-ProNHCH₂CH₃,
(366) N-Ac-Sar-Gly-Val-D-alloThr-Thr-Gln-Ile-Arg-ProNHCH₂CH₃,
(367) N-Ac-Sar-Gly-Val-D-Ser-Ser-Nva-Ile-Arg-ProNHCH₂CH₃,
(368) N-Ac-Sar-Gly-Val-D-Thr-Ser-Nva-Ile-Arg-ProNHCH₂CH₃,
(369) N-Ac-Sar-Gly-Val-D-alloThr-Ser-Nva-Ile-Arg-ProNHCH₂CH₃,
(370) N-Ac-Sar-Gly-Val-D-alloThr-Ser-Gln-Ile-Arg-ProNHCH₂CH₃,
(371) N-Ac-Sar-Gly-Val-D-Thr-Ser-Gln-Ile-Arg-ProNHCH₂CH₃,
(372) N-(6-Ac-Aca)-Sar-Gly-Val-D-Leu-Ser-Gln-Ile-Arg-ProNHCH₂CH₃,
(373) N-(6-Ac-Aca)-Sar-Gly-Val-D-Leu-Ser-Nva-Ile-Arg-ProNHCH₂CH₃,
(374) N-(4-Ac-Gaba)-Sar-Gly-Val-D-Leu-Ser-Gln-Ile-Arg-ProNHCH₂CH₃,
(375) N-(4-Ac-Gaba)-Sar-Gly-Val-D-Leu-Ser-Nva-Ile-Arg-ProNHCH₂CH₃,
(376) N-(2-Furoyl)-Sar-Gly-Val-D-Leu-Ser-Gln-Ile-Arg-ProNHCH₂CH₃,
(377) N-(2-Furoyl)-Sar-Gly-Val-D-Leu-Ser-Nva-Ile-Arg-ProNHCH₂CH₃,
(378) N-(Shikimyl)-Sar-Gly-Val-D-Leu-Ser-Gln-Ile-Arg-ProNHCH₂CH₃,
(379) N-(Shikimyl)-Sar-Gly-Val-D-Leu-Ser-Nva-Ile-Arg-ProNHCH₂CH₃,
(382) N-(2-Me-nicotinyl)-Sar-Gly-Val-D-Leu-Ser-Gln-Ile-Arg-ProNHCH₂CH₃,
(383) N-(2-Me-nicotinyl)-Sar-Gly-Val-D-Leu-Ser-Nva-Ile-Arg-ProNHCH₂CH₃,
(384) N-Ac-Sar-Gly-Val-D-Leu-Ser-Nva-Ile-Arg-ProNHéthyl-1-(R)-cyclohexyle,
(385) N-Ac-Sar-Gly-Val-D-Leu-Ser-Gln-Ile-Arg-ProNHéthyl-1-(R)-cyclohexyle,
(386) N-Ac-Sar-Gly-Val-DIIe-Thr-Ser-Ile-Arg-ProNHéthyl-1-(R)-cyclohexyle,
(387) N-Ac-Sar-Gly-Val-D-Leu-Thr-Nva-Ile-Arg-ProNHéthyl-1-(R)-cyclohexyle,
(388) N-Ac-Sar-Gly-Val-D-Leu-Ser-Ser-Ile-Arg-ProNHéthyl-1-(R)-cyclohexyle,
(389) N-Ac-Sar-Gly-Val-DIle-Thr-Nva-Ile-Arg-ProNHéthyl-l-(S)-cyclohexyle,
(390) N-Ac-Sar-Gly-Val-D-Pen-Ser-Nva-Ile-Arg-ProNHCH₂CH₃,
(391) N-Ac-Sar-Gly-Val-D-Pen-Gly-Nva-Ile-Arg-ProNHCH₂CH₃,
(392) N-Ac-Sar-Gly-Val-D-Pen-Thr-Gln-Ile-Arg-ProNHCH₂CH₃,
(393) N-Ac-Sar-Gly-Val-D-Pen-Ser-Nva-Ile-Arg-ProNHCH(CH₃)₂,
(394) N-Succinyl-Sar-Gly-Val-D-Pen-Ser-Nva-Ile-Arg-ProNHCH₂CH₃,
(395) N-Ac-Sar-Gly-Val-D-Pen-Ser-Nva-Ile-Arg-Pro-D-AlaNH₂,
(396) N-Ac-Sar-Gly-Val-D-Pen-Ser-Gln-Ile-Arg-ProNHCH₂CH₃,
(397) N-Ac-Sar-Gly-Val-D-Pen-Gly-Gln-Ile-Arg-ProNHCH₂CH₃,
(398) N-Ac-Sar-Gly-Val-D-Pen-Ser-Ser-Ile-Arg-ProNHCH₂CH₃,
(399) N-Ac-Sar-Gly-Val-D-Pen-Thr-Ser-Ile-Arg-ProNHCH₂CH₃,
(400) N-Ac-Sar-Gly-Val-D-Pen-Thr-Leu-Ile-Arg-ProNHCH₂CH₃,
(401) N-Ac-Sar-Gly-Val-D-Pen-Ser-Leu-Ile-Arg-ProNHCH₂CH₃,
(402) N-Succinyl-Sar-Gly-Val-D-Pen-Ser-Ser-Ile-Arg-ProNHCH₂CH₃,
(403) N-Succinyl-Sar-Gly-Val-D-Pen-Ser-Leu-Ile-Arg-ProNHCH₂CH₃,
(404) N-Succinyl-Sar-Gly-Val-D-Pen-Thr-Gln-Ile-Arg-ProNHCH(CH₃)₂,
(405) N-Ac-Sar-Gly-Val-D-Cys-Thr-Nva-Ile-Arg-ProNHCH₂CH₃,
(406) N-Ac-Sar-Gly-Val-D-Cys-Ser-Nva-Ile-Arg-ProNHCH₂CH₃,
(407) N-Ac-Sar-Gly-Val-D-Cys-Gly-Nva-Ile-Arg-ProNHCH₂CH₃,
(408) N-Ac-Sar-Gly-Val-D-Cys-Thr-Gln-Ile-Arg-ProNHCH₂CH₃,
(409) N-Ac-Sar-Gly-Val-D-Cys-Ser-Nva-Ile-Arg-ProNHCH(CH₃)₂,
(410) N-Succinyl-Sar-Gly-Val-D-Cys-Ser-Nva-Ile-Arg-ProNHCH₂CH₃,
(411) N-Ac-Sar-Gly-Val-D-Cys-Ser-Nva-Ile-Arg-Pro-D-AlaNH₂,
(412) N-Ac-Sar-Gly-Val-D-Cys-Ser-Gln-Ile-Arg-ProNHCH₂CH₃,
(413) N-Ac-Sar-Gly-Val-D-Cys-Gly-Gln-Ile-Arg-ProNHCH₂CH₃,
(414) N-Ac-Sar-Gly-Val-D-Cys-Ser-Ser-Ile-Arg-ProNHCH₂CH₃,
(415) N-Ac-Sar-Gly-Val-D-Cys-Thr-Ser-Ile-Arg-ProNHCH₂CH₃,
(416) N-Ac-Sar-Gly-Val-D-Cys-Thr-Leu-Ile-Arg-ProNHCH₂CH₃,
(417) N-Ac-Sar-Gly-Val-D-Cys-Ser-Leu-Ile-Arg-ProNHCH₂CH₃,
(418) N-Succinyl-Sar-Gly-Val-D-Cys-Ser-Ser-Ile-Arg-ProNHCH₂CH₃,
(419) N-Succinyl-Sar-Gly-Val-D-Cys-Ser-Leu-Ile-Arg-ProNHCH₂CH₃,
(431) N-Ac-Sar-Gly-Val-D-Leu-Pen-Nva-Ile-Arg-ProNHCH₂CH₃,
(432) N-Ac-Sar-Gly-Val-D-Ile-Pen-Nva-Ile-Arg-ProNHCH₂CH₃,
(433) N-Ac-Sar-Gly-Val-D-alloIle-Pen-Nva-Ile-Arg-ProNHCH₂CH₃,
(434) N-Ac-Sar-Gly-Val-D-Ile-Pen-Gln-Ile-Arg-ProNHCH₂CH₃,
(435) N-Ac-Sar-Gly-Val-D-Ile-Pen-Ser-Ile-Arg-ProNHCH₂CH₃,
(436) N-Ac-Sar-Gly-Val-D-Ile-Pen-Leu-Ile-Arg-ProNHCH₂CH₃,
(437) N-Ac-Sar-Gly-Val-D-Ile-Pen-Nva-Ile-Arg-ProNHCH(CH₃)₂,
(438) N-Ac-Sar-Gly- Val-D-Ile-Pen-Nva-Ile-Arg-Pro-D-AlaNH₂,
(439) N-Succinyl-Sar-Gly-Val-D-Ile-Pen-Nva-Ile-Arg-ProNHCH₂CH₃,
(440) N-Succinyl-Sar-Gly-Val-D-Ile-Pen-Gln-Ile-Arg-ProNHCH₂CH₃,
(441) N-Succinyl-Sar-Gly-Val-D-Ile-Pen-Gln-Ile-Arg-ProNHCH(CH₃)₂,
(442) N-Ac-Sar-Gly-Val-D-Ile-Thr-Pen-Ile-Arg-ProNHCH₂CH₂OCH₃,
(443) N-Ac-Sar-Gly-Val-D-alloIle-Thr-Pen-Ile-Arg-ProNHCH₂CH₃,
(444) N-Ac-Sar-Gly-Val-D-Leu-Thr-Pen-Ile-Arg-ProNHCH₂CH₃,
(445) N-Ac-Sar-Gly-Val-D-Ile-Thr-Pen-Ile-Arg-Pro-D-AlaNH₂,
(446) N-Succinyl-Sar-Gly-Val-D-Ile-Thr-Pen-Ile-Arg-ProNHCH₂CH₃,
(447) N-Ac-Sar-Gly-Val-D-Ile-Thr-Pen-Ile-Arg-ProNHCH(CH₃)₂,
(448) N-Ac-Sar-Gly-Val-D-Leu-Ser-Pen-Ile-Arg-ProNHCH₂CH₃,
(449) N-Ac-Sar-Gly-Val-D-Leu-Gly-Pen-Ile-Arg-ProNHCH₂CH₃,
(450) N-Succinyl-Sar-Gly-Val-D-Leu-Ser-Pen-Ile-Arg-ProNHCH₂CH₃,
(451) N-Ac-Sar-Gly-Val-D-Phe (3,4,5-triF)-Thr-Gln-Ile-Arg-ProNHCH₂CH₃,
(452) N-Ac-Sar-Gly-Val-D-Phe (3,4,5-triF)-Ser-Nva-Ile-Arg-ProNHCH₂CH₃,
(453) N-Ac-Sar-Gly-Val-D-Phe(3,4,5-triF)-Gly-Nva-Ile-Arg-ProNHCH₂CH₃,
(454) N-Ac-Sar-Gly-Val-D-Phe (3,4,5-triF)-Ser-Leu-Ile-Arg-ProNHCH₂CH₃,
(455) N-Ac-Sar-Gly-Val-D-Phe (3,4,5-triF)-Ser-Nva-Ile-Arg-Pro-D-AlaNH₂,
(456) N-Succinyl-Sar-Gly-Val-D-Phe (3,4,5-triF)-Thr-Gln-Ile-Arg-ProNHCH₂CH₃,
(457) N-Succinyl-Sar-Gly-Val-D-Phe(3,4,5-triF)-Ser-Gln-Ile-Arg-ProNHCH₂CH₃,
(458) N-Succinyl-Sar-Gly-Val-D-Phe(3,4,5-triF)-Thr-Gln-Ile-Arg-ProNHCH(CH₃)₂,
(459) N-Ac-Sar-Gly-Val-D-Phe(3,4,5-triF)-Ser-Gln-Ile-Arg-ProNHCH₂CH₃,
(460) N-Ac-Sar-Gly-Val-D-Phe(3,4,5-triF)-Ser-Ser-Ile-Arg-ProNHCH₂CH₃,
(470) N-(3-Ac-Bala)-Sar-Gly- Val-D-allolle- Thr-Nva-Ile-Arg-ProNHCH₂CH₃,
(471) N-(3-Ac-Bala)-Sar-Gly-Val-D-Ile-Thr-Gln-Ile-Arg-ProNHCH₂CH₃,
(472) N-(3-Ac-Bala)-Sar-Gly- Val-D-alloIle- Thr-Gln-Ile-Arg-ProNHCH₂CH₃,
(473) N-(3-Ac-Bala)-Sar-Gly-Val-D-Ile-Thr-Gln-Ile-Arg-Pro-DAlaNH₂,
(474) N-(3-Ac-Bala)-Sar-Gly-Val-D-allolle-Thr-Gln-Ile-Arg-Pro-DAlaNH₂,
(475) N-(3-Ac-Bala)-Sar-Gly-Val-D-alloIle-Thr-Gln-Ile-Arg-ProNHCH(CH₃)₂,
(476) N-(3-Ac-Bala)-Sar-Gly-Val-D-Leu-Ser-Nva-Ile-Arg-ProNHCH₂CH₃,
(477) N-(3-Ac-Bala)-Sar-Gly-Val-D-Leu-Thr-Nva-Ile-Arg-ProNHCH₂CH₃,
(478) N-(3-Ac-Bala)-Sar-Gly-Val-D-Pen-Thr-Nva-Ile-Arg-ProNHCH₂CH₃,
(479) N-(3-Ac-Bala)-Sar-Gly-Val-D-Ile-Ser-Nva-Ile-Arg-ProNHCH₂CH₃,
(484) N-Ac-Sar-Gly-Val-D-Ile-Thr-Nva-Ile-Arg-Pro-OH,
(485) N-Ac-Sar-Gly-Val-D-alloIle-Thr-Nva-Ile-Arg-Pro-OH,
(486) N-Ac-Sar-Gly-Val-D-Leu-Thr-Nva-Ile-Arg-Pro-OH,
(487) N-Ac-Sar-Gly-Val-D-Pen-Thr-Nva-Ile-Arg-Pro-OH,
(488) N-Ac-Sar-Gly-Val-D-Phe (3,4,5-triF)-Thr-Nva-Ile-Arg-Pro-OH,
(489) N-Ac-Sar-Gly-Val-D-Ile-Thr-Gln-Ile-Arg-Pro-OH,
(490) N-Ac-Sar-Gly-Val-D-Leu-Ser-Nva-Ile-Arg-Pro-OH,
(492) N-Ac-Sar-Gly-Val-D-Ile-Ser-Gln-Ile-Arg-Pro-OH,
(493) N-Succinyl-Sar-Gly-Val-D-Ile-Thr-Nva-Ile-Arg-Pro-OH,
(494) N-Succinyl-Sar-Gly-Val-D-Leu-Thr-Gln-Ile-Arg-Pro-OH,
(495) N-Ac-Sar-Gly-Val-allo-Ile-Thr-Nva-Ile-Arg-ProNHCH₂CH₃,
(496) N-Ac-Sar-Gly-Val-D-Lys-Thr-Nva-Ile-Arg-ProNHCH₂CH₃,
(497) N-Ac-Sar-Gly-Val-D-Trp-Thr-Nva-Ile-Arg-ProNHCH₂CH₃,
(498) N-Ac-Sar-Gly-Val-D-3,3-Diphénylala-Thr-Nva-Ile-Arg-ProNHCH₂CH₃,
(499) N-Ac-Sar-Gly-Val-D-3-Benzothiénylala-Thr-Nva-Ile-Arg-ProNHCH₂CH₃,
(500) N-Ac-Sar-Gly-Val-D-3,4-diF₇Phe-Thr-Nva-Ile-Arg-ProNHCH₂CH₃,
(501) N-Ac-Sar-Gly-Val-D-Pen(Bzl)-Thr-Nva-Ile-Arg-ProNHCH₂CH₃,
(502) N-Ac-Sar-Gly-Val-D-Leu-Thr-Gln-Ile-Arg-ProNHCH(CH₃)₂,
(503) N-Sar-Gly-Val-D-Leu-Thr-Gln-Ile-Arg-ProNHCH₂CH₃,
(508) N-Ac-Sar-Gly-Val-D-Ile-Thr-Nva-Ile-Arg-ProNHCH₂CH₂OH, et
(510) N-Ac-Sar-Gly-Val-D-Ile-Thr-Leu-Ile-Arg-ProNH((R)-1-cyclohexyléthyle). -

12. Composé selon la revendication 11, choisi parmi:
(1) N-Ac-Sar-Gly-Val-D-Ile-Thr-Nva-Ile-Arg-ProNHCH₂CH₃,
(2) N-Ac-Sar-Gty-Val-D-Ile-Thr-Nva-Ile-Arg-ProNHCH₂CH₂-(1-pyrrolidine),
(3) N-Ac-Sar-Gly-Val-D-Ile-Thr-Nva-Ile-Arg-ProNH (éthyl-1-(R)-cyclohexyle),
(4) N-Ac-Sar-Gly-Val-D-Ile-Thr-Nva-Ile-Arg-ProNH₂,
(5) N-Ac-Sar-Gly-Val-D-Ile-Thr-Nva-Ile-Arg-ProNHCH(CH₃)₂,
(6) N-Ac-Sar-Gly-Val-D-alloIle-Thr-Nva-Ile-Arg-ProNHCH₂CH₃,
(7) N-Ac-Sar-Gly-Val-D-Val-Thr-Nva-Ile-Arg-ProNHCH₂CH₃,
(8) N-Ac-Sar-Gly-Val-D-Nle-Thr-Nva-Ile-Arg-ProNHCH₂CH₃,
(9) N-Ac-Sar-Gly-Val-D-Phe-Thr-Nva-Ile-Arg-ProNHCH₂CH₃,
(10) N-Ac-Sar-Gly-Val-D-Cha-Thr-Nva-Ile-Arg-ProNHCH₂CH₃,
(12) N-Ac-Sar-Gly-Val-D-3-ClPhe-Thr-Nva-Ile-Arg-ProNHCH₂CH₃,
(13) N-Ac-Sar-Gly-Val-D-2-Thiénylala-Thr-Nva-Ile-Arg-ProNHCH₂CH₃,
(14) N-Ac-Sar-Gly-Val-D-3-CNPhe-Thr-Nva-Ile-Arg-ProNHCH₂CH₃,
(15) N-Ac-Sar-Gly-Val-D-Ile-Thr-Cha-Ile-Arg-ProNHCH₂CH₃,
(16) N-[2-THF-C(O)]-Sar-Gly-Val-D-Ile-Thr-Nva-Ile-Arg-ProNHCH₂CH₃,
(17) N-[6-N-acétyl-(CH₂)₅C(O)]-Sar-Gly-Val-D-Ile-Thr-Nva-Ile-Arg-ProNHCH₂CH₃,
(19) N-[4-N-Acétylaminobutyryl]-Sar-Gly-Val-D-Ile-Thr-Nva-Ile-Arg-ProNHCH₂CH₃,
(23) N-Ac-Sar-Gly-Val-D-Ile-Thr-Leu-Ile-Arg-ProNHCH₂CH₃,
(24) N-Ac-Sar-Gly-Val-D-Ile-Thr-Ser-Ile-Arg-ProNHCH₂CH₃,
(25) N-Ac-Sar-Gly-Val-D-Ile-Thr-Nva-Ile-Arg-Pro-D-AlaNH₂,
(30) N-Ac-Sar-Gly-Val-D-Leu-Ala-Nva-Ile-Arg-ProNHCH₂CH₃,
(31) N-Ac-Sar-Gly-Val-D-Leu-Trp-Nva-Ile-Arg-ProNHCH₂CH₃,
(32) N-Ac-Sar-Gly-Val-D-Leu-Tyr-Nva-Ile-Arg-ProNHCH₂CH₃,
(33) N-Ac-Sar-Gly-Val-D-Leu-Gly-Nva-Ile-Arg-ProNHCH₂CH₃,
(34) N-Ac-Sar-Gly-Val-D-Leu-2Na1-Nva-Ile-Arg-ProNHCH₂CH₃,
(35) N-Ac-Sar-Gly-Val-D-Leu-lNal-Nva-Ile-Arg-ProNHCH₂CH₃,
(36) N-Ac-Sar-Gly-Val-D-Leu-Octylgly-Nva-Ile-Arg-ProNHCH₂CH₃,
(37) N-Ac-Sar-Gly-Val-D-Leu-Ser-Nva-Ile-Arg-ProNHCH₂CH₃,
(38) N-Ac-Sar-Gly-Val-D-Leu-Allylgly-Nva-Ile-Arg-ProNHCH₂CH₃,
(39) N-Ac-Sar-Gly-Val-D-Leu-D-Thr-Nva-Ile-Arg-ProNHCH₂CH₃,
(40) N-Ac-Sar-Gly-Val-D-Ile-Thr-Tyr-Ile-Arg-ProNHCH₂CH₃,
(41) N-Ac-Sar-Gly-Val-D-Ile-Thr-Glu-Ile-Arg-ProNHCH₂CH₃,
(42) N-Ac-Sar-Gly-Val-D-Ile-Thr-Propargylgly-Ile-Arg-ProNHCH₂CH₃,
(43) N-Ac-Sar-Gly-Val-D-alloIle-Thr-Gln-Ile-Arg-ProNHCH₂CH₃,
(44) N-Ac-Bala-Sar-Gly-Val-D-Ile-Thr-Nva-Ile-Arg-ProNHCH₂CH₃,
(45) N-Phénylacétyl-Sar-Gly-Val-D-Ile-Thr-Nva-Ile-Arg-ProNHCH₂CH₃,
(46) N-Ac-Sar-Gly-Val-D-Ile-Thr-Nva-Ile-Arg-Pro-AzaglyNH₂,
(47) N-Ac-Sar-Gly-Val-D-Ile-Thr-Nva-Ile-Arg-Pro-SerNH₂,
(48) N-(6-Ac-Aca)-Sar-Gly-Val-D-Leu-Ser-Gln-Ile-Arg-ProNHCH₂CH₃,
(49) N-(6-Ac-Aca)-Sar-Gly-Val-D-Leu-Ser-Nva-Ile-Arg-ProNHCH₂CH₃,
(50) N-(4-Ac-Gaba)-Sar-Gly-Val-D-Leu-Ser-Gln-Ile-Arg-ProNHCH₂CH₃,
(51) N-(4-Ac-Gaba)-Sar-Gly-Val-D-Leu-Ser-Nva-Ile-Arg-ProNHCH₂CH₃,
(52) N-(2-Furoyl)-Sar-Gly-Val-D-Leu-Ser-Gln-Ile-Arg-ProNHCH₂CH₃,
(53) N-(2-Furoyl)-Sar-Gly-Val-D-Leu-Ser-Nva-Ile- Arg-ProNHCH₂CH₃,
(54) N-(Shikimyl)-Sar-Gly-Val-D-Leu-Ser-Gln-Ile-Arg-ProNHCH₂CH₃,
(55) N-(Shikimyl)-Sar-Gly-Val-D-Leu-Ser-Nva-Ile-Arg-ProNHCH₂CH₃,
(58) N-(2-Me-nicotinyl)-Sar-Gly-Val-D-Leu-Ser-Gln-Ile-Arg-ProNHCH₂CH₃,
(59) N-(2-Me-nicotinyl)-Sar-Gly-Val-D-Leu-Ser-Nva-Ile-Arg-ProNHCH₂CH₃,
(60) N-Ac-Sar-Gly-Val-D-Ile-Thr-Nva-Ile-Arg-Pro-OH,
(62) N-Ac-Sar-Gly-Val-D-Pen-Thr-Nva-Ile-Arg-ProNHCH₂CH₃,
(63) N-Ac-Sar-Gly-Val-D-Phe(3,4,5-triF)-Thr-Nva-Ile-Arg-ProNHCH₂CH₃, et
(64) N-Ac-Sar-Gly-Val-D-Phe(4-NH₂)-Thr-Nva-Ile-Arg-ProNHCH₂CH₃,

13. Composition pharmaceutique comprenant un composé selon la revendication 1 et un support pharmaceutiquement acceptable.

14. Utilisation d'une quantité thérapeutiquement efficace d'un composé selon la revendication 1 pour la fabrication d'un médicament pour le traitement d'un patient ayant besoin d'une thérapie anti-angiogenèse.

15. Composition pour le traitement d'une maladie choisie parmi le cancer, l'arthrite, le psoriasis, l'angiogenèse de l'oeil associée à une infection ou une intervention chirurgicale, la dégénérescence maculaire et la rétinopathie, comprenant un peptide tel que défini dans la revendication 1 en combinaison avec un support pharmaceutiquement acceptable.

16. Composé selon la revendication 11, choisi parmi :
N-Ac-Sar-Gly-Val-D-Ile-Thr-Nva-Ile-Arg-ProNHCH₂CH₃,
N-Ac-Sar-Gly-Val-D-alloIle-Thr-Nva-Ile-Arg-ProNHCH₂CH₃,
N-Ac-Sar-Gly-Val-D-Ile-Thr-Gln-Ile-Arg-ProNHCH₂CH₃, et
N-Ac-Sar-Gly-Val-D-allolle-Ser-Ser-Ile-Arg-ProNHCH₂CH₃.

17. Composé selon la revendication 16, qui est le
N-Ac-Sar-Gly-Val-D-Ile-Thr-Nva-Ile-Arg-ProNHCH₂CH₃.

18. Composé selon la revendication 16, qui est le
N-Ac-Sar-Gly-Val-D-alloIle-Thr-Nva-Ile-Arg-ProNHCH₂CH₃.

19. Composé selon la revendication 16, qui est le
N-Ac-Sar-Gly-Val-D-Ile-Thr-Gln-Ile-Arg-ProNHCH₂CH₃.

20. Composé selon la revendication 16, qui est le
N-Ac-Sar-Gly-Val-D-alloIle-Ser-Ser-Ile-Arg-ProNHCH₂CH₃.

21. Composition selon la revendication 13, comprenant un composé, ou un sel, ester, solvate ou précurseur médicamenteux pharmaceutiquement acceptable de celui-ci, choisi dans le groupe constitué par
N-Ac-Sar-Gly-Val-D-Ile-Thr-Nva-Ile-Arg-ProNHCH₂CH₃,
N-Ac-Sar-Gly-Val-D-alloIle-Thr-Nva-Ile-Arg-ProNHCH₂CH₃,
N-Ac-Sar-Gly-Val-D-Ile-Thr-Gln-Ile-Arg-ProNHCH₂CH₃, et
N-Ac-Sar-Gly-Val-D-alloIle-Ser-Ser-Ile-Arg-ProNHCH₂CH₃
et un support pharmaceutiquement acceptable.

22. Composition selon la revendication 21, comprenant le composé
N-Ac-Sar-Gly-Val-D-Ile-Thr-Nva-Ile-Arg-ProNHCH₂CH₃,
ou un sel, ester, solvate ou précurseur médicamenteux pharmaceutiquement acceptable de celui-ci, et un support pharmaceutiquement acceptable.

23. Composition selon la revendication 21, comprenant le composé
N-Ac-Sar-Gly-Val-D-allolle-Thr-Nva-IIe-Arg-ProNHCH₂CH₃,
ou un sel, ester, solvate ou précurseur médicamenteux pharmaceutiquement acceptable de celui-ci, et un support pharmaceutiquement acceptable.

24. Composition selon la revendication 21, comprenant le composé N-Ac-Sar-Gly-Val-D-Ile-Thr-Gln-Ile-Arg-ProNHCH₂CH₃,
ou un sel, ester, solvate ou précurseur médicamenteux pharmaceutiquement acceptable de celui-ci, et un support pharmaceutiquement acceptable.

25. Composition selon la revendication 21, comprenant le composé
N-Ac-Sar-Gly-Val-D-alloIle-Ser-Ser-Ile-Arg-ProNHCH₂CH₃,
ou un sel, ester, solvate ou précurseur médicamenteux pharmaceutiquement acceptable de celui-ci, et un support pharmaceutiquement acceptable.

26. Composition selon la revendication 15, comprenant un composé, ou un sel, ester, solvate ou précurseur médicamenteux pharmaceutiquement acceptable de celui-ci, choisi dans le groupe constitué par
N-Ac-Sar-Gly-Val-D-Ile-Thr-Nva-Ile-Arg-ProNHCH₂CH₃,
N-Ac-Sar-Gly-Val-D-alloIle-Thr-Nva-Ile-Arg-ProNHCH₂CH₃,
N-Ac-Sar-Gly-Val-D-Ile-Thr-Gln-Ile-Arg-ProNHCH₂CH₃, et
N-Ac-Sar-Gly-Val-D-alloIle-Ser-Ser-Ile-Arg-ProNHCH₂CH₃.

27. Composition selon la revendication 26, comprenant le composé
N-Ac-Sar-Gly-Val-D-Ile-Thr-Nva-Ile-Arg-ProNHCH₂CH₃,
ou un sel, ester, solvate ou précurseur médicamenteux pharmaceutiquement acceptable de celui-ci.

28. Composition selon la revendication 26, comprenant le composé
N-Ac-Sar-Gly-Val-D-alloIle-Thr-Nva-Ile-Arg-ProNHCH₂CH₃,
ou un sel, ester, solvate ou précurseur médicamenteux pharmaceutiquement acceptable de celui-ci.

29. Composition selon la revendication 26, comprenant le composé
N-Ac-Sar-Gly-Val-D-Ile-Thr-Gln-Ile-Arg-ProNHCH₂CH₃,
ou un sel, ester, solvate ou précurseur médicamenteux pharmaceutiquement acceptable de celui-ci.

30. Composition selon la revendication 26, comprenant le composé
N-Ac-Sar-Gly-Val-D-allolle-Ser-Ser-Ile-Arg-ProNHCH₂CH₃,
ou un sel, ester, solvate ou précurseur médicamenteux pharmaceutiquement acceptable de celui-ci.

31. Utilisation selon la revendication 14, dans laquelle le patient est un animal non humain.

32. Utilisation selon la revendication 14, dans laquelle le patient est un humain.

33. Utilisation selon la revendication 14, comprenant un composé selon l'une quelconque des revendications 17, 18, 19 ou 20.

34. Utilisation selon la revendication 33, dans laquelle le patient est un animal non humain.

35. Utilisation selon la revendication 33, dans laquelle le patient est un humain.
